(19) Europäisches Patentamt
European Patent Office
Office européen des brevets

(11) EP 1 793 006 A2

(12) EUROPEAN PATENT APPLICATION

(43) Date of publication:
**06.06.2007 Bulletin 2007/23**

(51) Int Cl.:
***C12Q 1/68*** *(2006.01)* ***C07H 21/02*** *(2006.01)*
***C07H 21/04*** *(2006.01)* ***C12N 15/10*** *(2006.01)*

(21) Application number: **06006842.6**

(22) Date of filing: **08.09.1994**

(84) Designated Contracting States:
**AT BE CH DE DK ES FR GB GR IE IT LI LU MC NL PT SE**

(30) Priority: **08.09.1993 US 117991**
**07.10.1993 US 134028**
**22.02.1994 US 199507**
**25.04.1994 US 233012**
**28.04.1994 US 234997**

(62) Document number(s) of the earlier application(s) in accordance with Art. 76 EPC:
**94927409.6 / 0 724 647**

(71) Applicant: **GILEAD SCIENCES, INC.**
**Foster City, CA 94404 (US)**

(72) Inventors:
- **Gold, Larry**
  **Boulder, CO 80303 (US)**
- **Pieken, Wolfgang**
  **Longmont, CO 80503 (US)**
- **Tasset, Diane**
  **Stevensville, Montana 59870 (US)**
- **Janjic, Nebojsa**
  **Boulder, CO 80301 (US)**
- **Kirschenheuter, Gary P.**
  **Arvada, CO 80005 (US)**
- **Polisky, Barry**
  **Boulder, CO 80304 (US)**
- **Jayasena, Sumedha**
  **Thousand Oaks, CA 91362 (US)**
- **Biesecker, Greg**
  **Boulder, CO 80301 (US)**
- **Smith, Drew**
  **Boulder, CO 80304 (US)**
- **Jenison, Robert D.**
  **Boulder, CO 80301 (US)**

(74) Representative: **Dörries, Hans Ulrich**
**Dörries, Frank-Molnia & Pohlman,**
**Triftstrasse 13**
**80538 München (DE)**

Remarks:
•This application was filed on 31 - 03 - 2006 as a divisional application to the application mentioned under INID code 62.
•The sequence listing, which is published as annex to the application documents, was filed after the date of filing. The applicant has declared that it does not include matter which goes beyond the content of the application as filed.

# (54) Nucleic acid ligands and improved methods for producing the same

(57) The present invention describes methods for the identification and production of nucleic acid ligands to a target molecule based on the SELEX process. The methods may be performed with modified nucleic acids, blended nucleic acids and with separate steps of contacting of the candidate mixture of nucleic acids with target and with nontarget molecules. Included in the invention are methods for the identification and preparation of nucleic acid ligands to thrombin, vascular endothelial growth factor, human neutrophil elastase, caffeine and theophylline and nucleic acid ligands to thrombin identified by the SELEX method.

EP 1 793 006 A2

Annex to the application documents - subsequently filed sequences listing

SEQUENCE LISTING

(1) GENERAL INFORMATION:

(i) APPLICANT: PIEKEN, WOLFGANG
TASSET, DIANE
JANJIC, NEBOJSA
GOLD, LARRY
KIRSCHENHEUTER, GARY P.
POLISKY, GARY
JENISON, ROBERT D.
JAYASENA, SUMEDHA
BIESECKER, GREG
SMITH, DREW

(ii) TITLE OF INVENTION: NUCLEIC ACID LIGANDS AND IMPROVED METHODS FOR PRODUCING THE SAME

(iii) NUMBER OF SEQUENCES: 276

(iv) CORRESPONDENCE ADDRESS:
(A) ADDRESSEE: Swanson & Bratschun, L.L.C.
(B) STREET: 8400 E. Prentice Place, Suite 200
(C) CITY: Englewood
(D) STATE: Colorado
(E) COUNTRY: USA
(F) ZIP: 80111

(v) COMPUTER READABLE FORM:
(A) MEDIUM TYPE: Diskette, 3.5 inch, 360 Kb storage
(B) COMPUTER: IBM compatible
(C) OPERATING SYSTEM: MS-DOS
(D) SOFTWARE: WordPerfect 5.1

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: 08/117,991
(B) FILING DATE: 8-SEPTEMBER-1993

(vii) PRIOR APPLICATION DATA:
(A) APPLICATION NUMBER: 08/134,029
(B) FILING DATE: 7-OCTOBER-1993

(vii) PRIOR APPLICATION DATA:
(A) APPLICATION NUMBER: 08/199,507
(B) FILING DATE: 22-FEB-1994

(vii) PRIOR APPLICATION DATA:
(A) APPLICATION NUMBER: 08/233,012
(B) FILING DATE: 25-APRIL-1994

(vii) PRIOR APPLICATION DATA:
(A) APPLICATION NUMBER: 08/234,997
(B) FILING DATE: 28-APRIL-1994

(viii) ATTORNEY/AGENT INFORMATION:

(A) NAME: Barry J. Swanson
(B) REGISTRATION NUMBER: 33,215
(C) REFERENCE/DOCKET NUMBER: NEX09/PCT

(ix) TELECOMMUNICATION INFORMATION:
(A) TELEPHONE: (303) 793-3333
(B) TELEFAX: (303) 793-3433

(2) INFORMATION FOR SEQ ID NO:1:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:1:
GGGAGAUGCC UGUCGAGCAU GCUGAGGAUC GAAGUUAGUA GGCUUUGUGU 50
GCUCGUAGCU AAACAGCUUU GUCGACUCU 79

(2) INFORMATION FOR SEQ ID NO:2:
(i) SEQUENCE CHARACTERISTICS:
(A) LENGTH: 76 base pairs
(B) TYPE: nucleic acid
(C) STRANDEDNESS: single
(D) TOPOLOGY: linear

(xi) SEQUENCE DESCRIPTION: SEQ ID NO:2:
GGGAGAUGCC UGUCGAGCAU GCUGUACUGG AUCGAAGGUA GUAGGCAGUC 50
ACGUAGCUAA ACAGCUUUGU GACUCU 76

(2) INFORMATION FOR SEQ ID NO:3:
(i) SEQUENCE CHARACTERISTICS:
(A) LENGTH: 76 base pairs
(B) TYPE: nucleic acid
(C) STRANDEDNESS: single
(D) TOPOLOGY: linear
(xi) SEQUENCE DESCRIPTION: SEQ ID NO:3:

GGGAGAUGCC UGUCGAGCAU GCUGAUAUCA CGGAUCGAAG GAAGUAGGCG 50
UGGUAGCUAA ACAGCUUUGU GACUCU 76

(2) INFORMATION FOR SEQ ID NO:4:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:4:
GGGAGAUGCC UGUCGAGCAU GCUGCCUUUC CCGGGUUCGA AGUCAGUAGG 50
CCGGGUAGCU AAACAGCUUU GUCGACUCU 79

(2) INFORMATION FOR SEQ ID NO:5:
(i) SEQUENCE CHARACTERISTICS:
(A) LENGTH: 78 base pairs
(B) TYPE: nucleic acid
(C) STRANDEDNESS: single
(D) TOPOLOGY: linear

(xi) SEQUENCE DESCRIPTION: SEQ ID NO:5:
GGGAGAUGCC UGUCGAGCAU GCUGCACCCG GAUCGAAGUU AGUAGGCGUG 50
AGUGUAGCUA AACAGCUUUG UCGACUCU 78

(2) INFORMATION FOR SEQ ID NO:6:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:6:
GGGAGAUGCC UGUCGAGCAU GCUGUGUACG GAUCGAAGGU AGUAGGCAGG 50
UUACGUAGCU AAACAGCUUU GUCGACUCU 79

(2) INFORMATION FOR SEQ ID NO:7:
(i) SEQUENCE CHARACTERISTICS:
(A) LENGTH: 78 base pairs
(B) TYPE: nucleic acid
(C) STRANDEDNESS: single
(D) TOPOLOGY: linear

(xi) SEQUENCE DESCRIPTION: SEQ ID NO:7:
GGGAGAUGCC UGUCGAGCAU GCUGCAUCCG GAUCGAAGUU AGUAGGCGGA 50
GUGGUAGCUA AACAGCUUUG CGACUCU 78

(2) INFORMATION FOR SEQ ID NO:8:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:8:
GGGAGAUGCC UGUCGAGCAU GCUGAUUGUU GCGGAUCGAA GUGGAGUAGG 50
CGCAGUAGCU AAACAGCUUU GUCGACUCU 79

(2) INFORMATION FOR SEQ ID NO:9:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:9:
GGGAGAUGCC UGUCGAGCAU GCUGUGUACU GGAUCGAAGG UAGUAGCGAG 50
UCACGUAGCU AAACAGCUUU GUCGACUCU 79

(2) INFORMATION FOR SEQ ID NO:10:
(i) SEQUENCE CHARACTERISTICS:
(A) LENGTH: 72 base pairs
(B) TYPE: nucleic acid
(C) STRANDEDNESS: single
(D) TOPOLOGY: linear

(xi) SEQUENCE DESCRIPTION: SEQ ID NO:10:
GGGAGAUGCC UGUCGAGCAU GCUGAUCGAA GUUAGUAGGA GCGUGUGGUA 50
GCUAAACAGC UUUGUCGACU CU 72

(2) INFORMATION FOR SEQ ID NO:11:
(i) SEQUENCE CHARACTERISTICS:
(A) LENGTH: 84 base pairs
(B) TYPE: nucleic acid
(C) STRANDEDNESS: single
(D) TOPOLOGY: linear

(xi) SEQUENCE DESCRIPTION: SEQ ID NO:11:
GGGAGAUGCC UGUCGAGCAU GCUGACGCUA GAGUCGGAUC GAAAGGUAAG 50
UAGGCGACUG UAGCUAAACA GCUUUGUCGA CUCU 84

(2) INFORMATION FOR SEQ ID NO:12:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:12:
GGGAGAUGCC UGUCGAGCAU GCUGGGGUCG GAUCGAAAGG UAAGUAGGCG 50
ACUGUAGCUA AACAGCUCUU GUCGACUCU 79

(2) INFORMATION FOR SEQ ID NO:13:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:13:
GGGAGAUGCC UGUCGAGCAU GCUGAUAUCA CGGAUCGAAA GAGAGUAGGC 50
GUGUAGCUAA ACAGCUUUGU CGACUCU 77

(2) INFORMATION FOR SEQ ID NO:14:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:14:
GGGAGAUGCC UGUCGAGCAU GCUGUGUACU GGAUCGAAGG UAGUAGGCAG 50
GCACGUAGCU AAACAGCUUU GUCGACUCU 79

(2) INFORMATION FOR SEQ ID NO:15:

(i) SEQUENCE CHARACTERISTICS:
(A) LENGTH: 78 base pairs
(B) TYPE: nucleic acid
(C) STRANDEDNESS: single
(D) TOPOLOGY: linear

(xi) SEQUENCE DESCRIPTION: SEQ ID NO:15:
GGGAGAUGCC UGUCGAGCAU GCUGAUAUCA CGGAUCGAAG GAAAGUAGGC 50
GUGGUAGCUA AACAGCUUUG UCGACUCU 78

(2) INFORMATION FOR SEQ ID NO:16:
(i) SEQUENCE CHARACTERISTICS:
(A) LENGTH: 75 base pairs

(B) TYPE: nucleic acid
(C) STRANDEDNESS: single
(D) TOPOLOGY: linear

(xi) SEQUENCE DESCRIPTION: SEQ ID NO:16:
GGGAGAUGCC UGUCGAGCAU GCUGGUGCGG CUUUGGGCGC CGUGCUUGGC 50
GUAGCUAAAC AGCUUUGUCG ACUCU 75

(2) INFORMATION FOR SEQ ID NO:17:
(i) SEQUENCE CHARACTERISTICS:
(A) LENGTH: 74 base pairs
(B) TYPE: nucleic acid
(C) STRANDEDNESS: single
(D) TOPOLOGY: linear

(xi) SEQUENCE DESCRIPTION: SEQ ID NO:17:
GGGAGAUGCC UGUCGAGCAU GCUGGUGCGG CUUUGGGCGC CGUGCUUACG 50
UAGCUAAACA GCUUUGUCGA CUCU 74

(2) INFORMATION FOR SEQ ID NO:18:
(i) SEQUENCE CHARACTERISTICS:
(A) LENGTH: 75 base pairs
(B) TYPE: nucleic acid
(C) STRANDEDNESS: single
(D) TOPOLOGY: linear

(xi) SEQUENCE DESCRIPTION: SEQ ID NO:18:
GGGAGAUGCC UGUCGAGCAU GCUGGUGCGG CUUUGGGCGC CGUGCUUGAC 50
GUAGCUAAAC AGCUUUGUCG ACUCU 75

(2) INFORMATION FOR SEQ ID NO:19:
(i) SEQUENCE CHARACTERISTICS:
(A) LENGTH: 74 base pairs
(B) TYPE: nucleic acid
(C) STRANDEDNESS: single
(D) TOPOLOGY: linear

(xi) SEQUENCE DESCRIPTION: SEQ ID NO:19:
GGGAGAUGCC UGUCGAGCAU GCUGGGGCGG CUUUGGGCGC CGUGCUUGAC 50
GUAGCUAAAC AGCUUUGUCG ACUC 74

(2) INFORMATION FOR SEQ ID NO:20:
(i) SEQUENCE CHARACTERISTICS:
(A) LENGTH: 79 base pairs
(B) TYPE: nucleic acid
(C) STRANDEDNESS: single
(D) TOPOLOGY:

(xi) SEQUENCE DESCRIPTION: SEQ ID NO:20:
GGGAGAUGCC UGUCGAGCAU GCUGUGAGCC UGCCAGUGUG UAUGUGGAAA 50
CAAGGUAGCU AAACAGCUUU GUCGACUCU 79

(2) INFORMATION FOR SEQ ID NO:21:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:21:
GGGAGAUGCC UGUCGAGCAU GCUGUGAGCC UGCCAGUGUG CAUGUGGAAA 50
CAAGGUAGCU AAACAGCUUU GUCGACUCU 79

(2) INFORMATION FOR SEQ ID NO:22:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:22:
GGGAGAUGCC UGUCGAGCAU GCUGUGAGCC AGCCAGUGUG CAUGUGGAAA 50
CAAGGUAGCU AAACAGCUUU GUCGACUCU 79

(2) INFORMATION FOR SEQ ID NO:23:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:23:
GGGAGAUGCC UGUCGAGCAU GCUGUGAGCC AGCCAGUGUG UAUGUGGAAA 50
CAAGGUAGCU AAACAGCUUU GUCGACUCU 79

(2) INFORMATION FOR SEQ ID NO:24:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:24:
GGGAGAUGCC UGUCGAGCAU GCUGUGAGCC GGCCAGUGUG CAUGUGGAAA 50
CAAGGUAGCU AAACAGCUUU GUCGACUCU 79

(2) INFORMATION FOR SEQ ID NO:25:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:25:
GGGAGAUGCC UGUCGAGCAU GCUGUGAGCC AGCCAGUGUG UAUGUGGAAA 50
CAAGGUAGCU AAACAGCUUU GUCGACUCU 79

(2) INFORMATION FOR SEQ ID NO:26:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:26:
GGGAGAUGCC UGUCGAGCAU GCUGUUACGG GGAGGUGUUA CGGAGUGUAC 50
CCGUAGCUAA ACAGCUUUGU CGACUCU 77

(2) INFORMATION FOR SEQ ID NO:27:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:27:
GGGAGAUGCC UGUCGAGCAU GCUGUUGCGG GGAGGUGUUA GGGAGUGUAC 50
CCGUAGCUAA ACAGCUUUGU CGACUCU 77

(2) INFORMATION FOR SEQ ID NO:28:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:28:
GGGAGAUGCC UGUCGAGCAU GCUGUUGCGG GGAGGUGUUA GNNAGUGUAC 50
CCGUAGCUAA ACAGCUUUGU CGACUCU 77

(2) INFORMATION FOR SEQ ID NO:29:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:29:
GGGAGAUGCC UGUCGAGCAU GCUGUUGCGG GGAGGUGUUA GGGAGUUCAC 50
CCGUAGCUAA ACAGCUUUGU CGACUCU 77

(2) INFORMATION FOR SEQ ID NO:30:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:30
GGGAGAUGCC UGUCGAGCAU GCUGCUGCGG GGAGGUGUUA GGGAGUGUAC 50
CCGUAGCUAA ACAGCUUUGU CGACUCU 77

(2) INFORMATION FOR SEQ ID NO:31
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:31:
GGGAGAUGCC UGUCGAGCAU GCUGCUGCGG GGAGGUGUUA GAGAGUGUAC 50
CCGUAGCUAA ACAGCUUUGU CGACUCU 77

(2) INFORMATION FOR SEQ ID NO:32
(i) SEQUENCE CHARACTERISTICS:
(A) LENGTH: 77 base pairs
(B) TYPE: nucleic acid

(C) STRANDEDNESS: single
(D) TOPOLOGY: linear

(xi) SEQUENCE DESCRIPTION: SEQ ID NO:32:
GGGAGAUGCC UGUCGAGCAU GCUGCUGCGG GGAGGUGUCA GAGAGUGUAC 50
CUGUAGCUAA ACAGCUUUGU CGACUCU 77

(2) INFORMATION FOR SEQ ID NO:33:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:33:
GGGAGAUGCC UGUCGAGCAU GCUGCUACGG GGAGGUGUUA GAGAGUGUAC 50
CUGUAGCUAA ACAGCUUUGU CGACUCU 77

(2) INFORMATION FOR SEQ ID NO:34:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:34:
GGGAGAUGCC UGUCGAGCAU GCUGCUACGG GGAGGUGUCG GAGAGUGUAC 50
CUGUAGCUAA ACAGCUUUGU CGACUCU 77

(2) INFORMATION FOR SEQ ID NO:35:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:35:
GGGAGAUGCC UGUCGAGCAU GCUGCACGAG GUGUCAGAGA GUGUAGUUCA 50
GCGUAGCUAA ACAGCUUUGU CGACUCU 77

(2) INFORMATION FOR SEQ ID NO:36:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:36:
GGGAGAUGCC UGUCGAGCAU GCUGCACGAG GUGUCAGAGA GUGUAGUGCA 50
GCGUAGCUAA ACAGCUUUGU CGACUCU 77

(2) INFORMATION FOR SEQ ID NO:37:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:37:

GGGAGAUGCC UGUCGAGCAU GCUGCACGAG GUGUAGAGGG UGUAGUGCAG 50
CAGUACGUAA ACAGCUUUGU CGACUCU 77

(2) INFORMATION FOR SEQ ID NO:38:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:38:
GGGAGAUGCC UGUCGAGCAU GCUGCACGAG GCGUCAGAGA GUGUAGUGCU 50
GCGUACGUAA ACAGCUUUGU CGACUCU 77

(2) INFORMATION FOR SEQ ID NO:39:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:39:
GGGAGAUGCC UGUCGAGCAU GCUGAGGAUC GAAGUUAGUA GGCUUUGUGU 50
ACUCGUAGCU AAACAGCUUU GUCCACUCU 79

(2) INFORMATION FOR SEQ ID NO:40:
(i) SEQUENCE CHARACTERISTICS:
(A) LENGTH: 6 amino acids
(B) TYPE: amino acid
(C) STRANDEDNESS: single
(D) TOPOLOGY: linear

(xi) SEQUENCE DESCRIPTION: SEQ ID NO:40:
Gly Arg Gly Asp Thr Pro 6

(2) INFORMATION FOR SEQ ID NO:41:
(i) SEQUENCE CHARACTERISTICS:
(A) LENGTH: 4 amino acids
(B) TYPE: amino acid
(C) STRANDEDNESS: single
(D) TOPOLOGY: linear

(xi) SEQUENCE DESCRIPTION: SEQ ID NO:41:

(ix) FEATURE:
(A) NAME/KEY: Xaa
(B) LOCATION: 1
(C) OTHER INFORMATION: This symbol stands for N-methylsuccinylalanine

(ix) FEATURE:
(A) NAME/KEY: Xaa
(B) LOCATION: 4
(C) OTHER INFORMATION: Valine chloromethyl ketone
Xaa Ala Pro Xaa 4

(2) INFORMATION FOR SEQ ID NO:42:
(i) SEQUENCE CHARACTERISTICS:
(A) LENGTH: 59 nucleotides

(B) TYPE: nucleic acid
(C) STRANDEDNESS: single
(D) TOPOLOGY: linear

(xi) SEQUENCE DESCRIPTION: SEQ ID NO:42:
TAGCGATACT GCGTGGGTTG GGGCGGGTAG GGCCAGCAGT CTCGTGCGGT 50
ACTTGAGCA 59

(2) INFORMATION FOR SEQ ID NO:43:
(i) SEQUENCE CHARACTERISTICS:
(A) LENGTH: 4 amino acids
(B) TYPE: amino acid
(C) STRANDEDNESS: single
(D) TOPOLOGY: linear

(xi) SEQUENCE DESCRIPTION: SEQ ID NO:43:

(ix) FEATURE:
(A) NAME/KEY: Xaa
(B) LOCATION: 1
(C) OTHER INFORMATION: N-methyoxysuccinyl alanine

(ix) FEATURE:
(A) NAME/KEY: Xaa
(B) LOCATION: 4
(C) OTHER INFORMATION: Valine p-nitroanilide
Xaa Ala Pro Xaa 4

(2) INFORMATION FOR SEQ ID NO:44:
(i) SEQUENCE CHARACTERISTICS:
(A) LENGTH: 4 amino acids
(B) TYPE: amino acid
(C) STRANDEDNESS: single
(D) TOPOLOGY: linear

(ix) FEATURE:
(A) NAME/KEY: Xaa
(B) LOCATION: 1
(C) OTHER INFORMATION: N-methyoxysuccinyl alanine

(xi) SEQUENCE DESCRIPTION: SEQ ID NO:44:
Xaa Ala Pro Val 4

(2) INFORMATION FOR SEQ ID NO:45:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:45:
GGGAGCUCAG AAUAAACGCU CAANNNNNNN NNNNNNNNNN NNNNNNNNNN 50
NNNUUCGACA UGAGGCCCGG AUCCGGC 77

(2) INFORMATION FOR SEQ ID NO:46:
(i) SEQUENCE CHARACTERISTICS:
(A) LENGTH: 48 base pairs
(B) TYPE: nucleic acid
(C) STRANDEDNESS: single

(D) TOPOLOGY: linear

(xi) SEQUENCE DESCRIPTION: SEQ ID NO:46:
CCGAAGCTTA ATACGACTCA CTATAGGGAG CTCAGAATAA ACGCTCAA 48

(2) INFORMATION FOR SEQ ID NO:47:
(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:47:
GCCGGATCCG GGCCTCATGT CGAA 24

(2) INFORMATION FOR SEQ ID NO:48:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:48:
UCAAGAGUGA UGCUCAUCCG CACUUGGUGA CGUU 34

(2) INFORMATION FOR SEQ ID NO:49:
(i) SEQUENCE CHARACTERISTICS:
(A) LENGTH: 37 base pairs
(B) TYPE: nucleic acid
(C) STRANDEDNESS: single
(D) TOPOLOGY: linear

(xi) SEQUENCE DESCRIPTION: SEQ ID NO:49:
CAAUACCGGC AUGCAUGUCC AUCGCUAGCG GUAUUCG 37

(2) INFORMATION FOR SEQ ID NO:50:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:50:
AAUGCGUGUU GUGACGCACA UCCGCACGCG CAUU 34

(2) INFORMATION FOR SEQ ID NO:51:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:51:
UCAAGGAGUG AUGCCCUAUC CGCACCUUGG CCCA 34

(2) INFORMATION FOR SEQ ID NO:52:
(i) SEQUENCE CHARACTERISTICS:
(A) LENGTH: 34 base pairs
(B) TYPE: nucleic acid

(C) STRANDEDNESS: single
(D) TOPOLOGY: linear

(xi) SEQUENCE DESCRIPTION: SEQ ID NO:52:
UCAAGCUUGA CNGCCCAUCC GAGCUUGAUC ACGC 34

(2) INFORMATION FOR SEQ ID NO:53:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:53
AAACGCUCAA UCCUUGAUGC GGAUCCGAGG AUGGGACGUU U 41

(2) INFORMATION FOR SEQ ID NO:54:
(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:54:
ACACCGUCGA CCUAUGAUGC GCAUCCGCAC UUCGAC 36

(2) INFORMATION FOR SEQ ID NO:55:
(i) SEQUENCE CHARACTERISTICS:
(A) LENGTH: 37 base pairs
(B) TYPE: nucleic acid
(C) STRANDEDNESS: single
(D) TOPOLOGY: linear

(xi) SEQUENCE DESCRIPTION: SEQ ID NO:55:
AACCGGUAGU CGCAUGGCCC AUCGCGCCCG GUUCGAC 37

(2) INFORMATION FOR SEQ ID NO:56:
(i) SEQUENCE CHARACTERISTICS:
(A) LENGTH: 38 base pairs
(B) TYPE: nucleic acid
(C) STRANDEDNESS: single
(D) TOPOLOGY: linear

(xi) SEQUENCE DESCRIPTION: SEQ ID NO:56:
ACGCUCAAGU CAGCAUGGCC CACCGCGCUU GACGUCUG 38

(2) INFORMATION FOR SEQ ID NO:57:
(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:57:
CACGGUUCGA UCUGUGACGU UCAUCCGCAC UUCGA 35

(2) INFORMATION FOR SEQ ID NO:58:
(i) SEQUENCE CHARACTERISTICS:
(A) LENGTH: 41 base pairs

(B) TYPE: nucleic acid
(C) STRANDEDNESS: single
(D) TOPOLOGY: linear

(xi) SEQUENCE DESCRIPTION: SEQ ID NO:58:
AACGCUCAAG GAGCAGUGAC GCACAUCCAC ACUCCAGCGU U 41

(2) INFORMATION FOR SEQ ID NO:59:
(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:59:
UUCGAAUGCC GAGGCUCGUG CCUUGACGGG UUC 33

(2) INFORMATION FOR SEQ ID NO:60:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:60:
UCGCGAAUGC CGACCACUCA GGUUGAUGGG UUCG 34

(2) INFORMATION FOR SEQ ID NO:61:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:61:
UCAAUGCCGG CCUGAUCGGC UGAUGGGUUG ACCG 34

(2) INFORMATION FOR SEQ ID NO:62:
(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:62:
GAAUGCCGAG CCCUAAGAGG CUUGAUGUGG UU 32

(2) INFORMATION FOR SEQ ID NO:63:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:63:
AACCUUNAUG UGGCNCGAAC UGCGUGCCGA GGUU 34

(2) INFORMATION FOR SEQ ID NO:64:
(i) SEQUENCE CHARACTERISTICS:

(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:64:
AAGCUUGAUG GGUGACACAC GUCAUGCCGA GCUU 34

(2) INFORMATION FOR SEQ ID NO:65:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:65:
GUCGUCCUGC AUGGGCCGUA UCGGUGCGCG 30

(2) INFORMATION FOR SEQ ID NO:66:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:66:
GCAGACGAAG GGAACCUGCG UCUCGGCACC UUCG 34

(2) INFORMATION FOR SEQ ID NO:67:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:67:
AAGGAGGANC CUGCGUCUCG GCACUCCGCA 30

(2) INFORMATION FOR SEQ ID NO:68:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:68:
UCAAGGGAAC CUGCGUUUCG GCACCUUGUU CCGU 34

(2) INFORMATION FOR SEQ ID NO:69:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:69:
AAAUGUGGGU UACCUGCGUU UCGGCACCAC GUUU 34

(2) INFORMATION FOR SEQ ID NO:70:

(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:70:
CGACGGUAGA GUCUGUCCCG UCAUCCCCCA 30

(2) INFORMATION FOR SEQ ID NO:71:
(i) SEQUENCE CHARACTERISTICS:
(A) LENGTH: 32 base pairs
(B) TYPE: nucleic acid
(C) STRANDEDNESS: single
(D) TOPOLOGY: linear
(xi) SEQUENCE DESCRIPTION: SEQ ID NO:71:
AAAGACCCCU GGUUGAGUCU GUCCCAGCCG UU 32

(2) INFORMATION FOR SEQ ID NO:72:
(i) SEQUENCE CHARACTERISTICS:
(A) LENGTH: 42 base pairs
(B) TYPE: nucleic acid
(C) STRANDEDNESS: single
(D) TOPOLOGY: linear

(xi) SEQUENCE DESCRIPTION: SEQ ID NO:72:
GACCCAUCGU CAACGGUUGA GUCUGUCCCG UUCGACAUGA GG 42

(2) INFORMATION FOR SEQ ID NO:73:
(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:73:
GCUCAAGGUU GAGUCUGUCC CUUCGAGUAU CUGAUC 36

(2) INFORMATION FOR SEQ ID NO:74:
(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:74:
UCGGACAGUU GGUUGAGUCU GUCCCAACUU UU 32

(2) INFORMATION FOR SEQ ID NO:75:
(i) SEQUENCE CHARACTERISTICS:
(A) LENGTH: 31 base pairs
(B) TYPE: nucleic acid
(C) STRANDEDNESS: single
(D) TOPOLOGY: linear

(xi) SEQUENCE DESCRIPTION: SEQ ID NO:75:
GACCAUGUGA CUGGUUGAGC CUGUCCCAGU U 31

(2) INFORMATION FOR SEQ ID NO:76:

(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:76:
AACGGUUGAG UCUGUCCCGU AAGAGAGCGC 30

(2) INFORMATION FOR SEQ ID NO:77:
(i) SEQUENCE CHARACTERISTICS:
(A) LENGTH: 38 base pairs
(B) TYPE: nucleic acid
(C) STRANDEDNESS: single
(D) TOPOLOGY: linear

(xi) SEQUENCE DESCRIPTION: SEQ ID NO:77:
UCGGAAUGUA GUUGACGUAU CCUUGUCCGA UUCGACAU 38

(2) INFORMATION FOR SEQ ID NO:78:
(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:78:
AGGGUGUAGUU GGGACCUAGU CCGCCGUACC UU 32

(2) INFORMATION FOR SEQ ID NO:79:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:79:
GGCAUAGUUG GGACCUCGUC CGCCGUGCCC 30

(2) INFORMATION FOR SEQ ID NO:80:
(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:80:
GCUCAAUAGU UGGAGGCCUG UCCUCGCCGU AGAGCG 36

(2) INFORMATION FOR SEQ ID NO:81:
(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:81:
AGGGGUUCUA GUGGAGACUC UGCCGCGGCC CUU 33

(2) INFORMATION FOR SEQ ID NO:82:
(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:82:
AACGGUUCUG UGUGUGGACU AGCCGCGGCC GUU 33

(2) INFORMATION FOR SEQ ID NO:83:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:83
GGGAUGUUUG GCUAUCUCGG AUAGUGCCCC 30

(2) INFORMATION FOR SEQ ID NO:84:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:84:
GCUUAAUACG ACUCACUNUA GGGAGCUCAG 30

(2) INFORMATION FOR SEQ ID NO:85:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:85:
UUGAGUGAUG UGCUUGACGU AUCGCUGCAC 30

(2) INFORMATION FOR SEQ ID NO:86:
(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: N
(B) LOCATION: 15
(C) OTHER INFORMATION: This symbol stands for the complimentary base for the N located in position 1

(xi) SEQUENCE DESCRIPTION: SEQ ID NO:86:
NUGAUGVNCA UCCGN 15

(2) INFORMATION FOR SEQ ID NO:87:
(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: S
(B) LOCATION: 11 and 12
(C) OTHER INFORMATION: This symbol stands for the complimentary base for the S located in positions 9 and 10

(xi) SEQUENCE DESCRIPTION: SEQ ID NO:87:
AAUGCCGASS SSUUGAUGGG UU 22

(2) INFORMATION FOR SEQ ID NO:88:
(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: H
(B) LOCATION: 24
(C) OTHER INFORMATION: This symbol stands for the complimentary base for the D located in position 2

(ix) FEATURE:
(A) NAME/KEY: Y
(B) LOCATION: 25
(C) OTHER INFORMATION: This symbol stands for the complimentary base for the R located in position 25

(ii) MOLECULAR TYPE: RNA

(xi) SEQUENCE DESCRIPTION: SEQ ID NO:88:
RDGGGAACCU GCGUYUCGGC ACCHY 25

(2) INFORMATION FOR SEQ ID NO:89:
(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: D
(B) LOCATION: 18 and 19

(C) OTHER INFORMATION: This symbol stands for the complimentary base for the H located in positions 1 and 2

(xi) SEQUENCE DESCRIPTION: SEQ ID NO:89:
HHGGUUGAGU CUGUCCCDD 19

(2) INFORMATION FOR SEQ ID NO:90:
(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: N
(B) LOCATION: 18-20 and 27
(C) OTHER INFORMATION: This symbol stands for the complimentary base for the N located in positions 1 and 10-12

(xi) SEQUENCE DESCRIPTION: SEQ ID NO:90:
NRUAGUUGGN NNCUNSUNNN CGCCGUN 27

(2) INFORMATION FOR SEQ ID NO:91:
(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: M
(B) LOCATION: 20
(C) OTHER INFORMATION: This symbol stands for the complimentary base for the K located in position 14

(ix) FEATURE:
(A) NAME/KEY: S
(B) LOCATION: 31
(C) OTHER INFORMATION: This symbol stands for the complimentary base for the S located in position 2

(xi) SEQUENCE DESCRIPTION: SEQ ID NO:91:
RSGGUUUCRU GUGKRGACUM UGCCGCGGCC SU 32

(2) INFORMATION FOR SEQ ID NO:92:
(i) SEQUENCE CHARACTERISTICS:
(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO:48:
GGGAACCUGC GUYUCGGCAC C 21

(2) INFORMATION FOR SEQ ID NO:93:
(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:93:
GGUUGAGUCU GUCCC 25

(2) INFORMATION FOR SEQ ID NO:94:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:94:
AAGCAGACGA AGGGAACCUG CGUCUCGGCA CCUUCGACAU 40

(2) INFORMATION FOR SEQ ID NO:95:
(i) SEQUENCE CHARACTERISTICS:
(A) LENGTH: 31 base pairs
(B) TYPE: nucleic acid
(C) STRANDEDNESS: single
(D) TOPOLOGY: linear

(xi) SEQUENCE DESCRIPTION: SEQ ID NO:95:
GGCCGGUAGU CGCAUGGCCC AUCGCGCCCG G 31

(2) INFORMATION FOR SEQ ID NO:96:
(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:96:
GGAAGCUUGA UGGGUGACAC ACGUCAUGCC GAGCU 35

(2) INFORMATION FOR SEQ ID NO:97:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:97:
GGAAGGGAAC CUGCGUCUCG GCACCUUCG 29

(2) INFORMATION FOR SEQ ID NO:98:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:98:
GGUCAACGGU UGAGUCUGUC CCGUUCGAC 29

(2) INFORMATION FOR SEQ ID NO:99:
(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:99:
GGCUCAAUAG UUGGAGGCCU GUCCUCGCCG UAGAGC 36

(2) INFORMATION FOR SEQ ID NO:100:
(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:100:
GGAACGGUUC UGUGUGUGGA CUAGCCGCGG CCGUU 35

(2) INFORMATION FOR SEQ ID NO:101:
(i) SEQUENCE CHARACTERISTICS:
(A) LENGTH: 76 base pairs
(B) TYPE: nucleic acid
(C) STRANDEDNESS: single
(D) TOPOLOGY: linear

(xi) SEQUENCE DESCRIPTION: SEQ ID NO:101:
GGGAGACAAG AAUAACGCUC AANNNNNNNN NNNNNNNNNN NNNNNNNNNN
NNUUCGACAG GAGGCUCACA ACAGGC 76

(2) INFORMATION FOR SEQ ID NO:102:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:102:
TAATACGACT CACTATAGGG AGACAAGAAU AACGCUCAA 39

(2) INFORMATION FOR SEQ ID NO:103:
(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:103:
GCCTGTTGTG AGCCTCCTGT CGAA 24

(2) INFORMATION FOR SEQ ID NO:104:
(i) SEQUENCE CHARACTERISTICS:
(A) LENGTH: 81 base pairs
(B) TYPE: nucleic acid
(C) STRANDEDNESS: single

(D) TOPOLOGY: linear

(xi) SEQUENCE DESCRIPTION: SEQ ID NO:104:
GGGAGGACGA UGCGGNNNNN NNNNNNNNNN NNNNNNNNNN NNNNNNNNNN 50
NNNNNNNNNN NNNNNCAGAC GACTCGCCCG A 81

(2) INFORMATION FOR SEQ ID NO:105:
(i) SEQUENCE CHARACTERISTICS:
(A) LENGTH: 22 base pairs
(B) TYPE: nucleic acid
(C) STRANDEDNESS: single
(D) TOPOLOGY: linear

(xi) SEQUENCE DESCRIPTION: SEQ ID NO:105:
TAATACGACT CACTATAGGG AGGACGAUGC GG 22

(2) INFORMATION FOR SEQ ID NO:106:
(i) SEQUENCE CHARACTERISTICS:
(A) LENGTH: 16 base pairs
(B) TYPE: nucleic acid
(C) STRANDEDNESS: single
(D) TOPOLOGY: linear

(xi) SEQUENCE DESCRIPTION: SEQ ID NO:106:
TCGGGCGAGT CGTCTG 16

(2) INFORMATION FOR SEQ ID NO:107:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:107:
UGGCUGUGAU CAAUGCGGGG AGGUGAGGAA GGGCCUUACA AAUCCUUCGG 50

(2) INFORMATION FOR SEQ ID NO:108:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:108:
UGUGAUCAAU GCGGUGGCGG GGUAUGGAUG GGAGUCUGGA AUGCUGCGCU 50

(2) INFORMATION FOR SEQ ID NO:109:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:109:
CGCUGUGUUC AAUGCGGGGA UCGGGCCGGA GGAUGAACAA AUGGCGGGU 49

(2) INFORMATION FOR SEQ ID NO:110:
(i) SEQUENCE CHARACTERISTICS:
(A) LENGTH: 50 base pairs

(B) TYPE: nucleic acid
(C) STRANDEDNESS: single
(D) TOPOLOGY: linear

(xi) SEQUENCE DESCRIPTION: SEQ ID NO:110:
UGUUGAGCAA GCACUCAUGU GGUCAAUGUG GGAGUGGGAG CUGGUGGGGU 50

(2) INFORMATION FOR SEQ ID NO:111:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:111:
CAAGGGAGCG UUAGAGCCAU GUGGUCAAUG AGGGGUGGGA UUGGUUGGGU 50

(2) INFORMATION FOR SEQ ID NO:112:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:112:
CAUGGUUGUG AACUGUUGUG AUCAAUGCGG GGAGGGUAAU GGUGGGUGGU 50

(2) INFORMATION FOR SEQ ID NO:113:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:113:
AUGAGUGACA CAUGUGCUCA AUGCGGGGUG GGUUGGUAGG GGUAGCACGG 50

(2) INFORMATION FOR SEQ ID NO:114:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:114:
UGUGGUCAAU GUGGGGUAGG GCUGGUAGGG CAUUCCGUAC UGGUGUGGU 49

(2) INFORMATION FOR SEQ ID NO:115:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:115:
CCGAGUUGUG CUCAAUGUGG GGUCUGGGUA CGGACGGGAA CAGAUCUGG 49

(2) INFORMATION FOR SEQ ID NO:116:
(i) SEQUENCE CHARACTERISTICS:

(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:116:
GUGCUCAGCA UUGUGUGCUC AAUGCGGGGG AGUUUGGGUU GGCGACGG 48

(2) INFORMATION FOR SEQ ID NO:117:
(i) SEQUENCE CHARACTERISTICS:
(A) LENGTH: 16 base pairs
(B) TYPE: nucleic acid
(C) STRANDEDNESS: single
(D) TOPOLOGY: linear

(xi) SEQUENCE DESCRIPTION: SEQ ID NO:117:
UGUGNUCAAU GNGGGG 16

(2) INFORMATION FOR SEQ ID NO:118:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:118:
CAUAGGCUUA CAACAGAGCG GGGGUUCUGA UGGUAGACGC CGGGACGCCC 50

(2) INFORMATION FOR SEQ ID NO:119:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:119:
UAUGAUGGUA GACGCCGUAC CGCAUCAGGC CAAGUCGUCA CAGAUCGUG 49

(2) INFORMATION FOR SEQ ID NO:120:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:120:
GCAACAGAGG CUGAUGGUAG ACGCCGGCCA 30

(2) INFORMATION FOR SEQ ID NO:121:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:121:
AGAGUCGCUG AUGGUAGACG CCGGCGGAUC 30

(2) INFORMATION FOR SEQ ID NO:122:

(i) SEQUENCE CHARACTERISTICS:
(A) LENGTH: base pairs
(B) TYPE: 29 nucleic acid
(C) STRANDEDNESS: single
(D) TOPOLOGY: linear

(xi) SEQUENCE DESCRIPTION: SEQ ID NO:122:
GAGGCUGAUG GCAGACGCGG CCGAAGACA 29

(2) INFORMATION FOR SEQ ID NO:123:
(i) SEQUENCE CHARACTERISTICS:
(A) LENGTH: base pairs
(B) TYPE: 29 nucleic acid
(C) STRANDEDNESS: single
(D) TOPOLOGY: linear

(xi) SEQUENCE DESCRIPTION: SEQ ID NO:123:
CCCUGAUGGU AGACGCCGGG GUGCCGGAA 29

(2) INFORMATION FOR SEQ ID NO:124:
(i) SEQUENCE CHARACTERISTICS:
(A) LENGTH: 17 base pairs
(B) TYPE: nucleic acid
(C) STRANDEDNESS: single
(D) TOPOLOGY: linear

(xi) SEQUENCE DESCRIPTION: SEQ ID NO:124:
CUGAUGGUAG ACGCCGG 17

(2) INFORMATION FOR SEQ ID NO:125:
(i) SEQUENCE CHARACTERISTICS:
(A) LENGTH: 51 base pairs
(B) TYPE: nucleic acid
(C) STRANDEDNESS: single
(D) TOPOLOGY: linear

(xi) SEQUENCE DESCRIPTION: SEQ ID NO:125:
CAGUGCUGAA CUAAUCGAAC GGGGUCAAGG AGGGUCGAAC GAGAUCUGCC G 51

(2) INFORMATION FOR SEQ ID NO:126:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:126:
CACCUUCGUG GGGUCAAGGA GGGUCGCGAG GCCGCAGGAU CAACCGUGUG 50

(2) INFORMATION FOR SEQ ID NO:127:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:127:
GGUCAAGUUG GGUCGAGGAA GCGCUCCCGA GUAUCGUAGU GUGCGACUGC 50

(2) INFORMATION FOR SEQ ID NO:128:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:128:
GAACUUGAUC GGGGUCAAGG CGGGACGAA 29

(2) INFORMATION FOR SEQ ID NO:129:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:129:
UGGCGGGACC AAGGAGGGAC GUGUAGGAAA 30

(2) INFORMATION FOR SEQ ID NO:130:
(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:130:
AAAAUGCACA AAUCGGGGUC AAGGAGGGAC GA 32

(2) INFORMATION FOR SEQ ID NO:131:
(i) SEQUENCE CHARACTERISTICS:
(A) LENGTH: 47 base pairs
(B) TYPE: nucleic acid
(C) STRANDEDNESS: single
(D) TOPOLOGY: linear

(xi) SEQUENCE DESCRIPTION: SEQ ID NO:131:
AUGGGUUCGU GUGGUGAAUG GAGGAGGUGG GCUCGCAUGC UACUGUG 47

(2) INFORMATION FOR SEQ ID NO:132:
(i) SEQUENCE CHARACTERISTICS:
(A) LENGTH: 12 base pairs
(B) TYPE: nucleic acid
(C) STRANDEDNESS: single
(D) TOPOLOGY: linear

(xi) SEQUENCE DESCRIPTION: SEQ ID NO:132:
GGUCAAGGNG GG 12

(2) INFORMATION FOR SEQ ID NO:133:
(i) SEQUENCE CHARACTERISTICS:
(A) LENGTH: 44 base pairs
(B) TYPE: nucleic acid
(C) STRANDEDNESS: single
(D) TOPOLOGY: linear

(xi) SEQUENCE DESCRIPTION: SEQ ID NO:133:

UGCACUAAGU CCGGGUAGUG GGAGUGGUUG GGCCUGGAGU GCGC 44

(2) INFORMATION FOR SEQ ID NO:134:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:134:
AUCAAAGGGU AGAGGGUGGG CUGUGGCAAG 30

(2) INFORMATION FOR SEQ ID NO:135:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:135:
AAUCGAGGGU AGCGGGCGCG GCUUGGCCAA 30

(2) INFORMATION FOR SEQ ID NO:136:
(i) SEQUENCE CHARACTERISTICS:
(A) LENGTH: 31 base pairs
(B) TYPE: nucleic acid
(C) STRANDEDNESS: single
(D) TOPOLOGY: linear

(xi) SEQUENCE DESCRIPTION: SEQ ID NO:136:
GCCUCGGAUC GGGCAGCGGG UGGGAUGGCA A 31

(2) INFORMATION FOR SEQ ID NO:137:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:137:
AACGGAGUGG UAGGCGUUGG GUGCCAGGAA 30

(2) INFORMATION FOR SEQ ID NO:138:
(i) SEQUENCE CHARACTERISTICS:
(A) LENGTH: 11 base pairs
(B) TYPE: nucleic acid
(C) STRANDEDNESS: single
(D) TOPOLOGY: linear

(xi) SEQUENCE DESCRIPTION: SEQ ID NO:138:
GGUAGNGGGN G 11

(2) INFORMATION FOR SEQ ID NO:139:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:139:
AACCGAGUCG UGUGGGUUGG GGCUCCAGUA CAUCCCCGGU CUGGGUGU 48

(2) INFORMATION FOR SEQ ID NO:140:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:140:
UAACAUACGC AGUCGUGUGG GUAGGGGAUC ACAAACUGCG UAUCGUGU 48

(2) INFORMATION FOR SEQ ID NO:141:
(i) SEQUENCE CHARACTERISTICS:
(A) LENGTH: 19 base pairs
(B) TYPE: nucleic acid
(C) STRANDEDNESS: single
(D) TOPOLOGY: linear

(xi) SEQUENCE DESCRIPTION: SEQ ID NO:141:
AGUCGUGUGG GUGGGGUCA 19

(2) INFORMATION FOR SEQ ID NO:142:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:142:
AGUGUAGGAU AGGGGAUGGG AGGUCCGGGA 30

(2) INFORMATION FOR SEQ ID NO:143:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:143:
ACUGUGGGCU CUAGGGCAGU GGGAGUGGAG 30

(2) INFORMATION FOR SEQ ID NO:144:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:144:
AGUGGGACAG GGAUUGCGGA GGGUGGAAGG 30

(2) INFORMATION FOR SEQ ID NO:145:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:145:
GUCAGGAGGA CUGGAAGGUG GGACUGGUGA 30

(2) INFORMATION FOR SEQ ID NO:146:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:146:
GCAGGAGAGA GGGUGUUGGG UGCGGAUACA 30

(2) INFORMATION FOR SEQ ID NO:147:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:147:
AGGGUAGGAG GCUAAGCAUA GUUCAGAGGA GGUGGCGCGU GCCCCCGUG 49

(2) INFORMATION FOR SEQ ID NO:148:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:148:
CAACAUUGGC ACCAAUGCUC UGUGUUAAUG UGGGGUGGGA ACGGCGCCG 49

(2) INFORMATION FOR SEQ ID NO:149:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:149:
ACCAAUGAUU GCAAUGAGGG CAGUGGGGGG GAAUUGGGCU CGUGUGGU 48

(2) INFORMATION FOR SEQ ID NO:150:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:150:
GCAGUGGGUG AGGUCCGGGC ACGAUUGAGU UUGAACGGUU CUGGCUUGGU 50

(2) INFORMATION FOR SEQ ID NO:151:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:151:
GUGGUAGGUG UAGAGUGGAU GGCGGAGGUC CUAGUAGUUC UGUGCCUGGU 50

(2) INFORMATION FOR SEQ ID NO:152:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:152:
CGCGGGAGAG GGUAGUGGGU GUGGUGCUUG GACAAGCAGC G 41

(2) INFORMATION FOR SEQ ID NO:153:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:153:
ACCCGCAUAC GGACCGCGGA GGGGGAAAUC UAGCCUCAGG GGUGGCGGGC 50

(2) INFORMATION FOR SEQ ID NO:154:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:154:
UGAAGAAGCG GGGACUGCAC GACGGGAUGG AGGGACACGA CUGCGGGGU 49

(2) INFORMATION FOR SEQ ID NO:155:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:155:
ACACCAGGAG AGUGGGUUCG GGUGAGGACG 30

(2) INFORMATION FOR SEQ ID NO:156:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:156:
GUGGCUGAUG GCAGACGCCG GCUGCUGACG 30

(2) INFORMATION FOR SEQ ID NO:157:
(i) SEQUENCE CHARACTERISTICS:
(A) LENGTH: 30 base pairs
(B) TYPE: nucleic acid
(C) STRANDEDNESS: single

(D) TOPOLOGY: linear

(xi) SEQUENCE DESCRIPTION: SEQ ID NO:157:
UCGUGCCAGG ACAUGGUGGC UCAUGGGUAA 30

(2) INFORMATION FOR SEQ ID NO:158:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:158:
AGGUACGGGG GAGGGAAGGA UAUAACGCGA 30

(2) INFORMATION FOR SEQ ID NO:159:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:159:
UGGAAAGGUG UGGAAAGAGG CAUCGGGGGG 30

(2) INFORMATION FOR SEQ ID NO:160:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:160:
UCAAUGGGCA GGAAGAGGGA AGGGAUGUGA 30

(2) INFORMATION FOR SEQ ID NO:161:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:161:
CAUGGGUAAG GGAGUGGGAG UGGUGAAUAG 30

(2) INFORMATION FOR SEQ ID NO:162:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:162:
GGAACGAGUA GGGCAGUGGG UGGUGAUGGC 30

(2) INFORMATION FOR SEQ ID NO:163:
(i) SEQUENCE CHARACTERISTICS:
(A) LENGTH: 30 base pairs
(B) TYPE: nucleic acid

(C) STRANDEDNESS: single
(D) TOPOLOGY: linear

(xi) SEQUENCE DESCRIPTION: SEQ ID NO:163:
UAGGGCAGAG GGAGUGGUUA GGGCUGUGAU 30

(2) INFORMATION FOR SEQ ID NO:164:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:164:
GGGUAGUGGG AAGGGUAAGG GCCGAGGUGG 30

(2) INFORMATION FOR SEQ ID NO:165:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:165:
AAUACACACC GCGGGAAGGG AGGGUGGAAA 30

(2) INFORMATION FOR SEQ ID NO:166:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:166:
AGACUACAGC GCGGGUUAGG GUUGAGGGAA 30

(2) INFORMATION FOR SEQ ID NO:167:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:167:
UACGAGCAAG CGGGCGAAGG GUUGAGGGAA 30

(2) INFORMATION FOR SEQ ID NO:168:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:168:
CAAGGUGGUG GAGGAGGAUA CGAUCUGCAG 30

(2) INFORMATION FOR SEQ ID NO:169:
(i) SEQUENCE CHARACTERISTICS:
(A) LENGTH: 29 base pairs

(B) TYPE: nucleic acid
(C) STRANDEDNESS: single
(D) TOPOLOGY: linear

(xi) SEQUENCE DESCRIPTION: SEQ ID NO:169:
GGAGGGAAGG AGGGCAGGUG AUGGGUCAG 29

(2) INFORMATION FOR SEQ ID NO:170:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:170:
UGAUGGCGGU AGUGGAGGUA AUGAGCGUGA 30

(2) INFORMATION FOR SEQ ID NO:171:
(i) SEQUENCE CHARACTERISTICS:
(A) LENGTH: 26 base pairs
(B) TYPE: nucleic acid
(C) STRANDEDNESS: single
(D) TOPOLOGY: linear

(xi) SEQUENCE DESCRIPTION: SEQ ID NO:171:
GCAACUGGGG GCGGGUGGUG UGAGGA 26

(2) INFORMATION FOR SEQ ID NO:172:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:172:
GGAGGGGCCU AUAGGGGUGG UGGUGUACGA 30

(2) INFORMATION FOR SEQ ID NO:173:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:173:
UAUAGGGUAG UGGGUGUAGG UAGGGCGACA 30

(2) INFORMATION FOR SEQ ID NO:174:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:174:
GAGGGUUGGA GGGCAUGGGG CAGGAACCGG 30

(2) INFORMATION FOR SEQ ID NO:175:
(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:175:
CGUAGAACUG GCGGGCAGUG GGGGGGAUGC 30

(2) INFORMATION FOR SEQ ID NO:176:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:176:
UGAGGGGACG AGGGAUGUGG GGAGCGGGAC 30

(2) INFORMATION FOR SEQ ID NO:177:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:177:
CGAGGGAUGG GAGGCGUGUG GAAGAUGCAA 30

(2) INFORMATION FOR SEQ ID NO:178:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:178:
GCAUCCGGGG ACAAGAUGGG UCGGUAAGGU 30

(2) INFORMATION FOR SEQ ID NO:179:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:179:
GUGUGCGGGG UCAAGACGGG UGGCGUGCG 29

(2) INFORMATION FOR SEQ ID NO:180:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:180:
UCAAACCAUG GGGCGGGUGG UACGAGGAAC 30

(2) INFORMATION FOR SEQ ID NO:181:

(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:181:
CGAGUCCGAG GGAUGGGUGG UGUGCGGCAA 30

(2) INFORMATION FOR SEQ ID NO:182:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:182:
CAGUGUCGGA GAGGAGGAUG GAGGUAUGAA 30

(2) INFORMATION FOR SEQ ID NO:183:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:183:
CACCACUACG CGGGAAGGGU AGGGUGGAUU ACAAGGUGUG ACCGCUCCGU 50

(2) INFORMATION FOR SEQ ID NO:184:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:184:
UACGGUUAAC GGGGGUGGUG UGGGAGGACA CAAAGCGCGU ACCUGCCCC 49

(2) INFORMATION FOR SEQ ID NO:185:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:185:
AGGUCCUCGA GGGUCUGGGU GUGGGAGUGG GCAUGGACCA AUACCGCGUG 50

(2) INFORMATION FOR SEQ ID NO:186:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:186:
AAACCCAUCC UGCGCGGGAU GGGAGGGUGG AAACACUAGA GCUUCGCCUG 50

(2) INFORMATION FOR SEQ ID NO:187:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:187:
AACUGGUGGU CACGCGUUGA GGUGGUGGAG GUGGAUUCAA CGGUCGAGGG 50

(2) INFORMATION FOR SEQ ID NO:188:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:188:
CAUGAAAGUA GGGUUAUGAA GGCGGUAGAU GGAGGAGGUU GGGUUGCCGC 50

(2) INFORMATION FOR SEQ ID NO:189:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:189:
GUCUAUUGGG UAGGUGUUUG CAAGAAUUCC GCACGAUAGG UAAAACAGUG 50

(2) INFORMATION FOR SEQ ID NO:190:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:190:
UGUAGGGGAA GUACGAGAGU GGGAGCGGCC GUAUAGGUGG GAGUGUGCU 49

(2) INFORMATION FOR SEQ ID NO:191:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:191:
GAGUAGGCUU GACGCCUGGG GGGGUAUGGC UUCGACUGCG 40

(2) INFORMATION FOR SEQ ID NO:192:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:192:
GAGUAGGCUU GACGCCUGGG GGGGUAUGGC UUCAACUGCG 40

(2) INFORMATION FOR SEQ ID NO:193:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:193:
GAGUAGGCUU GACGCUGGGG GGGUAUGGCU UCGACUUGCG 40

(2) INFORMATION FOR SEQ ID NO:194:
(i) SEQUENCE CHARACTERISTICS:
(A) LENGTH: 42 base pairs
(B) TYPE: nucleic acid
(C) STRANDEDNESS: single
(D) TOPOLOGY: linear

(xi) SEQUENCE DESCRIPTION: SEQ ID NO:194:
AGAGGUAGGC UUGACGCCUG GGGGGGUAUG GCUCCGACUG CG 42

(2) INFORMATION FOR SEQ ID NO:195:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:195:
GGUAGACAUA CGCCUUGGGG GGGUGUCAGG GUAUAUGGUA 40

(2) INFORMATION FOR SEQ ID NO:196:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:196:
GGCAGACAUA CGCCUUGGGG GGGUGUCAGG GUAUAUGGUA 40

(2) INFORMATION FOR SEQ ID NO:197:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:197:
GGUAGACAUA CGCCUUGGGG GGGUGCCAGG GUAUAUGGUA 40

(2) INFORMATION FOR SEQ ID NO:198:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:198:
GGUAGACAUA CGCCUUGGGG GGGUAUCAGG GUAUAUGGUA 40

(2) INFORMATION FOR SEQ ID NO:199:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:199:
GGCAGACAUA CGCCUUGGGG GGGUGUCAGG GUAUAUGGCA 40

(2) INFORMATION FOR SEQ ID NO:200:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:200:
GGCAGACAUA CGCCUUGGGG GGGUUUCAGG GUAUAUGGCA 40

(2) INFORMATION FOR SEQ ID NO:201:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:201:
AGAAUGAAUG UGAUGAACAG GUGUCGGGGU GGAUGGGUGG 40

(2) INFORMATION FOR SEQ ID NO:202:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:202:
GGAAUGAAUG UGAUGAACAG GUGUCGGGGU GGAUGGGUGG 40

(2) INFORMATION FOR SEQ ID NO:203:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:203:
GAAUGAAUGU GAUGAACAGG UGUCGGGGUG GAUGGGUGG 39

(2) INFORMATION FOR SEQ ID NO:204:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:204:
CGCCUUAGUC GCCAUAAUGC UGUCGGGGUG GAUAGGGUGG 40

(2) INFORMATION FOR SEQ ID NO:205:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:205:
UGCCCGGCAG UACUGCACGG CCUCGGGGGG GACCAGGGAG 40

(2) INFORMATION FOR SEQ ID NO:206:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:206:
AUCGGAUAGC GCCGCGAUGG CGUCUGGGGG GGACCAGGGA 40

(2) INFORMATION FOR SEQ ID NO:207:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:207:
GUACUACUGC AAGCCCGUGU GGCGCGGUCA GUGGGUGGC 39

(2) INFORMATION FOR SEQ ID NO:208:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:208:
GUACUACUGC AAGCCCGUGU GGCGCGGUCA GUGGGUGGCC 40

(2) INFORMATION FOR SEQ ID NO:209:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:209:
CGGAUCGGGC GGUCGUCUAG CGGGAUGGCG UGCGGGUGGA 40

(2) INFORMATION FOR SEQ ID NO:210:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:210:
UGACCGGGUC GACAUGCCUG AGGUGUGGCC AGUGGGUGG 39

(2) INFORMATION FOR SEQ ID NO:211:
(i) SEQUENCE CHARACTERISTICS:
(A) LENGTH: 37 base pairs
(B) TYPE: nucleic acid
(C) STRANDEDNESS: single
(D) TOPOLOGY: linear

(xi) SEQUENCE DESCRIPTION: SEQ ID NO:211:
GGCCCUCUGA ACCGUCUGGG CGUGGUUAGU GGGUGGC 37

(2) INFORMATION FOR SEQ ID NO:212:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:212:
UGACCGGGCC GACAUGCUUG AGGUGUGGCC CAGUGGGUGG 40

(2) INFORMATION FOR SEQ ID NO:213:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:213:
GACGCGUGCU GGCCUCGACC GUGUGGGUGC GGAUGGGUGG 40

(2) INFORMATION FOR SEQ ID NO:214:
(i) SEQUENCE CHARACTERISTICS:
(A) LENGTH: 44 base pairs
(B) TYPE: nucleic acid
(C) STRANDEDNESS: single
(D) TOPOLOGY: linear

(xi) SEQUENCE DESCRIPTION: SEQ ID NO:214:
UGAGGUUGAG UAGUCGUGGU AAUGGCCACG GCUGGGGGGG UCGG 44

(2) INFORMATION FOR SEQ ID NO:215:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:215:
UGGAUAGCGC UGCGUGACCG CGCUGGGGGG GUUGGCAGGG 40

(2) INFORMATION FOR SEQ ID NO:216:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:216:
ACCTTTTCCT CTCAGTCTTC TTATCTCGCC TATTATTATT 40

(2) INFORMATION FOR SEQ ID NO:217:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:217:
GCGTGGGTTG GGGCCGGGAG GGCCAGCAGT CTCGTGCGTC 40

(2) INFORMATION FOR SEQ ID NO:218:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:218:
CATTCATCTT CTCATTCTCG CCTAACTGTA CACATCTTT 39

(2) INFORMATION FOR SEQ ID NO:219:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:219:
GCGTGGGTTG GGGCCGGGAG GGCCAGCAGT CTCGTGCGT 39

(2) INFORMATION FOR SEQ ID NO:220:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:220:
GCGTGGGTTG GGGCCGGGAG GGCCAACAGT CTCGTGCGTC 40

(2) INFORMATION FOR SEQ ID NO:221:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:221:
CTACCCTTTC TTGACCACCG CCTCGTTTCA TCCACCTTAC 40

(2) INFORMATION FOR SEQ ID NO:222:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:222:
TTCTTTCTAT ACCCATATTA CCCTTCTTCA CACTCGTATC 40

(2) INFORMATION FOR SEQ ID NO:223:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:223:
CTTTATCCTT TCTCTTTCCT TGCACTCTAA CATCCTACTC 40

(2) INFORMATION FOR SEQ ID NO:224:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:224:
GCGTGGGTTG GGGCCGGTAG GGCCAGCAGT CTCGTTGCGT 40

(2) INFORMATION FOR SEQ ID NO:225:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:225:
CCTTCTTGTT ATATTGGTCG TTTTCTTCTT TTACTTTCTT 40

(2) INFORMATION FOR SEQ ID NO:226:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:226:
TCTTCATCAT TTCACTTCAT TCTGTCGGGC TATCTTCGGT 40

(2) INFORMATION FOR SEQ ID NO:227:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:227:
TTCCACGTCT CCTCAGCCCG GGAGGCCACC TTTTTATCTG 40

(2) INFORMATION FOR SEQ ID NO:228:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:228:
GAAGGCTTAA CCTAATTTTC CACCTTTCAT CCACTTTTCC 40

(2) INFORMATION FOR SEQ ID NO:229:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:229:
TCACCTCCCA TTTATATTTT CCCTTAATTT CTTCTTCTTA 40

(2) INFORMATION FOR SEQ ID NO:230:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:230:
CTTACTATGC ATCTTACTTA TTATTTTTTT TTACTTTCTA 40

(2) INFORMATION FOR SEQ ID NO:231:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:231:
TACTTCTTTT ACATCATTCC TCGATTTATT CATTCTCCAC 40

(2) INFORMATION FOR SEQ ID NO:232:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:232:
TTCACCCGTG TCATATCATA TTTCCCGGTC CTTCCTTTCC C 41

(2) INFORMATION FOR SEQ ID NO:233:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:233:
CAATTCAAAC CTTTTCTACA ATTTTCATCT TACATTCTTC 40

(2) INFORMATION FOR SEQ ID NO:234:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:234:
TCACTTGATC CTTCTTTACT TTTTTTCTCG TCTAATTATA 40

(2) INFORMATION FOR SEQ ID NO:235:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:235:
GCGTGGGTTG GGGCGGGATG GGCCAGCAGT CTCGTGCGGT 40

(2) INFORMATION FOR SEQ ID NO:236:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:236:
CTTTTTATTC CAACCCCCAT TCTTACTTAC AATATCTTGA 40

(2) INFORMATION FOR SEQ ID NO:237:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:237:
TATCCTTCTC CTTAACTCCT ACTTCTATCT ATAAAATTAT 40

(2) INFORMATION FOR SEQ ID NO:238:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:238:
GCGTGGGTAG GGGCCGGGAG GGCCAGCAGT CACGTGCGTA 40

(2) INFORMATION FOR SEQ ID NO:239:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:239:
GGGTGGGTTG GGGCCGGGAG GGCTAGCAGT CTCGTGCGTT 40

(2) INFORMATION FOR SEQ ID NO:240:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:240:
GCGTGGGATG GGGCCGGGAG GGCCAGCAGT CTCGTGCGTT 40

(2) INFORMATION FOR SEQ ID NO:241:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:241:
GCGTGGGTTG GGGCCGGGAG GGCCAGCAGT CTCGTGCGT 39

(2) INFORMATION FOR SEQ ID NO:242:
(i) SEQUENCE CHARACTERISTICS:
(A) LENGTH: 59 base pairs
(B) TYPE: nucleic acid
(C) STRANDEDNESS: single
(D) TOPOLOGY: linear

(xi) SEQUENCE DESCRIPTION: SEQ ID NO:242:
TAGCGATACT GCGTGGGTTG GGGCGGGTAG GGCCAGCAGT CTCGTGCGGT 50
ACTTGAGCA 59

(2) INFORMATION FOR SEQ ID NO:243:
(i) SEQUENCE CHARACTERISTICS:
(A) LENGTH: 56 base pairs
(B) TYPE: nucleic acid
(C) STRANDEDNESS: single
(D) TOPOLOGY: linear

(xi) SEQUENCE DESCRIPTION: SEQ ID NO:243:
TAGCGATACT GCGTGGGNGG GNGGGNGGGC CAGCAGTCTC GTGCGGTACT 50
TGAGCA 56

(2) INFORMATION FOR SEQ ID NO:244:
(i) SEQUENCE CHARACTERISTICS:
(A) LENGTH: 6 base pairs
(B) TYPE: nucleic acid
(C) STRANDEDNESS: single
(D) TOPOLOGY: linear

(xi) SEQUENCE DESCRIPTION: SEQ ID NO:244:
AUACCA 6

(2) INFORMATION FOR SEQ ID NO:245:
(i) SEQUENCE CHARACTERISTICS:
(A) LENGTH: 9 base pairs
(B) TYPE: nucleic acid
(C) STRANDEDNESS: single
(D) TOPOLOGY: linear

(xi) SEQUENCE DESCRIPTION: SEQ ID NO:245:
CCUUGGMAG 9

(2) INFORMATION FOR SEQ ID NO:246:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:246:
GAGAAAUACC AGUGACAACU CUCGAGAUCA CCCUUGGAAG 40

(2) INFORMATION FOR SEQ ID NO:247:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:247:
AUACCAUCGU GUAAGCAAGA GCACGACCUU GGCAGUGUGU G 41

(2) INFORMATION FOR SEQ ID NO:248:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:248:
GAUACCAGCA GCAUAUUUGC UGUCCUUGGA AGCAACGAGA 40

(2) INFORMATION FOR SEQ ID NO:249:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:249:
GUGAUACCAG CAUCGUCUUG AUGCCCUUGG CAGCACUUCA 40

(2) INFORMATION FOR SEQ ID NO:250:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:250:
UUGUCGAAUC GGAUACCAGC AAUGCAGCCC UUGGAAGCAG 40

(2) INFORMATION FOR SEQ ID NO:251:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:251:
GAUACCAACG GCAUAUUUGC UGUCCUUGGA AGCAACUAUA 40

(2) INFORMATION FOR SEQ ID NO:252:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:252:
CUCUCGAAAU ACCAACUACU CUCACAAUAG UCCUUGGAAG 40

(2) INFORMATION FOR SEQ ID NO:253:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:253:
UUCAUGUCGC UUGAUACCAU CAACAAUGAC CUUGGAAGCA 40

(2) INFORMATION FOR SEQ ID NO:254:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:254:
UGACUCGAAC CCUUGGAAGA CCUGAGUACA GGUAUACCAG 40

(2) INFORMATION FOR SEQ ID NO:255:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:255:
UCCUUGGAAG CCGUACGGAU ACCAAUUGAG UGGCCAUAUG 40

(2) INFORMATION FOR SEQ ID NO:256:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:256:
UAUCGAGUGG CCUUGGCAGA CCAGGCCCGG UAUACCACCA 40

(2) INFORMATION FOR SEQ ID NO:257:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:257:
CGAGAUUCAA CCUUGGAAGU CAAUCGUGAA UACCAUUGUU 40

(2) INFORMATION FOR SEQ ID NO:258:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:258:
UCAGAACCUU GGAAGCACUG AAUAAGAUCA GUUGAUACCA 40

(2) INFORMATION FOR SEQ ID NO:259:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:259:
GGACGAGACT CATCGTAACC TAGATGGTTG CCAGCATTTA 40

(2) INFORMATION FOR SEQ ID NO:260:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:260:
GGATGCTTAC AGCATAATCG GAATTGATTG CCAGCGGAAA 40

(2) INFORMATION FOR SEQ ID NO:261:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:261:
GGGGCAATAG AAGCCAACGC ACAGTCGTTG CCAGTGTTCG 40

(2) INFORMATION FOR SEQ ID NO:262:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:262:
GGGATGGATC TTCGGATACG TCAACCAAAG GTTGCCAGCG 40

(2) INFORMATION FOR SEQ ID NO:263:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:263:
GCGCATCGTA AAAAGGACAA ACGTCGTCGT GACCCCGATA 40

(2) INFORMATION FOR SEQ ID NO:264:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:264:
GTGCATCGTA AAAAGGACAA ACGTCGTCGT GACCCCGATA 40

(2) INFORMATION FOR SEQ ID NO:265:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:265:
AGACGGTGAA ACTGAAATCT AATCCGTCTG AACCCTGGAT 40

(2) INFORMATION FOR SEQ ID NO:266:
(i) SEQUENCE CHARACTERISTICS:
(A) LENGTH: 40 base pairs
(B) TYPE: nucleic acid
(C) STRANDEDNESS: single
(D) TOPOLOGY: linear
(xi) SEQUENCE DESCRIPTION: SEQ ID NO:266:
AGACGGTGAA GCTGAAATCT AATCCGTCTG AACCCTGGAC 40

(2) INFORMATION FOR SEQ ID NO:267:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:267:
GGGAGGTCAA GGACCTCACA CTTTTGTGTG CCAGCGCTAT 40

(2) INFORMATION FOR SEQ ID NO:268:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:268:
GAAAGCGTTG TGGCGTGCCA TCGCCCGCAG GCGAATAACA 40

(2) INFORMATION FOR SEQ ID NO:269:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:269:
ACGATGGGTT GTTATAGTGG AAACGGTAAG TTCGAGTCTG 40

(2) INFORMATION FOR SEQ ID NO:270:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:270:
ACGGTGATCC TCTAATCCGT CGACAGAATC GATGTCAATC 40

(2) INFORMATION FOR SEQ ID NO:271:
(i) SEQUENCE CHARACTERISTICS:
(A) LENGTH: 87 base pairs
(B) TYPE: nucleic acid
(C) STRANDEDNESS: single
(D) TOPOLOGY: linear

(xi) SEQUENCE DESCRIPTION: SEQ ID NO:271:
GCCGGATCCG GGCCTCATGT CGAANNNNNN NNNNNNNNNN NNNNNNNNNN 50
NNNNNNNNNN NNNNTTGAGC GTTTATTCTG AGCTCCC 87

(2) INFORMATION FOR SEQ ID NO:272:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:272:
CCGAAGCTTA ATACGACTCA CTATAGGGAG CTCAGAATAA ACGCTCAA 48

(2) INFORMATION FOR SEQ ID NO:273:
(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:273:
GCCGGATCCG GGCCTCATGT CGAA 24

(2) INFORMATION FOR SEQ ID NO:274:
(i) SEQUENCE CHARACTERISTICS:
(A) LENGTH: 87 base pairs
(B) TYPE: nucleic acid
(C) STRANDEDNESS: single
(D) TOPOLOGY: linear

(xi) SEQUENCE DESCRIPTION: SEQ ID NO:274:
CCCCTGCAGG TGATTTTGCT CAAGTNNNNN NNNNNNNNNN NNNNNNNNNN 50
NNNNNNNNNN NNNNNAGTAT CGCTAATCAG GCGGATC 87

(2) INFORMATION FOR SEQ ID NO:275:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:275:
CCGAAGCTTA ATACGACTCA CTATAGGGAT CCGCCTGATT AGCGATACT 49

(2) INFORMATION FOR SEQ ID NO:276:
(i) SEQUENCE CHARACTERISTICS:
(A) LENGTH: 25 base pairs
(B) TYPE: nucleic acid

(C) STRANDEDNESS: single
(D) TOPOLOGY: linear

(xi) SEQUENCE DESCRIPTION: SEQ ID NO:276:
CCCCTGCAGG TGATTTTGCT CAAGT 25

(2) INFORMATION FOR SEQ ID NO:277:
(i) SEQUENCE CHARACTERISTICS:
(A) LENGTH: 51 base pairs
(B) TYPE: nucleic acid
(C) STRANDEDNESS: single
(D) TOPOLOGY: linear

(xi) SEQUENCE DESCRIPTION: SEQ ID NO:277:
AACGCUCAAU ACCAUCGUGU AAGAAAGAGC ACGACCUUGG CAGUGUGUGU 50
U 51

(2) INFORMATION FOR SEQ ID NO:278:
(i) SEQUENCE CHARACTERISTICS:
(A) LENGTH: 42 base pairs
(B) TYPE: nucleic acid
(C) STRANDEDNESS: single
(D) TOPOLOGY: linear

(xi) SEQUENCE DESCRIPTION: SEQ ID NO:278:
AAGUGAUACC AGCAUCGUCU UGAUGCCCUU GGCAGCACUU CA 42

(2) INFORMATION FOR SEQ ID NO:279:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:279:
GAUACCAACA GCAUAUUUGC UGUCCUUGGA AGCAACGAGA 40

(2) INFORMATION FOR SEQ ID NO:280:
(i) SEQUENCE CHARACTERISTICS:
(A) LENGTH: 42 base pairs
(B) TYPE: nucleic acid
(C) STRANDEDNESS: single
(D) TOPOLOGY: linear

(xi) SEQUENCE DESCRIPTION: SEQ ID NO:280:
GAGAAAUACC AGUGACAACU CUCGAGAUCA CCCUUGGAAG UU 42

(2) INFORMATION FOR SEQ ID NO:281:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:281:
UAUCGAGUGG CCUUGGCAGA CCAGGCCGGG UAUACCACCA 40

(2) INFORMATION FOR SEQ ID NO:282:

(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:282:
CGAGAUUCAA CCUUGGAAGU CAAUCGUGAA UACCAUUGUU 40

(2) INFORMATION FOR SEQ ID NO:283:
(i) SEQUENCE CHARACTERISTICS:
(A) LENGTH: 45 base pairs
(B) TYPE: nucleic acid
(C) STRANDEDNESS: single
(D) TOPOLOGY: linear

(xi) SEQUENCE DESCRIPTION: SEQ ID NO:283:
UGACUCGAAC CCUUGGAAGA CCUGAGUACA GGUAUACCAG UUCGA 45

(2) INFORMATION FOR SEQ ID NO:284:
(i) SEQUENCE CHARACTERISTICS:
(A) LENGTH: 47 base pairs
(B) TYPE: nucleic acid
(C) STRANDEDNESS: single
(D) TOPOLOGY: linear

(xi) SEQUENCE DESCRIPTION: SEQ ID NO:284:
CGCUCAAUCC UUGGAAGCCG UACGGAUACC AAUUGAGUGG CCAUAUG 47

(2) INFORMATION FOR SEQ ID NO:285:
(i) SEQUENCE CHARACTERISTICS:
(A) LENGTH: 28 base pairs
(B) TYPE: nucleic acid
(C) STRANDEDNESS: single
(D) TOPOLOGY: linear

(xi) SEQUENCE DESCRIPTION: SEQ ID NO:285:
NNAUACCANN NNNNNNNCCU UGGMAGNN 28

(2) INFORMATION FOR SEQ ID NO:286:
(i) SEQUENCE CHARACTERISTICS:
(A) LENGTH: 38 base pairs
(B) TYPE: nucleic acid
(C) STRANDEDNESS: single
(D) TOPOLOGY: linear

(xi) SEQUENCE DESCRIPTION: SEQ ID NO:286:
GGUGAUACCA GCAUCGUCUU GAUGCCCUUG GCAGCACC 38

SEQUENCE LISTING

(1) GENERAL INFORMATION:

(i) APPLICANT: PIEKEN, WOLFGANG
TASSET, DIANE
JANJIC, NEBOJSA
GOLD, LARRY
KIRSCHENHEUTER, GARY P.
POLISKY, GARY
JENISON, ROBERT D.
JAYASENA, SUMEDHA
BIESECKER, GREG
SMITH, DREW

(ii) TITLE OF INVENTION: NUCLEIC ACID LIGANDS AND IMPROVED METHODS FOR PRODUCING THE SAME

(iii) NUMBER OF SEQUENCES: 286

(iv) CORRESPONDENCE ADDRESS:
(A) ADDRESSEE: Swanson & Bratschun, L.L.C.
(B) STREET: 8400 E. Prentice Place, Suite 200
(C) CITY: Englewood
(D) STATE: Colorado
(E) COUNTRY: USA
(F) ZIP: 80111

(v) COMPUTER READABLE FORM:
(A) MEDIUM TYPE: Diskette, 3.5 inch, 360 Kb storage
(B) COMPUTER: IBM compatible
(C) OPERATING SYSTEM: MS-DOS
(D) SOFTWARE: WordPerfect 5.1

(vi) CURRENT APPLICATION DATA:
(A) APPLICATION NUMBER:
(B) FILING DATE:
(C) CLASSIFICATION:

(vii)PRIOR APPLICATION DATA:
(A) APPLICATION NUMBER: 08/117,991
(B) FILING DATE: 8-SEPTEMBER-1993

(vii)PRIOR APPLICATION DATA:
(A) APPLICATION NUMBER: 08/134,029
(B) FILING DATE: 7-OCTOBER-1993

(vii)PRIOR APPLICATION DATA:
(A) APPLICATION NUMBER: 08/199,507
(B) FILING DATE: 22-FEB-1994

(vii)PRIOR APPLICATION DATA:
(A) APPLICATION NUMBER: 08/233,012
(B) FILING DATE: 25-APRIL-1994

(vii)PRIOR APPLICATION DATA:
(A) APPLICATION NUMBER: 08/234,997
(B) FILING DATE: 28-APRIL-1994

(viii)ATTORNEY/AGENT INFORMATION:
(A) NAME: Barry J. Swanson
(B) REGISTRATION NUMBER: 33,215
(C) REFERENCE/DOCKET NUMBER: NEX09/PCT

(ix) TELECOMMUNICATION INFORMATION:
(A) TELEPHONE: (303) 793-3333
(B) TELEFAX: (303) 793-3433

(2) INFORMATION FOR SEQ ID NO:1:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:1:
GGGAGAUGCC UGUCGAGCAU GCUGAGGAUC GAAGUUAGUA GGCUUUGUGU 50
GCUCGUAGCU AAACAGCUUU GUCGACUCU 79

(2) INFORMATION FOR SEQ ID NO:2:
(i) SEQUENCE CHARACTERISTICS:
(A) LENGTH: 76 base pairs
(B) TYPE: nucleic acid
(C) STRANDEDNESS: single
(D) TOPOLOGY: linear

(xi) SEQUENCE DESCRIPTION: SEQ ID NO:2:
GGGAGAUGCC UGUCGAGCAU GCUGUACUGG AUCGAAGGUA GUAGGCAGUC 50
ACGUAGCUAA ACAGCUUUGU GACUCU 76

(2) INFORMATION FOR SEQ ID NO:3:
(i) SEQUENCE CHARACTERISTICS:
(A) LENGTH: 76 base pairs
(B) TYPE: nucleic acid
(C) STRANDEDNESS: single
(D) TOPOLOGY: linear
(xi) SEQUENCE DESCRIPTION: SEQ ID NO:3:

GGGAGAUGCC UGUCGAGCAU GCUGAUAUCA CGGAUCGAAG GAAGUAGGCG 50
UGGUAGCUAA ACAGCUUUGU GACUCU 76

(2) INFORMATION FOR SEQ ID NO:4:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:4:
GGGAGAUGCC UGUCGAGCAU GCUGCCUUUC CCGGGUUCGA AGUCAGUAGG 50
CCGGGUAGCU AAACAGCUUU GUCGACUCU 79

(2) INFORMATION FOR SEQ ID NO:5:
(i) SEQUENCE CHARACTERISTICS:
(A) LENGTH: 78 base pairs
(B) TYPE: nucleic acid
(C) STRANDEDNESS: single
(D) TOPOLOGY: linear

(xi) SEQUENCE DESCRIPTION: SEQ ID NO:5:
GGGAGAUGCC UGUCGAGCAU GCUGCACCCG GAUCGAAGUU AGUAGGCGUG 50
AGUGUAGCUA AACAGCUUUG UCGACUCU 78

(2) INFORMATION FOR SEQ ID NO:6:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:6:
GGGAGAUGCC UGUCGAGCAU GCUGUGUACG GAUCGAAGGU AGUAGGCAGG 50
UUACGUAGCU AAACAGCUUU GUCGACUCU 79

(2) INFORMATION FOR SEQ ID NO:7:
(i) SEQUENCE CHARACTERISTICS:

(A) LENGTH: 78 base pairs
(B) TYPE: nucleic acid
(C) STRANDEDNESS: single
(D) TOPOLOGY: linear

(xi) SEQUENCE DESCRIPTION: SEQ ID NO:7:
GGGAGAUGCC UGUCGAGCAU GCUGCAUCCG GAUCGAAGUU AGUAGGCGGA 50
GUGGUAGCUA AACAGCUUUG UCGACUCU 78

(2) INFORMATION FOR SEQ ID NO:8:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:8:
GGGAGAUGCC UGUCGAGCAU GCUGAUUGUU GCGGAUCGAA GUGGAGUAGG 50
CGCAGUAGCU AAACAGCUUU GUCGACUCU 79

(2) INFORMATION FOR SEQ ID NO:9:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:9:
GGGAGAUGCC UGUCGAGCAU GCUGUGUACU GGAUCGAAGG UAGUAGCGAG 50
UCACGUAGCU AAACAGCUUU GUCGACUCU 79

(2) INFORMATION FOR SEQ ID NO:10:
(i) SEQUENCE CHARACTERISTICS:
(A) LENGTH: 72 base pairs
(B) TYPE: nucleic acid
(C) STRANDEDNESS: single
(D) TOPOLOGY: linear

(xi) SEQUENCE DESCRIPTION: SEQ ID NO:10:
GGGAGAUGCC UGUCGAGCAU GCUGAUCGAA GUUAGUAGGA GCGUGUGGUA 50
GCUAAACAGC UUUGUCGACU CU 72

(2) INFORMATION FOR SEQ ID NO:11:
(i) SEQUENCE CHARACTERISTICS:
(A) LENGTH: 84 base pairs
(B) TYPE: nucleic acid
(C) STRANDEDNESS: single
(D) TOPOLOGY: linear

(xi) SEQUENCE DESCRIPTION: SEQ ID NO:11:
GGGAGAUGCC UGUCGAGCAU GCUGACGCUA GAGUCGGAUC GAAAGGUAAG 50
UAGGCGACUG UAGCUAAACA GCUUUGUCGA CUCU 84

(2) INFORMATION FOR SEQ ID NO:12:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:12:
GGGAGAUGCC UGUCGAGCAU GCUGGGGUCG GAUCGAAAGG UAAGUAGGCG 50
ACUGUAGCUA AACAGCUCUU GUCGACUCU 79

(2) INFORMATION FOR SEQ ID NO:13:
(i) SEQUENCE CHARACTERISTICS:
(A) LENGTH: 77 base pairs
(B) TYPE: nucleic acid

(C) STRANDEDNESS: single
(D) TOPOLOGY: linear

(xi) SEQUENCE DESCRIPTION: SEQ ID NO:13:
GGGAGAUGCC UGUCGAGCAU GCUGAUAUCA CGGAUCGAAA GAGAGUAGGC 50
GUGUAGCUAA ACAGCUUUGU CGACUCU 77

(2) INFORMATION FOR SEQ ID NO:14:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:14:
GGGAGAUGCC UGUCGAGCAU GCUGUGUACU GGAUCGAAGG UAGUAGGCAG 50
GCACGUAGCU AAACAGCUUU GUCGACUCU 79

(2) INFORMATION FOR SEQ ID NO:15:

(i) SEQUENCE CHARACTERISTICS:
(A) LENGTH: 78 base pairs
(B) TYPE: nucleic acid
(C) STRANDEDNESS: single
(D) TOPOLOGY: linear

(xi) SEQUENCE DESCRIPTION: SEQ ID NO:15:
GGGAGAUGCC UGUCGAGCAU GCUGAUAUCA CGGAUCGAAG GAAAGUAGGC 50
GUGGUAGCUA AACAGCUUUG UCGACUCU 78

(2) INFORMATION FOR SEQ ID NO:16:
(i) SEQUENCE CHARACTERISTICS:
(A) LENGTH: 75 base pairs
(B) TYPE: nucleic acid
(C) STRANDEDNESS: single
(D) TOPOLOGY: linear

(xi) SEQUENCE DESCRIPTION: SEQ ID NO:16:
GGGAGAUGCC UGUCGAGCAU GCUGGUGCGG CUUUGGGCGC CGUGCUUGGC 50
GUAGCUAAAC AGCUUUGUCG ACUCU 75

(2) INFORMATION FOR SEQ ID NO:17:
(i) SEQUENCE CHARACTERISTICS:
(A) LENGTH: 74 base pairs
(B) TYPE: nucleic acid
(C) STRANDEDNESS: single
(D) TOPOLOGY: linear

(xi) SEQUENCE DESCRIPTION: SEQ ID NO:17:
GGGAGAUGCC UGUCGAGCAU GCUGGUGCGG CUUUGGGCGC CGUGCUUACG 50
UAGCUAAACA GCUUUGUCGA CUCU 74

(2) INFORMATION FOR SEQ ID NO:18:
(i) SEQUENCE CHARACTERISTICS:
(A) LENGTH: 75 base pairs
(B) TYPE: nucleic acid
(C) STRANDEDNESS: single
(D) TOPOLOGY: linear

(xi) SEQUENCE DESCRIPTION: SEQ ID NO:18:
GGGAGAUGCC UGUCGAGCAU GCUGGUGCGG CUUUGGGCGC CGUGCUUGAC 50
GUAGCUAAAC AGCUUUGUCG ACUCU 75

(2) INFORMATION FOR SEQ ID NO:19:
(i) SEQUENCE CHARACTERISTICS:
(A) LENGTH: 74 base pairs
(B) TYPE: nucleic acid
(C) STRANDEDNESS: single

(D) TOPOLOGY: linear

(xi) SEQUENCE DESCRIPTION: SEQ ID NO:19:
GGGAGAUGCC UGUCGAGCAU GCUGGGGCGG CUUUGGGCGC CGUGCUUGAC 50
GUAGCUAAAC AGCUUUGUCG ACUC 74

(2) INFORMATION FOR SEQ ID NO:20:
(i) SEQUENCE CHARACTERISTICS:
(A) LENGTH: 79 base pairs
(B) TYPE: nucleic acid
(C) STRANDEDNESS: single
(D) TOPOLOGY:

(xi) SEQUENCE DESCRIPTION: SEQ ID NO:20:
GGGAGAUGCC UGUCGAGCAU GCUGUGAGCC UGCCAGUGUG UAUGUGGAAA 50
CAAGGUAGCU AAACAGCUUU GUCGACUCU 79

(2) INFORMATION FOR SEQ ID NO:21:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:21:
GGGAGAUGCC UGUCGAGCAU GCUGUGAGCC UGCCAGUGUG CAUGUGGAAA 50
CAAGGUAGCU AAACAGCUUU GUCGACUCU 79

(2) INFORMATION FOR SEQ ID NO:22:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:22:
GGGAGAUGCC UGUCGAGCAU GCUGUGAGCC AGCCAGUGUG CAUGUGGAAA 50
CAAGGUAGCU AAACAGCUUU GUCGACUCU 79

(2) INFORMATION FOR SEQ ID NO:23:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:23:
GGGAGAUGCC UGUCGAGCAU GCUGUGAGCC AGCCAGUGUG UAUGUGGAAA 50
CAAGGUAGCU AAACAGCUUU GUCGACUCU 79

(2) INFORMATION FOR SEQ ID NO:24:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:24:
GGGAGAUGCC UGUCGAGCAU GCUGUGAGCC GGCCAGUGUG CAUGUGGAAA 50
CAAGGUAGCU AAACAGCUUU GUCGACUCU 79

(2) INFORMATION FOR SEQ ID NO:25:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:25:
GGGAGAUGCC UGUCGAGCAU GCUGUGAGCC AGCCAGUGUG UAUGUGGAAA 50
CAAGGUAGCU AAACAGCUUU GUCGACUCU 79

(2) INFORMATION FOR SEQ ID NO:26:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:26:
GGGAGAUGCC UGUCGAGCAU GCUGUUACGG GGAGGUGUUA CGGAGUGUAC 50
CCGUAGCUAA ACAGCUUUGU CGACUCU 77

(2) INFORMATION FOR SEQ ID NO:27:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:27:
GGGAGAUGCC UGUCGAGCAU GCUGUUGCGG GGAGGUGUUA GGGAGUGUAC 50
CCGUAGCUAA ACAGCUUUGU CGACUCU 77

(2) INFORMATION FOR SEQ ID NO:28:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:28:
GGGAGAUGCC UGUCGAGCAU GCUGUUGCGG GGAGGUGUUA GNNAGUGUAC 50
CCGUAGCUAA ACAGCUUUGU CGACUCU 77

(2) INFORMATION FOR SEQ ID NO:29:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:29:
GGGAGAUGCC UGUCGAGCAU GCUGUUGCGG GGAGGUGUUA GGGAGUUCAC 50
CCGUAGCUAA ACAGCUUUGU CGACUCU 77

(2) INFORMATION FOR SEQ ID NO:30:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:30
GGGAGAUGCC UGUCGAGCAU GCUGCUGCGG GGAGGUGUUA GGGAGUGUAC 50
CCGUAGCUAA ACAGCUUUGU CGACUCU 77

(2) INFORMATION FOR SEQ ID NO:31
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:31:
GGGAGAUGCC UGUCGAGCAU GCUGCUGCGG GGAGGUGUUA GAGAGUGUAC 50

CCGUAGCUAA ACAGCUUUGU CGACUCU 77

(2) INFORMATION FOR SEQ ID NO:32
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:32:
GGGAGAUGCC UGUCGAGCAU GCUGCUGCGG GGAGGUGUCA GAGAGUGUAC 50
CUGUAGCUAA ACAGCUUUGU CGACUCU 77

(2) INFORMATION FOR SEQ ID NO:33:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:33:
GGGAGAUGCC UGUCGAGCAU GCUGCUACGG GGAGGUGUUA GAGAGUGUAC 50
CUGUAGCUAA ACAGCUUUGU CGACUCU 77

(2) INFORMATION FOR SEQ ID NO:34:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:34:
GGGAGAUGCC UGUCGAGCAU GCUGCUACGG GGAGGUGUCG GAGAGUGUAC 50
CUGUAGCUAA ACAGCUUUGU CGACUCU 77

(2) INFORMATION FOR SEQ ID NO:35:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:35:
GGGAGAUGCC UGUCGAGCAU GCUGCACGAG GUGUCAGAGA GUGUAGUUCA 50
GCGUAGCUAA ACAGCUUUGU CGACUCU 77

(2) INFORMATION FOR SEQ ID NO:36:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:36:
GGGAGAUGCC UGUCGAGCAU GCUGCACGAG GUGUCAGAGA GUGUAGUGCA 50
GCGUAGCUAA ACAGCUUUGU CGACUCU 77

(2) INFORMATION FOR SEQ ID NO:37:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:37:
GGGAGAUGCC UGUCGAGCAU GCUGCACGAG GUGUAGAGGG UGUAGUGCAG 50
CAGUACGUAA ACAGCUUUGU CGACUCU 77

(2) INFORMATION FOR SEQ ID NO:38:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:38:
GGGAGAUGCC UGUCGAGCAU GCUGCACGAG GCGUCAGAGA GUGUAGUGCU 50
GCGUACGUAA ACAGCUUUGU CGACUCU 77

(2) INFORMATION FOR SEQ ID NO:39:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:39:
GGGAGAUGCC UGUCGAGCAU GCUGAGGAUC GAAGUUAGUA GGCUUUGUGU 50
ACUCGUAGCU AAACAGCUUU GUCCACUCU 79

(2) INFORMATION FOR SEQ ID NO:40:
(i) SEQUENCE CHARACTERISTICS:
(A) LENGTH: 6 amino acids
(B) TYPE: amino acid
(C) STRANDEDNESS: single
(D) TOPOLOGY: linear

(xi) SEQUENCE DESCRIPTION: SEQ ID NO:40:
Gly Arg Gly Asp Thr Pro 6

(2) INFORMATION FOR SEQ ID NO:41:
(i) SEQUENCE CHARACTERISTICS:
(A) LENGTH: 4 amino acids
(B) TYPE: amino acid
(C) STRANDEDNESS: single
(D) TOPOLOGY: linear

(xi) SEQUENCE DESCRIPTION: SEQ ID NO:41:

(ix) FEATURE:
(A) NAME/KEY: Xaa
(B) LOCATION: 1
(C) OTHER INFORMATION: This symbol stands for N-methylsuccinylalanine

(ix) FEATURE:
(A) NAME/KEY: Xaa
(B) LOCATION: 4
(C) OTHER INFORMATION: Valine chloromethyl ketone
Xaa Ala Pro Xaa 4

(2) INFORMATION FOR SEQ ID NO:42:
(i) SEQUENCE CHARACTERISTICS:
(A) LENGTH: 59 nucleotides
(B) TYPE: nucleic acid
(C) STRANDEDNESS: single
(D) TOPOLOGY: linear

(xi) SEQUENCE DESCRIPTION: SEQ ID NO:42:
TAGCGATACT GCGTGGGTTG GGGCGGGTAG GGCCAGCAGT CTCGTGCGGT 50
ACTTGAGCA 59

(2) INFORMATION FOR SEQ ID NO:43:
(i) SEQUENCE CHARACTERISTICS:
(A) LENGTH: 4 amino acids

(B) TYPE: amino acid
(C) STRANDEDNESS: single
(D) TOPOLOGY: linear

(xi) SEQUENCE DESCRIPTION: SEQ ID NO:43:

(ix) FEATURE:
(A) NAME/KEY: Xaa
(B) LOCATION: 1
(C) OTHER INFORMATION: N-methyoxysuccinyl alanine

(ix) FEATURE:
(A) NAME/KEY: Xaa
(B) LOCATION: 4
(C) OTHER INFORMATION: Valine p-nitroanilide

Xaa Ala Pro Xaa 4

(2) INFORMATION FOR SEQ ID NO:44:
(i) SEQUENCE CHARACTERISTICS:
(A) LENGTH: 4 amino acids
(B) TYPE: amino acid
(C) STRANDEDNESS: single
(D) TOPOLOGY: linear

(ix) FEATURE:
(A) NAME/KEY: Xaa
(B) LOCATION: 1
(C) OTHER INFORMATION: N-methyoxysuccinyl alanine

(xi) SEQUENCE DESCRIPTION: SEQ ID NO:44:
Xaa Ala Pro Val 4

(2) INFORMATION FOR SEQ ID NO:45:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:45:
GGGAGCUCAG AAUAAACGCU CAANNNNNNN NNNNNNNNNN NNNNNNNNNN 50
NNNUUCGACA UGAGGCCCGG AUCCGGC 77

(2) INFORMATION FOR SEQ ID NO:46:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:46:
CCGAAGCTTA ATACGACTCA CTATAGGGAG CTCAGAATAA ACGCTCAA 48

(2) INFORMATION FOR SEQ ID NO:47:
(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:47:
GCCGGATCCG GGCCTCATGT CGAA 24

(2) INFORMATION FOR SEQ ID NO:48:
(i) SEQUENCE CHARACTERISTICS:

(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:48:
UCAAGAGUGA UGCUCAUCCG CACUUGGUGA CGUU 34

(2) INFORMATION FOR SEQ ID NO:49:
(i) SEQUENCE CHARACTERISTICS:
(A) LENGTH: 37 base pairs
(B) TYPE: nucleic acid
(C) STRANDEDNESS: single
(D) TOPOLOGY: linear

(xi) SEQUENCE DESCRIPTION: SEQ ID NO:49:
CAAUACCGGC AUGCAUGUCC AUCGCUAGCG GUAUUCG 37

(2) INFORMATION FOR SEQ ID NO:50:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:50:
AAUGCGUGUU GUGACGCACA UCCGCACGCG CAUU 34

(2) INFORMATION FOR SEQ ID NO:51:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:51:
UCAAGGAGUG AUGCCCUAUC CGCACCUUGG CCCA 34

(2) INFORMATION FOR SEQ ID NO:52:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:52:
UCAAGCUUGA CNGCCCAUCC GAGCUUGAUC ACGC 34

(2) INFORMATION FOR SEQ ID NO:53:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:53
AAACGCUCAA UCCUUGAUGC GGAUCCGAGG AUGGGACGUU U 41

(2) INFORMATION FOR SEQ ID NO:54:
(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:54:
ACACCGUCGA CCUAUGAUGC GCAUCCGCAC UUCGAC 36

(2) INFORMATION FOR SEQ ID NO:55:
(i) SEQUENCE CHARACTERISTICS:
(A) LENGTH: 37 base pairs
(B) TYPE: nucleic acid
(C) STRANDEDNESS: single
(D) TOPOLOGY: linear

(xi) SEQUENCE DESCRIPTION: SEQ ID NO:55:
AACCGGUAGU CGCAUGGCCC AUCGCGCCCG GUUCGAC 37

(2) INFORMATION FOR SEQ ID NO:56:
(i) SEQUENCE CHARACTERISTICS:
(A) LENGTH: 38 base pairs
(B) TYPE: nucleic acid
(C) STRANDEDNESS: single
(D) TOPOLOGY: linear

(xi) SEQUENCE DESCRIPTION: SEQ ID NO:56:
ACGCUCAAGU CAGCAUGGCC CACCGCGCUU GACGUCUG 38

(2) INFORMATION FOR SEQ ID NO:57:
(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:57:
CACGGUUCGA UCUGUGACGU UCAUCCGCAC UUCGA 35

(2) INFORMATION FOR SEQ ID NO:58:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:58:
AACGCUCAAG GAGCAGUGAC GCACAUCCAC ACUCCAGCGU U 41

(2) INFORMATION FOR SEQ ID NO:59:
(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:59:
UUCGAAUGCC GAGGCUCGUG CCUUGACGGG UUC 33

(2) INFORMATION FOR SEQ ID NO:60:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:60:
UCGCGAAUGC CGACCACUCA GGUUGAUGGG UUCG 34

(2) INFORMATION FOR SEQ ID NO:61:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:61:

UCAAUGCCGG CCUGAUCGGC UGAUGGGUUG ACCG 34

(2) INFORMATION FOR SEQ ID NO:62:
(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:62:
GAAUGCCGAG CCCUAAGAGG CUUGAUGUGG UU 32

(2) INFORMATION FOR SEQ ID NO:63:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:63:
AACCUUNAUG UGGCNCGAAC UGCGUGCCGA GGUU 34

(2) INFORMATION FOR SEQ ID NO:64:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:64:
AAGCUUGAUG GGUGACACAC GUCAUGCCGA GCUU 34

(2) INFORMATION FOR SEQ ID NO:65:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:65:
GUCGUCCUGC AUGGGCCGUA UCGGUGCGCG 30

(2) INFORMATION FOR SEQ ID NO:66:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:66:
GCAGACGAAG GGAACCUGCG UCUCGGCACC UUCG 34

(2) INFORMATION FOR SEQ ID NO:67:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:67:
AAGGAGGANC CUGCGUCUCG GCACUCCGCA 30

(2) INFORMATION FOR SEQ ID NO:68:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:68:
UCAAGGGAAC CUGCGUUUCG GCACCUUGUU CCGU 34

(2) INFORMATION FOR SEQ ID NO:69:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:69:
AAAUGUGGGU UACCUGCGUU UCGGCACCAC GUUU 34

(2) INFORMATION FOR SEQ ID NO:70:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:70:
CGACGGUAGA GUCUGUCCCG UCAUCCCCCA 30

(2) INFORMATION FOR SEQ ID NO:71:
(i) SEQUENCE CHARACTERISTICS:
(A) LENGTH: 32 base pairs
(B) TYPE: nucleic acid
(C) STRANDEDNESS: single
(D) TOPOLOGY: linear
(xi) SEQUENCE DESCRIPTION: SEQ ID NO:71:
AAAGACCCCU GGUUGAGUCU GUCCCAGCCG UU 32

(2) INFORMATION FOR SEQ ID NO:72:
(i) SEQUENCE CHARACTERISTICS:
(A) LENGTH: 42 base pairs
(B) TYPE: nucleic acid
(C) STRANDEDNESS: single
(D) TOPOLOGY: linear

(xi) SEQUENCE DESCRIPTION: SEQ ID NO:72:
GACCCAUCGU CAACGGUUGA GUCUGUCCCG UUCGACAUGA GG 42

(2) INFORMATION FOR SEQ ID NO:73:
(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:73:
GCUCAAGGUU GAGUCUGUCC CUUCGAGUAU CUGAUC 36

(2) INFORMATION FOR SEQ ID NO:74:
(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:74:
UCGGACAGUU GGUUGAGUCU GUCCCAACUU UU 32

(2) INFORMATION FOR SEQ ID NO:75:
(i) SEQUENCE CHARACTERISTICS:
(A) LENGTH: 31 base pairs
(B) TYPE: nucleic acid
(C) STRANDEDNESS: single

(D) TOPOLOGY: linear

(xi) SEQUENCE DESCRIPTION: SEQ ID NO:75:
GACCAUGUGA CUGGUUGAGC CUGUCCCAGU U 31

(2) INFORMATION FOR SEQ ID NO:76:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:76:
AACGGUUGAG UCUGUCCCGU AAGAGAGCGC 30

(2) INFORMATION FOR SEQ ID NO:77:
(i) SEQUENCE CHARACTERISTICS:
(A) LENGTH: 38 base pairs
(B) TYPE: nucleic acid
(C) STRANDEDNESS: single
(D) TOPOLOGY: linear

(xi) SEQUENCE DESCRIPTION: SEQ ID NO:77:
UCGGAAUGUA GUUGACGUAU CCUUGUCCGA UUCGACAU 38

(2) INFORMATION FOR SEQ ID NO:78:
(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:78:
AGGGUGUAGU UGGGACCUAG UCCGCCGUAC CUU 33

(2) INFORMATION FOR SEQ ID NO:79:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:79:
GGCAUAGUUG GGACCUCGUC CGCCGUGCCC 30

(2) INFORMATION FOR SEQ ID NO:80:
(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:80:
GCUCAAUAGU UGGAGGCCUG UCCUCGCCGU AGAGCG 36

(2) INFORMATION FOR SEQ ID NO:81:
(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:81:
AGGGGUUCUA GUGGAGACUC UGCCGCGGCC CUU 33

(2) INFORMATION FOR SEQ ID NO:82:
(i) SEQUENCE CHARACTERISTICS:
(A) LENGTH: 33 base pairs

(B) TYPE: nucleic acid
(C) STRANDEDNESS: single
(D) TOPOLOGY: linear

(xi) SEQUENCE DESCRIPTION: SEQ ID NO:82:
AACGGUUCUG UGUGUGGACU AGCCGCGGCC GUU 33

(2) INFORMATION FOR SEQ ID NO:83:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:83
GGGAUGUUUG GCUAUCUCGG AUAGUGCCCC 30

(2) INFORMATION FOR SEQ ID NO:84:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:84:
GCUUAAUACG ACUCACUNUA GGGAGCUCAG 30

(2) INFORMATION FOR SEQ ID NO:85:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:85:
UUGAGUGAUG UGCUUGACGU AUCGCUGCAC 30

(2) INFORMATION FOR SEQ ID NO:86:
(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: N
(B) LOCATION: 15
(C) OTHER INFORMATION: This symbol stands for the complimentary base for the N located in position 1

(xi) SEQUENCE DESCRIPTION: SEQ ID NO:86:
NUGAUGVNCA UCCGN 15

(2) INFORMATION FOR SEQ ID NO:87:
(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: S
(B) LOCATION: 11 and 12

(C) OTHER INFORMATION: This symbol stands for the complimentary base for the S located in positions 9 and 10

(xi) SEQUENCE DESCRIPTION: SEQ ID NO:87:
AAUGCCGASS SSUUGAUGGG UU 22

(2) INFORMATION FOR SEQ ID NO:88:
(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: H
(B) LOCATION: 24
(C) OTHER INFORMATION: This symbol stands for the complimentary base for the D located in position 2

(ix) FEATURE:
(A) NAME/KEY: Y
(B) LOCATION: 25
(C) OTHER INFORMATION: This symbol stands for the complimentary base for the R located in position 25

(ii) MOLECULAR TYPE: RNA

(xi) SEQUENCE DESCRIPTION: SEQ ID NO:88:
RDGGGAACCU GCGUYUCGGC ACCHY 25

(2) INFORMATION FOR SEQ ID NO:89:
(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: D
(B) LOCATION: 18 and 19
(C) OTHER INFORMATION: This symbol stands for the complimentary base for the H located in positions 1 and 2

(xi) SEQUENCE DESCRIPTION: SEQ ID NO:89:
HHGGUUGAGU CUGUCCCDD 19

(2) INFORMATION FOR SEQ ID NO:90:
(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: N
(B) LOCATION: 18-20 and 27
(C) OTHER INFORMATION: This symbol stands for the complimentary base for the N

located in positions 1 and 10-12

(xi) SEQUENCE DESCRIPTION: SEQ ID NO:90:
NRUAGUUGGN NNCUNSUNNN CGCCGUN 27

(2) INFORMATION FOR SEQ ID NO:91:
(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: M
(B) LOCATION: 20
(C) OTHER INFORMATION: This symbol stands for the complimentary base for the K located in position 14

(ix) FEATURE:
(A) NAME/KEY: S
(B) LOCATION: 31
(C) OTHER INFORMATION: This symbol stands for the complimentary base for the S located in position 2

(xi) SEQUENCE DESCRIPTION: SEQ ID NO:91:
RSGGUUUCRU GUGKRGACUM UGCCGCGGCC SU 32

(2) INFORMATION FOR SEQ ID NO:92:
(i) SEQUENCE CHARACTERISTICS:
(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO:92:
GGGAACCUGC GUYUCGGCAC C 21

(2) INFORMATION FOR SEQ ID NO:93:
(i) SEQUENCE CHARACTERISTICS:
(A) LENGTH: 15 base pairs
(B) TYPE: nucleic acid
(C) STRANDEDNESS: single
(D) TOPOLOGY: linear

(xi) SEQUENCE DESCRIPTION: SEQ ID NO:93:
GGUUGAGUCU GUCCC 15

(2) INFORMATION FOR SEQ ID NO:94:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:94:
AAGCAGACGA AGGGAACCUG CGUCUCGGCA CCUUCGACAU 40

(2) INFORMATION FOR SEQ ID NO:95:
(i) SEQUENCE CHARACTERISTICS:
(A) LENGTH: 31 base pairs
(B) TYPE: nucleic acid
(C) STRANDEDNESS: single
(D) TOPOLOGY: linear

(xi) SEQUENCE DESCRIPTION: SEQ ID NO:95:
GGCCGGUAGU CGCAUGGCCC AUCGCGCCCG G 31

(2) INFORMATION FOR SEQ ID NO:96:
(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:96:
GGAAGCUUGA UGGGUGACAC ACGUCAUGCC GAGCU 35

(2) INFORMATION FOR SEQ ID NO:97:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:97:
GGAAGGGAAC CUGCGUCUCG GCACCUUCG 29

(2) INFORMATION FOR SEQ ID NO:98:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:98:
GGUCAACGGU UGAGUCUGUC CCGUUCGAC 29

(2) INFORMATION FOR SEQ ID NO:99:
(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:99:
GGCUCAAUAG UUGGAGGCCU GUCCUCGCCG UAGAGC 36

(2) INFORMATION FOR SEQ ID NO:100:
(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:100:
GGAACGGUUC UGUGUGUGGA CUAGCCGCGG CCGUU 35

(2) INFORMATION FOR SEQ ID NO:101:
(i) SEQUENCE CHARACTERISTICS:
(A) LENGTH: 76 base pairs
(B) TYPE: nucleic acid
(C) STRANDEDNESS: single
(D) TOPOLOGY: linear

(xi) SEQUENCE DESCRIPTION: SEQ ID NO:101:
GGGAGACAAG AAUAACGCUC AANNNNNNNN NNNNNNNNNN NNNNNNNNNN
NNUUCGACAG GAGGCUCACA ACAGGC 76

(2) INFORMATION FOR SEQ ID NO:102:
(i) SEQUENCE CHARACTERISTICS:
(A) LENGTH: 39 base pairs
(B) TYPE: nucleic acid

(C) STRANDEDNESS: single
(D) TOPOLOGY: linear

(xi) SEQUENCE DESCRIPTION: SEQ ID NO:102:
TAATACGACT CACTATAGGG AGACAAGAAU AACGCUCAA 39

(2) INFORMATION FOR SEQ ID NO:103:
(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:103:
GCCTGTTGTG AGCCTCCTGT CGAA 24

(2) INFORMATION FOR SEQ ID NO:104:
(i) SEQUENCE CHARACTERISTICS:
(A) LENGTH: 81 base pairs
(B) TYPE: nucleic acid
(C) STRANDEDNESS: single
(D) TOPOLOGY: linear

(xi) SEQUENCE DESCRIPTION: SEQ ID NO:104:
GGGAGGACGA UGCGGNNNNN NNNNNNNNNN NNNNNNNNNN NNNNNNNNNN 50
NNNNNNNNNN NNNNNCAGAC GACTCGCCCG A 81

(2) INFORMATION FOR SEQ ID NO:105:
(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:105:
TAATACGACT CACTATAGGG AGGACGAUGC GG 32

(2) INFORMATION FOR SEQ ID NO:106:
(i) SEQUENCE CHARACTERISTICS:
(A) LENGTH: 16 base pairs
(B) TYPE: nucleic acid
(C) STRANDEDNESS: single
(D) TOPOLOGY: linear

(xi) SEQUENCE DESCRIPTION: SEQ ID NO:106:
TCGGGCGAGT CGTCTG 16

(2) INFORMATION FOR SEQ ID NO:107:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:107:
UGGCUGUGAU CAAUGCGGGG AGGUGAGGAA GGGCCUUACA AAUCCUUCGG 50

(2) INFORMATION FOR SEQ ID NO:108:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:108:
UGUGAUCAAU GCGGUGGCGG GGUAUGGAUG GGAGUCUGGA AUGCUGCGCU 50

(2) INFORMATION FOR SEQ ID NO:109:

(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:109:
CGCUGUGUUC AAUGCGGGGA UCGGGCCGGA GGAUGAACAA AUGGCGGGU 49

(2) INFORMATION FOR SEQ ID NO:110:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:110:
UGUUGAGCAA GCACUCAUGU GGUCAAUGUG GGAGUGGGAG CUGGUGGGGU 50

(2) INFORMATION FOR SEQ ID NO:111:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:111:
CAAGGGAGCG UUAGAGCCAU GUGGUCAAUG AGGGGUGGGA UUGGUUGGGU 50

(2) INFORMATION FOR SEQ ID NO:112:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:112:
CAUGGUUGUG AACUGUUGUG AUCAAUGCGG GGAGGGUAAU GGUGGGUGGU 50

(2) INFORMATION FOR SEQ ID NO:113:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:113:
AUGAGUGACA CAUGUGCUCA AUGCGGGGUG GGUUGGUAGG GGUAGCACGG 50

(2) INFORMATION FOR SEQ ID NO:114:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:114:
UGUGGUCAAU GUGGGGUAGG GCUGGUAGGG CAUUCCGUAC UGGUGUGGU 49

(2) INFORMATION FOR SEQ ID NO:115:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:115:
CCGAGUUGUG CUCAAUGUGG GGUCUGGGUA CGGACGGGAA CAGAUCUGG 49

(2) INFORMATION FOR SEQ ID NO:116:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:116:
GUGCUCAGCA UUGUGUGCUC AAUGCGGGGG AGUUUGGGUU GGCGACGG 48

(2) INFORMATION FOR SEQ ID NO:117:
(i) SEQUENCE CHARACTERISTICS:
(A) LENGTH: 16 base pairs
(B) TYPE: nucleic acid
(C) STRANDEDNESS: single
(D) TOPOLOGY: linear

(xi) SEQUENCE DESCRIPTION: SEQ ID NO:117:
UGUGNUCAAU GNGGGG 16

(2) INFORMATION FOR SEQ ID NO:118:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:118:
CAUAGGCUUA CAACAGAGCG GGGGUUCUGA UGGUAGACGC CGGGACGCCC 50

(2) INFORMATION FOR SEQ ID NO:119:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:119:
UAUGAUGGUA GACGCCGUAC CGCAUCAGGC CAAGUCGUCA CAGAUCGUG 49

(2) INFORMATION FOR SEQ ID NO:120:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:120:
GCAACAGAGG CUGAUGGUAG ACGCCGGCCA 30

(2) INFORMATION FOR SEQ ID NO:121:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:121:
AGAGUCGCUG AUGGUAGACG CCGGCGGAUC 30

(2) INFORMATION FOR SEQ ID NO:122:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:122:
GAGGCUGAUG GCAGACGCGG CCGAAGACA 29

(2) INFORMATION FOR SEQ ID NO:123:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:123:
CCCUGAUGGU AGACGCCGGG GUGCCGGAA 29

(2) INFORMATION FOR SEQ ID NO:124:
(i) SEQUENCE CHARACTERISTICS:
(A) LENGTH: 17 base pairs
(B) TYPE: nucleic acid
(C) STRANDEDNESS: single
(D) TOPOLOGY: linear

(xi) SEQUENCE DESCRIPTION: SEQ ID NO:124:
CUGAUGGUAG ACGCCGG 17

(2) INFORMATION FOR SEQ ID NO:125:
(i) SEQUENCE CHARACTERISTICS:
(A) LENGTH: 51 base pairs
(B) TYPE: nucleic acid
(C) STRANDEDNESS: single
(D) TOPOLOGY: linear

(xi) SEQUENCE DESCRIPTION: SEQ ID NO:125:
CAGUGCUGAA CUAAUCGAAC GGGGUCAAGG AGGGUCGAAC GAGAUCUGCC G 51

(2) INFORMATION FOR SEQ ID NO:126:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:126:
CACCUUCGUG GGGUCAAGGA GGGUCGCGAG GCCGCAGGAU CAACCGUGUG 50

(2) INFORMATION FOR SEQ ID NO:127:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:127:
GGUCAAGUUG GGUCGAGGAA GCGCUCCCGA GUAUCGUAGU GUGCGACUGC 50

(2) INFORMATION FOR SEQ ID NO:128:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:128:
GAACUUGAUC GGGGUCAAGG CGGGACGAA 29

(2) INFORMATION FOR SEQ ID NO:129:
(i) SEQUENCE CHARACTERISTICS:
(A) LENGTH: 30 base pairs
(B) TYPE: nucleic acid
(C) STRANDEDNESS: single

(D) TOPOLOGY: linear

(xi) SEQUENCE DESCRIPTION: SEQ ID NO:129:
UGGCGGGACC AAGGAGGGAC GUGUAGGAAA 30

(2) INFORMATION FOR SEQ ID NO:130:
(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:130:
AAAAUGCACA AAUCGGGGUC AAGGAGGGAC GA 32

(2) INFORMATION FOR SEQ ID NO:131:
(i) SEQUENCE CHARACTERISTICS:
(A) LENGTH: 47 base pairs
(B) TYPE: nucleic acid
(C) STRANDEDNESS: single
(D) TOPOLOGY: linear

(xi) SEQUENCE DESCRIPTION: SEQ ID NO:131:
AUGGGUUCGU GUGGUGAAUG GAGGAGGUGG GCUCGCAUGC UACUGUG 47

(2) INFORMATION FOR SEQ ID NO:132:
(i) SEQUENCE CHARACTERISTICS:
(A) LENGTH: 12 base pairs
(B) TYPE: nucleic acid
(C) STRANDEDNESS: single
(D) TOPOLOGY: linear

(xi) SEQUENCE DESCRIPTION: SEQ ID NO:132:
GGUCAAGGNG GG 12

(2) INFORMATION FOR SEQ ID NO:133:
(i) SEQUENCE CHARACTERISTICS:
(A) LENGTH: 44 base pairs
(B) TYPE: nucleic acid
(C) STRANDEDNESS: single
(D) TOPOLOGY: linear

(xi) SEQUENCE DESCRIPTION: SEQ ID NO:133:
UGCACUAAGU CCGGGUAGUG GGAGUGGUUG GGCCUGGAGU GCGC 44

(2) INFORMATION FOR SEQ ID NO:134:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:134:
AUCAAAGGGU AGAGGGUGGG CUGUGGCAAG 30

(2) INFORMATION FOR SEQ ID NO:135:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:135:
AAUCGAGGGU AGCGGGCGCG GCUUGGCCAA 30

(2) INFORMATION FOR SEQ ID NO:136:
(i) SEQUENCE CHARACTERISTICS:

(A) LENGTH: 31 base pairs
(B) TYPE: nucleic acid
(C) STRANDEDNESS: single
(D) TOPOLOGY: linear

(xi) SEQUENCE DESCRIPTION: SEQ ID NO:136:
GCCUCGGAUC GGGCAGCGGG UGGGAUGGCA A 31

(2) INFORMATION FOR SEQ ID NO:137:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:137:
AACGGAGUGG UAGGCGUUGG GUGCCAGGAA 30

(2) INFORMATION FOR SEQ ID NO:138:
(i) SEQUENCE CHARACTERISTICS:
(A) LENGTH: 11 base pairs
(B) TYPE: nucleic acid
(C) STRANDEDNESS: single
(D) TOPOLOGY: linear

(xi) SEQUENCE DESCRIPTION: SEQ ID NO:138:
GGUAGNGGGN G 11

(2) INFORMATION FOR SEQ ID NO:139:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:139:
AACCGAGUCG UGUGGGUUGG GGCUCCAGUA CAUCCCCGGU CUGGGUGU 48

(2) INFORMATION FOR SEQ ID NO:140:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:140:
UAACAUACGC AGUCGUGUGG GUAGGGGAUC ACAAACUGCG UAUCGUGU 48

(2) INFORMATION FOR SEQ ID NO:141:
(i) SEQUENCE CHARACTERISTICS:
(A) LENGTH: 19 base pairs
(B) TYPE: nucleic acid
(C) STRANDEDNESS: single
(D) TOPOLOGY: linear

(xi) SEQUENCE DESCRIPTION: SEQ ID NO:141:
AGUCGUGUGG GUGGGGUCA 19

(2) INFORMATION FOR SEQ ID NO:142:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:142:
AGUGUAGGAU AGGGGAUGGG AGGUCCGGGA 30

(2) INFORMATION FOR SEQ ID NO:143:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:143:
ACUGUGGGCU CUAGGGCAGU GGGAGUGGAG 30

(2) INFORMATION FOR SEQ ID NO:144:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:144:
AGUGGGACAG GGAUUGCGGA GGGUGGAAGG 30

(2) INFORMATION FOR SEQ ID NO:145:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:145:
GUCAGGAGGA CUGGAAGGUG GGACUGGUGA 30

(2) INFORMATION FOR SEQ ID NO:146:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:146:
GCAGGAGAGA GGGUGUUGGG UGCGGAUACA 30

(2) INFORMATION FOR SEQ ID NO:147:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:147:
AGGGUAGGAG GCUAAGCAUA GUUCAGAGGA GGUGGCGCGU GCCCCCGUG 49

(2) INFORMATION FOR SEQ ID NO:148:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:148:
CAACAUUGGC ACCAAUGCUC UGUGUUAAUG UGGGGUGGGA ACGGCGCCG 49

(2) INFORMATION FOR SEQ ID NO:149:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:149:

ACCAAUGAUU GCAAUGAGGG CAGUGGGGGG GAAUUGGGCU CGUGUGGU 48

(2) INFORMATION FOR SEQ ID NO:150:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:150:
GCAGUGGGUG AGGUCCGGGC ACGAUUGAGU UUGAACGGUU CUGGCUUGGU 50

(2) INFORMATION FOR SEQ ID NO:151:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:151:
GUGGUAGGUG UAGAGUGGAU GGCGGAGGUC CUAGUAGUUC UGUGCCUGGU 50

(2) INFORMATION FOR SEQ ID NO:152:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:152:
CGCGGGAGAG GGUAGUGGGU GUGGUGCUUG GACAAGCAGC G 41

(2) INFORMATION FOR SEQ ID NO:153:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:153:
ACCCGCAUAC GGACCGCGGA GGGGGAAAUC UAGCCUCAGG GGUGGCGGGC 50

(2) INFORMATION FOR SEQ ID NO:154:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:154:
UGAAGAAGCG GGGACUGCAC GACGGGAUGG AGGGACACGA CUGCGGGGU 49

(2) INFORMATION FOR SEQ ID NO:155:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:155:
ACACCAGGAG AGUGGGUUCG GGUGAGGACG 30

(2) INFORMATION FOR SEQ ID NO:156:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:156:
GUGGCUGAUG GCAGACGCCG GCUGCUGACG 30

(2) INFORMATION FOR SEQ ID NO:157:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:157:
UCGUGCCAGG ACAUGGUGGC UCAUGGGUAA 30

(2) INFORMATION FOR SEQ ID NO:158:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:158:
AGGUACGGGG GAGGGAAGGA UAUAACGCGA 30

(2) INFORMATION FOR SEQ ID NO:159:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:159:
UGGAAAGGUG UGGAAAGAGG CAUCGGGGGG 30

(2) INFORMATION FOR SEQ ID NO:160:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:160:
UCAAUGGGCA GGAAGAGGGA AGGGAUGUGA 30

(2) INFORMATION FOR SEQ ID NO:161:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:161:
CAUGGGUAAG GGAGUGGGAG UGGUGAAUAG 30

(2) INFORMATION FOR SEQ ID NO:162:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:162:
GGAACGAGUA GGGCAGUGGG UGGUGAUGGC 30

(2) INFORMATION FOR SEQ ID NO:163:
(i) SEQUENCE CHARACTERISTICS:
(A) LENGTH: 30 base pairs

(B) TYPE: nucleic acid
(C) STRANDEDNESS: single
(D) TOPOLOGY: linear

(xi) SEQUENCE DESCRIPTION: SEQ ID NO:163:
UAGGGCAGAG GGAGUGGUUA GGGCUGUGAU 30

(2) INFORMATION FOR SEQ ID NO:164:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:164:
GGGUAGUGGG AAGGGUAAGG GCCGAGGUGG 30

(2) INFORMATION FOR SEQ ID NO:165:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:165:
AAUACACACC GCGGGAAGGG AGGGUGGAAA 30

(2) INFORMATION FOR SEQ ID NO:166:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:166:
AGACUACAGC GCGGGUUAGG GUUGAGGGAA 30

(2) INFORMATION FOR SEQ ID NO:167:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:167:
UACGAGCAAG CGGGCGAAGG GUUGAGGGAA 30

(2) INFORMATION FOR SEQ ID NO:168:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:168:
CAAGGUGGUG GAGGAGGAUA CGAUCUGCAG 30

(2) INFORMATION FOR SEQ ID NO:169:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:169:
GGAGGGAAGG AGGGCAGGUG AUGGGUCAG 29

(2) INFORMATION FOR SEQ ID NO:170:

(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:170:
UGAUGGCGGU AGUGGAGGUA AUGAGCGUGA 30

(2) INFORMATION FOR SEQ ID NO:171:
(i) SEQUENCE CHARACTERISTICS:
(A) LENGTH: 26 base pairs
(B) TYPE: nucleic acid
(C) STRANDEDNESS: single
(D) TOPOLOGY: linear

(xi) SEQUENCE DESCRIPTION: SEQ ID NO:171:
GCAACUGGGG GCGGGUGGUG UGAGGA 26

(2) INFORMATION FOR SEQ ID NO:172:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:172:
GGAGGGGCCU AUAGGGGUGG UGGUGUACGA 30

(2) INFORMATION FOR SEQ ID NO:173:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:173:
UAUAGGGUAG UGGGUGUAGG UAGGGCGACA 30

(2) INFORMATION FOR SEQ ID NO:174:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:174:
GAGGGUUGGA GGGCAUGGGG CAGGAACCGG 30

(2) INFORMATION FOR SEQ ID NO:175:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:175:
CGUAGAACUG GCGGGCAGUG GGGGGGAUGC 30

(2) INFORMATION FOR SEQ ID NO:176:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:176:
UGAGGGGACG AGGGAUGUGG GGAGCGGGAC 30

(2) INFORMATION FOR SEQ ID NO:177:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:177:
CGAGGGAUGG GAGGCGUGUG GAAGAUGCAA 30

(2) INFORMATION FOR SEQ ID NO:178:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:178:
GCAUCCGGGG ACAAGAUGGG UCGGUAAGGU 30

(2) INFORMATION FOR SEQ ID NO:179:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:179:
GUGUGCGGGG UCAAGACGGG UGGCGUGCG 29

(2) INFORMATION FOR SEQ ID NO:180:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:180:
UCAAACCAUG GGGCGGGUGG UACGAGGAAC 30

(2) INFORMATION FOR SEQ ID NO:181:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:181:
CGAGUCCGAG GGAUGGGUGG UGUGCGGCAA 30

(2) INFORMATION FOR SEQ ID NO:182:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:182:
CAGUGUCGGA GAGGAGGAUG GAGGUAUGAA 30

(2) INFORMATION FOR SEQ ID NO:183:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:183:
CACCACUACG CGGGAAGGGU AGGGUGGAUU ACAAGGUGUG ACCGCUCCGU 50

(2) INFORMATION FOR SEQ ID NO:184:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:184:
UACGGUUAAC GGGGGUGGUG UGGGAGGACA CAAAGCGCGU ACCUGCCCC 49

(2) INFORMATION FOR SEQ ID NO:185:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:185:
AGGUCCUCGA GGGUCUGGGU GUGGGAGUGG GCAUGGACCA AUACCGCGUG 50

(2) INFORMATION FOR SEQ ID NO:186:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:186:
AAACCCAUCC UGCGCGGGAU GGGAGGGUGG AAACACUAGA GCUUCGCCUG 50

(2) INFORMATION FOR SEQ ID NO:187:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:187:
AACUGGUGGU CACGCGUUGA GGUGGUGGAG GUGGAUUCAA CGGUCGAGGG 50

(2) INFORMATION FOR SEQ ID NO:188:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:188:
CAUGAAAGUA GGGUUAUGAA GGCGGUAGAU GGAGGAGGUU GGGUUGCCGC 50

(2) INFORMATION FOR SEQ ID NO:189:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:189:
GUCUAUUGGG UAGGUGUUUG CAAGAAUUCC GCACGAUAGG UAAAACAGUG 50

(2) INFORMATION FOR SEQ ID NO:190:
(i) SEQUENCE CHARACTERISTICS:
(A) LENGTH: 49 base pairs
(B) TYPE: nucleic acid

(C) STRANDEDNESS: single
(D) TOPOLOGY: linear

(xi) SEQUENCE DESCRIPTION: SEQ ID NO:190:
UGUAGGGGAA GUACGAGAGU GGGAGCGGCC GUAUAGGUGG GAGUGUGCU 49

(2) INFORMATION FOR SEQ ID NO:191:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:191:
GAGUAGGCUU GACGCCUGGG GGGGUAUGGC UUCGACUGCG 40

(2) INFORMATION FOR SEQ ID NO:192:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:192:
GAGUAGGCUU GACGCCUGGG GGGGUAUGGC UUCAACUGCG 40

(2) INFORMATION FOR SEQ ID NO:193:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:193:
GAGUAGGCUU GACGCUGGGG GGGUAUGGCU UCGACUUGCG 40

(2) INFORMATION FOR SEQ ID NO:194:
(i) SEQUENCE CHARACTERISTICS:
(A) LENGTH: 42 base pairs
(B) TYPE: nucleic acid
(C) STRANDEDNESS: single
(D) TOPOLOGY: linear

(xi) SEQUENCE DESCRIPTION: SEQ ID NO:194:
AGAGGUAGGC UUGACGCCUG GGGGGGUAUG GCUCCGACUG CG 42

(2) INFORMATION FOR SEQ ID NO:195:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:195:
GGUAGACAUA CGCCUUGGGG GGGUGUCAGG GUAUAUGGUA 40

(2) INFORMATION FOR SEQ ID NO:196:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:196:
GGCAGACAUA CGCCUUGGGG GGGUGUCAGG GUAUAUGGUA 40

(2) INFORMATION FOR SEQ ID NO:197:
(i) SEQUENCE CHARACTERISTICS:

(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:197:
GGUAGACAUA CGCCUUGGGG GGGUGCCAGG GUAUAUGGUA 40

(2) INFORMATION FOR SEQ ID NO:198:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:198:
GGUAGACAUA CGCCUUGGGG GGGUAUCAGG GUAUAUGGUA 40

(2) INFORMATION FOR SEQ ID NO:199:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:199:
GGCAGACAUA CGCCUUGGGG GGGUGUCAGG GUAUAUGGCA 40

(2) INFORMATION FOR SEQ ID NO:200:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:200:
GGCAGACAUA CGCCUUGGGG GGGUUUCAGG GUAUAUGGCA 40

(2) INFORMATION FOR SEQ ID NO:201:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:201:
AGAAUGAAUG UGAUGAACAG GUGUCGGGGU GGAUGGGUGG 40

(2) INFORMATION FOR SEQ ID NO:202:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:202:
GGAAUGAAUG UGAUGAACAG GUGUCGGGGU GGAUGGGUGG 40

(2) INFORMATION FOR SEQ ID NO:203:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:203:
GAAUGAAUGU GAUGAACAGG UGUCGGGGUG GAUGGGUGG 39

(2) INFORMATION FOR SEQ ID NO:204:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:204:
CGCCUUAGUC GCCAUAAUGC UGUCGGGGUG GAUAGGGUGG 40

(2) INFORMATION FOR SEQ ID NO:205:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:205:
UGCCCGGCAG UACUGCACGG CCUCGGGGGG GACCAGGGAG 40

(2) INFORMATION FOR SEQ ID NO:206:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:206:
AUCGGAUAGC GCCGCGAUGG CGUCUGGGGG GGACCAGGGA 40

(2) INFORMATION FOR SEQ ID NO:207:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:207:
GUACUACUGC AAGCCCGUGU GGCGCGGUCA GUGGGUGGC 39

(2) INFORMATION FOR SEQ ID NO:208:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:208:
GUACUACUGC AAGCCCGUGU GGCGCGGUCA GUGGGUGGCC 40

(2) INFORMATION FOR SEQ ID NO:209:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:209:
CGGAUCGGGC GGUCGUCUAG CGGGAUGGCG UGCGGGUGGA 40

(2) INFORMATION FOR SEQ ID NO:210:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:210:

UGACCGGGUC GACAUGCCUG AGGUGUGGCC AGUGGGUGG 39

(2) INFORMATION FOR SEQ ID NO:211:
(i) SEQUENCE CHARACTERISTICS:
(A) LENGTH: 37 base pairs
(B) TYPE: nucleic acid
(C) STRANDEDNESS: single
(D) TOPOLOGY: linear

(xi) SEQUENCE DESCRIPTION: SEQ ID NO:211:
GGCCCUCUGA ACCGUCUGGG CGUGGUUAGU GGGUGGC 37

(2) INFORMATION FOR SEQ ID NO:212:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:212:
UGACCGGGCC GACAUGCUUG AGGUGUGGCC CAGUGGGUGG 40

(2) INFORMATION FOR SEQ ID NO:213:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:213:
GACGCGUGCU GGCCUCGACC GUGUGGGUGC GGAUGGGUGG 40

(2) INFORMATION FOR SEQ ID NO:214:
(i) SEQUENCE CHARACTERISTICS:
(A) LENGTH: 44 base pairs
(B) TYPE: nucleic acid
(C) STRANDEDNESS: single
(D) TOPOLOGY: linear

(xi) SEQUENCE DESCRIPTION: SEQ ID NO:214:
UGAGGUUGAG UAGUCGUGGU AAUGGCCACG GCUGGGGGGG UCGG 44

(2) INFORMATION FOR SEQ ID NO:215:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:215:
UGGAUAGCGC UGCGUGACCG CGCUGGGGGG GUUGGCAGGG 40

(2) INFORMATION FOR SEQ ID NO:216:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:216:
ACCTTTTCCT CTCAGTCTTC TTATCTCGCC TATTATTATT 40

(2) INFORMATION FOR SEQ ID NO:217:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:217:
GCGTGGGTTG GGGCCGGGAG GGCCAGCAGT CTCGTGCGTC 40

(2) INFORMATION FOR SEQ ID NO:218:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:218:
CATTCATCTT CTCATTCTCG CCTAACTGTA CACATCTTT 39

(2) INFORMATION FOR SEQ ID NO:219:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:219:
GCGTGGGTTG GGGCCGGGAG GGCCAGCAGT CTCGTGCGT 39

(2) INFORMATION FOR SEQ ID NO:220:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:220:
GCGTGGGTTG GGGCCGGGAG GGCCAACAGT CTCGTGCGTC 40

(2) INFORMATION FOR SEQ ID NO:221:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:221:
CTACCCTTTC TTGACCACCG CCTCGTTTCA TCCACCTTAC 40

(2) INFORMATION FOR SEQ ID NO:222:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:222:
TTCTTTCTAT ACCCATATTA CCCTTCTTCA CACTCGTATC 40

(2) INFORMATION FOR SEQ ID NO:223:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:223:
CTTTATCCTT TCTCTTTCCT TGCACTCTAA CATCCTACTC 40

(2) INFORMATION FOR SEQ ID NO:224:
(i) SEQUENCE CHARACTERISTICS:
(A) LENGTH: 40 base pairs
(B) TYPE: nucleic acid

(C) STRANDEDNESS: single
(D) TOPOLOGY: linear

(xi) SEQUENCE DESCRIPTION: SEQ ID NO:224:
GCGTGGGTTG GGGCCGGTAG GGCCAGCAGT CTCGTTGCGT 40

(2) INFORMATION FOR SEQ ID NO:225:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:225:
CCTTCTTGTT ATATTGGTCG TTTTCTTCTT TTACTTTCTT 40

(2) INFORMATION FOR SEQ ID NO:226:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:226:
TCTTCATCAT TTCACTTCAT TCTGTCGGGC TATCTTCGGT 40

(2) INFORMATION FOR SEQ ID NO:227:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:227:
TTCCACGTCT CCTCAGCCCG GGAGGCCACC TTTTTATCTG 40

(2) INFORMATION FOR SEQ ID NO:228:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:228:
GAAGGCTTAA CCTAATTTTC CACCTTTCAT CCACTTTTCC 40

(2) INFORMATION FOR SEQ ID NO:229:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:229:
TCACCTCCCA TTTATATTTT CCCTTAATTT CTTCTTCTTA 40

(2) INFORMATION FOR SEQ ID NO:230:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:230:
CTTACTATGC ATCTTACTTA TTATTTTTTT TTACTTTCTA 40

(2) INFORMATION FOR SEQ ID NO:231:
(i) SEQUENCE CHARACTERISTICS:

(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:231:
TACTTCTTTT ACATCATTCC TCGATTTATT CATTCTCCAC 40

(2) INFORMATION FOR SEQ ID NO:232:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:232:
TTCACCCGTG TCATATCATA TTTCCCGGTC CTTCCTTTCC C 41

(2) INFORMATION FOR SEQ ID NO:233:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:233:
CAATTCAAAC CTTTTCTACA ATTTTCATCT TACATTCTTC 40

(2) INFORMATION FOR SEQ ID NO:234:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:234:
TCACTTGATC CTTCTTTACT TTTTTTCTCG TCTAATTATA 40

(2) INFORMATION FOR SEQ ID NO:235:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:235:
GCGTGGGTTG GGGCGGGATG GGCCAGCAGT CTCGTGCGGT 40

(2) INFORMATION FOR SEQ ID NO:236:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:236:
CTTTTTATTC CAACCCCCAT TCTTACTTAC AATATCTTGA 40

(2) INFORMATION FOR SEQ ID NO:237:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:237:
TATCCTTCTC CTTAACTCCT ACTTCTATCT ATAAAATTAT 40

(2) INFORMATION FOR SEQ ID NO:238:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:238:
GCGTGGGTAG GGGCCGGGAG GGCCAGCAGT CACGTGCGTA 40

(2) INFORMATION FOR SEQ ID NO:239:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:239:
GGGTGGGTTG GGGCCGGGAG GGCTAGCAGT CTCGTGCGTT 40

(2) INFORMATION FOR SEQ ID NO:240:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:240:
GCGTGGGATG GGGCCGGGAG GGCCAGCAGT CTCGTGCGTT 40

(2) INFORMATION FOR SEQ ID NO:241:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:241:
GCGTGGGTTG GGGCCGGGAG GGCCAGCAGT CTCGTGCGT 39

(2) INFORMATION FOR SEQ ID NO:242:
(i) SEQUENCE CHARACTERISTICS:
(A) LENGTH: 59 base pairs
(B) TYPE: nucleic acid
(C) STRANDEDNESS: single
(D) TOPOLOGY: linear

(xi) SEQUENCE DESCRIPTION: SEQ ID NO:242:
TAGCGATACT GCGTGGGTTG GGGCGGGTAG GGCCAGCAGT CTCGTGCGGT 50
ACTTGAGCA 59

(2) INFORMATION FOR SEQ ID NO:243:
(i) SEQUENCE CHARACTERISTICS:
(A) LENGTH: 56 base pairs
(B) TYPE: nucleic acid
(C) STRANDEDNESS: single
(D) TOPOLOGY: linear

(xi) SEQUENCE DESCRIPTION: SEQ ID NO:243:
TAGCGATACT GCGTGGGNGG GNGGGNGGGC CAGCAGTCTC GTGCGGTACT 50
TGAGCA 56

(2) INFORMATION FOR SEQ ID NO:244:
(i) SEQUENCE CHARACTERISTICS:
(A) LENGTH: 6 base pairs
(B) TYPE: nucleic acid
(C) STRANDEDNESS: single
(D) TOPOLOGY: linear

(xi) SEQUENCE DESCRIPTION: SEQ ID NO:244:
AUACCA 6

(2) INFORMATION FOR SEQ ID NO:245:
(i) SEQUENCE CHARACTERISTICS:
(A) LENGTH: 9 base pairs
(B) TYPE: nucleic acid
(C) STRANDEDNESS: single
(D) TOPOLOGY: linear

(xi) SEQUENCE DESCRIPTION: SEQ ID NO:245:
CCUUGGMAG 9

(2) INFORMATION FOR SEQ ID NO:246:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:246:
GAGAAAUACC AGUGACAACU CUCGAGAUCA CCCUUGGAAG 40

(2) INFORMATION FOR SEQ ID NO:247:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:247:
AUACCAUCGU GUAAGCAAGA GCACGACCUU GGCAGUGUGU G 41

(2) INFORMATION FOR SEQ ID NO:248:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:248:
GAUACCAGCA GCAUAUUUGC UGUCCUUGGA AGCAACGAGA 40

(2) INFORMATION FOR SEQ ID NO:249:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:249:
GUGAUACCAG CAUCGUCUUG AUGCCCUUGG CAGCACUUCA 40

(2) INFORMATION FOR SEQ ID NO:250:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:250:
UUGUCGAAUC GGAUACCAGC AAUGCAGCCC UUGGAAGCAG 40

(2) INFORMATION FOR SEQ ID NO:251:
(i) SEQUENCE CHARACTERISTICS:
(A) LENGTH: 40 base pairs
(B) TYPE: nucleic acid

(C) STRANDEDNESS: single
(D) TOPOLOGY: linear

(xi) SEQUENCE DESCRIPTION: SEQ ID NO:251:
GAUACCAACG GCAUAUUUGC UGUCCUUGGA AGCAACUAUA 40

(2) INFORMATION FOR SEQ ID NO:252:
(i) SEQUENCE CHARACTERISTICS:
(A) LENGTH: 40 base pairs
(B) TYPE: nucleic acid
(C) STRANDEDNESS: single
(D) TOPOLOGY: linear
(xi) SEQUENCE DESCRIPTION: SEQ ID NO:252:
CUCUCGAAAU ACCAACUACU CUCACAAUAG UCCUUGGAAG 40

(2) INFORMATION FOR SEQ ID NO:253:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:253:
UUCAUGUCGC UUGAUACCAU CAACAAUGAC CUUGGAAGCA 40

(2) INFORMATION FOR SEQ ID NO:254:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:254:
UGACUCGAAC CCUUGGAAGA CCUGAGUACA GGUAUACCAG 40

(2) INFORMATION FOR SEQ ID NO:255:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:255:
UCCUUGGAAG CCGUACGGAU ACCAAUUGAG UGGCCAUAUG 40

(2) INFORMATION FOR SEQ ID NO:256:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:256:
UAUCGAGUGG CCUUGGCAGA CCAGGCCCGG UAUACCACCA 40

(2) INFORMATION FOR SEQ ID NO:257:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:257:
CGAGAUUCAA CCUUGGAAGU CAAUCGUGAA UACCAUUGUU 40

(2) INFORMATION FOR SEQ ID NO:258:
(i) SEQUENCE CHARACTERISTICS:
(A) LENGTH: 40 base pairs

(B) TYPE: nucleic acid
(C) STRANDEDNESS: single
(D) TOPOLOGY: linear

(xi) SEQUENCE DESCRIPTION: SEQ ID NO:258:
UCAGAACCUU GGAAGCACUG AAUAAGAUCA GUUGAUACCA 40

(2) INFORMATION FOR SEQ ID NO:259:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:259:
GGACGAGACT CATCGTAACC TAGATGGTTG CCAGCATTTA 40

(2) INFORMATION FOR SEQ ID NO:260:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:260:
GGATGCTTAC AGCATAATCG GAATTGATTG CCAGCGGAAA 40

(2) INFORMATION FOR SEQ ID NO:261:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:261:
GGGGCAATAG AAGCCAACGC ACAGTCGTTG CCAGTGTTCG 40

(2) INFORMATION FOR SEQ ID NO:262:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:262:
GGGATGGATC TTCGGATACG TCAACCAAAG GTTGCCAGCG 40

(2) INFORMATION FOR SEQ ID NO:263:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:263:
GCGCATCGTA AAAAGGACAA ACGTCGTCGT GACCCCGATA 40

(2) INFORMATION FOR SEQ ID NO:264:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:264:
GTGCATCGTA AAAAGGACAA ACGTCGTCGT GACCCCGATA 40

(2) INFORMATION FOR SEQ ID NO:265:

(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:265:
AGACGGTGAA ACTGAAATCT AATCCGTCTG AACCCTGGAT 40

(2) INFORMATION FOR SEQ ID NO:266:
(i) SEQUENCE CHARACTERISTICS:
(A) LENGTH: 40 base pairs
(B) TYPE: nucleic acid
(C) STRANDEDNESS: single
(D) TOPOLOGY: linear
(xi) SEQUENCE DESCRIPTION: SEQ ID NO:266:
AGACGGTGAA GCTGAAATCT AATCCGTCTG AACCCTGGAC 40

(2) INFORMATION FOR SEQ ID NO:267:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:267:
GGGAGGTCAA GGACCTCACA CTTTTGTGTG CCAGCGCTAT 40

(2) INFORMATION FOR SEQ ID NO:268:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:268:
GAAAGCGTTG TGGCGTGCCA TCGCCCGCAG GCGAATAACA 40

(2) INFORMATION FOR SEQ ID NO:269:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:269:
ACGATGGGTT GTTATAGTGG AAACGGTAAG TTCGAGTCTG 40

(2) INFORMATION FOR SEQ ID NO:270:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:270:
ACGGTGATCC TCTAATCCGT CGACAGAATC GATGTCAATC 40

(2) INFORMATION FOR SEQ ID NO:271:
(i) SEQUENCE CHARACTERISTICS:
(A) LENGTH: 87 base pairs
(B) TYPE: nucleic acid
(C) STRANDEDNESS: single
(D) TOPOLOGY: linear

(xi) SEQUENCE DESCRIPTION: SEQ ID NO:271:
GCCGGATCCG GGCCTCATGT CGAANNNNNN NNNNNNNNNN NNNNNNNNNN 50
NNNNNNNNNN NNNNTTGAGC GTTTATTCTG AGCTCCC 87

(2) INFORMATION FOR SEQ ID NO:272:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:272:
CCGAAGCTTA ATACGACTCA CTATAGGGAG CTCAGAATAA ACGCTCAA 48

(2) INFORMATION FOR SEQ ID NO:273:
(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:273:
GCCGGATCCG GGCCTCATGT CGAA 24

(2) INFORMATION FOR SEQ ID NO:274:
(i) SEQUENCE CHARACTERISTICS:
(A) LENGTH: 87 base pairs
(B) TYPE: nucleic acid
(C) STRANDEDNESS: single
(D) TOPOLOGY: linear

(xi) SEQUENCE DESCRIPTION: SEQ ID NO:274:
CCCCTGCAGG TGATTTTGCT CAAGTNNNNN NNNNNNNNNN NNNNNNNNNN 50
NNNNNNNNNN NNNNNAGTAT CGCTAATCAG GCGGATC 87

(2) INFORMATION FOR SEQ ID NO:275:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:275:
CCGAAGCTTA ATACGACTCA CTATAGGGAT CCGCCTGATT AGCGATACT 49

(2) INFORMATION FOR SEQ ID NO:276:
(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:276:
CCCCTGCAGG TGATTTTGCT CAAGT 25

(2) INFORMATION FOR SEQ ID NO:277:
(i) SEQUENCE CHARACTERISTICS:
(A) LENGTH: 51 base pairs
(B) TYPE: nucleic acid
(C) STRANDEDNESS: single
(D) TOPOLOGY: linear

(xi) SEQUENCE DESCRIPTION: SEQ ID NO:277:
AACGCUCAAU ACCAUCGUGU AAGAAAGAGC ACGACCUUGG CAGUGUGUGU 50
U 51

(2) INFORMATION FOR SEQ ID NO:278:
(i) SEQUENCE CHARACTERISTICS:
(A) LENGTH: 42 base pairs
(B) TYPE: nucleic acid

(C) STRANDEDNESS: single
(D) TOPOLOGY: linear

(xi) SEQUENCE DESCRIPTION: SEQ ID NO:278:
AAGUGAUACC AGCAUCGUCU UGAUGCCCUU GGCAGCACUU CA 42

(2) INFORMATION FOR SEQ ID NO:279:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:279:
GAUACCAACA GCAUAUUUGC UGUCCUUGGA AGCAACGAGA 40

(2) INFORMATION FOR SEQ ID NO:280:
(i) SEQUENCE CHARACTERISTICS:
(A) LENGTH: 42 base pairs
(B) TYPE: nucleic acid
(C) STRANDEDNESS: single
(D) TOPOLOGY: linear

(xi) SEQUENCE DESCRIPTION: SEQ ID NO:280:
GAGAAAUACC AGUGACAACU CUCGAGAUCA CCCUUGGAAG UU 42

(2) INFORMATION FOR SEQ ID NO:281:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:281:
UAUCGAGUGG CCUUGGCAGA CCAGGCCGGG UAUACCACCA 40

(2) INFORMATION FOR SEQ ID NO:282:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:282:
CGAGAUUCAA CCUUGGAAGU CAAUCGUGAA UACCAUUGUU 40

(2) INFORMATION FOR SEQ ID NO:283:
(i) SEQUENCE CHARACTERISTICS:
(A) LENGTH: 45 base pairs
(B) TYPE: nucleic acid
(C) STRANDEDNESS: single
(D) TOPOLOGY: linear

(xi) SEQUENCE DESCRIPTION: SEQ ID NO:283:
UGACUCGAAC CCUUGGAAGA CCUGAGUACA GGUAUACCAG UUCGA 45

(2) INFORMATION FOR SEQ ID NO:284:
(i) SEQUENCE CHARACTERISTICS:
(A) LENGTH: 47 base pairs
(B) TYPE: nucleic acid
(C) STRANDEDNESS: single
(D) TOPOLOGY: linear

(xi) SEQUENCE DESCRIPTION: SEQ ID NO:284:
CGCUCAAUCC UUGGAAGCCG UACGGAUACC AAUUGAGUGG CCAUAUG 47

(2) INFORMATION FOR SEQ ID NO:285:
(i) SEQUENCE CHARACTERISTICS:

(A) LENGTH: 28 base pairs
(B) TYPE: nucleic acid
(C) STRANDEDNESS: single
(D) TOPOLOGY: linear

(xi) SEQUENCE DESCRIPTION: SEQ ID NO:285:
NNAUACCANN NNNNNNNCCU UGGMAGNN 28

(2) INFORMATION FOR SEQ ID NO:286:
(i) SEQUENCE CHARACTERISTICS:
(A) LENGTH: 38 base pairs
(B) TYPE: nucleic acid
(C) STRANDEDNESS: single
(D) TOPOLOGY: linear

(xi) SEQUENCE DESCRIPTION: SEQ ID NO:286:
GGUGAUACCA GCAUCGUCUU GAUGCCCUUG GCAGCACC 38

SEQUENCE LISTING

(1) GENERAL INFORMATION:
(i) APPLICANT: PIEKEN, WOLFGANG
TASSET, DIANE
JANJIC, NEBOJSA
GOLD, LARRY
KIRSCHENHEUTER, GARY P.
POLISKY, GARY
JENISON, ROBERT D.
JAYASENA, SUMEDHA
BIESECKER, GREG
SMITH, DREW

(ii) TITLE OF INVENTION: NUCLEIC ACID LIGANDS AND IMPROVED METHODS FOR PRODUCING THE SAME.

(iii) NUMBER OF SEQUENCES: 286

(iv) CORRESPONDENCE ADDRESS:
(A) ADDRESSEE: Swanson & Bratschun, L.L.C.
(B) STREET: 8400 E. Prentice Place, Suite 200
(C) CITY: Englewood
(D) STATE: Colorado
(E) COUNTRY: USA
(F) ZIP: 80111

(v) COMPUTER READABLE FORM:
(A) MEDIUM TYPE: Diskette, 3.5 inch, 360 Kb storage
(B) COMPUTER: IBM compatible
(C) OPERATING SYSTEM: MS-DOS
(D) SOFTWARE: WordPerfect 5.1

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: 08/117,991
(B) FILING DATE: 8-SEPTEMBER-1993

(vii) PRIOR APPLICATION DATA:
(A) APPLICATION NUMBER: 08/134,029
(B) FILING DATE: 7-OCTOBER-1993

(vii) PRIOR APPLICATION DATA:
(A) APPLICATION NUMBER: 08/199,507
(B) FILING DATE: 22-FEB-1994

(vii) PRIOR APPLICATION DATA:
(A) APPLICATION NUMBER: 08/233,012
(B) FILING DATE: 25-APRIL-1994

(vii) PRIOR APPLICATION DATA:
(A) APPLICATION NUMBER: 08/234,997
(B) FILING DATE: 28-APRIL-1994

(viii) ATTORNEY/AGENT INFORMATION:

SEQUENCE LISTING

(1) GENERAL INFORMATION:

(i) APPLICANT: PIEKEN, WOLFGANG
TASSET, DIANE
JANJIC, NEBOJSA
GOLD, LARRY
KIRSCHENHEUTER, GARY P.
POLISKY, GARY
JENISON, ROBERT D.
JAYASENA, SUMEDHA
BIESECKER, GREG
SMITH, DREW

(ii) TITLE OF INVENTION: NUCLEIC ACID LIGANDS AND IMPROVED METHODS FOR PRODUCING THE SAME

(iii) NUMBER OF SEQUENCES: 286

(iv) CORRESPONDENCE ADDRESS:
(A) ADDRESSEE: Swanson & Bratschun, L.L.C.
(B) STREET: 8400 E. Prentice Place, Suite 200
(C) CITY: Englewood
(D) STATE: Colorado
(E) COUNTRY: USA
(F) ZIP: 80111

(v) COMPUTER READABLE FORM:
(A) MEDIUM TYPE: Diskette, 3.5 inch, 360 Kb storage
(B) COMPUTER: IBM compatible
(C) OPERATING SYSTEM: MS-DOS
(D) SOFTWARE: WordPerfect 5.1

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: 08/117,991
(B) FILING DATE: 8-SEPTEMBER-1993

(vii) PRIOR APPLICATION DATA:
(A) APPLICATION NUMBER: 08/134,029
(B) FILING DATE: 7-OCTOBER-1993

(vii) PRIOR APPLICATION DATA:
(A) APPLICATION NUMBER: 08/199,507
(B) FILING DATE: 22-FEB-1994

(vii) PRIOR APPLICATION DATA:
(A) APPLICATION NUMBER: 08/233,012
(B) FILING DATE: 25-APRIL-1994

(vii) PRIOR APPLICATION DATA:
(A) APPLICATION NUMBER: 08/234,997
(B) FILING DATE: 28-APRIL-1994

(viii) ATTORNEY/AGENT INFORMATION:
(A) NAME: Barry J. Swanson
(B) REGISTRATION NUMBER: 33,215
(C) REFERENCE/DOCKET NUMBER: NEX09/PCT

(ix) TELECOMMUNICATION INFORMATION:
(A) TELEPHONE: (303) 793-3333
(B) TELEFAX: (303) 793-3433

(2) INFORMATION FOR SEQ ID NO:1:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:1:
GGGAGAUGCC UGUCGAGCAU GCUGAGGAUC GAAGUUAGUA GGCUUUGUGU 50
GCUCGUAGCU AAACAGCUUU GUCGACUCU 79

(2) INFORMATION FOR SEQ ID NO:2:
(i) SEQUENCE CHARACTERISTICS:
(A) LENGTH: 76 base pairs
(B) TYPE: nucleic acid
(C) STRANDEDNESS: single
(D) TOPOLOGY: linear

(xi) SEQUENCE DESCRIPTION: SEQ ID NO:2:
GGGAGAUGCC UGUCGAGCAU GCUGUACUGG AUCGAAGGUA GUAGGCAGUC 50
ACGUAGCUAA ACAGCUUUGU GACUCU 76

(2) INFORMATION FOR SEQ ID NO:3:
(i) SEQUENCE CHARACTERISTICS:
(A) LENGTH: 76 base pairs
(B) TYPE: nucleic acid
(C) STRANDEDNESS: single
(D) TOPOLOGY: linear
(xi) SEQUENCE DESCRIPTION: SEQ ID NO:3:

GGGAGAUGCC UGUCGAGCAU GCUGAUAUCA CGGAUCGAAG GAAGUAGGCG 50
UGGUAGCUAA ACAGCUUUGU GACUCU 76

(2) INFORMATION FOR SEQ ID NO:4:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:4:
GGGAGAUGCC UGUCGAGCAU GCUGCCUUUC CCGGGUUCGA AGUCAGUAGG 50
CCGGGUAGCU AAACAGCUUU GUCGACUCU 79

(2) INFORMATION FOR SEQ ID NO:5:
(i) SEQUENCE CHARACTERISTICS:
(A) LENGTH: 78 base pairs
(B) TYPE: nucleic acid
(C) STRANDEDNESS: single
(D) TOPOLOGY: linear

(xi) SEQUENCE DESCRIPTION: SEQ ID NO:5:
GGGAGAUGCC UGUCGAGCAU GCUGCACCCG GAUCGAAGUU AGUAGGCGUG 50
AGUGUAGCUA AACAGCUUUG UCGACUCU 78

(2) INFORMATION FOR SEQ ID NO:6:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:6:
GGGAGAUGCC UGUCGAGCAU GCUGUGUACG GAUCGAAGGU AGUAGGCAGG 50
UUACGUAGCU AAACAGCUUU GUCGACUCU 79

(2) INFORMATION FOR SEQ ID NO:7:
(i) SEQUENCE CHARACTERISTICS:

(A) LENGTH: 78 base pairs
(B) TYPE: nucleic acid
(C) STRANDEDNESS: single
(D) TOPOLOGY: linear

(xi) SEQUENCE DESCRIPTION: SEQ ID NO:7:
GGGAGAUGCC UGUCGAGCAU GCUGCAUCCG GAUCGAAGUU AGUAGGCGGA 50
GUGGUAGCUA AACAGCUUUG CGACUCU 78

(2) INFORMATION FOR SEQ ID NO:8:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:8:
GGGAGAUGCC UGUCGAGCAU GCUGAUUGUU GCGGAUCGAA GUGGAGUAGG 50
CGCAGUAGCU AAACAGCUUU GUCGACUCU 79

(2) INFORMATION FOR SEQ ID NO:9:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:9:
GGGAGAUGCC UGUCGAGCAU GCUGUGUACU GGAUCGAAGG UAGUAGCGAG 50
UCACGUAGCU AAACAGCUUU GUCGACUCU 79

(2) INFORMATION FOR SEQ ID NO:10:
(i) SEQUENCE CHARACTERISTICS:
(A) LENGTH: 72 base pairs
(B) TYPE: nucleic acid
(C) STRANDEDNESS: single
(D) TOPOLOGY: linear

(xi) SEQUENCE DESCRIPTION: SEQ ID NO:10:
GGGAGAUGCC UGUCGAGCAU GCUGAUCGAA GUUAGUAGGA GCGUGUGGUA 50
GCUAAACAGC UUUGUCGACU CU 72

(2) INFORMATION FOR SEQ ID NO:11:
(i) SEQUENCE CHARACTERISTICS:
(A) LENGTH: 84 base pairs
(B) TYPE: nucleic acid
(C) STRANDEDNESS: single
(D) TOPOLOGY: linear

(xi) SEQUENCE DESCRIPTION: SEQ ID NO:11:
GGGAGAUGCC UGUCGAGCAU GCUGACGCUA GAGUCGGAUC GAAAGGUAAG 50
UAGGCGACUG UAGCUAAACA GCUUUGUCGA CUCU 84

(2) INFORMATION FOR SEQ ID NO:12:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:12:
GGGAGAUGCC UGUCGAGCAU GCUGGGGUCG GAUCGAAAGG UAAGUAGGCG 50
ACUGUAGCUA AACAGCUCUU GUCGACUCU 79

(2) INFORMATION FOR SEQ ID NO:13:
(i) SEQUENCE CHARACTERISTICS:
(A) LENGTH: 77 base pairs
(B) TYPE: nucleic acid

(C) STRANDEDNESS: single
(D) TOPOLOGY: linear

(xi) SEQUENCE DESCRIPTION: SEQ ID NO:13:
GGGAGAUGCC UGUCGAGCAU GCUGAUAUCA CGGAUCGAAA GAGAGUAGGC 50
GUGUAGCUAA ACAGCUUUGU CGACUCU 77

(2) INFORMATION FOR SEQ ID NO:14:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:14:
GGGAGAUGCC UGUCGAGCAU GCUGUGUACU GGAUCGAAGG UAGUAGGCAG 50
GCACGUAGCU AAACAGCUUU GUCGACUCU 79

(2) INFORMATION FOR SEQ ID NO:15:

(i) SEQUENCE CHARACTERISTICS:
(A) LENGTH: 78 base pairs
(B) TYPE: nucleic acid
(C) STRANDEDNESS: single
(D) TOPOLOGY: linear

(xi) SEQUENCE DESCRIPTION: SEQ ID NO:15:
GGGAGAUGCC UGUCGAGCAU GCUGAUAUCA CGGAUCGAAG GAAAGUAGGC 50
GUGGUAGCUA AACAGCUUUG UCGACUCU 78

(2) INFORMATION FOR SEQ ID NO:16:
(i) SEQUENCE CHARACTERISTICS:
(A) LENGTH: 75 base pairs
(B) TYPE: nucleic acid
(C) STRANDEDNESS: single
(D) TOPOLOGY: linear

(xi) SEQUENCE DESCRIPTION: SEQ ID NO:16:
GGGAGAUGCC UGUCGAGCAU GCUGGUGCGG CUUUGGGCGC CGUGCUUGGC 50
GUAGCUAAAC AGCUUUGUCG ACUCU 75

(2) INFORMATION FOR SEQ ID NO:17:
(i) SEQUENCE CHARACTERISTICS:
(A) LENGTH: 74 base pairs
(B) TYPE: nucleic acid
(C) STRANDEDNESS: single
(D) TOPOLOGY: linear

(xi) SEQUENCE DESCRIPTION: SEQ ID NO:17:
GGGAGAUGCC UGUCGAGCAU GCUGGUGCGG CUUUGGGCGC CGUGCUUACG 50
UAGCUAAACA GCUUUGUCGA CUCU 74

(2) INFORMATION FOR SEQ ID NO:18:
(i) SEQUENCE CHARACTERISTICS:
(A) LENGTH: 75 base pairs
(B) TYPE: nucleic acid
(C) STRANDEDNESS: single
(D) TOPOLOGY: linear

(xi) SEQUENCE DESCRIPTION: SEQ ID NO:18:
GGGAGAUGCC UGUCGAGCAU GCUGGUGCGG CUUUGGGCGC CGUGCUUGAC 50
GUAGCUAAAC AGCUUUGUCG ACUCU 75

(2) INFORMATION FOR SEQ ID NO:19:
(i) SEQUENCE CHARACTERISTICS:
(A) LENGTH: 74 base pairs
(B) TYPE: nucleic acid
(C) STRANDEDNESS: single

(D) TOPOLOGY: linear

(xi) SEQUENCE DESCRIPTION: SEQ ID NO:19:

GGGAGAUGCC UGUCGAGCAU GCUGGGGCGG CUUUGGGCGC CGUGCUUGAC 50
GUAGCUAAAC AGCUUUGUCG ACUC 74

(2) INFORMATION FOR SEQ ID NO:20:

(i) SEQUENCE CHARACTERISTICS:

(A) LENGTH: 79 base pairs
(B) TYPE: nucleic acid
(C) STRANDEDNESS: single
(D) TOPOLOGY:

(xi) SEQUENCE DESCRIPTION: SEQ ID NO:20:

GGGAGAUGCC UGUCGAGCAU GCUGUGAGCC UGCCAGUGUG UAUGUGGAAA 50
CAAGGUAGCU AAACAGCUUU GUCGACUCU 79

(2) INFORMATION FOR SEQ ID NO:21:

(i) SEQUENCE CHARACTERISTICS:

(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:21:

GGGAGAUGCC UGUCGAGCAU GCUGUGAGCC UGCCAGUGUG CAUGUGGAAA 50
CAAGGUAGCU AAACAGCUUU GUCGACUCU 79

(2) INFORMATION FOR SEQ ID NO:22:

(i) SEQUENCE CHARACTERISTICS:

(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:22:

GGGAGAUGCC UGUCGAGCAU GCUGUGAGCC AGCCAGUGUG CAUGUGGAAA 50
CAAGGUAGCU AAACAGCUUU GUCGACUCU 79

(2) INFORMATION FOR SEQ ID NO:23:

(i) SEQUENCE CHARACTERISTICS:

(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:23:

GGGAGAUGCC UGUCGAGCAU GCUGUGAGCC AGCCAGUGUG UAUGUGGAAA 50
CAAGGUAGCU AAACAGCUUU GUCGACUCU 79

(2) INFORMATION FOR SEQ ID NO:24:

(i) SEQUENCE CHARACTERISTICS:

(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:24:

GGGAGAUGCC UGUCGAGCAU GCUGUGAGCC GGCCAGUGUG CAUGUGGAAA 50
CAAGGUAGCU AAACAGCUUU GUCGACUCU 79

(2) INFORMATION FOR SEQ ID NO:25:

(i) SEQUENCE CHARACTERISTICS:

(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:25:
GGGAGAUGCC UGUCGAGCAU GCUGUGAGCC AGCCAGUGUG UAUGUGGAAA 50
CAAGGUAGCU AAACAGCUUU GUCGACUCU 79

(2) INFORMATION FOR SEQ ID NO:26:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:26:
GGGAGAUGCC UGUCGAGCAU GCUGUUACGG GGAGGUGUUA CGGAGUGUAC 50
CCGUAGCUAA ACAGCUUUGU CGACUCU 77

(2) INFORMATION FOR SEQ ID NO:27:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:27:
GGGAGAUGCC UGUCGAGCAU GCUGUUGCGG GGAGGUGUUA GGGAGUGUAC 50
CCGUAGCUAA ACAGCUUUGU CGACUCU 77

(2) INFORMATION FOR SEQ ID NO:28:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:28:
GGGAGAUGCC UGUCGAGCAU GCUGUUGCGG GGAGGUGUUA GNNAGUGUAC 50
CCGUAGCUAA ACAGCUUUGU CGACUCU 77

(2) INFORMATION FOR SEQ ID NO:29:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:29:
GGGAGAUGCC UGUCGAGCAU GCUGUUGCGG GGAGGUGUUA GGGAGUUCAC 50
CCGUAGCUAA ACAGCUUUGU CGACUCU 77

(2) INFORMATION FOR SEQ ID NO:30:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:30
GGGAGAUGCC UGUCGAGCAU GCUGCUGCGG GGAGGUGUUA GGGAGUGUAC 50
CCGUAGCUAA ACAGCUUUGU CGACUCU 77

(2) INFORMATION FOR SEQ ID NO:31
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:31:
GGGAGAUGCC UGUCGAGCAU GCUGCUGCGG GGAGGUGUUA GAGAGUGUAC 50

CCGUAGCUAA ACAGCUUUGU CGACUCU 77

(2) INFORMATION FOR SEQ ID NO:32
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:32:
GGGAGAUGCC UGUCGAGCAU GCUGCUGCGG GGAGGUGUCA GAGAGUGUAC 50
CUGUAGCUAA ACAGCUUUGU CGACUCU 77

(2) INFORMATION FOR SEQ ID NO:33:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:33:
GGGAGAUGCC UGUCGAGCAU GCUGCUACGG GGAGGUGUUA GAGAGUGUAC 50
CUGUAGCUAA ACAGCUUUGU CGACUCU 77

(2) INFORMATION FOR SEQ ID NO:34:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:34:
GGGAGAUGCC UGUCGAGCAU GCUGCUACGG GGAGGUGUCG GAGAGUGUAC 50
CUGUAGCUAA ACAGCUUUGU CGACUCU 77

(2) INFORMATION FOR SEQ ID NO:35:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:35:
GGGAGAUGCC UGUCGAGCAU GCUGCACGAG GUGUCAGAGA GUGUAGUUCA 50
GCGUAGCUAA ACAGCUUUGU CGACUCU 77

(2) INFORMATION FOR SEQ ID NO:36:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:36:
GGGAGAUGCC UGUCGAGCAU GCUGCACGAG GUGUCAGAGA GUGUAGUGCA 50
GCGUAGCUAA ACAGCUUUGU CGACUCU 77

(2) INFORMATION FOR SEQ ID NO:37:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:37:
GGGAGAUGCC UGUCGAGCAU GCUGCACGAG GUGUAGAGGG UGUAGUGCAG 50
CAGUACGUAA ACAGCUUUGU CGACUCU 77

(2) INFORMATION FOR SEQ ID NO:38:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:38:
GGGAGAUGCC UGUCGAGCAU GCUGCACGAG GCGUCAGAGA GUGUAGUGCU 50
GCGUACGUAA ACAGCUUUGU CGACUCU 77

(2) INFORMATION FOR SEQ ID NO:39:
(i) SEQUENCE CHARACTERISTICS:
(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:39:
GGGAGAUGCC UGUCGAGCAU GCUGAGGAUC GAAGUUAGUA GGCUUUGUGU 50
ACUCGUAGCU AAACAGCUUU GUCCACUCU 79

(2) INFORMATION FOR SEQ ID NO:40:
(i) SEQUENCE CHARACTERISTICS:
(A) LENGTH: 6 amino acids
(B) TYPE: amino acid
(C) STRANDEDNESS: single
(D) TOPOLOGY: linear

(xi) SEQUENCE DESCRIPTION: SEQ ID NO:40:
Gly Arg Gly Asp Thr Pro 6

(2) INFORMATION FOR SEQ ID NO:41:
(i) SEQUENCE CHARACTERISTICS:
(A) LENGTH: 4 amino acids
(B) TYPE: amino acid
(C) STRANDEDNESS: single
(D) TOPOLOGY: linear

(xi) SEQUENCE DESCRIPTION: SEQ ID NO:41:

(ix) FEATURE:
(A) NAME/KEY: Xaa
(B) LOCATION: 1
(C) OTHER INFORMATION: This symbol stands for N-methylsuccinylalanine

(ix) FEATURE:
(A) NAME/KEY: Xaa
(B) LOCATION: 4
(C) OTHER INFORMATION: Valine chloromethyl ketone
Xaa Ala Pro Xaa 4

(2) INFORMATION FOR SEQ ID NO:42:
(i) SEQUENCE CHARACTERISTICS:
(A) LENGTH: 59 nucleotides
(B) TYPE: nucleic acid
(C) STRANDEDNESS: single
(D) TOPOLOGY: linear

(xi) SEQUENCE DESCRIPTION: SEQ ID NO:42:
TAGCGATACT GCGTGGGTTG GGGCGGGTAG GGCCAGCAGT CTCGTGCGGT 50
ACTTGAGCA 59

(2) INFORMATION FOR SEQ ID NO:43:
(i) SEQUENCE CHARACTERISTICS:
(A) LENGTH: 4 amino acids

(B) TYPE: amino acid
(C) STRANDEDNESS: single
(D) TOPOLOGY: linear

(xi) SEQUENCE DESCRIPTION: SEQ ID NO:43:

(ix) FEATURE:
(A) NAME/KEY: Xaa
(B) LOCATION: 1
(C) OTHER INFORMATION: N-methyoxysuccinyl alanine

(ix) FEATURE:
(A) NAME/KEY: Xaa
(B) LOCATION: 4
(C) OTHER INFORMATION: Valine p-nitroanilide

Xaa Ala Pro Xaa 4

(2) INFORMATION FOR SEQ ID NO:44:
(i) SEQUENCE CHARACTERISTICS:
(A) LENGTH: 4 amino acids
(B) TYPE: amino acid
(C) STRANDEDNESS: single
(D) TOPOLOGY: linear

(ix) FEATURE:
(A) NAME/KEY: Xaa
(B) LOCATION: 1
(C) OTHER INFORMATION: N-methyoxysuccinyl alanine

(xi) SEQUENCE DESCRIPTION: SEQ ID NO:44:
Xaa Ala Pro Val 4

(2) INFORMATION FOR SEQ ID NO:45:
(i) SEQUENCE CHARACTERISTICS:
(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:45:
GGGAGCUCAG AAUAAACGCU CAANNNNNNN NNNNNNNNNN NNNNNNNNNN 50
NNNUUCGACA UGAGGCCCGG AUCCGGC 77

(2) INFORMATION FOR SEQ ID NO:46:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:46:
CCGAAGCTTA ATACGACTCA CTATAGGGAG CTCAGAATAA ACGCTCAA 48

(2) INFORMATION FOR SEQ ID NO:47:
(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:47:
GCCGGATCCG GGCCTCATGT CGAA 24

(2) INFORMATION FOR SEQ ID NO:48:
(i) SEQUENCE CHARACTERISTICS:

(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:48:
UCAAGAGUGA UGCUCAUCCG CACUUGGUGA CGUU 34

(2) INFORMATION FOR SEQ ID NO:49:
(i) SEQUENCE CHARACTERISTICS:
(A) LENGTH: 37 base pairs
(B) TYPE: nucleic acid
(C) STRANDEDNESS: single
(D) TOPOLOGY: linear

(xi) SEQUENCE DESCRIPTION: SEQ ID NO:49:
CAAUACCGGC AUGCAUGUCC AUCGCUAGCG GUAUUCG 37

(2) INFORMATION FOR SEQ ID NO:50:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:50:
AAUGCGUGUU GUGACGCACA UCCGCACGCG CAUU 34

(2) INFORMATION FOR SEQ ID NO:51:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:51:
UCAAGGAGUG AUGCCCUAUC CGCACCUUGG CCCA 34

(2) INFORMATION FOR SEQ ID NO:52:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:52:
UCAAGCUUGA CNGCCCAUCC GAGCUUGAUC ACGC 34

(2) INFORMATION FOR SEQ ID NO:53:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:53
AAACGCUCAA UCCUUGAUGC GGAUCCGAGG AUGGGACGUU U 41

(2) INFORMATION FOR SEQ ID NO:54:
(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:54:
ACACCGUCGA CCUAUGAUGC GCAUCCGCAC UUCGAC 36

(2) INFORMATION FOR SEQ ID NO:55:
(i) SEQUENCE CHARACTERISTICS:
(A) LENGTH: 37 base pairs
(B) TYPE: nucleic acid
(C) STRANDEDNESS: single
(D) TOPOLOGY: linear

(xi) SEQUENCE DESCRIPTION: SEQ ID NO:55:
AACCGGUAGU CGCAUGGCCC AUCGCGCCCG GUUCGAC 37

(2) INFORMATION FOR SEQ ID NO:56:
(i) SEQUENCE CHARACTERISTICS:
(A) LENGTH: 38 base pairs
(B) TYPE: nucleic acid
(C) STRANDEDNESS: single
(D) TOPOLOGY: linear

(xi) SEQUENCE DESCRIPTION: SEQ ID NO:56:
ACGCUCAAGU CAGCAUGGCC CACCGCGCUU GACGUCUG 38

(2) INFORMATION FOR SEQ ID NO:57:
(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:57:
CACGGUUCGA UCUGUGACGU UCAUCCGCAC UUCGA 35

(2) INFORMATION FOR SEQ ID NO:58:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:58:
AACGCUCAAG GAGCAGUGAC GCACAUCCAC ACUCCAGCGU U 41

(2) INFORMATION FOR SEQ ID NO:59:
(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:59:
UUCGAAUGCC GAGGCUCGUG CCUUGACGGG UUC 33

(2) INFORMATION FOR SEQ ID NO:60:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:60:
UCGCGAAUGC CGACCACUCA GGUUGAUGGG UUCG 34

(2) INFORMATION FOR SEQ ID NO:61:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:61:

UCAAUGCCGG CCUGAUCGGC UGAUGGGUUG ACCG 34

(2) INFORMATION FOR SEQ ID NO:62:
(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:62:
GAAUGCCGAG CCCUAAGAGG CUUGAUGUGG UU 32

(2) INFORMATION FOR SEQ ID NO:63:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:63:
AACCUUNAUG UGGCNCGAAC UGCGUGCCGA GGUU 34

(2) INFORMATION FOR SEQ ID NO:64:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:64:
AAGCUUGAUG GGUGACACAC GUCAUGCCGA GCUU 34

(2) INFORMATION FOR SEQ ID NO:65:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:65:
GUCGUCCUGC AUGGGCCGUA UCGGUGCGCG 30

(2) INFORMATION FOR SEQ ID NO:66:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:66:
GCAGACGAAG GGAACCUGCG UCUCGGCACC UUCG 34

(2) INFORMATION FOR SEQ ID NO:67:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:67:
AAGGAGGANC CUGCGUCUCG GCACUCCGCA 30

(2) INFORMATION FOR SEQ ID NO:68:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:68:
UCAAGGGAAC CUGCGUUUCG GCACCUUGUU CCGU 34

(2) INFORMATION FOR SEQ ID NO:69:
(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:69:
AAAUGUGGGU UACCUGCGUU UCGGCACCAC GUUU 34

(2) INFORMATION FOR SEQ ID NO:70:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:70:
CGACGGUAGA GUCUGUCCCG UCAUCCCCCA 30

(2) INFORMATION FOR SEQ ID NO:71:
(i) SEQUENCE CHARACTERISTICS:
(A) LENGTH: 32 base pairs
(B) TYPE: nucleic acid
(C) STRANDEDNESS: single
(D) TOPOLOGY: linear
(xi) SEQUENCE DESCRIPTION: SEQ ID NO:71:
AAAGACCCCU GGUUGAGUCU GUCCCAGCCG UU 32

(2) INFORMATION FOR SEQ ID NO:72:
(i) SEQUENCE CHARACTERISTICS:
(A) LENGTH: 42 base pairs
(B) TYPE: nucleic acid
(C) STRANDEDNESS: single
(D) TOPOLOGY: linear

(xi) SEQUENCE DESCRIPTION: SEQ ID NO:72:
GACCCAUCGU CAACGGUUGA GUCUGUCCCG UUCGACAUGA GG 42

(2) INFORMATION FOR SEQ ID NO:73:
(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:73:
GCUCAAGGUU GAGUCUGUCC CUUCGAGUAU CUGAUC 36

(2) INFORMATION FOR SEQ ID NO:74:
(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:74:
UCGGACAGUU GGUUGAGUCU GUCCCAACUU UU 32

(2) INFORMATION FOR SEQ ID NO:75:
(i) SEQUENCE CHARACTERISTICS:
(A) LENGTH: 31 base pairs
(B) TYPE: nucleic acid
(C) STRANDEDNESS: single

(D) TOPOLOGY: linear

(xi) SEQUENCE DESCRIPTION: SEQ ID NO:75:
GACCAUGUGA CUGGUUGAGC CUGUCCCAGU U 31

(2) INFORMATION FOR SEQ ID NO:76:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:76:
AACGGUUGAG UCUGUCCCGU AAGAGAGCGC 30

(2) INFORMATION FOR SEQ ID NO:77:
(i) SEQUENCE CHARACTERISTICS:
(A) LENGTH: 38 base pairs
(B) TYPE: nucleic acid
(C) STRANDEDNESS: single
(D) TOPOLOGY: linear

(xi) SEQUENCE DESCRIPTION: SEQ ID NO:77:
UCGGAAUGUA GUUGACGUAU CCUUGUCCGA UUCGACAU 38

(2) INFORMATION FOR SEQ ID NO:78:
(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:78:
AGGGUGUAGUU GGGACCUAGU CCGCCGUACC UU 32

(2) INFORMATION FOR SEQ ID NO:79:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:79:
GGCAUAGUUG GGACCUCGUC CGCCGUGCCC 30

(2) INFORMATION FOR SEQ ID NO:80:
(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:80:
GCUCAAUAGU UGGAGGCCUG UCCUCGCCGU AGAGCG 36

(2) INFORMATION FOR SEQ ID NO:81:
(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:81:
AGGGGUUCUA GUGGAGACUC UGCCGCGGCC CUU 33

(2) INFORMATION FOR SEQ ID NO:82:
(i) SEQUENCE CHARACTERISTICS:
(A) LENGTH: 33 base pairs

(B) TYPE: nucleic acid
(C) STRANDEDNESS: single
(D) TOPOLOGY: linear

(xi) SEQUENCE DESCRIPTION: SEQ ID NO:82:
AACGGUUCUG UGUGUGGACU AGCCGCGGCC GUU 33

(2) INFORMATION FOR SEQ ID NO:83:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:83
GGGAUGUUUG GCUAUCUCGG AUAGUGCCCC 30

(2) INFORMATION FOR SEQ ID NO:84:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:84:
GCUUAAUACG ACUCACUNUA GGGAGCUCAG 30

(2) INFORMATION FOR SEQ ID NO:85:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:85:
UUGAGUGAUG UGCUUGACGU AUCGCUGCAC 30

(2) INFORMATION FOR SEQ ID NO:86:
(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: N
(B) LOCATION: 15
(C) OTHER INFORMATION: This symbol stands for the complimentary base for the N located in position 1

(xi) SEQUENCE DESCRIPTION: SEQ ID NO:86:
NUGAUGVNCA UCCGN 15

(2) INFORMATION FOR SEQ ID NO:87:
(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: S
(B) LOCATION: 11 and 12

(C) OTHER INFORMATION: This symbol stands for the complimentary base for the S located in positions 9 and 10

(xi) SEQUENCE DESCRIPTION: SEQ ID NO:87:
AAUGCCGASS SSUUGAUGGG UU 22

(2) INFORMATION FOR SEQ ID NO:88:
(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: H
(B) LOCATION: 24
(C) OTHER INFORMATION: This symbol stands for the complimentary base for the D located in position 2

(ix) FEATURE:
(A) NAME/KEY: Y
(B) LOCATION: 25
(C) OTHER INFORMATION: This symbol stands for the complimentary base for the R located in position 25

(ii) MOLECULAR TYPE: RNA

(xi) SEQUENCE DESCRIPTION: SEQ ID NO:88:
RDGGGAACCU GCGUYUCGGC ACCHY 25

(2) INFORMATION FOR SEQ ID NO:89:
(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: D
(B) LOCATION: 18 and 19
(C) OTHER INFORMATION: This symbol stands for the complimentary base for the H located in positions 1 and 2

(xi) SEQUENCE DESCRIPTION: SEQ ID NO:89:
HHGGUUGAGU CUGUCCCDD 19

(2) INFORMATION FOR SEQ ID NO:90:
(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: N
(B) LOCATION: 18-20 and 27
(C) OTHER INFORMATION: This symbol stands for the complimentary base for the N

located in positions 1 and 10-12

(xi) SEQUENCE DESCRIPTION: SEQ ID NO:90:
NRUAGUUGGN NNCUNSUNNN CGCCGUN 27

(2) INFORMATION FOR SEQ ID NO:91:
(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULAR TYPE: RNA

(ix) FEATURE:
(A) NAME/KEY: M
(B) LOCATION: 20
(C) OTHER INFORMATION: This symbol stands for the complimentary base for the K located in position 14

(ix) FEATURE:
(A) NAME/KEY: S
(B) LOCATION: 31
(C) OTHER INFORMATION: This symbol stands for the complimentary base for the S located in position 2

(xi) SEQUENCE DESCRIPTION: SEQ ID NO:91:
RSGGUUUCRU GUGKRGACUM UGCCGCGGCC SU 32

(2) INFORMATION FOR SEQ ID NO:92:
(i) SEQUENCE CHARACTERISTICS:
(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO:48:
GGGAACCUGC GUYUCGGCAC C 21

(2) INFORMATION FOR SEQ ID NO:93:
(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:93:
GGUUGAGUCU GUCCC 25

(2) INFORMATION FOR SEQ ID NO:94:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:94:
AAGCAGACGA AGGGAACCUG CGUCUCGGCA CCUUCGACAU 40

(2) INFORMATION FOR SEQ ID NO:95:
(i) SEQUENCE CHARACTERISTICS:
(A) LENGTH: 31 base pairs
(B) TYPE: nucleic acid
(C) STRANDEDNESS: single
(D) TOPOLOGY: linear

(xi) SEQUENCE DESCRIPTION: SEQ ID NO:95:
GGCCGGUAGU CGCAUGGCCC AUCGCGCCCG G 31

(2) INFORMATION FOR SEQ ID NO:96:
(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:96:
GGAAGCUUGA UGGGUGACAC ACGUCAUGCC GAGCU 35

(2) INFORMATION FOR SEQ ID NO:97:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:97:
GGAAGGGAAC CUGCGUCUCG GCACCUUCG 29

(2) INFORMATION FOR SEQ ID NO:98:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:98:
GGUCAACGGU UGAGUCUGUC CCGUUCGAC 29

(2) INFORMATION FOR SEQ ID NO:99:
(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:99:
GGCUCAAUAG UUGGAGGCCU GUCCUCGCCG UAGAGC 36

(2) INFORMATION FOR SEQ ID NO:100:
(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:100:
GGAACGGUUC UGUGUGUGGA CUAGCCGCGG CCGUU 35

(2) INFORMATION FOR SEQ ID NO:101:
(i) SEQUENCE CHARACTERISTICS:
(A) LENGTH: 76 base pairs
(B) TYPE: nucleic acid
(C) STRANDEDNESS: single
(D) TOPOLOGY: linear

(xi) SEQUENCE DESCRIPTION: SEQ ID NO:101:
GGGAGACAAG AAUAACGCUC AANNNNNNNN NNNNNNNNNN NNNNNNNNNN
NNUUCGACAG GAGGCUCACA ACAGGC 76

(2) INFORMATION FOR SEQ ID NO:102:
(i) SEQUENCE CHARACTERISTICS:
(A) LENGTH: 39 base pairs
(B) TYPE: nucleic acid

(C) STRANDEDNESS: single
(D) TOPOLOGY: linear

(xi) SEQUENCE DESCRIPTION: SEQ ID NO:102:
TAATACGACT CACTATAGGG AGACAAGAAU AACGCUCAA 39

(2) INFORMATION FOR SEQ ID NO:103:
(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:103:
GCCTGTTGTG AGCCTCCTGT CGAA 24

(2) INFORMATION FOR SEQ ID NO:104:
(i) SEQUENCE CHARACTERISTICS:
(A) LENGTH: 81 base pairs
(B) TYPE: nucleic acid
(C) STRANDEDNESS: single
(D) TOPOLOGY: linear

(xi) SEQUENCE DESCRIPTION: SEQ ID NO:104:
GGGAGGACGA UGCGGNNNNN NNNNNNNNNN NNNNNNNNNN NNNNNNNNNN 50
NNNNNNNNNN NNNNNCAGAC GACTCGCCCG A 81

(2) INFORMATION FOR SEQ ID NO:105:
(i) SEQUENCE CHARACTERISTICS:
(A) LENGTH: 22 base pairs
(B) TYPE: nucleic acid
(C) STRANDEDNESS: single
(D) TOPOLOGY: linear

(xi) SEQUENCE DESCRIPTION: SEQ ID NO:105:
TAATACGACT CACTATAGGG AGGACGAUGC GG 22

(2) INFORMATION FOR SEQ ID NO:106:
(i) SEQUENCE CHARACTERISTICS:
(A) LENGTH: 16 base pairs
(B) TYPE: nucleic acid
(C) STRANDEDNESS: single
(D) TOPOLOGY: linear

(xi) SEQUENCE DESCRIPTION: SEQ ID NO:106:
TCGGGCGAGT CGTCTG 16

(2) INFORMATION FOR SEQ ID NO:107:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:107:
UGGCUGUGAU CAAUGCGGGG AGGUGAGGAA GGGCCUUACA AAUCCUUCGG 50

(2) INFORMATION FOR SEQ ID NO:108:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:108:
UGUGAUCAAU GCGGUGGCGG GGUAUGGAUG GGAGUCUGGA AUGCUGCGCU 50

(2) INFORMATION FOR SEQ ID NO:109:

(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:109:
CGCUGUGUUC AAUGCGGGGA UCGGGCCGGA GGAUGAACAA AUGGCGGGU 49

(2) INFORMATION FOR SEQ ID NO:110:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:110:
UGUUGAGCAA GCACUCAUGU GGUCAAUGUG GGAGUGGGAG CUGGUGGGGU 50

(2) INFORMATION FOR SEQ ID NO:111:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:111:
CAAGGGAGCG UUAGAGCCAU GUGGUCAAUG AGGGGUGGGA UUGGUUGGGU 50

(2) INFORMATION FOR SEQ ID NO:112:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:112:
CAUGGUUGUG AACUGUUGUG AUCAAUGCGG GGAGGGUAAU GGUGGGUGGU 50

(2) INFORMATION FOR SEQ ID NO:113:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:113:
AUGAGUGACA CAUGUGCUCA AUGCGGGGUG GGUUGGUAGG GGUAGCACGG 50

(2) INFORMATION FOR SEQ ID NO:114:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:114:
UGUGGUCAAU GUGGGGUAGG GCUGGUAGGG CAUUCCGUAC UGGUGUGGU 49

(2) INFORMATION FOR SEQ ID NO:115:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:115:
CCGAGUUGUG CUCAAUGUGG GGUCUGGGUA CGGACGGGAA CAGAUCUGG 49

(2) INFORMATION FOR SEQ ID NO:116:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:116:
GUGCUCAGCA UUGUGUGCUC AAUGCGGGGG AGUUUGGGUU GGCGACGG 48

(2) INFORMATION FOR SEQ ID NO:117:
(i) SEQUENCE CHARACTERISTICS:
(A) LENGTH: 16 base pairs
(B) TYPE: nucleic acid
(C) STRANDEDNESS: single
(D) TOPOLOGY: linear

(xi) SEQUENCE DESCRIPTION: SEQ ID NO:117:
UGUGNUCAAU GNGGGG 16

(2) INFORMATION FOR SEQ ID NO:118:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:118:
CAUAGGCUUA CAACAGAGCG GGGGUUCUGA UGGUAGACGC CGGGACGCCC 50

(2) INFORMATION FOR SEQ ID NO:119:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:119:
UAUGAUGGUA GACGCCGUAC CGCAUCAGGC CAAGUCGUCA CAGAUCGUG 49

(2) INFORMATION FOR SEQ ID NO:120:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:120:
GCAACAGAGG CUGAUGGUAG ACGCCGGCCA 30

(2) INFORMATION FOR SEQ ID NO:121:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:121:
AGAGUCGCUG AUGGUAGACG CCGGCGGAUC 30

(2) INFORMATION FOR SEQ ID NO:122:
(i) SEQUENCE CHARACTERISTICS:
(A) LENGTH: base pairs
(B) TYPE: 29 nucleic acid
(C) STRANDEDNESS: single
(D) TOPOLOGY: linear

(xi) SEQUENCE DESCRIPTION: SEQ ID NO:122:
GAGGCUGAUG GCAGACGCGG CCGAAGACA 29

(2) INFORMATION FOR SEQ ID NO:123:
(i) SEQUENCE CHARACTERISTICS:
(A) LENGTH: base pairs
(B) TYPE: 29 nucleic acid
(C) STRANDEDNESS: single
(D) TOPOLOGY: linear

(xi) SEQUENCE DESCRIPTION: SEQ ID NO:123:
CCCUGAUGGU AGACGCCGGG GUGCCGGAA 29

(2) INFORMATION FOR SEQ ID NO:124:
(i) SEQUENCE CHARACTERISTICS:
(A) LENGTH: 17 base pairs
(B) TYPE: nucleic acid
(C) STRANDEDNESS: single
(D) TOPOLOGY: linear

(xi) SEQUENCE DESCRIPTION: SEQ ID NO:124:
CUGAUGGUAG ACGCCGG 17

(2) INFORMATION FOR SEQ ID NO:125:
(i) SEQUENCE CHARACTERISTICS:
(A) LENGTH: 51 base pairs
(B) TYPE: nucleic acid
(C) STRANDEDNESS: single
(D) TOPOLOGY: linear

(xi) SEQUENCE DESCRIPTION: SEQ ID NO:125:
CAGUGCUGAA CUAAUCGAAC GGGGUCAAGG AGGGUCGAAC GAGAUCUGCC G 51

(2) INFORMATION FOR SEQ ID NO:126:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:126:
CACCUUCGUG GGGUCAAGGA GGGUCGCGAG GCCGCAGGAU CAACCGUGUG 50

(2) INFORMATION FOR SEQ ID NO:127:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:127:
GGUCAAGUUG GGUCGAGGAA GCGCUCCCGA GUAUCGUAGU GUGCGACUGC 50

(2) INFORMATION FOR SEQ ID NO:128:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:128:
GAACUUGAUC GGGGUCAAGG CGGGACGAA 29

(2) INFORMATION FOR SEQ ID NO:129:
(i) SEQUENCE CHARACTERISTICS:
(A) LENGTH: 30 base pairs
(B) TYPE: nucleic acid
(C) STRANDEDNESS: single

(D) TOPOLOGY: linear

(xi) SEQUENCE DESCRIPTION: SEQ ID NO:129:
UGGCGGGACC AAGGAGGGAC GUGUAGGAAA 30

(2) INFORMATION FOR SEQ ID NO:130:
(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:130:
AAAAUGCACA AAUCGGGGUC AAGGAGGGAC GA 32

(2) INFORMATION FOR SEQ ID NO:131:
(i) SEQUENCE CHARACTERISTICS:
(A) LENGTH: 47 base pairs
(B) TYPE: nucleic acid
(C) STRANDEDNESS: single
(D) TOPOLOGY: linear

(xi) SEQUENCE DESCRIPTION: SEQ ID NO:131:
AUGGGUUCGU GUGGUGAAUG GAGGAGGUGG GCUCGCAUGC UACUGUG 47

(2) INFORMATION FOR SEQ ID NO:132:
(i) SEQUENCE CHARACTERISTICS:
(A) LENGTH: 12 base pairs
(B) TYPE: nucleic acid
(C) STRANDEDNESS: single
(D) TOPOLOGY: linear

(xi) SEQUENCE DESCRIPTION: SEQ ID NO:132:
GGUCAAGGNG GG 12

(2) INFORMATION FOR SEQ ID NO:133:
(i) SEQUENCE CHARACTERISTICS:
(A) LENGTH: 44 base pairs
(B) TYPE: nucleic acid
(C) STRANDEDNESS: single
(D) TOPOLOGY: linear

(xi) SEQUENCE DESCRIPTION: SEQ ID NO:133:
UGCACUAAGU CCGGGUAGUG GGAGUGGUUG GGCCUGGAGU GCGC 44

(2) INFORMATION FOR SEQ ID NO:134:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:134:
AUCAAAGGGU AGAGGGUGGG CUGUGGCAAG 30

(2) INFORMATION FOR SEQ ID NO:135:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:135:
AAUCGAGGGU AGCGGGCGCG GCUUGGCCAA 30

(2) INFORMATION FOR SEQ ID NO:136:
(i) SEQUENCE CHARACTERISTICS:

(A) LENGTH: 31 base pairs
(B) TYPE: nucleic acid
(C) STRANDEDNESS: single
(D) TOPOLOGY: linear

(xi) SEQUENCE DESCRIPTION: SEQ ID NO:136:
GCCUCGGAUC GGGCAGCGGG UGGGAUGGCA A 31

(2) INFORMATION FOR SEQ ID NO:137:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:137:
AACGGAGUGG UAGGCGUUGG GUGCCAGGAA 30

(2) INFORMATION FOR SEQ ID NO:138:
(i) SEQUENCE CHARACTERISTICS:
(A) LENGTH: 11 base pairs
(B) TYPE: nucleic acid
(C) STRANDEDNESS: single
(D) TOPOLOGY: linear

(xi) SEQUENCE DESCRIPTION: SEQ ID NO:138:
GGUAGNGGGN G 11

(2) INFORMATION FOR SEQ ID NO:139:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:139:
AACCGAGUCG UGUGGGUUGG GGCUCCAGUA CAUCCCCGGU CUGGGUGU 48

(2) INFORMATION FOR SEQ ID NO:140:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:140:
UAACAUACGC AGUCGUGUGG GUAGGGGAUC ACAAACUGCG UAUCGUGU 48

(2) INFORMATION FOR SEQ ID NO:141:
(i) SEQUENCE CHARACTERISTICS:
(A) LENGTH: 19 base pairs
(B) TYPE: nucleic acid
(C) STRANDEDNESS: single
(D) TOPOLOGY: linear

(xi) SEQUENCE DESCRIPTION: SEQ ID NO:141:
AGUCGUGUGG GUGGGGUCA 19

(2) INFORMATION FOR SEQ ID NO:142:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:142:
AGUGUAGGAU AGGGGAUGGG AGGUCCGGGA 30

(2) INFORMATION FOR SEQ ID NO:143:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:143:
ACUGUGGGCU CUAGGGCAGU GGGAGUGGAG 30

(2) INFORMATION FOR SEQ ID NO:144:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:144:
AGUGGGACAG GGAUUGCGGA GGGUGGAAGG 30

(2) INFORMATION FOR SEQ ID NO:145:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:145:
GUCAGGAGGA CUGGAAGGUG GGACUGGUGA 30

(2) INFORMATION FOR SEQ ID NO:146:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:146:
GCAGGAGAGA GGGUGUUGGG UGCGGAUACA 30

(2) INFORMATION FOR SEQ ID NO:147:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:147:
AGGGUAGGAG GCUAAGCAUA GUUCAGAGGA GGUGGCGCGU GCCCCCGUG 49

(2) INFORMATION FOR SEQ ID NO:148:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:148:
CAACAUUGGC ACCAAUGCUC UGUGUUAAUG UGGGGUGGGA ACGGCGCCG 49

(2) INFORMATION FOR SEQ ID NO:149:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:149:

ACCAAUGAUU GCAAUGAGGG CAGUGGGGGG GAAUUGGGCU CGUGUGGU 48

(2) INFORMATION FOR SEQ ID NO:150:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:150:
GCAGUGGGUG AGGUCCGGGC ACGAUUGAGU UUGAACGGUU CUGGCUUGGU 50

(2) INFORMATION FOR SEQ ID NO:151:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:151:
GUGGUAGGUG UAGAGUGGAU GGCGGAGGUC CUAGUAGUUC UGUGCCUGGU 50

(2) INFORMATION FOR SEQ ID NO:152:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:152:
CGCGGGAGAG GGUAGUGGGU GUGGUGCUUG GACAAGCAGC G 41

(2) INFORMATION FOR SEQ ID NO:153:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:153:
ACCCGCAUAC GGACCGCGGA GGGGGAAAUC UAGCCUCAGG GGUGGCGGGC 50

(2) INFORMATION FOR SEQ ID NO:154:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:154:
UGAAGAAGCG GGGACUGCAC GACGGGAUGG AGGGACACGA CUGCGGGGU 49

(2) INFORMATION FOR SEQ ID NO:155:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:155:
ACACCAGGAG AGUGGGUUCG GGUGAGGACG 30

(2) INFORMATION FOR SEQ ID NO:156:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:156:
GUGGCUGAUG GCAGACGCCG GCUGCUGACG 30

(2) INFORMATION FOR SEQ ID NO:157:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:157:
UCGUGCCAGG ACAUGGUGGC UCAUGGGUAA 30

(2) INFORMATION FOR SEQ ID NO:158:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:158:
AGGUACGGGG GAGGGAAGGA UAUAACGCGA 30

(2) INFORMATION FOR SEQ ID NO:159:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:159:
UGGAAAGGUG UGGAAAGAGG CAUCGGGGGG 30

(2) INFORMATION FOR SEQ ID NO:160:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:160:
UCAAUGGGCA GGAAGAGGGA AGGGAUGUGA 30

(2) INFORMATION FOR SEQ ID NO:161:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:161:
CAUGGGUAAG GGAGUGGGAG UGGUGAAUAG 30

(2) INFORMATION FOR SEQ ID NO:162:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:162:
GGAACGAGUA GGGCAGUGGG UGGUGAUGGC 30

(2) INFORMATION FOR SEQ ID NO:163:
(i) SEQUENCE CHARACTERISTICS:
(A) LENGTH: 30 base pairs

(B) TYPE: nucleic acid
(C) STRANDEDNESS: single
(D) TOPOLOGY: linear

(xi) SEQUENCE DESCRIPTION: SEQ ID NO:163:
UAGGGCAGAG GGAGUGGUUA GGGCUGUGAU 30

(2) INFORMATION FOR SEQ ID NO:164:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:164:
GGGUAGUGGG AAGGGUAAGG GCCGAGGUGG 30

(2) INFORMATION FOR SEQ ID NO:165:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:165:
AAUACACACC GCGGGAAGGG AGGGUGGAAA 30

(2) INFORMATION FOR SEQ ID NO:166:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:166:
AGACUACAGC GCGGGUUAGG GUUGAGGGAA 30

(2) INFORMATION FOR SEQ ID NO:167:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:167:
UACGAGCAAG CGGGCGAAGG GUUGAGGGAA 30

(2) INFORMATION FOR SEQ ID NO:168:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:168:
CAAGGUGGUG GAGGAGGAUA CGAUCUGCAG 30

(2) INFORMATION FOR SEQ ID NO:169:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:169:
GGAGGGAAGG AGGGCAGGUG AUGGGUCAG 29

(2) INFORMATION FOR SEQ ID NO:170:

(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:170:
UGAUGGCGGU AGUGGAGGUA AUGAGCGUGA 30

(2) INFORMATION FOR SEQ ID NO:171:
(i) SEQUENCE CHARACTERISTICS:
(A) LENGTH: 26 base pairs
(B) TYPE: nucleic acid
(C) STRANDEDNESS: single
(D) TOPOLOGY: linear

(xi) SEQUENCE DESCRIPTION: SEQ ID NO:171:
GCAACUGGGG GCGGGUGGUG UGAGGA 26

(2) INFORMATION FOR SEQ ID NO:172:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:172:
GGAGGGGCCU AUAGGGGUGG UGGUGUACGA 30

(2) INFORMATION FOR SEQ ID NO:173:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:173:
UAUAGGGUAG UGGGUGUAGG UAGGGCGACA 30

(2) INFORMATION FOR SEQ ID NO:174:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:174:
GAGGGUUGGA GGGCAUGGGG CAGGAACCGG 30

(2) INFORMATION FOR SEQ ID NO:175:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:175:
CGUAGAACUG GCGGGCAGUG GGGGGGAUGC 30

(2) INFORMATION FOR SEQ ID NO:176:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:176:
UGAGGGGACG AGGGAUGUGG GGAGCGGGAC 30

(2) INFORMATION FOR SEQ ID NO:177:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:177:
CGAGGGAUGG GAGGCGUGUG GAAGAUGCAA 30

(2) INFORMATION FOR SEQ ID NO:178:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:178:
GCAUCCGGGG ACAAGAUGGG UCGGUAAGGU 30

(2) INFORMATION FOR SEQ ID NO:179:
(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:179:
GUGUGCGGGG UCAAGACGGG UGGCGUGCG 29

(2) INFORMATION FOR SEQ ID NO:180:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:180:
UCAAACCAUG GGGCGGGUGG UACGAGGAAC 30

(2) INFORMATION FOR SEQ ID NO:181:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:181:
CGAGUCCGAG GGAUGGGUGG UGUGCGGCAA 30

(2) INFORMATION FOR SEQ ID NO:182:
(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:182:
CAGUGUCGGA GAGGAGGAUG GAGGUAUGAA 30

(2) INFORMATION FOR SEQ ID NO:183:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:183:
CACCACUACG CGGGAAGGGU AGGGUGGAUU ACAAGGUGUG ACCGCUCCGU 50

(2) INFORMATION FOR SEQ ID NO:184:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:184:
UACGGUUAAC GGGGGUGGUG UGGGAGGACA CAAAGCGCGU ACCUGCCCC 49

(2) INFORMATION FOR SEQ ID NO:185:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:185:
AGGUCCUCGA GGGUCUGGGU GUGGGAGUGG GCAUGGACCA AUACCGCGUG 50

(2) INFORMATION FOR SEQ ID NO:186:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:186:
AAACCCAUCC UGCGCGGGAU GGGAGGGUGG AAACACUAGA GCUUCGCCUG 50

(2) INFORMATION FOR SEQ ID NO:187:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:187:
AACUGGUGGU CACGCGUUGA GGUGGUGGAG GUGGAUUCAA CGGUCGAGGG 50

(2) INFORMATION FOR SEQ ID NO:188:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:188:
CAUGAAAGUA GGGUUAUGAA GGCGGUAGAU GGAGGAGGUU GGGUUGCCGC 50

(2) INFORMATION FOR SEQ ID NO:189:
(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:189:
GUCUAUUGGG UAGGUGUUUG CAAGAAUUCC GCACGAUAGG UAAAACAGUG 50

(2) INFORMATION FOR SEQ ID NO:190:
(i) SEQUENCE CHARACTERISTICS:
(A) LENGTH: 49 base pairs
(B) TYPE: nucleic acid

(C) STRANDEDNESS: single
(D) TOPOLOGY: linear

(xi) SEQUENCE DESCRIPTION: SEQ ID NO:190:
UGUAGGGGAA GUACGAGAGU GGGAGCGGCC GUAUAGGUGG GAGUGUGCU 49

(2) INFORMATION FOR SEQ ID NO:191:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:191:
GAGUAGGCUU GACGCCUGGG GGGGUAUGGC UUCGACUGCG 40

(2) INFORMATION FOR SEQ ID NO:192:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:192:
GAGUAGGCUU GACGCCUGGG GGGGUAUGGC UUCAACUGCG 40

(2) INFORMATION FOR SEQ ID NO:193:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:193:
GAGUAGGCUU GACGCUGGGG GGGUAUGGCU UCGACUUGCG 40

(2) INFORMATION FOR SEQ ID NO:194:
(i) SEQUENCE CHARACTERISTICS:
(A) LENGTH: 42 base pairs
(B) TYPE: nucleic acid
(C) STRANDEDNESS: single
(D) TOPOLOGY: linear

(xi) SEQUENCE DESCRIPTION: SEQ ID NO:194:
AGAGGUAGGC UUGACGCCUG GGGGGGUAUG GCUCCGACUG CG 42

(2) INFORMATION FOR SEQ ID NO:195:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:195:
GGUAGACAUA CGCCUUGGGG GGGUGUCAGG GUAUAUGGUA 40

(2) INFORMATION FOR SEQ ID NO:196:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:196:
GGCAGACAUA CGCCUUGGGG GGGUGUCAGG GUAUAUGGUA 40

(2) INFORMATION FOR SEQ ID NO:197:
(i) SEQUENCE CHARACTERISTICS:

(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:197:
GGUAGACAUA CGCCUUGGGG GGGUGCCAGG GUAUAUGGUA 40

(2) INFORMATION FOR SEQ ID NO:198:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:198:
GGUAGACAUA CGCCUUGGGG GGGUAUCAGG GUAUAUGGUA 40

(2) INFORMATION FOR SEQ ID NO:199:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:199:
GGCAGACAUA CGCCUUGGGG GGGUGUCAGG GUAUAUGGCA 40

(2) INFORMATION FOR SEQ ID NO:200:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:200:
GGCAGACAUA CGCCUUGGGG GGGUUUCAGG GUAUAUGGCA 40

(2) INFORMATION FOR SEQ ID NO:201:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:201:
AGAAUGAAUG UGAUGAACAG GUGUCGGGGU GGAUGGGUGG 40

(2) INFORMATION FOR SEQ ID NO:202:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:202:
GGAAUGAAUG UGAUGAACAG GUGUCGGGGU GGAUGGGUGG 40

(2) INFORMATION FOR SEQ ID NO:203:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:203:
GAAUGAAUGU GAUGAACAGG UGUCGGGGUG GAUGGGUGG 39

(2) INFORMATION FOR SEQ ID NO:204:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:204:
CGCCUUAGUC GCCAUAAUGC UGUCGGGGUG GAUAGGGUGG 40

(2) INFORMATION FOR SEQ ID NO:205:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:205:
UGCCCGGCAG UACUGCACGG CCUCGGGGGG GACCAGGGAG 40

(2) INFORMATION FOR SEQ ID NO:206:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:206:
AUCGGAUAGC GCCGCGAUGG CGUCUGGGGG GGACCAGGGA 40

(2) INFORMATION FOR SEQ ID NO:207:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:207:
GUACUACUGC AAGCCCGUGU GGCGCGGUCA GUGGGUGGC 39

(2) INFORMATION FOR SEQ ID NO:208:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:208:
GUACUACUGC AAGCCCGUGU GGCGCGGUCA GUGGGUGGCC 40

(2) INFORMATION FOR SEQ ID NO:209:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:209:
CGGAUCGGGC GGUCGUCUAG CGGGAUGGCG UGCGGGUGGA 40

(2) INFORMATION FOR SEQ ID NO:210:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:210:

UGACCGGGUC GACAUGCCUG AGGUGUGGCC AGUGGGUGG 39

(2) INFORMATION FOR SEQ ID NO:211:
(i) SEQUENCE CHARACTERISTICS:
(A) LENGTH: 37 base pairs
(B) TYPE: nucleic acid
(C) STRANDEDNESS: single
(D) TOPOLOGY: linear

(xi) SEQUENCE DESCRIPTION: SEQ ID NO:211:
GGCCCUCUGA ACCGUCUGGG CGUGGUUAGU GGGUGGC 37

(2) INFORMATION FOR SEQ ID NO:212:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:212:
UGACCGGGCC GACAUGCUUG AGGUGUGGCC CAGUGGGUGG 40

(2) INFORMATION FOR SEQ ID NO:213:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:213:
GACGCGUGCU GGCCUCGACC GUGUGGGUGC GGAUGGGUGG 40

(2) INFORMATION FOR SEQ ID NO:214:
(i) SEQUENCE CHARACTERISTICS:
(A) LENGTH: 44 base pairs
(B) TYPE: nucleic acid
(C) STRANDEDNESS: single
(D) TOPOLOGY: linear

(xi) SEQUENCE DESCRIPTION: SEQ ID NO:214:
UGAGGUUGAG UAGUCGUGGU AAUGGCCACG GCUGGGGGGG UCGG 44

(2) INFORMATION FOR SEQ ID NO:215:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:215:
UGGAUAGCGC UGCGUGACCG CGCUGGGGGG GUUGGCAGGG 40

(2) INFORMATION FOR SEQ ID NO:216:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:216:
ACCTTTTCCT CTCAGTCTTC TTATCTCGCC TATTATTATT 40

(2) INFORMATION FOR SEQ ID NO:217:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:217:
GCGTGGGTTG GGGCCGGGAG GGCCAGCAGT CTCGTGCGTC 40

(2) INFORMATION FOR SEQ ID NO:218:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:218:
CATTCATCTT CTCATTCTCG CCTAACTGTA CACATCTTT 39

(2) INFORMATION FOR SEQ ID NO:219:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:219:
GCGTGGGTTG GGGCCGGGAG GGCCAGCAGT CTCGTGCGT 39

(2) INFORMATION FOR SEQ ID NO:220:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:220:
GCGTGGGTTG GGGCCGGGAG GGCCAACAGT CTCGTGCGTC 40

(2) INFORMATION FOR SEQ ID NO:221:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:221:
CTACCCTTTC TTGACCACCG CCTCGTTTCA TCCACCTTAC 40

(2) INFORMATION FOR SEQ ID NO:222:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:222:
TTCTTTCTAT ACCCATATTA CCCTTCTTCA CACTCGTATC 40

(2) INFORMATION FOR SEQ ID NO:223:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:223:
CTTTATCCTT TCTCTTTCCT TGCACTCTAA CATCCTACTC 40

(2) INFORMATION FOR SEQ ID NO:224:
(i) SEQUENCE CHARACTERISTICS:
(A) LENGTH: 40 base pairs
(B) TYPE: nucleic acid

(C) STRANDEDNESS: single
(D) TOPOLOGY: linear

(xi) SEQUENCE DESCRIPTION: SEQ ID NO:224:
GCGTGGGTTG GGGCCGGTAG GGCCAGCAGT CTCGTTGCGT 40

(2) INFORMATION FOR SEQ ID NO:225:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:225:
CCTTCTTGTT ATATTGGTCG TTTTCTTCTT TTACTTTCTT 40

(2) INFORMATION FOR SEQ ID NO:226:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:226:
TCTTCATCAT TTCACTTCAT TCTGTCGGGC TATCTTCGGT 40

(2) INFORMATION FOR SEQ ID NO:227:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:227:
TTCCACGTCT CCTCAGCCCG GGAGGCCACC TTTTTATCTG 40

(2) INFORMATION FOR SEQ ID NO:228:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:228:
GAAGGCTTAA CCTAATTTTC CACCTTTCAT CCACTTTTCC 40

(2) INFORMATION FOR SEQ ID NO:229:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:229:
TCACCTCCCA TTTATATTTT CCCTTAATTT CTTCTTCTTA 40

(2) INFORMATION FOR SEQ ID NO:230:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:230:
CTTACTATGC ATCTTACTTA TTATTTTTTT TTACTTTCTA 40

(2) INFORMATION FOR SEQ ID NO:231:
(i) SEQUENCE CHARACTERISTICS:

(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:231:
TACTTCTTTT ACATCATTCC TCGATTTATT CATTCTCCAC 40

(2) INFORMATION FOR SEQ ID NO:232:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:232:
TTCACCCGTG TCATATCATA TTTCCCGGTC CTTCCTTTCC C 41

(2) INFORMATION FOR SEQ ID NO:233:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:233:
CAATTCAAAC CTTTTCTACA ATTTTCATCT TACATTCTTC 40

(2) INFORMATION FOR SEQ ID NO:234:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:234:
TCACTTGATC CTTCTTTACT TTTTTTCTCG TCTAATTATA 40

(2) INFORMATION FOR SEQ ID NO:235:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:235:
GCGTGGGTTG GGGCGGGATG GGCCAGCAGT CTCGTGCGGT 40

(2) INFORMATION FOR SEQ ID NO:236:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:236:
CTTTTTATTC CAACCCCCAT TCTTACTTAC AATATCTTGA 40

(2) INFORMATION FOR SEQ ID NO:237:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:237:
TATCCTTCTC CTTAACTCCT ACTTCTATCT ATAAAATTAT 40

(2) INFORMATION FOR SEQ ID NO:238:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:238:
GCGTGGGTAG GGGCCGGGAG GGCCAGCAGT CACGTGCGTA 40

(2) INFORMATION FOR SEQ ID NO:239:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:239:
GGGTGGGTTG GGGCCGGGAG GGCTAGCAGT CTCGTGCGTT 40

(2) INFORMATION FOR SEQ ID NO:240:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:240:
GCGTGGGATG GGGCCGGGAG GGCCAGCAGT CTCGTGCGTT 40

(2) INFORMATION FOR SEQ ID NO:241:
(i) SEQUENCE CHARACTERISTICS:
(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:241:
GCGTGGGTTG GGGCCGGGAG GGCCAGCAGT CTCGTGCGT 39

(2) INFORMATION FOR SEQ ID NO:242:
(i) SEQUENCE CHARACTERISTICS:
(A) LENGTH: 59 base pairs
(B) TYPE: nucleic acid
(C) STRANDEDNESS: single
(D) TOPOLOGY: linear

(xi) SEQUENCE DESCRIPTION: SEQ ID NO:242:
TAGCGATACT GCGTGGGTTG GGGCGGGTAG GGCCAGCAGT CTCGTGCGGT 50
ACTTGAGCA 59

(2) INFORMATION FOR SEQ ID NO:243:
(i) SEQUENCE CHARACTERISTICS:
(A) LENGTH: 56 base pairs
(B) TYPE: nucleic acid
(C) STRANDEDNESS: single
(D) TOPOLOGY: linear

(xi) SEQUENCE DESCRIPTION: SEQ ID NO:243:
TAGCGATACT GCGTGGGNGG GNGGGNGGGC CAGCAGTCTC GTGCGGTACT 50
TGAGCA 56

(2) INFORMATION FOR SEQ ID NO:244:
(i) SEQUENCE CHARACTERISTICS:
(A) LENGTH: 6 base pairs
(B) TYPE: nucleic acid
(C) STRANDEDNESS: single
(D) TOPOLOGY: linear

(xi) SEQUENCE DESCRIPTION: SEQ ID NO:244:
AUACCA 6

(2) INFORMATION FOR SEQ ID NO:245:
(i) SEQUENCE CHARACTERISTICS:
(A) LENGTH: 9 base pairs
(B) TYPE: nucleic acid
(C) STRANDEDNESS: single
(D) TOPOLOGY: linear

(xi) SEQUENCE DESCRIPTION: SEQ ID NO:245:
CCUUGGMAG 9

(2) INFORMATION FOR SEQ ID NO:246:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:246:
GAGAAAUACC AGUGACAACU CUCGAGAUCA CCCUUGGAAG 40

(2) INFORMATION FOR SEQ ID NO:247:
(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:247:
AUACCAUCGU GUAAGCAAGA GCACGACCUU GGCAGUGUGU G 41

(2) INFORMATION FOR SEQ ID NO:248:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:248:
GAUACCAGCA GCAUAUUUGC UGUCCUUGGA AGCAACGAGA 40

(2) INFORMATION FOR SEQ ID NO:249:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:249:
GUGAUACCAG CAUCGUCUUG AUGCCCUUGG CAGCACUUCA 40

(2) INFORMATION FOR SEQ ID NO:250:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:250:
UUGUCGAAUC GGAUACCAGC AAUGCAGCCC UUGGAAGCAG 40

(2) INFORMATION FOR SEQ ID NO:251:
(i) SEQUENCE CHARACTERISTICS:
(A) LENGTH: 40 base pairs
(B) TYPE: nucleic acid

(C) STRANDEDNESS: single
(D) TOPOLOGY: linear

(xi) SEQUENCE DESCRIPTION: SEQ ID NO:251:
GAUACCAACG GCAUAUUUGC UGUCCUUGGA AGCAACUAUA 40

(2) INFORMATION FOR SEQ ID NO:252:
(i) SEQUENCE CHARACTERISTICS:
(A) LENGTH: 40 base pairs
(B) TYPE: nucleic acid
(C) STRANDEDNESS: single
(D) TOPOLOGY: linear
(xi) SEQUENCE DESCRIPTION: SEQ ID NO:252:
CUCUCGAAAU ACCAACUACU CUCACAAUAG UCCUUGGAAG 40

(2) INFORMATION FOR SEQ ID NO:253:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:253:
UUCAUGUCGC UUGAUACCAU CAACAAUGAC CUUGGAAGCA 40

(2) INFORMATION FOR SEQ ID NO:254:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:254:
UGACUCGAAC CCUUGGAAGA CCUGAGUACA GGUAUACCAG 40

(2) INFORMATION FOR SEQ ID NO:255:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:255:
UCCUUGGAAG CCGUACGGAU ACCAAUUGAG UGGCCAUAUG 40

(2) INFORMATION FOR SEQ ID NO:256:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:256:
UAUCGAGUGG CCUUGGCAGA CCAGGCCCGG UAUACCACCA 40

(2) INFORMATION FOR SEQ ID NO:257:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:257:
CGAGAUUCAA CCUUGGAAGU CAAUCGUGAA UACCAUUGUU 40

(2) INFORMATION FOR SEQ ID NO:258:
(i) SEQUENCE CHARACTERISTICS:
(A) LENGTH: 40 base pairs

(B) TYPE: nucleic acid
(C) STRANDEDNESS: single
(D) TOPOLOGY: linear

(xi) SEQUENCE DESCRIPTION: SEQ ID NO:258:
UCAGAACCUU GGAAGCACUG AAUAAGAUCA GUUGAUACCA 40

(2) INFORMATION FOR SEQ ID NO:259:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:259:
GGACGAGACT CATCGTAACC TAGATGGTTG CCAGCATTTA 40

(2) INFORMATION FOR SEQ ID NO:260:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:260:
GGATGCTTAC AGCATAATCG GAATTGATTG CCAGCGGAAA 40

(2) INFORMATION FOR SEQ ID NO:261:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:261:
GGGGCAATAG AAGCCAACGC ACAGTCGTTG CCAGTGTTCG 40

(2) INFORMATION FOR SEQ ID NO:262:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:262:
GGGATGGATC TTCGGATACG TCAACCAAAG GTTGCCAGCG 40

(2) INFORMATION FOR SEQ ID NO:263:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:263:
GCGCATCGTA AAAAGGACAA ACGTCGTCGT GACCCCGATA 40

(2) INFORMATION FOR SEQ ID NO:264:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:264:
GTGCATCGTA AAAAGGACAA ACGTCGTCGT GACCCCGATA 40

(2) INFORMATION FOR SEQ ID NO:265:

(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:265:
AGACGGTGAA ACTGAAATCT AATCCGTCTG AACCCTGGAT 40

(2) INFORMATION FOR SEQ ID NO:266:
(i) SEQUENCE CHARACTERISTICS:
(A) LENGTH: 40 base pairs
(B) TYPE: nucleic acid
(C) STRANDEDNESS: single
(D) TOPOLOGY: linear
(xi) SEQUENCE DESCRIPTION: SEQ ID NO:266:
AGACGGTGAA GCTGAAATCT AATCCGTCTG AACCCTGGAC 40

(2) INFORMATION FOR SEQ ID NO:267:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:267:
GGGAGGTCAA GGACCTCACA CTTTTGTGTG CCAGCGCTAT 40

(2) INFORMATION FOR SEQ ID NO:268:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:268:
GAAAGCGTTG TGGCGTGCCA TCGCCCGCAG GCGAATAACA 40

(2) INFORMATION FOR SEQ ID NO:269:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:269:
ACGATGGGTT GTTATAGTGG AAACGGTAAG TTCGAGTCTG 40

(2) INFORMATION FOR SEQ ID NO:270:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:270:
ACGGTGATCC TCTAATCCGT CGACAGAATC GATGTCAATC 40

(2) INFORMATION FOR SEQ ID NO:271:
(i) SEQUENCE CHARACTERISTICS:
(A) LENGTH: 87 base pairs
(B) TYPE: nucleic acid
(C) STRANDEDNESS: single
(D) TOPOLOGY: linear

(xi) SEQUENCE DESCRIPTION: SEQ ID NO:271:
GCCGGATCCG GGCCTCATGT CGAANNNNNN NNNNNNNNNN NNNNNNNNNN 50
NNNNNNNNNN NNNNTTGAGC GTTTATTCTG AGCTCCC 87

(2) INFORMATION FOR SEQ ID NO:272:
(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:272:
CCGAAGCTTA ATACGACTCA CTATAGGGAG CTCAGAATAA ACGCTCAA 48

(2) INFORMATION FOR SEQ ID NO:273:
(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:273:
GCCGGATCCG GGCCTCATGT CGAA 24

(2) INFORMATION FOR SEQ ID NO:274:
(i) SEQUENCE CHARACTERISTICS:
(A) LENGTH: 87 base pairs
(B) TYPE: nucleic acid
(C) STRANDEDNESS: single
(D) TOPOLOGY: linear

(xi) SEQUENCE DESCRIPTION: SEQ ID NO:274:
CCCCTGCAGG TGATTTTGCT CAAGTNNNNN NNNNNNNNNN NNNNNNNNNN 50
NNNNNNNNNN NNNNNAGTAT CGCTAATCAG GCGGATC 87

(2) INFORMATION FOR SEQ ID NO:275:
(i) SEQUENCE CHARACTERISTICS:
(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:275:
CCGAAGCTTA ATACGACTCA CTATAGGGAT CCGCCTGATT AGCGATACT 49

(2) INFORMATION FOR SEQ ID NO:276:
(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:276:
CCCCTGCAGG TGATTTTGCT CAAGT 25

(2) INFORMATION FOR SEQ ID NO:277:
(i) SEQUENCE CHARACTERISTICS:
(A) LENGTH: 51 base pairs
(B) TYPE: nucleic acid
(C) STRANDEDNESS: single
(D) TOPOLOGY: linear

(xi) SEQUENCE DESCRIPTION: SEQ ID NO:277:
AACGCUCAAU ACCAUCGUGU AAGAAAGAGC ACGACCUUGG CAGUGUGUGU 50
U 51

(2) INFORMATION FOR SEQ ID NO:278:
(i) SEQUENCE CHARACTERISTICS:
(A) LENGTH: 42 base pairs
(B) TYPE: nucleic acid

(C) STRANDEDNESS: single
(D) TOPOLOGY: linear

(xi) SEQUENCE DESCRIPTION: SEQ ID NO:278:
AAGUGAUACC AGCAUCGUCU UGAUGCCCUU GGCAGCACUU CA 42

(2) INFORMATION FOR SEQ ID NO:279:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:279:
GAUACCAACA GCAUAUUUGC UGUCCUUGGA AGCAACGAGA 40

(2) INFORMATION FOR SEQ ID NO:280:
(i) SEQUENCE CHARACTERISTICS:
(A) LENGTH: 42 base pairs
(B) TYPE: nucleic acid
(C) STRANDEDNESS: single
(D) TOPOLOGY: linear

(xi) SEQUENCE DESCRIPTION: SEQ ID NO:280:
GAGAAAUACC AGUGACAACU CUCGAGAUCA CCCUUGGAAG UU 42

(2) INFORMATION FOR SEQ ID NO:281:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:281:
UAUCGAGUGG CCUUGGCAGA CCAGGCCGGG UAUACCACCA 40

(2) INFORMATION FOR SEQ ID NO:282:
(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:282:
CGAGAUUCAA CCUUGGAAGU CAAUCGUGAA UACCAUUGUU 40

(2) INFORMATION FOR SEQ ID NO:283:
(i) SEQUENCE CHARACTERISTICS:
(A) LENGTH: 45 base pairs
(B) TYPE: nucleic acid
(C) STRANDEDNESS: single
(D) TOPOLOGY: linear

(xi) SEQUENCE DESCRIPTION: SEQ ID NO:283:
UGACUCGAAC CCUUGGAAGA CCUGAGUACA GGUAUACCAG UUCGA 45

(2) INFORMATION FOR SEQ ID NO:284:
(i) SEQUENCE CHARACTERISTICS:
(A) LENGTH: 47 base pairs
(B) TYPE: nucleic acid
(C) STRANDEDNESS: single
(D) TOPOLOGY: linear

(xi) SEQUENCE DESCRIPTION: SEQ ID NO:284:
CGCUCAAUCC UUGGAAGCCG UACGGAUACC AAUUGAGUGG CCAUAUG 47

(2) INFORMATION FOR SEQ ID NO:285:
(i) SEQUENCE CHARACTERISTICS:

(A) LENGTH: 28 base pairs
(B) TYPE: nucleic acid
(C) STRANDEDNESS: single
(D) TOPOLOGY: linear

(xi) SEQUENCE DESCRIPTION: SEQ ID NO:285:
NNAUACCANN NNNNNNNCCU UGGMAGNN 28

(2) INFORMATION FOR SEQ ID NO:286:
(i) SEQUENCE CHARACTERISTICS:
(A) LENGTH: 38 base pairs
(B) TYPE: nucleic acid
(C) STRANDEDNESS: single
(D) TOPOLOGY: linear

(xi) SEQUENCE DESCRIPTION: SEQ ID NO:286:
GGUGAUACCA GCAUCGUCUU GAUGCCCUUG GCAGCACC 38

## Description

## FIELD OF THE INVENTION

Described herein are high affinity nucleic acid ligands to vascular endothelial growth factor (VEGF) human neutrophil elastase, theophylline, caffeine and thrombin. The method utilized herein for identifying such nucleic acid ligands is called SELEX, an acronym for Systematic Evolution of Ligands by EXponential enrichment. Further described herein are methods for identifying highly specific nucleic acid ligands able to discriminate between closely related molecules, termed "counter-SELEX", methods for combining nucleic acids with other functional units for generation of high affinity ligands, termed "blended SELEX" and methods for preparing modified oligonucleotides capable of binding target molecules with high affinity. Specifically disclosed herein are high-affinity nucleic acid ligands to VEGF, elastase, caffeine and theophylline, including single-stranded RNA and DNA ligands, single-stranded RNA ligands able to discriminate between theophylline and caffeine and RNA ligands to thrombin containing 2'-$NH_2$-modifications.

The modified oligonucleotides of the present invention contain one or more modified nucleotide bases, which include 5-X and/or 2'-Y substitutions in pyrimidine bases and 8-X and/or 2'Y substitutions in purine bases. The invention includes nuclease-resistant oligonucleotide ligands containing the modified nucleotides of the present invention. The invention further includes methods for synthesizing the substituted nucleotides, bases and intermediates described herein. The oligonucleotides of the present invention are modified by incorporation of chemically-modified nucleotide derivatives. The nucleotide derivatives incorporated into the oligonucleotides of the present invention also introduce means for incorporating additional functional groups into the nucleic acid ligands. This invention includes modified high affinity nucleic acid ligands which are single-stranded DNA and RNA ligands.

Specific examples are provided of oligonucleotides containing nucleotide derivatives chemically modified at the 5- and 2'- positions of pyrimidine. Further disclosed are specific RNA ligands to thrombin containing $2^1$-$NH_2$-modifications.

The modified oligonucleotides of the present invention increase the chemical diversity of the candidate mixture for the SELEX process, producing improved nucleic acid ligands to specific target molecules. In many cases, the modifications also provide the oligonucleotide with increased relative resistance to endonucleases in serum. The modified oligonucleotides of the present invention are useful as pharmaceuticals, diagnostic agents, and as part of gene therapy treatments.

The blended nucleic acid ligands of the present invention consist of at least one nucleic acid ligand unit and at least one functional unit. Examples of functional units that may be coupled to nucleic acids include proteins, peptides, photoreactive groups, chemically-reactive groups, active site directed compounds, lipids, biotin and fluorescent compounds.

The nucleic acid ligand unit(s) of the blended nucleic acid ligand serve in whole or in part as ligands to a given target. The functional unit(s) can be designed to serve in a large variety of functions. For example, the functional unit may independently or in combination with the nucleic acid ligand have specific affinity for the target, and in some cases may be a ligand to a different site of interaction with the target than the nucleic acid ligand. The functional unit(s) may be added which covalently react and couple the ligand to the target molecule, catalytic groups may be added to aid in the selection of protease or nuclease activity, and reporter molecules such as biotin- or fluorescence-tagged oligonucleotides may be added for use as diagnostic reagents.

## BACKGROUND OF THE INVENTION

The SELEX method (hereinafter termed SELEX), was first described in U.S. Application Serial No. 07/536,428, filed June 11, 1990, entitled Systematic Evolution of Ligands By Exponential Enrichment, now abandoned. U.S. Patent Application Serial No. 07/714,131, filed June 10, 1991 and U.S. Patent Application Serial No. 07/931,473, filed August 17, 1992, now U.S. Patent No. 5,270,163, both entitled Nucleic Acid Ligands, further disclose the basic SELEX process. (See also PCT/US91/04078). Each of these applications are herein specifically incorporated by reference. The SELEX process provides a class of products which are referred to as nucleic acid ligands, such ligands having a unique sequence, and which have the property of binding specifically to a desired target compound or molecule. Each SELEX-identified nucleic acid ligand is a specific ligand of a given target compound or molecule. SELEX is based on the unique insight that nucleic acids have sufficient capacity for forming a variety of two- and three-dimensional structures and sufficient chemical versatility available within their monomers to act as ligands (form specific binding pairs) with virtually any chemical compound, whether monomeric or polymeric. Molecules of any size can serve as targets.

The SELEX method involves selection from a mixture of candidates and step-wise iterations of binding, partitioning, and amplification, using the same general selection theme, to achieve virtually any desired criterion of binding affinity and selectivity. Starting from a mixture of nucleic acids, preferably comprising a segment of randomized sequence, the method includes steps of contacting the mixture with the target under conditions favorable for binding, partitioning unbound nucleic acids from those nucleic acids which have bound to target molecules, dissociating the nucleic acid-target pairs, amplifying the nucleic acids dissociated from the nucleic acid-target pairs to yield a ligand-enriched mixture of nucleic acids, then reiterating the steps of binding, partitioning, dissociating and amplifying through as many cycles as desired. A variety of techniques can be used to partition members in the pool of nucleic acids that have a higher affinity to the target than the bulk of the nucleic acids in the mixture.

While not bound by theory, SELEX is based on the inventors' insight that within a nucleic acid mixture containing a large number of possible sequences and structures there is a wide range of binding affinities for a given target. A nucleic acid mixture comprising, for example, a 20 nucleotide randomized segment, can have $4^{20}$ candidate possibilities. Those which have the higher affinity constants for the target are most likely to bind to the target. After partitioning, dissociation and amplification, a second nucleic acid mixture is generated, enriched for the higher binding affinity candidates. Additional rounds of selection progressively favor the best ligands until the resulting nucleic acid mixture is predominantly composed of only one or a few sequences. These can then be cloned, sequenced and individually tested for binding affinity as pure ligands.

Cycles of selection, partition and amplification are repeated until a desired goal is achieved. In the most general case, selection/partition/amplification is continued until no significant improvement in binding strength is achieved on repetition of the cycle. The method may be used to sample as many as about $10^{18}$ different nucleic acid species. The nucleic acids of the test mixture preferably include a randomized sequence portion as well as conserved sequences necessary for efficient amplification. Nucleic acid sequence variants can be produced in a number of ways including synthesis of randomized nucleic acid sequences and size selection from randomly cleaved cellular nucleic acids. The variable sequence portion may contain a fully or partially random sequence; it may also contain subportions of conserved sequence incorporated with randomized sequence. Sequence variation in test nucleic acids can be introduced or increased by mutagenesis before or during the selection/partition/amplification iterations.

In one embodiment of the method of the SELEX Patent Applications, the selection process is so efficient at isolating those nucleic acid ligands that bind most strongly to the selected target, that only one cycle of selection and amplification is required. Such an efficient selection may occur, for example, in a chromatographic-type process wherein the ability of nucleic acids to associate with targets bound on a column operates in such a manner that the column is sufficiently able to allow separation and isolation of the highest affinity nucleic acid ligands.

In many cases, it is not necessarily desirable to perform the iterative steps of SELEX until a single nucleic acid ligand is identified. The target-specific nucleic acid ligand solution may include a family of nucleic acid structures or motifs that have a number of conserved sequences and a number of sequences which can be substituted or added without significantly affecting the affinity of the nucleic acid ligands to the target. By terminating the SELEX process prior to completion, it is possible to determine the sequence of a number of members of the nucleic acid ligand solution family.

A variety of nucleic acid primary, secondary and tertiary structures are known to exist. The structures or motifs that have been shown most commonly to be involved in non-Watson-Crick type interactions are referred to as hairpin loops, symmetric and asymmetric bulges, pseudoknots and myriad combinations of the same. Almost all known cases of such motifs suggest that they can be formed in a nucleic acid sequence of no more than 30 nucleotides. For this reason, it is often preferred that SELEX procedures with contiguous randomized segments be initiated with nucleic acid sequences containing a randomized segment of between about 20-50 nucleotides.

The basic SELEX method may be modified to achieve specific objectives. For example, U.S. Patent Application Serial No. 07/960,093, filed October 14, 1992, entitled Method for Selecting Nucleic Acids on the Basis of Structure, describes the use of SELEX in conjunction with gel electrophoresis to select nucleic acid molecules with specific structural characteristics, such as bent DNA. U.S. Patent Application Serial No. 08/123,935, filed September 17, 1993, entitled Photoselection of Nucleic Acid Ligands, describes a SELEX based method for selecting nucleic acid ligands containing photoreactive groups capable of binding and/or photocrosslinking to and/or photoinactivating a target molecule. U.S. Patent Application Serial No. 08/143,564, filed October 25, 1993, entitled Systematic Evolution of Ligands by EXponential Enrichment: Solution SELEX, describes a SELEX-based method which achieves highly efficient partitioning between oligonucleotides having high and low affinity for a target molecule *(See* also PCT/US93/09296).

The SELEX Patent Applications describe methods for obtaining nucleic acid ligands that bind to more than one site on the target molecule, and to nucleic acid ligands that include non-nucleic acid species that bind to specific sites on the target. The SELEX method provides means for isolating and identifying nucleic acid ligands which bind to any envisionable target. However, in preferred embodiments the SELEX method is applied to situations where the target is a protein, including both nucleic acid-binding proteins and proteins not known to bind nucleic acids as part of their biological function. For example, U.S. Patent Application Serial No. 08/061,691, filed April 22, 1993, entitled High-Affinity RNA Ligands of Basic Fibroblast Growth Factor (bFGF), discloses RNA ligand inhibitors of bFGF and U.S. Patent Application Serial No. 07/973,333, filed November 6, 1992, entitled Ligands of Thrombin, herein specifically incorporated by reference, describes nucleic acid ligands to thrombin.

Basic fibroblast growth factor (bFGF) is a multifunctional effector for many cells of mesenchymal and neuroectodermal origin (Rifkin & Moscatelli (1989) J. Cell Biol. 109:1; Baird & Bohlen (1991) in Peptide Growth Factors and Their Receptors (Sporn, M. B. & Roberts, A. B., eds.); pp. 369-418, Springer, N.Y.; Basilico & Moscatelli (1992) Adv. Cancer Res. 59:115). It is one of the most studied and best characterized members of a family of related proteins that also includes acidic FGF (Jaye et al. (1986) Science 233:541; Abraham et al. (1986) Science 233:545), int-2 (Moore et al. (1986) EMBO J. 5:919), kFGF/hst/KS3 (Delli-Bovi et al. (1987) Cell 50:729; Taira et al. (1987) Proc. Natl. Acad. Sci. USA 84:2980), FGF-5 (Zhan et al. (1988) Mol. Cell. Biol. 8:3487), FGF-6 (Marics et *al*. (1989) Oncogene 4:335) and keratinocyte growth factor/FGF-7 (Finch et *al*. (1989) Science 245:752).

In vitro, bFGF stimulates cell proliferation, migration and induction of plasminogen activator and collagenase activities (Presta et *al.* (1986) Mol. Cell. Biol. 6:4060; Moscatelli et *al.* (1986) Proc. Natl. Acad. Sci. USA 83:2091; Mignatti *et al.* (1989) J. Cell Biol. 108:671). In vivo, it is one of the most potent inducers of neovascularization. Its angiogenic activity in vivo suggests a role in tissue remodeling and wound healing, as well as, in some disease states that are characterized by pathological neovascularization such as tumor proliferation, tumor metastasis, diabetic retinopathy and rheumatoid arthritis (Folkman & Klagsbrun (1987) Science 235:442; Gospodarowitz (1991) Cell Biology Reviews 25:307).

bFGF is also known to play a key role in the development of smooth-muscle cell lesions following vascular injury (Reidy et al. Circulation, Suppl. III 86:III-43). Over expression of bFGF (and other members of the FGF family) is correlated with many malignant disorders (Halaban et al. (1991) Ann. N. Y. Acad. Sci. 638:232; Takahashi et al. (1990) Proc. Natl. Acad. Sci. USA 87:5710; Fujimoto et al. (1991) Biochem. Biophys. Res. Commun. 180:386). Recently, neutralizing anti-bFGF antibodies have been found to suppress solid tumor growth in vivo by inhibiting tumor-linked angiogenesis (Hori et al. (1991) Cancer Res. 51:6180). Notable in this regard is the recent therapeutic examination of suramin, a polysulfonated naphthalene derivative with known antiprotozoal activity, as an anti-tumor agent. Suramin is believed to inhibit the activity of bFGF through binding in the polyanion binding site and disrupting interaction of the growth factor with its receptor (Middaugh et *al.* (1992) Biochemistry 31:9016; Eriksson et *al.* (1992) Proc. Natl. Acad. Sci. USA 88:3441). In addition to having a number of undesirable side effects and substantial toxicity, suramin is known to interact with several other heparin-binding growth factors which makes linking of its beneficial therapeutic effects to specific drug-protein interactions difficult (La Rocca et al. (1990) Cancer Cells 2:106). Anti-angiogenic properties of certain heparin preparations have also been observed (Folkman et *al*. (1983) Science 221:719; Crum et *al*. (1985) Science 250:1375) and these effects are probably based at least in part on their ability to interfere with bFGF signaling. While the specific heparin fraction that contributes to bFGF binding is now partially elucidated (Ishai-Michaeli et *al.* (1992) Biochemistry 31:2080; Turnbull et *al*. (1992) J. Biol. Chem. 267:10337), a typical heparin preparation is heterogeneous with respect to size, degree of sulfonation and iduronic acid content. Additionally, heparin also affects many enzymes and growth factors. Thus, apart from monoclonal antibodies, specific antagonists of bFGF are not known.

Thrombin is a multifunctional serine protease that has important procoagulant and anticoagulant activities. As a procoagulant enzyme thrombin cleaves fibrinogen, activates clotting factors V, VIII, and XIII, and activates platelets. The specific cleavage of fibrinogen by thrombin initiates the polymerization of fibrin monomers, a primary event in blood clot formation. The central event in the formation of platelet thrombi is the activation of platelets from the "nonbinding" to the "binding" mode and thrombin is the most potent physiologic activator of platelet aggregation (Berndt and Phillips (1981) in Platelets in Biology and Pathology, J.L. Gordon, ed. (Amsterdam: Elsevier/North Holland Biomedical Press), pp. 43-74; Hansen and Harker (1988) Proc. Natl. Acad. Sci. USA 85:3184-3188; Eidt et al. (1989) J. Clin. Invest. 84: 18-27). Thus, as a procoagulant, thrombin plays a key role in the arrest of bleeding (physiologic hemostasis) and formation of vasoocclusive thrombi (pathologic thrombosis).

As an anticoagulant thrombin binds to thrombomodulin (TM), a glycoprotein expressed on the surface of vascular endothelial cells. TM alters substrate specificity from fibrinogen and platelets to protein C through a combination of an allosteric change in the active site conformation and an overlap of the TM and fibrinogen binding sites on thrombin. Activated protein C, in the presence of a phospholipid surface, $Ca^{2+}$, and a second vitamin K-dependent protein cofactor, protein S, inhibits coagulation by proteolytically degrading factors Va and VIIIa. Thus, the formation of the thrombin-TM complex converts thrombin from a procoagulant to an anticoagulant enzyme, and the normal balance between these opposing activities is critical to the regulation of hemostasis. It is therefore of interest to produce a high affinity nucleic acid ligand of thrombin capable of inhibiting its anticoagulant activity.

Integrin gpIIbIIIa is a protein expressed on activated platelets which mediate platelet adhesion to fibrinogen and fibrin clots (Phillips et al. (1988) Blood 71:831; Frojmovic et al. (1991) Blood 78:369). Members of the integrin superfamily of cell adhesion receptors, including gpIIbIIIa, are known to recognize the peptide arginine-glycine-aspartic acid sequence (RGD). When gpIIbIIIa binds a RGD-containing ligand, a signal is generated which triggers platelet granule release, shape change, aggregation and adhesion (Loftus et *al.* (1990) Science 249:915; Ware et *al*. (1993) N. Eng. J. Med. 328:628). Inhibitors of gpIIbIIIa-mediated platelet clot formation may have therapeutic potential in a variety of vascular diseases including reducing the occurrence of heart attacks following angioplasty.

Currently known integrin inhibitors are limited by a lack of specificity. The development of a high specificity integrin inhibitor, which does not cross react with other integrin proteins, would have significant therapeutic potential.

Human neutrophil elastase (hereinafter referred to as elastase), is a major protein stored in the azurophilic granules of human polymorphonuclear granulocytes (Dewald et *al*. (1975) J. Exp. Med. 141:709) that is secreted upon inflammatory stimuli (Bonney et *al*. (1989) J. Cell. Biochem. 39:47). Elastase is a serine protease with broad substrate specificity able to digest many macromolecules found in connective tissues. For example, elastase can hydrolyze macromolecules such as elastin, type III and type IV collagen, and fibronectin. In addition to connective tissue components, many plasma proteins such as immunoglobulins, clotting factors and complement proteins can also be hydrolyzed by elastase.

Elastase is a single-chain glycoprotein 218 amino acids in length (Sinha et *al.* (1987) Proc. Natl. Acad. Sci. USA 84:2228). Elastase has two N-glycosylation sites at positions Asn-95 and Asn-144. Its molecular weight is about 29,500 daltons, and its isoelectric point (pI) lies between 8-9. The crystal structure of elastase complexed with an inhibitor has been determined by Navia et *al*. (1989) Proc. Natl. Acad. Sci. USA 86:7.

Several natural proteinase inhibitors have been shown to be effective in regulating the activity of elastase. Human α-1-proteinase inhibitor (α-1-PI) is the primary protease inhibitor found in plasma (Heimburger et *al.* (1970) in Proceedings of the First International Research Conference on Proteinase Inhibitors, Walter de Gruyter, New York, pp 1-21). In some individuals, α-1-PI is present in unusually low amounts due to an underlying genetic defect; this low level causes familial emphysema (Garver et *al.* (1986) N. Engl. J. Med. 314:762). In other cases, the inhibitor is nonfunctional due to oxidative destruction by cigarette smoke (Janus et *al.* (1985) Lancet i:152). Secretory leucocyte proteinase inhibitor (SLPI) is another elastase inhibitor found in all mucous secretions (Thompson & ohlsson (1986) Proc. Natl. Acad. Sci. USA 83:6692) which is believed to be the major elastase inhibitor present in the upper airways of the lung. Recently, another natural elastase inhibitor, elafin, was characterized from human skin (Wiedow et *al.* (1990) J. Biol. Chem. 265:14791).

An excess of elastase activity has been implicated in several diseases, including pulmonary emphysema (Kalpan et *al*. (1973) J. Lab. Clin. Med. 82:349; Powers (1983) Am. Rev. Respir. Dis. 127:554), cystic fibrosis, rheumatoid arthritis (Barrett (1978) Agents and Actions 8:1), chronic bronchitis, bronchopulmonary dysplasia in premature infants, and adult respiratory distress syndrome (ARDS) (Weiland et al. (1986) Am. Rev. Respir. Dis. 133:218). In most cases, the pathogenesis of these diseases has been correlated with the inactivation or the insufficiency of natural inhibitors of elastase, whose primary role is to keep excess enzyme activity under control.

The development of elastase-specific inhibitors has been a major goal in the pharmaceutical industry for some time. As a result, different types of inhibitors have been developed. These include irreversible inhibitors such as peptide chloromethyl ketones (Powers et al. (1977) Biochim. Biophys. Acta 484:156), reversible inhibitors such as peptide boronic acids (Stone et *al.* (1990) Am. Rev. Respir. Dis. 141:47), cephalosporins (Doherty et *al*. (1986) Nature 322:192), and peptide aldehydes (Kennedy et *al.* (1987) Eur. J. Respir. Dis. 71:472). However, many of these inhibitors are nonspecific inhibitors of other serine proteases as well (Hemmi *et al*. (1985) Biochemistry 24:1841). Moreover, some of the more specific peptide-based inhibitors of elastase that have been developed suffer from a lack of oral availability (Williams et *al.* (1991) Am rev. Respir. Dis. 144:875). Some of the β-lactam inhibitors have both stability problems and lack oral availability (Knight et al. (1992) Biochemistry 31:4980). In addition to synthetic inhibitors, biosynthetically derived naturally occurring inhibitors such as α-1-proteinase (Gadek et *al.* (1981) J. Clin. Invest. 68:1158), eglin C (Snider et *al.* (1985) Am. Rev. Respir. Dis. 132:1155), and SLPI (Gauthier et *al*. (1982) Biochim. Biophys. Acta 700:178; Gast et *al.* (1990) Am. Rev. Respir. Dis. 141: 889) are under development.

Polynucleotides including synthetic RNA homopolymers (Simon et al. (1988) Exp. Lung Res. 14:85), tRNAs and DNAs (Lestienne & Bieth (1983) Biochimie 65:49) have been shown to inhibit elastase to some extent. The enzyme inhibition caused by these polyanions is not likely due to simple electrostatic interactions, because other polyanions lacking hydrophobic constituents such as heparin and polyanionic polysaccharides have been shown to be ineffective inhibitors. Cell extracts of certain pneumococcal species (Pneumococci type I, type II smooth, and type II rough) yield high molecular weight RNAs upon autolysis that act as elastase inhibitors (Vered et al. (1988) Exp. Lung Res. 14:67).

The development of high affinity ligands capable of inhibiting elastase would be useful in the treatment of diseases such as pulmonary emphysema (Kalpan et al. (1973) J. Lab. Clin. Med. 82:349; Powers (1983) Am. Rev. Respir. Dis. 127:554), cystic fibrosis, rheumatoid arthritis (Barrett (1978) Agents and Actions 8:1), chronic bronchitis, bronchopulmonary dysplasia in premature infants, and adult respiratory distress syndrome (ARDS) (Weiland et *al*. (1986) Am. Rev. Respir. Dis. 133:218).

Vascular endothelial growth factor (VEGF), like bFGF, is a protein capable of inducing neovascularization or angiogenesis in vivo. Neovascularization or angiogenesis is the process in which sprouting new blood vessels are formed from the existing endothelium in response to external stimuli that signal inadequate blood supply. Angiogenesis is generally rare under normal physiological conditions, but frequently accompanies certain pathological conditions such as psoriasis, rheumatoid arthritis, hemangioma, solid tumor growth and metastasis (Folkman & Klagsbrun, 1987) Science 235, 442-447; Kim et *al.,* (1993) Nature 362, 841-844). To date, several growth factors, capable of inducing angiogenesis in vivo have been identified including, basic and acidic fibroblast growth factors (aFGF, bFGF), transforming growth factors α and β (TGFα, TGFβ), platelet derived growth factor (PDGF), angiogenin, platelet-derived endothelial cell growth factor (PD-ECGF), interleukin-8 (IL-8), and vascular endothelial growth factor (VEGF).

VEGF was originally purified from guinea pig ascites and tumor cell cultures as a factor that increases vascular permeability (Senger, D.R. et al. (1983), Science 219:983-985) and it has therefore also been referred to as vascular permeability factor (VPF). VEGF is a heat and acid-stable, disulfide-linked homodimer. Four isoforms have been described (121, 165, 189 and 206 amino acids, respectively) and are believed to be the result of alternative splicing of mRNA. Despite the presence of an identical N-terminal hydrophobic signal sequence in all molecular isoforms of VEGF, only the two lower molecular weight species are efficiently secreted (Ferrara, N. et *al*. (1991) J. Cell. Biochem. 47: 211-218). The predominant VEGF isoform in most cells and tissues is the 165 amino acid species. Although VEGF is typically glycosylated, glycosylation is required only for efficient secretion, not for activity (Yeo, T-.K. et *al.* (1991) Biochem. Biophys. Res. Commun. 179:1568-1575; Peretz, D. et *al.* (1992) Biochem. Biophys. Res. Commun. 182:1340-1347). The amino acid sequence of VEGF is highly conserved across species and exhibits a modest but significant homology (18-20%) to PDGF A and B (Jakeman L.B. et *al.* (1992) J. Clin. Invest. 89:244-253; Ferrara et *al.* (1992) Endocrine Rev. 13:18-32).

Unlike other angiogenic growth factors, target cell specificity of VEGF is limited to vascular endothelial cells. The biological actions of VEGF are mediated through its interaction with specific cell-associated receptors which have been identified in the majority of tissues and organs (Jakeman, L.B. (1992) J. Clin. Invest. 89:244-253). Three high-affinity receptors for VEGF have been cloned to date: flt1, kdr/flk-1 and flt4 (Vaisman, N. et *al.* (1990) J. Biol. Chem. 265:19461-19466; de Vries, C. et *al.* (1992) Science 255:989-991; Galland, F. et *al*. (1993) Oncogene 8:1233-1240). These receptors belong to a family of transmembrane tyrosine kinases and bind VEGF with dissociation constants between $10^{-11}$ M to $10^{-12}$ M. Recent experiments have shown that binding of VEGF to its high-affinity receptors is significantly enhanced by heparin or cell surface-associated heparin-like molecules (Gitay-Goren, H. (1992) J. Biol. Chem. 267:6093-6098).

In addition to promoting the growth of vascular endothelial cells and inducing vascular leakage, VEGF is also known to induce the proteolytic enzymes interstitial collagenase, urokinase-type plasminogen activator (uPA) and tissue-type plasminogen activator (tPA) (Unemori E. et *al*. (1993) J. Biol. Chem. in press; Pepper, M.S. et al. (1992) Biochem. Biophys. Res. Commun. 181:902).
These enzymes are known to play a prominent role in angiogenesis-related extracellular matrix degradation.

As a secreted and specific mitogen for endothelial cells, VEGF may be one of the major angiogenesis inducers in vivo. Several recent observations have supported this notion. For example, the expression of VEGF and its receptors accompanies angiogenesis associated with (i) embryonic development (Breier, G. et al. (1992) Development 114: 521-532); (ii) hormonally-regulated reproductive cycle; and (iii) tumor growth (Dvorak, H.F. (1991) J. Exp. Med. 174: 1275-1278; Shweiki, D. et al. (1992) Nature 359:843-845; Plate, K.H. et al. (1992) Nature 359:845-848). It is relevant to note that aggressive tumor growth is accompanied by the generation of necrotic areas where oxygen and nutrient supplies are inadequate. Oxygen deprivation (hypoxia) in tissues is a known angiogenesis stimulant. Interestingly, VEGF expression was found to be the highest in tumor cells facing the necrotic areas (Shweiki, D. et *al*. (1992) Nature 359: 843-845; Plate, K.H. et *al*. (1992) Nature 359:845-848). It has therefore been suggested that VEGF plays a key role in hypoxia-induced angiogenesis.

Recent experiments with neutralizing monoclonal antibodies (MAbs) to VEGF have been especially meaningful for establishing that this growth factor is an important tumor angiogenesis inducer in vivo (Kim, K.J. et *al.* (1993) Nature 362:841-844). Immunocompromized (nude) mice injected with human rhabdomyosarcoma, glioblastoma or leiomyosarcoma cell lines rapidly develop tumors. Specific neutralizing MAbs to VEGF were found to inhibit the growth of these tumors. The density of tumor vasculature was decreased in MAb-treated animals as compared to controls. The same MAb, on the other hand, had no effect on the growth rate of the tumor cells in vitro suggesting that the growth inhibition was not mediated at the cellular level and appears to be mediated by the 165-amino acid isoform of VEGF.

Theophylline (1,3-dimethylxanthine)(Figure 47) is a naturally occurring alkaloid that is widely used as an effective bronchodilator in the treatment of asthma, bronchitis, and emphysema (Hendeles and Weinberger (1983) Pharmacotherapy 3:2). Because of its narrow therapeutic index, serum levels must be monitored carefully to avoid serious toxicity. Theophylline is closely related structurally to caffeine (1,3,7-trimethylxanthine)(Figure 47) and theobromine (3,7-dimethylxanthine), both of which are often present in serum samples. Analytical diagnostic techniques utilizing spectroscopic characteristics or immunological reagents must therefore discriminate efficiently among these and other alkaloids (Jolley et al. (1981) Clin. Chem. 27:1575; Broussard (1981) Clin. Chem. 27:1931; Reinecke et al. (1986) Ann. Emergency Med. 15:147).

Native oligonucleotides are sensitive to degradation by nucleases. Two kinds of ribonucleases are known. The first, termed exonucleases, degrade an oligoribonucleotide sequentially from either the 3'- or the 5'-end. Exonucleases cleave the phosphodiester chain through catalyzing direct hydrolysis, with water as the attacking agent.

The more prevailing mode of degradation of oligoribonucleotides proceeds through catalysis by endonucleases. Endonucleases cleave RNA within the chain, 3' to the specific base they recognize. The mechanism of cleavage involves activation of the 2'-hydroxyl (2'-OH) to attack the phosphorous of the internucleotidic linkage (Saenger (1984) in: Principles of Nucleic Acid Structure, Springer Verlag, New York, p. 174). This initial step leads to chain cleavage with formation of the 2',3'-cyclic phosphate end on the 5'-product and a free 5'-OH end on the 3'-product. The major degradation of oligoribonucleotides in serum proceeds through pyrimidine-specific endonuclease (Pieken et al. (1990) Science 253: 314).

The resistance of 2'-amino, 2'-deoxy pyrimidine homopolymers to degradation by pancreatic ribonuclease (RNAse A) has been reported. Both poly(2'-amino,2'-deoxyuridine) and poly(2'-amino,2'-deoxycytidine) are essentially completely stable towards RNAse A degradation. As expected, these polymers are readily degraded by snake venom phosphodiesterase, an enzyme that catalyzes water-hydrolysis of the phosphodiester backbone. The stability of 2'-amino,2'-deoxy pyrimidine containing oligonucleotides in rabbit serum is reported to be 1200-fold increased compared to unmodified oligoribonucleotides (Pieken et *al.* (1990) Science 253:314). This technology has been applied to the preparation of nuclease resistant hammerhead ribozymes (PCT Patent Application Publication WO 92/07065).

Oligonucleotides modified so as to exhibit resistance to nucleases are known to the art. For example, Ikehara et al. (1984) Eur. J. Biochem. 139:447 reported the synthesis of a mixed octamer containing a 2'-deoxy-2'-fluoroguanosine residue or a 2'-deoxy-2'-fluoroadenine residue. Ikehara et al. (1978) Nucleic Acids Res. 5:3315, showed that a 2'-chloro or bromo substituent in poly(2'-deoxyadenylic acid) provided nuclease resistance. Eckstein et *al.* (1972) Biochemistry 11:4336, showed that poly(2'-chloro-2'-deoxyuridylic acid) and poly(2'-chloro-2'-deoxycytidylic acid) are resistant to various nucleases. Inoue et *al.* (1987) Nucleic Acids Res. 15:6131, described the synthesis of mixed oligonucleotide sequences containing 2'-$OCH_3$ at every nucleotide unit. The mixed 2'-$OCH_3$ substituted sequences hybridized to their RNAs as strongly at the non-substituted RNAs. Shibahara et *al.* (1987) Nucleic Acids Res. 17:239, also described the synthesis of mixed oligonucleotide sequences containing 2'-$OCH_3$ at every nucleotide unit.

The stability of oligoribonucleotides against endonuclease degradation may be achieved by replacement of the 2'-OH group of the ribose moiety with an alternate substituent such as an amino group or a fluoro group (Pieken et *al.* (1990) Science 253:314). Both 2'-amino and 2'-fluoro nucleoside 5-triphosphates are substrates for T7 RNA polymerase, albeit with somewhat decreased incorporation efficiency (Aurup et *al*. (1992) Biochemistry 31:9636). Other 2'-substituted nucleotides such as 2'-O-methyl, 2'-O-alkyl, or 2'-deoxy nucleoside 5-triphosphates are not recognized as substrates by T7 RNA polymerase.

The 2'-amino,2'-deoxy pyrimidine nucleosides have been prepared previously (Verheyden et al. (1971) J. Org. Chem. 36:250; U.S. Patent No. 3,755,295, issued August 28, 1973). However, the reported method for preparation of the crucial 2'-azido,2'-deoxyuridine precursor is laborious and time consuming, with only moderate yields.

## SUMMARY OF THE INVENTION

The present invention includes methods for identifying and producing nucleic acid ligands using the SELEX procedure. The invention also includes the nucleic acid ligands so identified and produced. The SELEX method described above allows for identification of a single nucleic acid ligand or a family of nucleic acid ligands to a given target. The methods of the present invention allow for the analysis of the nucleic acid ligand or family of nucleic acid ligands obtained by SELEX in order to identify and produce improved nucleic acid ligands.

In one embodiment of the present invention, nucleic acid ligands are desired for their ability to inhibit one or more of the biological activities of the target. In such cases, methods are provided for determining whether the nucleic acid ligand effectively inhibits the desired biological activity.

The present invention further includes methods for producing high affinity nucleic acid ligands which incorporate chemically-modified nucleotides. In one embodiment, SELEX is performed with a candidate mixture of oligonucleotides containing modified nucleotides. In another embodiment, SELEX is performed with a candidate mixture of oligonucleotides not containing modified nucleotides and the selected high affinity ligands are subsequently modified by incorporation of modified nucleotides. Incorporation of modified nucleotides into oligonucleotides provides means for introducing additional functional groups into the nucleic acid ligands via the modified nucleotides. The method of the present invention provides increased enrichment of the chemical diversity of a nucleic acid candidate mixture from which ligands to specific targets are identified through the SELEX process. Further, the method of the present invention provides nucleic acid ligands with increased in vivo stability relative to the non-modified ligand.

The present invention includes oligonucleotides containing one or more modified bases. The modified pyrimidine bases of the present invention have substitutions of the general formula 5-X and/or 2'-Y, and the modified purine bases have modifications of the general formula 8-X and/or 2'-Y. The group X includes the halogens I, Br, Cl, or an azide or amino group. The group Y includes an amino group, fluoro, or a methoxy group as shown in Figure 1. Other functional substitutions that would serve the same function are also included.

The oligonucleotide ligands of the present invention may have one or more X-modified bases, or one or more Y-modified bases, or a combination of X- and Y-modified bases. The present invention encompasses derivatives of these substituted pyrimidines and purines such as 5'-triphosphates, and 5'-dimethoxytrityl, 3'-b-cyanoethyl, N, N-diisopropyl phosphoramidites with isobutyryl protected bases in the case of adenosine and guanosine, or acyl protection in the case of cytosine. Further included in the present invention are oligonucleotides bearing any of the nucleotide analogs herein disclosed. The present invention encompasses specific nucleotide analogs modified at the 5 and 2' positions, including 5-(3-aminoallyl)uridine triphosphate (5-AA-UTP), 5-(3-aminoallyl) deoxyuridine triphosphate (5-AA-dUTP), 5-fluorescein-12-uridine triphosphate (5-F-12-UTP), 5-digoxygenin-11-uridine triphosphate (5-Dig-11-UTP), 5-bromouridine triphosphate (5-Br-UTP), 2'-amino-uridine triphosphate (2'-$NH_2$-UTP), 2'-amino-cytidine triphosphate (2'-$NH_2$-CTP), 2'-fluoro-cytidine triphosphate (2'-F-CTP) and 2'-fluro-uridine triphosphate (2'-F-UTP).

Further included in the present invention are nuclease-resistant oligonucleotide ligands containing the modified nucleotides of the present invention.

The present invention further includes a method for generating blended nucleic acid ligands comprised of functional unit(s) added to provide a nucleic acid ligand with additional functions. In the preferred embodiment, the functional unit provides additional affinity or a desired effect such as inhibition or induction between the blended nucleic acid ligand and the target molecule. This method for combining nucleic acids with other functional groups to use in molecular evolution is herein referred to as blended SELEX.

The method of this invention provides novel means for generating nucleic acid ligands with specifically selected functionalities. For example, high affinity ligands are generated by the method of this invention which are highly specific inhibitors of a target enzyme.

The present invention encompasses nucleic acid ligands coupled to a non-nucleic acid functional unit. In one example of the blended nucleic acid ligands generated by the method of this invention, a peptide-conjugated nucleotide was produced by coupling the peptide Gly-Arg-Gly-Asp-Thr-Pro (SEQ ID NO:40) to the derivatized base 5-(3-aminoallyl)-uridine triphosphate (RGD-UTP). RGD-UTP containing oligonucleotides (RGD-RNA) were then generated by the method of this invention and shown to bind the RGD-binding protein integrin gpIIbIIIa. It is expected that RGD-RNA is a highly specific inhibitor of gpIIbIIIa.

In another example of the blended nucleic acid ligands produced by the method of this invention, a blended nucleic acid ligand to elastase with the ability to specifically inhibit elastase activity was generated. An inhibitory peptide was coupled to a single-stranded DNA ligand to elastase and the blended nucleic acid ligand shown to specifically inhibit elastase activity. These non-limiting examples provided in the present disclosure are explanatory and exemplary of the method of the present invention.

Further included in this invention is the isolation and characterization of binding properties of a set of high-affinity RNA ligands to VEGF, identified through the SELEX method. These ligands were selected from an initial pool of about $10^{14}$ RNA molecules randomized at thirty contiguous positions. The evolved RNA ligands bind VEGF with affinities in the low nanomolar range.

Also included herein are modified RNA ligands to VEGF. Such modified RNA ligands may be prepared after the identification of 2'-OH RNA ligands or by performing SELEX using a candidate mixture of modified RNAs. For example, 2'-$NH_2$ pyrimidine RNA ligands to VEGF are described herein.

The present invention includes the method of identifying nucleic acid ligands and ligand sequences to VEGF comprising the steps of a) contacting a candidate mixture of nucleic acids with VEGF; b) partitioning between members of said candidate mixture on the basis of affinity to VEGF; and c) amplifying the increased affinity nucleic acids to yield a mixture of nucleic acids enriched for nucleic acid sequences with relatively higher affinity for binding to VEGF.

Also included herein are nucleic acid ligands to human neutrophil elastase identified through the SELEX procedure. This invention includes 2'-$NH_2$ RNA and single-stranded DNA ligands to elastase, specifically the 2'-$NH_2$ RNA sequences shown in Table 4 and the DNA sequences shown in Table 9. Also included are RNA and DNA ligands to elastase identified through the SELEX method that are substantially homologous to those shown in Tables 4 and 7 and that have substantially the same ability to bind elastase.

This invention also includes nucleic acid ligands which inhibit the biological activity of elastase.

Further included in this invention is a method of identifying nucleic acid ligands and ligand sequences to elastase comprising the steps of a) contacting a candidate mixture of single-stranded nucleic acids with elastase; b) partitioning the increased affinity nucleic acids from the remainder of the candidate mixture; and c) amplifying the increased affinity nucleic acids to yield a ligand-enriched mixture of nucleic acids, whereby nucleic acid ligands to elastase may be identified.

The present invention further includes methods for identifying and producing nucleic acid ligands of theophylline and caffeine, and the nucleic acid ligands so identified and produced.

Nucleic acid sequences identified through the SELEX process are provided that are ligands of theophylline. Specifically, RNA sequences are provided that are capable of binding with high affinity to theophylline. Included within the invention are the nucleic acid ligand sequences shown in Figure 48 (TR8 and TCT8) (SEQ ID NOS:3-10). Also included are RNA ligands to theophylline identified through the SELEX method that are substantially homologous to those shown in Figure 48 and that have substantially the same ability to bind theophylline.

Nucleic acid sequences identified through the SELEX process are provided that are ligands of caffeine. Specifically, RNA sequences are provided that are capable of binding with high affinity to caffeine. Included within the invention are the nucleic acid ligand sequences shown in Figure 54 (CR8) (SEQ ID NOS:16-27). Also included are RNA ligands to caffeine identified through the SELEX method that are substantially homologous to those shown in Figure 54 and that have substantially the same ability to bind caffeine.

Further included in this invention is an extension of the SELEX method of identifying nucleic acid ligands, termed counter-SELEX. Counter-SELEX is a method for improving the specificity of nucleic acid ligands to a target molecule by eliminating nucleic acid ligand sequences with cross-reactivity to one or more non-target molecules. Counter-SELEX is comprised of the steps of (a) contacting a candidate mixture with a target molecule, wherein nucleic acids having an increased affinity to the target relative to the candidate mixture may be partitioned from the remainder of the candidate mixture; b) partitioning the increased affinity nucleic acids from the remainder of the candidate mixture; c) contacting the increased affinity nucleic acids with one or more non-target molecules such that nucleic acid ligands with specific affinity for the non-target molecule(s) are removed; and d) amplifying the nucleic acids with specific affinity to the target molecule to yield a mixture of nucleic acids enriched for nucleic acid sequences with a relatively higher affinity and specificity for binding to the target molecule.

The present invention includes the nucleic acid ligands to theophylline identified according to the above-described counter-SELEX method, including those ligands listed in Figure 48 (TCT8). Also included are nucleic acid ligands to theophylline that are substantially homologous to any of the given ligands and that have substantially the same ability to bind theophylline.

## BRIEF DESCRIPTION OF THE FIGURES

FIGURE 1 illustrates some of the 2'- and 5-substituted nucleosides which may be utilized by the method of the present invention.

FIGURE 2 illustrates the structures of 5-allylamino-UTP (5-AA-UTP), 5-bromo-UTP (5-Br-UTP), 5-fluorescein-12-UTP (5-F-12-UTP), 5-digoxygenin-11-UTP (5-Dig-11-UTP).

FIGURE 3 shows the autoradiogram of a 15% denaturing polyacrylamide gel for transcription of an 87-mer of defined sequence. Four time points are 0.5, 1.0, 2.0, and 3.0 hours.

FIGURE 4 shows examples of side chains on 5-AA-UTP (for RNA SELEX) or 5-AA-dUTP (for DNA SELEX) that direct the binding of an oligonucleotide to a specific site of a target.

FIGURE 5 shows how a variety of functional groups can be introduced into the primary amine group of 5-AA-UTP and 5-AA-dUTP by the carbodiimide/N-hydroxy succinimide coupling.

FIGURE 6 depicts nucleotide sequences of RNA ligands isolated by SELEX for human thrombin. Each sequence is divided into 3 blocks from left to right: 1) the 5' fixed region, 2) the 30 base pair (30N) variable region, and 3) the 3' fixed region. Figure 6A depicts 2'-OH RNA ligands separated into class I and II. Figure 6B depicts 2'-$NH_2$ RNA ligands separated into groups I, II, and III.

FIGURE 7 shows proposed secondary structures of RNA ligands. Figure 7A shows the sequence of the class I 2'-OH RNA clone 16 and of the class II 2'-OH RNA clone 27.

Figure 7B shows the sequence of the group I 2'-$NH_2$ RNA clone 32, the group II sub A 2'-$NH_2$ RNA clone 37 and sub B clone 17, and the group III 2'-$NH_2$ RNA clone 29.

FIGURE 8 depicts binding curves for thrombin ligands. Figure 8A shows thrombin binding curves for nonselected 30N3 RNA (•), and for the 2'-OH RNA ligand class I clone 16 (0) and class II clone 27 (x). Figure 8B shows the thrombin binding curves for 2'-$NH_2$-30N3 (nonselected) (•) and for 2'-$NH_2$ RNA ligand clones 32, 37, and 17 (0).

FIGURE 9 illustrates the four step synthesis of 5-iodo-2'-amino,2'-deoxyuridine.

FIGURE 10 shows the synthesis of 2'-amino,2'-deoxyuridine 5'-triphosphate.

FIGURE 11 shows the plasma kinetic curves obtained for each of 5 experimental rats injected with $^{32}P$ labelled RNA ligand for thrombin. Blood samples were collected at 1, 2, 4, 7, and 24 hours.

FIGURE 12 illustrates the structure of 5-(3-aminoallyl)-uridine triphosphate (AA-UTP) and the synthetic scheme for production of Arg-Gly-Asp-UTP (RGD-UTP). Details of the reaction conditions are as described in Example 8.

FIGURE 13A shows the isolation of succinyl-UTP by Mono Q anion exchange column chromatography. FIGURE 13B shows the fractions versus the optical density at 290 nm. As described in Example 8, succinyl-UTP eluted in fractions 43-49.

FIGURE 14A shows the isolation of RGD-UTP by Mono Q anion exchange column chromatography. FIGURE 14B shows the fractions versus the optical density at 290 nm. As described in Example 8, RGD-UTP eluted in fractions 35-38.

FIGURE 15 shows the results of thin layer chromatography of aminoallyl-UTP, succinyl-UTP, and RGD-UTP.

FIGURE 16 shows the results of the RGD-UTP RNA transcript purification by anion exchange chromatography. The elution of RGD-UTP RNA (□) and RNA (♦) from a Mono Q anion exchange column is shown.

FIGURE 17 shows the separation of RGD-30n7 RNA and gpIIbIIIa by size exclusion chromatography on Superdex 200 (Pharmacia). The elution profiles of RGD-30n7 bound to gpIIbIIIa (0) and RGD-30n7 (♦) are shown.

FIGURE 18 shows the chemistry of the attachment of the inhibitory substrate peptide N-methoxysuccinyl Ala-Ala-Pro-Val-chloromethyl (SEQ ID NO:41) ketone to a high affinity single-stranded DNA (DNA-17).

FIGURE 19 shows the inactivation of 1.95 mM N-methoxysuccinyl Ala-Ala-Pro-Val-chloromethyl (SEQ ID NO:41) ketone by 50 mM DTT.

FIGURE 20 shows the inhibition of the blended nucleic acid ligand in the presence of 20 mM DTT.

FIGURE 21 shows the inhibition of elastase by DNA-17 conjugated at the 5' end with chloromethyl ketone (O), and DNA-17 conjugated at the 3' end with chloromethyl ketone (●).

FIGURE 22 is a Lineweaver-Burk plot of the inhibition of elastase by N-methoxysuccinyl Ala-Ala-Pro-Val (SEQ ID NO:44) (●), peptide-conjugated oligonucleotide (□) and substrate alone (O).

FIGURE 23 shows the inhibition of elastase by DNA ligands with and without the inhibitors conjugated at the 3' end compared with the effect of conjugated and nonconjugated ligands on urokinase and thrombin.

FIGURE 24 depicts the valyl phosphonate moiety attached to a nucleic acid segment as used in Example 11 below, and the reaction of the species with human neutrophil elastase.

FIGURE 25 depicts the first order rate constant of a pool of splint blended SELEX nucleic acid ligands after five rounds of SELEX measured by gel electrophoresis; TBE pH 8 (■) and MAE pH 6 (●).

FIGURE 26 depicts the starting RNA and PCR primers used in the VEGF SELEX experiment described in Examples 12 and 13.

FIGURE 27 depicts the aligned sequences and predicted secondary structures for the six families (grouped by primary sequence homology) of RNA ligands to VEGF. Arrows underline the inverted repeats of the double stranded (stem) regions. Lowercase and uppercase letters are used to distinguish nucleotides in the constant and the evolved sequence regions, respectively. Positions are numbered consecutively starting (arbitrarily) with the evolved nucleotide closest to the 5' end of the shown window.

FIGURE 28 shows the consensus sequences and predicted secondary structures for certain of the VEGF ligand families. Plain text is used to designate positions that occur at >60% but <80% frequencies. Positions where individual nucleotides are strongly conserved (frequencies >80%) are outlined. Residues in parenthesis occur at that position with equal frequencies to gaps. The numbering system described in the legend to Figure 27 is used. R = A or G; Y = C or U; M = A or C; D = A, G or U; V = G, A or C; S = G or C; K = G or U; N = any base and prime (') indicates a complementary base.

FIGURE 29 depicts the binding curves for a representative set of high-affinity ligands to VEGF. Full-length (o) and truncated (Δ) ligands tested were 100 (SEQ ID NO:55) and 100t (SEQ ID NO:95) (family 1, Figure 29A), 44 (SEQ ID NO:64) and 44t (SEQ ID NO:96) (family 2, Figure 29B), 12 (SEQ ID NO:66) (SEQ ID NO: ) and 12t (SEQ ID NO:

97) (family 3, Figure 29), 40 (SEQ ID NO:28) and 40t (SEQ ID N0:98) (family 4, Figure 29D), 84 (SEQ ID N0:80) and 84t (SEQ ID N0:99) (family 5, Figure 29E), and 126 (SEQ ID NO:82) and 126t (SEQ ID NO:100) (family 6, Figure 29F). The fraction of $^{32}P$-labeled RNA bound to nitrocellulose filters is plotted as a function of total protein concentration and the lines represent the fit of the data points to eq. 2 (40t, 84 and 84t) or to eq. 5 (all other ligands). RNA concentrations were determined from their absorbance reading at 260 nm (and were typically < 50 pM). Binding reactions were done at 37˚C in phosphate buffered saline containing 0.01% human serum albumin.

FIGURE 30 depicts the results of the determination of the 3'- and 5'-boundaries for a representative high-affinity VEGF ligand (ligand 12 (SEQ ID NO:66)). The 3'-boundary determination (Figure 30A) showing partially hydrolyzed 5'-end labeled RNA (lane 4), hydrolytic fragments retained on nitrocellulose filters following incubation of the partially hydrolyzed RNA with VEGF at 5 nM (lane 1), 0.5 nM (lane 2), or 0.125 nM (lane 3) and partial digest of the 5'-end labeled RNA with RNAse $T_1$ (lane 5) resolved on an 8% denaturing polyacrylamide gel. The 5'-boundary (Figure 30B) was determined in an identical manner except that RNA radiolabeled at the 3'-end was used. Shown are RNase $T_1$ digest (lane 1), partial alkaline hydrolysis (lane 2), and hydrolytic fragments retained on nitrocellulose filters following incubation with VEGF at 5 nM (lane 3), 0.5 nM (lane 4), or 0.125 nM (lane 5). Arrows indicate the 3'- and the 5'-boundaries of the minimal ligand 12 (italized).

FIGURE 31 depicts the Scotchard analysis of $^{125}I$-VEGF binding to HUVECS. Data points are averages of two determinations. Increasing concentrations of $^{125}I$-VEGF were incubated with $2X10^5$ cells in the presence or absence of 50-fold excess of unlabeled VEGF to determine the amount of total (o), specific (□) and non-specific (Δ) binding of $^{125}1$-VEGF as a function of free $^{125}I$-VEGF concentration (insert).

FIGURE 32 depicts the effect of random RNA (o) and representative high affinity RNA ligands 100t (SEQ ID NO: 95) (family 1) (Δ) and 44t (SEQ ID NO:96) (family 2) (□) on binding of $^{125}I$-VEGF to cell-surface receptors as a function of RNA concentration. The inhibitory affect of high affinity ligands representing other sequence families is virtually identical to that of ligands 100t and 44t.

FIGURE 33 depicts the starting random RNAs for experiments A and B, and PCR primers used in identifying 2'-$NH_2$-RNA ligands to VEGF (Example 15).

FIGURE 34 depicts 2'-$NH_2$-RNA ligands to VEGF identified via the SELEX technology as described in Example 15.

FIGURE 35 shows the binding of representative ligand 14 (SEQ ID NO:200) to human (•) (Kd = 14.5 nM) and porcine (o) (Kd = 1.3 μM) elastase wherein elastase concentrations were varied from $10^{-10}$ to $10^{-6}$ M.

FIGURE 36 shows the elastase inhibitory activity of representative ligand 34 (SEQ ID N0:197). Generation of p-nitroanilide was measured in the absence of elastase (o), in the presence of the irreversible inhibitor N-methoxysuccinyl-Ala-Ala-Pro-Val-chloromethyl ketone (•), in the absence of any inhibitor (□), and in the presence of 180 nM ligand 34 (0).

FIGURE 37A shows the results of competition experiments carried out between ligands 24 (SEQ ID NO:198) and 30 (SEQ ID NO:191) (Classes I and II).

Figure 37B shows the results of competition experiments carried out between ligands 56 (SEQ ID NO:195) and 19 (SEQ ID NO:205) (Classes II and IV).

FIGURE 38 shows the binding of representative ligand 34 (SEQ ID NO:197) to elastase irreversibly bound to N-methoxysuccinyl-Ala-Ala-Pro-Val-chloromethyl ketone (O) (Kd = 26 nM) or to native elastase (●) (Kd = 25 nM).

FIGURE 39 shows the binding of representative ligand 34 (SEQ ID NO:197) to elastase complexed with α-1-proteinase inhibitor (α-1PI) (o) or native elastase (•).

FIGURE 40 shows the additive effect of representative ligand 19 (SEQ ID NO:205) on α-1PI-induced inhibition of elastase. Generation of p-nitroanilide was measured in the presence of elastase alone (♦), 5 nM α-1PI (○ and ●), 5 nM α-1PI and 325 nM ligand 19 (□ and Δ).

FIGURE 41 shows the results of a cell detachment assay conducted with elastase only, or elastase in the presence of an inhibitory ligand 24 (SEQ ID NO:198), 30 (SEQ ID NO:191) or 19 (SEQ ID NO:205).

FIGURE 42 shows the predicted secondary structure of the G-quartet sequence (SEQ ID NO:197).

FIGURE 43 shows the binding affinities of ligands 21 (SEQ ID NO:195), 24 (SEQ ID NO:198), and 34 (SEQ ID NO: 197) to elastase in the presence and absence of 6 mM KCl.

FIGURE 44 shows the effect of pH on binding of 2'-$NH_2$ RNA ligand 14 (SEQ ID NO:200) (3'-fixed sequence truncate) and non-modified single-stranded DNA ligand 17 (SEQ ID NO:191) to elastase.

FIGURE 45 shows a predicted G-quartet structure and the mimetic molecule wherein the connecting loop nucleotides are replaced by a synthetic ethylene glycol linker tether.

FIGURE 46 shows the 2'-$NH_2$-RNA SELEX primer-template constructs (Primer-Template-Mixture I) and ssDNA SELEX primer template constructs (Primer-Template-Mixture II) .

FIGURE 47 shows the structures of theophylline and caffeine.

FIGURE 48 shows the aligned sequences for two classes of RNA molecules with affinity for theophylline selected through the SELEX method (designated TR8 (SEQ ID NOS:251-253, 256-258)) and counter-SELEX method (designated TCT8 (SEQ ID NOS:247-250, 254-255)). Figure 2A shows the Class I sequences, Figure 2B shows the Class II sequences. The clone number from which the sequence was derived is shown at the left of the sequence. In some cases, multiple isolates were obtained. Sequences shown comprised the 40 nucleotide sequence that was initially random at the start of the SELEX process. The bold uppercase sequence is conserved and provides the basis for the alignment. The arrows overlay regions of potential intramolecular complementary bases. The asterisk marks the single position in motif 1 that shows variability. Dashes represent absence of a nucleotide. Class I and II are related by circular permutation.

FIGURE 49 illustrates the predicted secondary structures for theophylline binding RNA species obtained from the TR8 SELEX and TCT8 SELEX experiments. Bases in shadow text were initially present in either the fixed 5' or 3' regions which flanked the random region. Note that either fixed region can contribute to the proposed structure. The arrows in the TCT8-4 ligand (SEQ ID NO:249) represent the termini of the mini-derivative mTCT8-4 with the exception that the position 1-38 AU pair was changed to a GC pair in mTCT8-4.

FIGURE 50 illustrates the predicted secondary structure of theophylline binding RNA species. N and N' are any complementary base pair. Numbers represent the size range of the various domains observed in the ligands obtained.

FIGURE 51 shows the binding properties of TCT8-4 (SEQ ID NO:249) and mTCT8-4 RNAs.

FIGURE 52 shows the predicted secondary structure for mTCT8-4.

FIGURE 53 shows the competition binding analysis of xanthine derivatives with TCT8-4 RNA (SEQ ID NO:249).

FIGURE 54 shows the sequences of RNA ligands for caffeine (CR8 (SEQ ID NOS:259-270)). These ligands were identified after 8 SELEX selection rounds.

FIGURE 55 illustrates the chemical structure of theophylline with a series of derivatives that were used in competitive binding experiments with TCT8-4 RNA as described in Example 27. The number in parenthesis represents the effectiveness of the competitor relative to theophylline, Kc(c)/Kc(t). Kc(c) is the individual competitor dissociation constant and Kc(t) is the competitive dissociation constant of theophylline. 1-cp theophylline is 1-carboxypropyl theophylline. Certain data, denoted by ">" are minimum values that were limited by solubility of the competitor. Each experiment was carried out in duplicate.

## DETAILED DESCRIPTION OF THE PREFERRED EMBODIMENTS

This application is an extension and an improvement of the method for identifying nucleic acid ligands referred to as the SELEX method. The SELEX method (hereinafter termed SELEX) was first described in U.S. Application Serial No. 07/536,428 filed June 11, 1990, entitled Systematic Evolution of Ligands by EXponential Enrichment, now abandoned, U.S. Patent Application Serial No. 07/714,131, filed June 10, 1991 and U.S. Patent Application Serial No. 07/931,473, filed August 17, 1992, now U.S. Patent No. 5, 270,163, both entitled Nucleic Acid Ligands. *(See* also PCT/US91/04078 and PCT/US93/09296). The full text of these applications, collectively referred to as the SELEX applications, including but not limited to, all definitions and descriptions of the SELEX process, are specifically incorporated herein by reference.

In its most basic form, the SELEX process may be defined by the following series of steps:

1) A candidate mixture of nucleic acids of differing sequence is prepared. The candidate mixture generally includes regions of fixed sequences (i.e., each of the members of the candidate mixture contains the same sequences in the same location) and regions of randomized sequences. The fixed sequence regions are selected either: a) to assist in the amplification steps described below; b) to mimic a sequence known to bind to the target; or c) to enhance the potential of a given structural arrangement of the nucleic acids in the candidate mixture. The randomized sequences can be totally randomized (i.e., the probability of finding a base at any position being one in four) or only partially randomized (e.g., the probability of finding a base at any location can be selected at any level between 0 and 100 percent).

2) The candidate mixture is contacted with the selected target under conditions favorable for binding between the target and members of the candidate mixture. Under these circumstances, the interaction between the target and the nucleic acids of the candidate mixture can be considered as forming nucleic acid-target pairs between the target and the nucleic acids having the strongest affinity for the target.

3) The nucleic acids with the highest affinity for the target are partitioned from those nucleic acids with lesser affinity to the target. Because only an extremely small number of sequences (and possibly only one molecule of nucleic acid) corresponding to the highest affinity nucleic acids exist in the candidate mixture, it is generally desirable to set the partitioning criteria so that a significant amount of the nucleic acids in the candidate mixture (approximately 5-10%) is retained during partitioning.

4) Those nucleic acids selected during partitioning as having the relatively higher affinity to the target are then amplified to create a new candidate mixture that is enriched in nucleic acids having a relatively higher affinity for the target.

5) By repeating the partitioning and amplifying steps above, the newly formed candidate mixture contains fewer and fewer unique sequences, and the average degree of affinity of the nucleic acid mixture to the target will generally increase. Taken to its extreme, the SELEX process will yield a candidate mixture containing one or a small number of unique nucleic acids representing those nucleic acids from the original candidate mixture having the highest affinity to the target molecule.

Partitioning means any process whereby ligands bound to target molecules can be separated from nucleic acids not bound to target molecules. More broadly stated, partitioning allows for the separation of all the nucleic acids in a candidate mixture into at least two pools based on their relative affinity to the target molecule. Partitioning can be accomplished by various methods known in the art. Nucleic acid-protein pairs can be bound to nitrocellulose filters while unbound nucleic acids are not. Columns which specifically retain nucleic acid-target complexes can be used for partitioning. For example, oligonucleotides able to associate with a target molecule bound on a column allow use of column chromatography for separating and isolating the highest affinity nucleic acid ligands. Liquid-liquid partitioning can be used as well as filtration gel retardation, and density gradient centrifugation.

The SELEX Patent Applications describe and elaborate on this process in great detail. Included are targets that can be used in the process; methods for the preparation of the initial candidate mixture; methods for partitioning nucleic acids within a candidate mixture; and methods for amplifying partitioned nucleic acids to generate enriched candidate mixtures. The SELEX Patent Applications also describe ligand solutions obtained to a number of target species, including both protein targets wherein the protein is and is not a nucleic acid binding protein.

SELEX provides high affinity ligands of a target molecule. This represents a singular achievement that is unprecedented in the field of nucleic acids research.

The present invention includes the application of the SELEX procedure to the specific targets of VEGF, elastase, thrombin, theophylline and caffeine.

In order to produce nucleic acids desirable for use as a pharmaceutical, it is preferred that the nucleic acid ligand 1) binds to the target in a manner capable of achieving the desired effect on the target; 2) be as small as possible to obtain the desired effect; 3) be as stable as possible; and 4) be a specific ligand to the chosen target. In most, if not all, situations it is preferred that the nucleic acid ligand have the highest possible affinity to the target.

In co-pending and commonly assigned U.S. Patent Application Serial No. 07/964,624, filed October 21, 1992 ('624), methods are described for obtaining improved nucleic acid ligands after SELEX has been performed.

This application, entitled Methods of Producing Nucleic Acid Ligands is specifically incorporated herein by reference. Included in the '624 application are methods relating to: assays of ligand effects on target molecules; affinity assays of the ligands; information boundaries determination; quantitative and qualitative assessment of individual nucleotide contributions to affinity via secondary SELEX, nucleotide substitution, and chemical modification experiments; and structural determination *(See also* PCT/US93/09296).

The present invention includes methods for producing improved nucleic acid ligands based on modifications of the basic SELEX process. The application includes separate sections covering the following embodiments of the invention: I. High Affinity Nucleic Acid Ligands Containing Modified Nucleotides; II. High Affinity Nucleic Acid Ligands Containing Blended Nucleotides - Blended SELEX; III. High-Affinity Oligonucleotide Ligands to Vascular Endothelial Growth Factor (VEGF); IV. Nucleic Acid Ligand Inhibitors of Human Neutrophil Elastase; and V. High-Affinity Nucleic Acid Ligands that Discriminate Between Theophylline and Caffeine - Counter-SELEX.

Modified and non-modified nucleic acid ligands to VEGF, elastase, thrombin, theophylline and caffeine are disclosed and claimed herein. The scope of the ligands covered by the invention extends to all ligands of VEGF, elastase, thrombin, theophylline and caffeine identified herein: This invention also includes modified and non-modified nucleic acid sequences that are substantially homologous to and that have substantially the same ability to bind thrombin as the specific nucleic acid ligands disclosed herein. By substantially homologous, it is meant, a degree of primary sequence homology in excess of 70%, most preferably in excess of 80%. Substantially the same ability to bind VEGF, elastase, thrombin, theophylline and caffeine means that the affinity is within two orders of magnitude of the affinity of the substantially homologous sequences described herein. It is well within the skill of those of ordinary skill in the art to determine whether a given sequence -- substantially homologous to those specifically described herein -- has substantially the same ability to bind VEGF, elastase, thrombin, theophylline and caffeine.

As has been shown, sequences that have little or no primary sequence homology may still have substantially the same ability to bind the target molecule. It is clear that binding is controlled by the secondary or tertiary structure of the nucleic acid ligand. For these reasons, the present invention includes nucleic acid ligands that have substantially the same structural form as the ligands presented herein and that have substantially the same ability to bind VEGF, elastase, thrombin, theophylline and caffeine as the modified and unmodified nucleic acid ligands disclosed herein. Wherein substantially the same structure includes all nucleic acid ligands having the common structural elements as the ligands disclosed herein that lead to the affinity to VEGF, elastase, thrombin, theophyline and caffeine. This invention further includes ligands containing a variety of modified nucleotides, such as 2'-fluoro modifications.

I. HIGH AFFINITY NUCLEIC ACID LIGANDS CONTAINING MODIFIED NUCLEOTIDES.

The present invention is directed at methods for producing improved nucleic acid ligands. In one embodiment of the method of the present invention, SELEX is performed with a candidate mixture of oligonucleotides containing one or more modified nucleotides. The presence of the modified nucleotides increases the chemical diversity of the candidate mixture, allowing improved ligands to a particular target molecule to be identified. In another embodiment of the method of the present invention, nucleic acid ligands selected through the SELEX process are modified by incorporation of modified nucleotides.

Important modifications or derivatizations of the ligand to which the method of the present invention are directed are those that confer resistance to degradation and clearance *in vivo* during therapy. Further improvements conferred by the method of the present invention may include enhanced capacity to cross various tissue or cell membrane barriers, or any other accessory properties that do not significantly interfere with affinity for the target molecule.

The increased chemical diversity achieved by carrying out SELEX with nucleotide analogs can introduce ligand properties such as enhanced stability against nucleases, incorporation of reporter groups, introduction of moieties capable of covalent crosslinking to a target, or introduction of intra-ligand crosslinks for the generation of stable conformers and novel oligonucleotide shapes.

Introduction of 2'-amino,2'-deoxy pyrimidines into the SELEX candidate mixture library requires preparation of their 5'-triphosphate derivatives. This is the form that is recognized as a substrate for DNA-dependent RNA polymerases or for DNA-dependent DNA polymerases. Furthermore, analogs also have to be prepared as the phosphoramidite in order to be incorporated into the final oligonucleotide ligand by automated chemical synthesis. These derivatives have been described, along with their method of preparation (Aurup et al. (1992) Biochemistry 31:9636). The synthesis of oligonucleotides containing 2'-amino,2'-deoxy pyrimidines by T7 RNA polymerase transcription of DNA templates has also been previously reported (Id.; Pieken et *al.* (1990) Science 253:314). Homopolymers of the 2'-amino,2'-deoxy pyrimidine nucleotides have also been prepared by polymerization of their 5'-diphosphate derivatives (Hobbs *et al.* (1973) Biochem 12:5138). Oligoribonucleotides containing 2'-amino,2'-deoxy pyrimidines have also been prepared by automated solid phase synthesis. The trifluoroacetyl group has been used for protection of the 2'-amino group in preparation of phosphoramidite monomers (Pieken et *al.* (1990) supra).

As described above and in the SELEX patent applications, the SELEX technology identifies specific high affinity oligonucleotide ligands to a given molecular target by iterative enrichment from a vast pool of species. In one embodiment of the present invention, the amplified oligodeoxyribonucleotide sequences are transcribed to its oligoribonucleotide homolog with T7 RNA polymerase. Thus, during each step in the enrichment process, the library is reassembled from its nucleoside triphosphate building blocks. This feature allows the introduction of chemically modified nucleoside triphosphates, and thus the enrichment of ligands bearing chemical functionalities not found in native RNA. Examples of such modifications include chemical substitutions at the ribose and/or phosphate positions of a given RNA sequence. *See, e.g.,* Cook et *al.* PCT Application WO 9203568; U.S. Patent No. 5,118,672 of Schinazi et al.; Hobbs et al. (1973) Biochem 12:5138; Guschlbauer et al. (1977) Nucleic Acids Res. 4:1933; Shibaharu et *al.* (1987) Nucl. Acids. Res. 15:4403; Pieken et *al.* (1990) supra, each of which is specifically incorporated herein by reference.

The increased chemical diversity achieved by the method of the present invention requires the chemical synthesis of a number of compounds. It is desirable to have a highly divergent synthetic methodology at hand that allows preparation of a variety of desired nucleotide analogs from a common intermediate. These analogs in all cases need to infer nuclease resistance to the oligonucleotide.

A large variety of modifying groups can be introduced to the 5-position of pyrimidines. Such methods are described in U.S. Patent Application Serial No. 08/076,735 (USSN 08/076,735), filed June 14, 1993, entitled Method for Palladium Catalyzed Carbon-Carbon Coupling and Products, herein specifically incorporated by reference. The process described in USSN 08/076,735 requires the 5-iodo or 5-bromo pyrimidine precursor. In order to generate base-modified pyrimidines that are also nuclease resistant, it is desirable to depart from a 2'-modified precursor for the coupling technology (USSN 08/076,735).

5-iodo-2'-amino,2'-deoxyuridine has been prepared previously (Verheyden et *al*. (1971) J. Org. Chem. 36:250). It has not been applied to the uses discussed herein. The 5-iodo,2'-deoxyuridine is introduced into the SELEX library of candidate oligonucleotides as the 5'-triphosphate derivative. The 5-iodo substituent is not compatible with the reaction conditions used in standard phosphorylation of 5-iodo,2'amino,2'-deoxy pyrimidines. Instead, the 5'-triphosphate derivative, which has not been previously described, is prepared from the 2'-amino, 2'-deoxy pyrimidine 5'-triphosphate by mercuration of the 5-position (Dale et *al.* (1975) Biochemistry 14:2447) with subsequent iodination (Dale et al. (1975) Nucleic Acids Res. 2:915). These modified bases are central intermediates in the generation of base-modified nuclease resistant oligonucleotide ligands.

The modified nucleic acid ligands of the present invention may include one or more modified nucleotides. The proportion of modified to non-modified nucleotides contained in the nucleic acid ligands of the present invention may range from 1-100%.

In one embodiment of the method of the present invention wherein SELEX is performed with a library candidate mixture of oligonucleotides containing modified nucleotides, the desired amount of modified nucleotide incorporation in the oligonucleotides is achieved by including one or more modified nucleotides in the oligonucleotide synthesis mixture.

In a second embodiment of the method of the present invention, wherein one or more modified nucleotides are incorporated into a non-modified nucleotide-containing nucleic acid ligand identified through SELEX, SELEX is performed to completion with an initial candidate mixture of 2'-OH oligonucleotides, such that high-affinity nucleic acid ligands or family of ligands to a target molecule are identified. These ligands are then transcribed in the presence of modified nucleotides such that high-affinity ligands to the target molecule containing modified nucleotides are produced. The present invention includes oligonucleotide ligands modified at specific positions. Nucleic acids ligands containing modified nucleotides at specific positions may be produced by synthesis.

In another embodiment of the method of the present invention, SELEX is performed for a few selection rounds but not to completion, such that a candidate mixture of 2'-OH oligonucleotides is selected for partially enhanced affinity to the target molecule. Modified nucleotides are then incorporated into the partially-selected oligonucleotides by transcription in the presence of modified nucleotides.

Example 1 describes the general experimental procedures for performing SELEX. In order to be useful in the existing SELEX protocol, the modified nucleotides must be substrates for polymerase(s) in their triphosphate forms and the resulting modified oligonucleotides must be templates for amplification. Example 2 demonstrates that four prototypic 5-modified uridines, 5-AA-UTP, 5-F-12-UTP, 5-Dig-11-UTP, and 5-Br-UTP, shown in Figure 2, meet the above requirements and can be used in SELEX. These modified nucleotides are incorporated into RNA by the T7 RNA polymerase under standard transcription conditions. Modified transcripts were reversed transcribed into cDNA by AMV reverse transcriptase and amplified by PCR. These results show that modified nucleotides can be directly incorporated into the SELEX procedure.

As discussed above, 2'-amino modified pyrimidines and purines exhibit increased resistance to endonuclease activity. Example 3 describes SELEX selection of 2'-$NH_2$ ligands to the human thrombin. The affinities of SELEX identified 2'-OH and 2'-$NH_2$ ligands to thrombin are compared. While Example 3 describes SELEX identification of RNA ligands, the same procedure may be performed for the SELEX identification of DNA ligands to a specific target molecule. The only enzymatic requirement for DNA SELEX is that the modified deoxynucleoside triphosphates serve as substrates for Taq DNA polymerase or another suitable polymerase. It is known that digoxygenin-11-deoxyuridine triphosphate can be used as a replacement substrate for TPP in PCR (Lanzillo (1990) BioTechniques 8:621).

This invention includes the specific 2'-OH and 2'-$NH_2$ nucleic acid ligands of thrombin shown in Figure 6. This invention also includes modified and non-modified nucleic acid sequences that are substantially homologous to and that have substantially the same ability to bind thrombin as the specific nucleic acid ligands shown in Figure 6.

As has been shown, sequences that have little or no primary sequence homology may still have substantially the same ability to bind the target molecule. It is clear that binding is controlled by the secondary or tertiary structure of the nucleic acid ligand. For these reasons, the present invention includes nucleic acid ligands that have substantially the same structural form as the ligands presented herein and that have substantially the same ability to bind thrombin as the modified and unmodified nucleic acid ligands shown in Figure 6. Wherein substantially the same structure includes all nucleic acid ligands having the common structural elements shown in Figure 7 that lead to the affinity to thrombin. This invention further includes ligands containing a variety of modified nucleotides, such as 2'-fluoro modifications.

Example 4 describes a novel method for the synthesis of 5-iodo, 2'-amino deoxyuridine. The synthesis of the intermediate 5-iodo, 2'-amino deoxyuridine, shown in Figure 9, achieves a greater yield of the compound over the prior art method with fewer synthetic steps.

Example 5 describes the synthesis of 5-iodo, 2'-amino, 2'-deoxy pyrimidine 5'-triphosphate. 5-iodo, 2'-amino, 2'-deoxy pyrimidine 5'-triphosphates cannot be prepared from their 5-iodo, 2'-amino, 2'-deoxy pyrimidine nucleoside precursors because the conditions employed in the phosphorylation reaction are not compatible with the 5-iodo group. However, they may be prepared from the 2'-amino,2'-deoxy pyrimidine 5'-triphosphates by mercuration and subsequent iodination of the C-5 position.

Example 6 describes the in vivo stability of 2'-$NH_2$ modified ligands of thrombin.

The method of the present invention further includes incorporation of functional groups into oligonucleotides via the modified nucleotides. One of the products of the SELEX procedure is a consensus of primary and secondary structures that enables the chemical or enzymatic synthesis of oligonucleotide ligands whose design is based on that consensus. Because the replication machinery of SELEX requires that rather limited variation at the subunit level (ribonucleotides, for example), such ligands imperfectly fill the available atomic space of a target molecule's binding surface. However, these ligands can be thought of as high-affinity scaffolds that can be derivatized to make additional contacts with the target molecule. In addition, the consensus contains atomic group descriptors that are pertinent to binding and atomic group descriptors that are coincidental to the pertinent atomic group interactions. The present invention further includes nucleic acid ligands containing additional functional groups introduced via the modified nucleotides. A strategy for introduction of functional groups is described in Example 7.

## II. HIGH AFFINITY NUCLEIC ACID LIGANDS CONTAINING BLENDED NUCLEOTIDES - BLENDED SELEX.

The present invention further includes a method for generating high affinity blended nucleic acid ligands to specific target molecules. The method generates blended nucleic acid molecules comprised of at least one functional unit.

Functional units that can be coupled to nucleotides or oligonucleotides include peptides, amino acids, aliphatic groups and lipid chains, or larger compounds such as peptide motifs, recognizable by the target molecule. These non-nucleic acid components of oligonucleotides may fit into specific binding pockets to form a tight binding via appropriate hydrogen bonds, salt bridges, or van der Walls interactions.

In certain embodiments of this invention, the blended nucleic acid ligands generated may guide SELEX-generated ligands to specific sites of the target molecule. Further blended nucleic acid ligands may be prepared after the SELEX process for post-SELEX modification to add functionality to the ligand, for example, to increase RNA hydrophobicity and enhance binding, membrane partitioning and/or permeability, or to add reporter molecules, such as biotin- or fluorescence-tagged reporter oligonucleotides, for use as diagnostics. Blended nucleic acid ligands may be generated by the addition of chemical groups which covalently react and couple the SELEX ligand to the target molecule. Additionally, catalytic groups can be added to nucleic acids to aid in the selection of SELEX ligands with protease or nuclease activity. The functional units may also serve as toxins or radiochemicals that are delivered to specific locations in the body determined by the specificity of the SELEX devised nucleic acid ligand.

Blended nucleic acid ligands are defined herein as comprising at least one nucleic acid ligand and at least one functional unit. A nucleic acid ligand is defined as any nucleic acid identified generally according to the SELEX process as described in the SELEX Patent Applications. The functional unit may be any chemical species not naturally associated with nucleic acids, and may have any number of functions as enumerated herein.

In one preferred embodiment of the invention, the blended nucleic acid ligand is prepared by performing the SELEX method utilizing an initial candidate mixture wherein each nucleic acid sequence of the candidate mixture contains at least one functional unit, or a "blended candidate mixture". This may be accomplished using a candidate mixture wherein each nucleic acid sequence of the candidate mixture has 1) a single functional unit attached at either the 5' or 3' end of nucleic acid sequence; 2) functional units at both the 5' and 3' ends of the nucleic acid sequence; 3) functional units added to individual nucleic acid residues; 4) functional units attached to all or a portion of all pyrimidine or purine residues; or 5) functional units attached to all or a portion of all nucleotides of a given type. The functional units may also be attached only to the fixed or to the randomized regions of each nucleic acid sequence of the candidate mixture.

In an alternate preferred embodiment of the invention, one or more functional units may be attached to a nucleic acid ligand identified according to the SELEX method wherein a blended candidate mixture is not used. Again the points of attachment of the functional unit(s) to the nucleic acid ligand may vary depending on the ultimate requirements for the blended nucleic acid ligands.

Examples 8-11 below describe methods for generating the blended nucleic acid ligands of the present invention. As these examples establish, nucleotides and oligonucleotides containing a new functional unit are useful in generating blended nucleic acid ligands to specific sites of a target molecule.

Two examples are described for coupling peptide molecules to SELEX nucleic acid ligands in order to target specific peptide binding pockets. In the first example, a peptide containing the sequence arginine-glycine-aspartic acid (RGD) is coupled to a nucleotide and enzymatically incorporated into unselected polyribonucleotide. The RGD-containing peptide is recognized and bound by the gpIIbIIIa integrin protein, causing gpIIbIIIa-mediated platelet aggregation. A SELEX RGD-nucleic acid ligand may be generated with high specificity for gpIIbIIIa that would not cross react with other integrins such as receptors for fibronectin, vetronectin, collagen, or laminin. SELEX blended nucleic acid ligands containing the RGD peptide could bind at or near the gpIIbIIIa ligand site and specifically inhibit gpIIbIIIa activity. In the second example, a SELEX-identified single stranded DNA ligand to elastase was produced and coupled to inhibitory substrate peptide chloromethyl ketone. The resulting blended nucleic acid ligand was shown to specifically inhibit elastase.

Example 8 describes the generation of a peptide-conjugated RNA. The peptide Gly-Arg-Gly-Asp-Thr-Pro (SEQ ID NO:40) was coupled to the derivatized base, 5-(3-aminoallyl)-uridine triphosphate to produce the peptide-conjugated UTP (RGD-UTP), as shown in Figure 12. RGD-UTP may be used in SELEX T7-catalyzed template-dependent transcription to produce an RNA oligonucleotide containing the RGD peptide at every uridine position.
The Gly-Arg-Gly-Asp-Thr-Pro (SEQ ID NO:40) peptide was chosen because the Arg-Gly-Asp (RGD) motif in matrix proteins is recognized and bound specifically by proteins of the integrin superfamily of cell adhesion receptors. Integrins bind to such proteins as fibronectin, laminin and vitronectin, at sites containing the RGD sequence. This binding is inhibited by short RGD-containing peptides which bind integrin proteins with a Kd of approximately $10^{-5}$ M.

Because of the specificity of RGD-containing peptides for integrins, the inventors of this application concluded that an RGD-containing peptide conjugated to an oligonucleotide may facilitate selection of high-affinity ligands to a RGD-binding target molecule. However, according to this embodiment of the invention utilizing a blended candidate mixture, peptide-conjugated oligonucleotides must also be compatible with the SELEX method, that is, the peptide-conjugated nucleotide must be incorporated with reasonable efficiency into transcribed RNA and partitioned RNA in turn must be reasonably efficiently transcribed into complementary DNA for amplification and additional rounds of SELEX. Examples 8 and 9 below demonstrate that all of the conditions required for successful SELEX identification of nucleic acid ligands containing new functional groups are met by the method of this invention. The peptide-UTP derivative was enzymatically incorporated into a blended RNA oligonucleotide as shown by increased size and altered charge compared with the native or unmodified oligonucleotide and by the UV shoulder at 310 nm.

Example 9 describes the binding of RGD-RNA to RGD-binding integrin gpIIbIIIa. After separation, the bound RGD-RNA was reversed transcribed into DNA using normal SELEX protocols. The efficient transcription, partitioning, and reverse transcription shows that the site-directed blended nucleic acid ligands of this invention are compatible with the basic SELEX procedure.

The methods described herein do not include all of the schemes for introducing non-nucleic acid functional units, such as peptides, into an oligonucleotide. However, such methods would be well within the skill of those ordinarily practicing in the art. Putting a peptide on every uridine, as done in Example 8, has several advantages as compared with other labelling methods for use in the SELEX procedure. First, the peptide is introduced throughout both the random and fixed regions, so that evolved RNA ligands could bind close to the peptide binding site. Second, distributing the peptide at multiple sites does not restrict the geometry of RNA and does not interfere with SELEX identification of the optimal peptide position. Third, one can use pre-derivatized nucleotides with SELEX. Post-transcription modification may require additional time and expertise and introduces the additional variable of coupling efficiency.

Other methods for coupling non-nucleic acid functional units to nucleic acids may be used to yield evolved ligands with a non-overlapping spectrum of binding sites. For instance, a peptide could be placed at the 5' or 3' end of SELEX identified RNA ligands. Another embodiment of this invention for introducing a non-nucleic acid functional unit at random positions and amounts is by use of a template-directed reaction with non-traditional base pairs. This method uses molecular evolution to select the best placement of the non-nucleic acid group on the SELEX identified ligand. For example, a X-dY base pair could be used, where X is a derivatizable ribonucleotide and the deoxynucleotide dY would pair only with X. The X-RNA would contain the non-nucleic acid functional unit only at positions opposite dY in the dY-DNA template; the derivatized X base could be positioned in either the fixed or random regions or both; and the amount of X at each position could vary between 0-100%. The sequence space of non-evolved SELEX ligands would be increased from $N^4$ to $N^5$ by substituting this fifth base without requiring changes in the SELEX protocol.

This embodiment may be used with photo-SELEX (U.S. Patent Application Serial No. 08/123,935, filed September 17, 1993, specifically incorporated herein by reference), where photo-active bases are placed at random sites, and these blended ligands partitioned after photolysis, then high affinity ligands are selected with the minimum number of substituted bases needed to crosslink with the target. In the same way, functional units that react covalently at enzyme active sites, such as chloromethylketones, may be incorporated to produce irreversible enzyme inhibitors. Use of directed incorporation could be used also for post-SELEX incorporation of fluorescence tags, biotin, radiolabel, lipid groups, or to cap the oligonucleotide with a uniquely modified base for protection against nuclease digestion.

Incorporation of non-nucleic acid functional units to produce blended SELEX ligands increases the repertoire of structures and interactions available to produce high affinity binding ligands. Various types of functional units can be incorporated to produce a spectrum of molecular structures. At one end of this structural spectrum are normal polynucleic acids where the ligand interactions involve only nucleic acid functional units. At the other, are fully substituted nucleic acid ligands where ligand interactions involve only non-nucleic acid functional units. Since the nucleic acid topology is determined by the sequence, and sequence partitioning and amplification are the basic SELEX steps, the best ligand topology is selected by nucleic acid evolution.

Example 10 below describes the preparation of a blended nucleic acid ligand to elastase to act as an elastase inhibitor. N-methoxysuccinyl-Ala-Ala-Pro-Val-chloromethyl (SEQ ID NO:41) ketone is an effective irreversible inhibitor for elastase. However, the nonspecific highly reactive nature of the chloromethyl ketone functionality in conjunction with mM range Kd of the tetrapeptide makes the inhibitor molecule unsuitable as a therapeutic agent. The enzyme inhibition of nucleic acid ligands to elastase may be improved by coupling such ligands to the substrate tetrapeptide in a nonhydrolyzable manner such that the blended nucleic acid will inhibit the enzyme by occupying the substrate binding pocket. In such a blended nucleic acid affinity and specificity is provided by the SELEX-derived nucleic acid ligand, whereas inhibition is provided by the peptide.

In one embodiment of the invention, referred to as splint blended SELEX, the functional unit of the blended nucleic acid ligand is attached to the SELEX derived nucleic acid ligand via the attachment of the functional unit to a nucleic acid that hybridizes to a region of the nucleic acid sequence of the ligand. In the preferred embodiment, the functional unit oligonucleotide is DNA, and hybridizes to the fixed region of the nucleic acid ligand or at least a region of the nucleic acid ligand that is not involved in the binding reaction to the target.

In one variation of this embodiment, the SELEX process is accomplished by the preparation of a candidate mixture of nucleic acid sequences comprised of fixed and randomized regions. The candidate mixture also contains an oligonucleotide attached to a selected functional group. The oligonucleotide is complementary to the fixed region of the nucleic acid candidate mixture, and is able to hybridize under the conditions employed in SELEX for the partitioning of high affinity ligands from the bulk of the candidate mixture. Following partitioning, the conditions can be adjusted so that the oligo-functional unit dissociates from the nucleic acid sequences.

Advantages to this embodiment include the following: 1) it places a single functional unit, such as a peptide analog, at a site where it is available for interaction with the random region of nucleic acid sequences of the candidate mixture; 2) because the functional unit is coupled to a separate molecule, the coupling reaction need only be performed once, whereas when the functional unit is coupled directly to the SELEX ligand, the coupling reaction must be performed at every SELEX cycle (aliquots from this reaction can be withdrawn for use at every cycle of SELEX); 3) the coupling chemistry between the functional unit and the oligonucleotide need not be compatible with RNA integrity or solubility -- thus simplifying the task of coupling; 4) in cases where the functional unit forms a covalent complex with the target, the SELEX derived nucleic acid ligand portion of the selected members of the candidate mixture can be released from the target for amplification or identification; and 5) following the successful identification of a blended nucleic ligand, the tethered portion of nucleic acid can be made into a hairpin loop to covalently attach the two portions of the blended nucleic acid ligand. An example of splint blended nucleic acid ligand for human neutrophil elastase is shown in Example 11.

## III. <u>HIGH-AFFINITY OLIGONUCLEOTIDE LIGANDS TO VASCULAR ENDOTHELIAL GROWTH FACTOR (VEGF).</u>

The present invention applies the SELEX and modified SELEX procedures to the specific target of vascular endothelial growth factor (VEGF). In Examples 12-15 below, the experimental parameters used to isolate and identify the nucleic acid ligand solutions to VEGF are described.

This invention includes the specific RNA ligands to VEGF shown in Figure 27 (SEQ ID NOS:48-82). The scope of the ligands covered by this invention extends to all RNA ligands of VEGF identified according to the SELEX procedure. More specifically, this invention includes nucleic acid sequences that are substantially homologous to and that have substantially the same ability to bind VEGF as the specific nucleic acid ligands shown in Figure 27.

This invention also includes the 2'-$NH_2$ modified RNA ligands to VEGF as shown in Figure 34. The scope of the present invention extends, therefore, to all modified nucleic acid ligands identified according to the SELEX method as well as to all sequences that are substantially homologous to and that have substantially the same ability to bind VEGF as the specific nucleic acid ligands shown in Figure 34.

This invention encompasses the use of the disclosed ligands to identify a second ligand. In one embodiment, a first SELEX identified ligand which binds to a specific site of the target molecule is used to elute secondary ligands binding to the same site. In another embodiment, a first SELEX identified ligand binding to a specific site of the target molecule is used to select secondary ligands which do not bind to the same site. In this case, SELEX is conducted in the presence of the first ligand such that the binding site is saturated with the first ligand and selection occurs for ligands binding elsewhere on the target molecule. In a further embodiment analogous to the generation of anti-idiotype antibodies, a SELEX identified ligand to VEGF may itself be used as a target molecule to identify secondary ligands resembling the VEGF binding site. Such secondary ligands may compete with VEGF-substrate binding and inhibit the biological activity of VEGF.

A review of the sequence homologies of the RNA ligands of VEGF shown in Figures 27 and 34 shows that sequences with little or no primary homology may have substantially the same ability to bind VEGF. For these reasons, this invention also includes nucleic acid ligands that have substantially the same structure as the ligands presented herein and that have substantially the same ability to bind VEGF as the nucleic acid ligands shown in Figures 27 and 34.

Example 12 describes the experimental procedures used to generate high-affinity nucleic acid ligands to VEGF. Example 13 describes the high-affinity RNA ligands to VEGF shown in Figure 27. Example 14 describes the specificity of truncated RNA ligands to VEGF. Example 15 describes the experimental procedures used to generate 2'-$NH_2$ pyrimidine modified RNA ligands to VEGF.

IV. <u>NUCLEIC ACID LIGAND INHIBITORS OF HUMAN NEUTROPHIL ELASTASE.</u>

The present invention also applies the SELEX and modified SELEX procedures to the specific target of human neutrophil elastase (elastase). In Examples 16-22 below, the experimental parameters used to isolate and identify the nucleic acid ligands to elastase are described.

Specifically, the invention includes the 2'-$NH_2$ RNA ligands of elastase shown in Table 4. The scope of the ligands covered extends to all RNA ligands of elastase, modified and unmodified, identified according to the SELEX procedure, and further, ligands obtained by use of the ligands of the present invention. More specifically, this invention includes nucleic acid sequences that are substantially homologous to and that have substantially the same ability to bind elastase as the specific nucleic acid ligands shown in Table 4.

This invention also includes the specific DNA ligands of elastase shown in Table 9, identified by the method described in Example 16. The scope of the ligands covered extends to all DNA ligands of elastase, modified and unmodified, identified according to the SELEX procedure, and further, ligands obtained by use of the ligands of the present invention. More specifically, this invention includes nucleic acid sequences that are substantially homologous to and that have substantially the same ability to bind elastase as the specific nucleic acid ligands shown in Table 9.

This invention encompasses the use of the disclosed ligands to identify a second ligand. In one embodiment, a first SELEX identified ligand which binds to a specific site of the target molecule is used to elute secondary ligands binding to the same site. In another embodiment, a first SELEX identified ligand binding to a specific site of the target molecule is used to select secondary ligands which do not bind to the same site. In this case, SELEX is conducted in the presence of the first ligand such that the binding site is saturated with the first ligand and selection occurs for ligands binding elsewhere on the target molecule. In a further embodiment analogous to the generation of anti-idiotype antibodies, a SELEX identified ligand to elastase may itself be used as a target molecule to identify secondary ligands resembling the elastase binding site. Such secondary ligands may compete with elastase-substrate binding and inhibit the biological activity of elastase.

A review of the sequence homologies of the RNA ligands of elastase shown in Table 4 or the DNA ligands shown in Table 9 shows that sequences with little or no primary homology may have substantially the same ability to bind elastase. For these reasons, this invention also includes nucleic acid ligands that have substantially the same three dimensional structure as the ligands presented herein and substantially the same ability to bind elastase as the nucleic acid ligands shown in Tables 4 and 9.

The present invention identifies and describes 2'-$NH_2$-modified RNA ligands which inhibit elastase activity (Example 18). Several of these inhibitory ligands were truncated by removal of the 3'-fixed sequence and the ability of the truncated ligands to inhibit elastase examined (Example 19). Based on primary sequence homology, the ligands of elastase have been grouped into a number of classes. The results show that for class I and II ligands, 3'-truncated ligands had similar abilities to inhibit elastase as the full-length ligands. However, for class III and IV ligands, the 3'-fixed sequence truncated ligands lost their ability to inhibit elastase.

The effect that the presence of small monovalent cations such as $K^+$ and $Li^+$ on affinity of ligands to elastase was examined (Example 20). The results suggest that $K^+$ may favor the formation of higher affinity secondary structures than $Li^+$.

<u>Effect of pH on Binding.</u> The overall effect of pH on ligand binding involves an interplay of factors contributed both by the protein and the ligand. In order to isolate the factors contributed by elastase, the binding of a single-stranded DNA (ssDNA) ligand to elastase was examined wherein the effect of pH on binding should derive primarily from the effect of pH on elastase (Example 21). The ssDNA ligand bound better at a low pH, and the Kd of binding increased approximately two fold over two pH units. In contrast, the binding of the 2'-$NH_2$ RNA ligand to elastase was profoundly affected by pH. The Kd of the binding increased more than 10 fold when the pH was decreased from 6.5 to 6.25. The optimum binding for the 2'-$NH_2$-modified RNA ligand to elastase was at pH 7.0, the pH at which SELEX was conducted.

The observation that binding of the 2'-$NH_2$ modified RNA ligands is pH dependent effects how the ligands may best be utilized. For example, by using two buffers with different pH, it may be possible to develop two ligands for a given target, each of which binds at a different pH. Likewise, one may tailor ligands with high affinity binding for a given target molecule in one cell compartment with specific pH, but not to the same protein in another cell compartment with a different pH. Similarly, ligands that are active specifically in certain cell types, for example tumor cells which are known to have lower internal pH than normal cells, may be discovered with 2'-$NH_2$ modified RNAs.

Nucleic Acid Mimetics. Several classes of elastase ligands identified herein appear to assume a "G-quartet" type structure. Sequences rich in repeated runs of contiguous guanines undergo structural rearrangement to form planar quartets joined by Hoogstein hydrogen bonds of four guanine residues. Based on X-ray fiber diffraction data, a G-quartet was described for poly rG (Zimmerman et *al.* (1975) J. Mol. Biol. 92:181-192). In the late 1980s, this structure was identified in DNA sequences containing short runs of guanines interrupted by several bases, and such DNA sequences are found in immunoglobulin switch regions (Sen, D. and Gilbert, W. (1988) Nature 334:364-366) and in telomeres (Williamson et *al.* (1989) Cell 59:871-880). The G-quartet structure can occur within a single DNA strand (intramolecular G-quartet) or between two (Sundquist et al. (1989) Nature ,342:825-829: Kang et al. (1992) Nature 356:126-131) or four strands (intermolecular G-quartets) (Sen, (1988) supra). After the initial report on G-quartet structure on the homopolymer, poly rG, much attention on the structure has been focussed on DNA. However, in recent reports (Sundquist et *al*. (1993) Proc. Natl. Acad. Sci. USA 90:3393-3397; Awang et *al*. (1993) Biochem. 32:11453-11457) the G-quartet structure has been identified in heterogenous RNA molecules such as HIV-1 genomic RNA. Nucleic acid ligands with G-quartet structures are attractive candidates for developing nucleic acid mimetic molecules. The replacement of connecting loop nucleotides by a synthetic linker as a possible basic modification in an intramolecular tetraplex was examined in Example 22. In such a molecule, the synthetic tether should allow the folding to assume a G-quartet structure, as shown in Figure 44.

## V. HIGH-AFFINITY NUCLEIC ACID LIGANDS THAT DISCRIMINATE BETWEEN THEOPHYLLINE AND CAFFEINE - COUNTER-SELEX.

The present invention applies the SELEX and counter-SELEX procedures to the specific targets of caffeine and theophylline. Examples 23-29 describe the experimental parameters used to isolate and identify the nucleic acid ligands to theophylline and caffeine.

Specifically, this invention includes the nucleic acid ligands of theophylline shown in Figures 48A and 48B and the specific nucleic acid ligands of caffeine shown in Figure 54. The scope of the ligands covered extends to all ligands of theophylline and caffeine modified and unmodified, identified according to the SELEX and counter-SELEX procedures. More specifically, this invention includes nucleic acid sequences that are substantially homologous to and that have substantially the same ability to bind theophylline and caffeine as the specific nucleic acid ligands shown in Figures 48 and 54.

A review of the sequence homologies of the ligands of theophylline and caffeine shown in Figures 48 and 54 shows that sequences with little or no primary homology may have substantially the same ability to bind theophylline and caffeine. For these reasons, this invention also includes nucleic acid ligands that have substantially the same three dimensional structure as the ligands presented herein and substantially the same ability to bind theophylline and caffeine as the nucleic acid ligands shown in Figures 48 and 54.

Specifically included within the scope of this invention are RNA ligands of theophylline and caffeine that contain 2'-$NH_2$ and/or 2'-F modifications of certain riboses of the RNA ligand.

The nucleic acid ligands and nucleic acid ligand solutions to theophylline and caffeine described herein are useful as diagnostic reagents. Further, the nucleic acid ligands to theophylline and caffeine described herein may be used beneficially for therapeutic purposes.

The present invention shows how SELEX can be applied to generate oligonucleotides with binding and discriminatory properties for theophylline and caffeine superior in certain respects to those of monoclonal antibodies. As is shown below, SELEX selected nucleic acid ligands to theophylline have an affinity for theophylline comparable to that of monoclonal antibodies but with a superior selectivity.

The present invention includes a novel modification of the SELEX method, herein termed "counter-SELEX". The counter-SELEX method is a powerful means of eliminating undesired sequences from a SELEX experiment. The significance of this technique is that it provides a means for increased specificity of SELEX pools. Also it provides a methodology for decreasing the number of SELEX selection rounds required to identify a high affinity and high specificity nucleic acid ligand. Further, it provides a methodology for identifying a nucleic acid ligand of a target molecule that does not cross-react with other molecules, including closely related molecules.

Generally, the method of counter-SELEX is comprised of the steps of: a) preparing a candidate mixture of nucleic acids (The candidate mixture may contain nucleic acids with one or more types of modified nucleotides.); b) contacting the candidate mixture with the target molecule, wherein nucleic acids having an increased affinity to the target relative to the candidate mixture will bind the target molecule and may be partitioned from the remainder of the candidate mixture (The target molecule may be, but is not necessarily attached to a column); c) partitioning the increased affinity nucleic acids from the remainder of the candidate mixture; d) contacting the increased affinity nucleic acids with one or more non-target molecules such that nucleic acids bound to the target molecule with affinity to a non-target molecule are removed;.and e) amplifying the nucleic acids with specific affinity to the target molecule to yield a mixture of nucleic acids enriched for sequences with a relatively higher affinity and specificity to the target molecule.

In one embodiment of the counter-SELEX method, counter-SELEX is conducted with oligonucleotides bound to a column. This embodiment is used to eliminate oligonucleotides with undesired cross-reactivities which bind the target molecule. Briefly, the target molecule is bound to a column, and a candidate mixture of oligonucleotides applied to the column such that oligonucleotides binding the target molecule are retained in the column. The column is then washed with the binding buffer to remove non-binding oligonucleotides. Then one or more non-target molecules, or counter-ligands, to which cross-reactivity is undesirable, are applied in solution to the column in vast molar excess. Molar excess may be defined as $10^3$-$10^4$ times the amount bound to the column. This results in the removal of bound oligonucleotides from the column which also bind the counter-ligand(s). The target molecule is then applied to the column in solution, in vast molar excess, to remove oligonucleotides with the desired ligand binding specificity. Counter-SELEX may be conducted with a single counter-ligand molecule, with sequential exposure to more than one counter-ligand, or with a cocktail of several related counter-ligands, to eliminate oligonucleotides with cross-reactivity to several compounds. Further, counter-SELEX may be conducted after pre-selection of oligonucleotides for affinity for a target molecule by one or more selection rounds of SELEX.

In a second embodiment of the counter-SELEX method, counter-SELEX is conducted in solution. This approach is designed to eliminate oligonucleotides with undesired cross-reactivity in solution. In this embodiment of counter-SELEX, the binding of oligonucleotides to a target on a column is performed with the counter-ligand present in the binding buffer. Once the oligonucleotides are bound to the target, the column is vigorously washed. This procedure prevents oligonucleotides with cross-reactivity to the counter-ligand from binding to the column. This embodiment of the counter-SELEX method may be used in SELEX experiments for binding to peptides with sequences related to other peptides. This embodiment is intended to eliminate possible side effects due to cross-reactivity of SELEX selected oligonucleotides with other target molecules *in vivo.* For example, SELEX may be performed to identify nucleic acid ligands to a peptide hormone such as vasointestinal peptide (VIP). The amino acid sequence of VIP is similar to that of the peptide hormone glucagon, which has several essential roles in vivo. With the second embodiment of counter-SELEX, nucleic acid ligands to VIP are identified which do not cross-react with glucagon in solution, and therefore, would not cross-react with glucagon in vivo. This embodiment of the counter-SELEX procedure may be performed with one or more counter-ligand molecules in solution to eliminate nucleic acid ligands with cross-reactivity to more than one target molecule in *vivo.* Further, counter-SELEX may be conducted after one or more selection rounds of SELEX.

The utility of the counter-SELEX procedure for accelerating the enrichment of the target molecule binding nucleic acid species is illustrated by comparing the binding properties of pooled RNA after an identical number of selection rounds of SELEX and counter-SELEX. After three SELEX selection rounds, 12% of RNA initially bound to immobilized theophylline was resistant to subsequent elution with caffeine (TR8). After three counter-SELEX selection rounds with caffeine, 45% of initially bound RNA was resistant to caffeine elution (TCT8).

DNA sequence analysis was carried out on double-stranded cDNA populations derived from the pools of the 8th selection round SELEX RNAs (TR8) (Example 23) and counter-SELEX (TCT8) (Example 24). This analysis revealed that both pools were decidedly non-random in sequence (data not shown). The cDNAs were inserted into plasmids and bacterial clones generated. From plasmid DNAs isolated from the bacterial clones, the relevant regions encoding the RNA ligands were amplified by PCR and their sequences determined. The sequence of eleven TCT8 and eight TR8 clones are shown in Figure 48A and B (SEQ ID NOS:246-258). The ligands selected to bind to theophylline show a remarkable 15 base sequence which is completely conserved at 14 positions and present in each TCT8 and TR8 clone. This sequence is comprised of two separate motifs: motif 1 is 5'-AUACCA-3' (SEQ ID NO:244) and motif 2 is 5'-CCUUGG (C/A)AG-3' (SEQ ID NO:245). The sequences and spacing between motifs 1 and 2 are variable among the ligands, ranging from 8 to 20 residues.

Inspection of the sequences located between the motifs in the different ligands provides important information about the potential secondary structures of these RNAs. It is possible to fold all of the TCT8 and TR8 ligands into a similar generic secondary structural conformation, shown in Figure 50. These conformations show the variable region and limited regions of the flanking fixed regions postulated to participate in the secondary structure. Several features of these conformations are notable. Specifically, each ligand contains two stems of variable length and sequence loop, termed "vstem1" and "vstem2", and a variable length and sequence loop, termed "vloop". These structural features flank the conserved motifs in the model. Motif 1 and 2 are based-paired through interaction of the CCA triplet of motif 2 with the UGG triplet of motif 1. In all cases, the CCU triplet of motif 1 is depicted as an asymmetric bulge. The model accommodates the remaining six nucleotides of the conserved region as a symmetric bulge consisting of the AUA of motif 2 opposed to (C/A)AG of motif 1. This bulge is closed by a stem of variable length and sequence. The relative spatial orientation of the CCU and (C/A)AG components is clearly specific in this family of ligands since their orientations are preserved whether the CCU component is located 3' (e.g., TCT8-5, TCT8-7, TCT8-8, TCT8-1, TCT8-6 (SEQ ID NOS:247, 250, 252, 248)) or 5' (e.g., TCT8-11, TCT8-3 (SEQ ID NOS:254-255)). It is notable that closure of the symmetric bulge often involves participation of the fixed sequences, either from the 5' (e.g., TCT8-5 (SEQ ID NO:247)) or 3' regions (e.g., TCT8-7 (SEQ ID NO:250)). The conservation of the 15 nucleotides in each clone suggests that the remaining structural components of variable sequence such as vloop and vstems 1 and 2 do not play a direct role in binding theophylline. The vloop and vstems may play a structural role in positioning a binding surface or pocket comprised of the conserved bases. A consensus secondary model is depicted in Figure 50.

The present invention shows that nucleic acids can interact specifically with a therapeutically important small molecule, further confirming the possible use of these oligonucleotides as diagnostic reagents. Currently, monoclonal antibodies are commonly used to determine serum theophylline levels (Poncelet et al. (1990) J. Immunoassay 11:77). The affinity of the RNA ligands for theophylline is comparable to that of the antibodies used in clinical assays (Poncelet et *al.* (1990) supra). Although these antibodies vary in their ability to discriminate amongst related compounds, typically they show 0.1-1% cross-reactivity with compounds such as caffeine and theobromine. The discrimination of the RNA ligands described here is at least an order of magnitude greater than such antibodies. These results help establish the fact that oligonucleotides can display unexpected recognition and specificity properties.

In addition to the work described here, the structural versatility of RNA is attested to by its ability to interact with other small molecules. These include the interaction of type I introns with guanosine (Bass and Cech (1984) Nature 308:820) and arginine (Yarus (1988) Science 240:1751), the binding of arginine by HIV TAR RNA (Tao and Frankel (1992) Proc. Natl. Acad. Sci. USA 89:2723), and the binding of a set of uncharacterized RNAs with amino-linked tryptophan agarose (Famulok and Szostak (1992) J. Amer. Chem. Soc. 114:3990). Of particular relevance to the work described here are the observations of stereoselective recognition by RNA of arginine (Yarus (1988) *supra)* and tryptophan (Famulok and Szostak (1992) supra). The detailed structure of the binding pockets of these ligands has been the subject of intense study. An important feature of the SELEX process is that it yields families of sequences whose similarities reveal important higher order structural clues. These clues provide starting points for modeling and analysis of structure by chemical and physical methods. In addition, the counter-SELEX procedure described herein may be used to accelerate the rate at which specific ligands are obtained, and more importantly, to selectively remove from the oligonucleotide population those species with affinity for targets that are structurally closely related to the target of interest. The present invention verifies the potential application of SELEX to include virtually any molecule of biomedical interest, irrespective of size and chemical class.

Example 23 describes the general experimental procedures used in the present work, including the equilibrium filtration analysis and the selection of the theophylline family of ligands TR8 after 8 rounds of selection of SELEX. Example 24 describes the selection of the theophylline ligand family TCT8, selected after 5 selection rounds of SELEX and 3 selection rounds of counter-SELEX with caffeine. Example 25 describes the identification of the caffeine ligand family CR8 after eight rounds of selection of SELEX. Example 26 describes the binding characteristics of a representative theophylline RNA ligand (TCT8-4 (SEQ ID NO:249)). The results show that TCT8-4 bound theophylline with a Kd of 0.58 μM, similar to that observed for some monoclonal antibodies. A truncated ligand was constructed containing the conserved motif of TCT8-4 (mTCT8-4), and found to bind theophylline with a Kd of 0.11 μ*M,* approximately 5.8-fold greater than the full-length ligand. Example 27 describes competition binding studies conducted with xanthine derivatives for RNA ligands for theophylline. These studies allow investigation of the relative contributions of various constituents to the overall binding. Example 28 describes aspects of molecular modeling of high affinity ligands for theophylline.

The following examples are explanatory and exemplary of the present invention.

## EXAMPLE 1. EXPERIMENTAL PROCEDURES.

SELEX. Essential features of the SELEX protocol have been described in detail in U.S. Patent No. 5,270,163 as well in previous papers from these laboratories (See, *e.g.,* Tuerk & Gold (1990) Science 249:505; Tuerk et *al*. (1992a) Proc. Natl. Acad. Sci. USA 89:6988; Tuerk et *al*. (1992b) in Polymerase Chain Reaction (Ferre, F, Mullis, K., Gibbs, R. & Ross, A., eds.) Birkhauser, NY)). Briefly, DNA templates for in vitro transcription (that contain a region of thirty random positions flanked by constant sequence regions) and the corresponding PCR primers were synthesized chemically (Operon). The random region was generated by utilizing an equimolar mixture of the four nucleotides during oligonucleotide synthesis. The two constant regions were designed to contain PCR primer annealing sites, a primer annealing site for cDNA synthesis, T7 RNA polymerase promoter region, and restriction enzyme sites that allow cloning into vectors.

### Nitrocellulose Filter Binding Assay.

Oligonucleotides bound to proteins can be effectively separated from the unbound species by filtration through nitrocellulose membrane filters (Yarus & Berg (1970) Anal. Biochem. 35:450; Lowary & Uhlenbeck (1987) Nucleic Acids Res. 15:10483; Tuerk & Gold (1990) supra). Nitrocellulose filters (Millipore, 0.45 um pore size, type HA) were secured on a filter manifold and washed with 4-10 ml of buffer. Following incubations of $^{32}P$-labeled RNA with serial dilutions of the protein (5-10 min) at 37˚C in buffer (PBS) containing 0.01% human serum albumin (HSA), the solutions were applied to the filters under gentle vacuum in 45 ul aliquots and washed with 5 ml of PBS. The filters were then dried under an infrared lamp and counted in a scintillation counter.

Cloning and Sequencing. Individual members of the enriched pools were cloned into pUC18 vector and sequenced as described (Schneider et *al*. (1992) J. Mol. Biol. (in press); Tuerk & Gold (1990) supra).

## EXAMPLE 2. MODIFIED RNAS.

Incorporation of 5-modified uridines into RNA. Four prototypic 5-modified uridines, 5-AA-UTP, 5-F-12-UTP, 5-Dig-11-UTP, and 5-Br-UTP (shown in Figure 2) were incorporated into RNA by the T7.RNA polymerase under standard transcription conditions (40 mM Tris-Cl, pH 8.0, 12 mM $MgCl_2$, 5 mM DTT, 1 mM spermidine, 0.002% Triton X-100, 4% PEG, 37˚C). Autoradiogram of a 15% denaturing polyacrylamide gel showing products of transcription done with UTP, 5-AA-UTP, and 5-Br-UTP is shown in Figure 3. The efficiency of transcription was estimated from the total amounts of $^{32}P$-radiolabelled transcript produced after three hours of incubation with 2 mM ribonucleotide triphosphates, 100 nM double-stranded DNA template, and 5 units/ul of T7 RNA polymerase (NEB). 5-AA-UTP, 5-F-12-UTP, 5-Dig-11-UTP, and 5-Br-UTP were incorporated with 28%, 51%, 23%, and 50% efficiency, respectively, as compared to UTP. No transcription was detectable when UTP was omitted from the transcription mixture.

Modified transcripts containing 5-AA-UTP, 5-F-12-UTP, 5-Dig-11-UTP, or 5-Br-UTP were reverse transcribed into cDNA by AMV reverse transcriptase with efficiencies comparable to that of the unmodified RNA (done in 50 mM Tris, pH 8.3, 60 mM NaCl, 6 mM $Mg(OAc)_2$, 10 mM DTT, 37˚C) (data not shown). These cDNAs were then amplified by PCR. Sequencing of the PCR products derived from UTP, 5-AA-UTP, and 5-Br-UTP transcriptions revealed that identical products were obtained in all three cases (Figure 4). These results show that modified nucleotides can be directly incorporated into the RNA SELEX procedure.

## EXAMPLE 3. 2'-$NH_2$ RNA LIGANDS FOR THROMBIN.

2'-$NH_2$ modified RNA. RNA was transcribed with T7 RNA polymerase from double-stranded DNA in a reaction containing ATP, GTP, 2'-$NH_2$-UTP, and 2'-$NH_2$-CTP.

2'-OH and 2'-$NH_2$ RNA SELEX for thrombin. SELEX experiments with thrombin were done with 2'-OH RNA and 2'-$NH_2$ RNA under almost identical conditions. A 30N3 template/primer set was used in both experiments. SELEX with 2'-OH RNA was conducted in 1X binding buffer (50 mM Tris-Cl, pH 7.7, 100 mM NaCl, 1 mM $MgCl_2$, and 1 mM DTT). SELEX with 2'-$NH_2$ RNA was conducted in 1X binding buffer without DTT. Approximately equimolar concentrations of RNA and protein were used in the 2'-OH RNA experiment. 2'-$NH_2$ bulk RNA had a higher starting affinity for thrombin ($kD_{2'-OH}$ = >1.0 x $10^{-6}$; $kD_{2'-}$NH2' = < 1.0 x $10^{-6}$) and thus the protein concentrations used in this SELEX experiment were higher. Both of the experiments began with the same concentration of RNA (2.5 x $10^{-7}$) and were done at equimolar RNA and protein, or protein excess.

Sequence. Two predominant classes (2'-OH) or groups (2'-$NH_2$) of sequences were obtained in each experiment (Figure 6 (SEQ ID NOS:1-39)). For the 2'-OH RNAs, class I contained 22/26 clones, and class II contained 6/28 clones. For the 2'-$NH_2$ RNAs, group I contained 15/47 clones, group II contained 30/45 clones, and group III contained 2/47 clones. Each class showed remarkable homology within the 30N region.

Secondary Structural Analysis. Secondary structures were predicted for the high affinity 2'-OH and 2'-$NH_2$ RNA ligands to thrombin shown in Figure 7. Clone 17 (SEQ ID NO:36) is representative of the A subgroup of the group II 2'-$NH_2$ ligands. Clone 16 (SEQ ID NO:7) is representative of the class I ligands of the 2'-OH ligands and clone 27 (SEQ ID NO:18) is representative of the class II ligands of the 2'-OH ligands.

Binding Analysis. All binding analyses were done by nitrocellulose filter binding. Binding curves as determined by increasing protein concentration in the presence of a fixed RNA concentration lower than the protein concentration are shown for the 2'-OH ligands in Figure 8A and for the bulk 2'-$NH_2$ ligands in Figure 8B. Under these conditions, the RNAs 16 and 27 have kDs of 30 nM and 60 nM, respectively. The kD for the bulk 2'-$NH_2$ round 15 ligands which had been sequenced is approximately 200 nM.

## EXAMPLE 4. SYNTHESIS OF 5-IODO,2'-AMINO DEOXYURIDINE.

The reported synthesis of 5-iodo,2'-amino,2'-deoxyuridine (Verheyden et al. (1971) J. Org. Chem. 36:250) involved five steps and gave the desired compound in an overall yield of 3%. Presented herein is a method of preparing 5-iodo,2'-amino,2'-deoxyuridine in four steps from uridine with an overall yield of 46%. The synthesis of 5-iodo,2'-amino,2'-deoxyuridine is shown in Figure 9. In the method presented below, uridine is initially dehydrated to the 2,2'-anhydrouridine by the standard method in 92% yield. *Id.* This intermediate is then converted to the 2'-azido,2'-deoxyuridine in 84% yield according to Example 7 below (Synthesis of 2'-Azido-2'-Deoxyuridine (ADU)). ADU is iodinated at the C-5 position without protection of the 5'- and 3'-OH groups in 76% yield in adaptation of the method of Robins et *al.* (1982) Can. J. Chem. 60:554. Reduction of the 5-iodo,2'-amino,2'-deoxyuridine with triphenylphosphine gave 78% yield of the desired compound.

Synthesis of 2,2'-Anhydrouridine. To a suspension of uridine (200 g, 819 mmoles) in hexamethylphosphoramide (HMPA) (400 ml) was added sodium bicarbonate (5.3 g, 63.1 mmoles, 0.077 equivalents (eq.)) and diphenyl carbonate (228.1 g, 1064.7 mmoles, 1.3 eq.). The mixture was heated to 135 °C for 11 h. After cooling to room temperature, diethyl ether (2 1) was added and the precipitate was filtered off. The solid was washed three times with ether (3x1 l). Water (1 1) was added to the filtrate, the aqueous layer was separated and extracted three times with methylene chloride (3 x 1 1). The aqueous phase was evaporated to dryness. The combined solids were recrystallized from hot methanol to yield 170 g (92%) of 2,2'-anhydrouridine. The spectral properties agreed with those reported in the literature (Hampton and Nichol (1966) Biochemistry 5:2076).

Synthesis of 2'-Azido, 2'-deoxyuridine. The synthesis of 2'-azido-2'-deoxyuridine (ADU) has been reported by Moffat et *al.* (1971) J. Org. Chem. 36:250. According to this procedure, anhydrouridine is treated with 7.1 equivalents of lithium azide in HMPA at 150 °C. The desired product is obtained in only 50% yield after separation from a number of by-products. It is desirable to have a process by which ADU is synthesized in high yield without the necessity of further purification by chromatographic processes. It is also desirable to avoid the use of HMPA, a known carcinogen.

A procedure was developed which utilizes an *in* situ generation of lithium azide in the presence of N',N',N",N"-tetramethylethylenediamine (TMEDA) in DMF at 100-110 °C. When anhydrouridine is allowed to react with 1.5 equivalents of this lithium azide:TMEDA complex in DMF at 105-110 °C for 24-48 h, ADU is formed in high yield as the sole product. The process described represents a substantial improvement over the previously known method.

Lithium fluoride (0.93 g, 36 mmol) was suspended in 20 ml of DMF heated to 105 °C. To the stirred suspension was added 20 ml of TMEDA followed by azidotrimethylsilane (4.15 g, 36 mmol). After stirring for 30 min, anhydrouridine (4.52 g, 20 mmol) was added and the reaction allowed to proceed for 48 h. The solvents were removed under vacuum and the residue co-evaporated from methanol three times. The residue was dissolved in 10 ml of methanol and 40 ml of ethyl acetate was added to precipitate most of the salt and residual starting material. The filtered solution was applied to 170 g of flash silica gel and eluted with 20% methanol/ethyl acetate. The product was obtained as an off-white solid (3.38 g, 63%). $^{1}H$ NMR (DMSO-$d_6$, 300 MHz) d 3.57 (dd, 1H, J=2.9, 12.2 Hz, $H_{5A'}$), 3.66 (dd, 1H, J=2.4, 1.2. Hz, $H_{5B'}$), 3.89 (dd, 1H, J=2.9, 4.4 Hz, $H_{4'}$), 4.05 (dd, 1H, J=5.4, $H_{2'}$), 4.30 (dd, 1H, J=4.4, 5.4 Hz, $H_{3'}$), 5,18 (br s, 1H, -OH), 5.67 (d, 1H, J=8.3 Hz), 5.88 (d, 1H, J=5.4 Hz, $H_{1'}$), 5.96 (br s, 1H, -OH), 7.86 (d, 1H, J=7.8 Hz), 11.4 (br s, 1H, NH). $^{13}C$ NMR (DMSO-$d_6$, 75 MHz) d 60.20, 64.57, 70.42, 85.21, 85.55, 102.03, 139.98, 150.40, 162.96.

Synthesis of 5-Iodo,2'-azido,2'-deoxyuridine. To a solution of 2'-azido,2'-deoxyuridine (2.5 g, 9.3 mmoles) in methanol (20 ml) is added ICl (3.3 g, 20.5 mmoles, 2.2 eq.). The mixture is heated at 50°C for 30 min. After cooling to room temperature, an equal volume of ether is added and the white precipitate is filtered off. The precipitate is stirred in methanolic ammonia (20 ml) at room temperature for 15 min, and then evaporated to dryness to yield 2.81 g (76%) of 5-iodo,2'-azido,2'-deoxyuridine. $^{1}H$ NMR (300 MHz, methanol-$d_4$) $\delta$ 8.58 (s, 1H, $H_6$), 5.88 (d, 1H, $H_{1'}$, $J_{H1'}$ ,H2' = 3 . 5 Hz), 4.46 (t, 1H, $H_{3'}$, J = 5.8 Hz), 4.11 (dd, 1H, $H_{2'}$, $JH_{1',H2'}$ = 3.9 Hz, J $H_{2',H3'}$ = 5.3 Hz), 4.01 (m, 1H, $H_{4'}$) , 3.91 (dd, 1H, $H_{5'A}$ $J_{gem}$ = 12.3 Hz, $J_{H5',H4'}$ = 2.4 Hz) 3.76 (dd, 1H, $H_{5'B}$, $J_{gem}$ = 12.3 Hz, $J_{H5',H4'}$ = 2.4 Hz). $^{13}C$ NMR (75 MHz, methanol-$d_4$) $\delta$ 163.03, 152.17, 146.50, 88.88, 86.18, 71.21, 67/67, 61.00, 48.15. FAB mass spectrum, (M+H) m/z 396.

Synthesis of 5-iodo,2'-amino,2'-deoxyuridine. To a solution of 5-iodo, 2'-azido, 2'-deoxyuridine (2.48 g, 6.3 mmoles) in dioxane (33 ml) is added triphenylphosphine (5.8 g, 22 mmoles, 3.5 eq.) and the mixture is stirred at room temperature for 1 h, during which time a white precipitate forms. The precipitate is collected and washed three times with diethyl ether (3 x 50 ml). The filtrate is evaporated to dryness, taken up in a minimum amount of dioxane, and stored at -20°C for 48 h. The precipitate is collected and washed with diethyl ether. The combined precipitates gave 1.81 g of 5-iodo,2'-amino,2'-deoxyuridine (78% yield). The spectral properties agreed with those reported in the literature (Verheyden et al. (1971) supra).

## EXAMPLE 5. SYNTHESIS OF 5-IODO.2'-AMINO.2'-DEOXY PYRIMIDINE 5'-TRIPHOSPHATES.

The synthesis of 2'-amino,2'-deoxy pyrimidine 5'-triphosphates has not previously been reported in full detail. The 2'-amino,2'-deoxyuridine 5'-triphosphate was prepared via 5'-phosphorylation of 2'-azido,2'-deoxyuridine and subsequent reduction (Aurup et *al.* (1992) Biochemistry 31:9636). This route requires protection of the 3'-OH group, which can only be achieved selectively with intermittent protection of the 5'-OH group. Herein described is a method of preparing the compound in only two steps from 2'-amino,2'-deoxyuridine. The strategy relied on herein is based on the hypothesis that protection of the 3'-OH group is not necessary if the 2'-$NH_2$ group carries a protecting group that blocks the 3'-OH during phosphorylation. The synthesis of 2'-amino,2'-deoxyuridine 5'-triphosphate herein described is shown in Figure 10.

The 2'-amino group of 2'-amino,2'-deoxyuridine is trifluoroacetylated (Imazawa et *al.* (1979) J. Org. Chem. 44: 2039) in 82% yield. This intermediate is phosphorylated at the 5'-position (Ludwig and Eckstein (1989) J. Org. Chem. 54:631) in 61% yield. The 5-iodo derivative of the 2'-amino,2'-deoxyuridine 5'-triphosphate was prepared by mercuration (Dale et *al.* (1975) Biochemistry 14:2447) in a single step. Both 2'-amino,2'-deoxyuridine 5'-triphosphate and 5-iodo,2'-amino,2'-deoxyuridine 5'-triphosphate were shown to be substrates for T7 RNA polymerase. This has never been demonstrated before for the latter compound.

2'-Trifluoroacetylamino,2'-deoxyuridine. To a suspension of 2'-amino,2'-deoxyuridine (2.0 g, 8.22 mmoles) in methanol (150 ml) was added S-ethyl trifluorothioacetate (1.6 ml, 12.48 mmoles, 1.5 eq.). This mixture was stirred at room temperature overnight. Nitrogen was bubbled through the reaction mixture for 2 h to remove volatile thio-byproducts. The mixture was evaporated to dryness and the solid was recrystallized from methanol/chloroform to yield 2.3 g (82%) of 2'-trifluoroacetylamino,2'-deoxyuridine. The analytical data agreed with the literature (Imazawa (1979) *supra).*

2'-Amino,2'-deoxyuridine 5'-triphosphate. 2'-(N-trifluoroacetyl)amino,2'-deoxyuridine (230 mg, 0.68 mmoles) was dried under vacuum for 3 h prior to dissolution in anhydrous dioxane (2 ml) and anhydrous pyridine (0.68 ml). To this solution was added a freshly prepared solution of 2-chloro-4H-1,2,3-dioxaphosphorinone in anhydrous dioxane (152 mg, dissolved to 1M). The reaction was stirred at room temperature for 10 min prior to addition of a solution of bis(tri-n-butylammonium) pyrophosphate in anhydrous DMF (2.04 ml, 1.02 mmoles, 1.5 eq.) and tributylamine (162 μl, 0.68 mmoles). After 15 min at room temperature an iodine solution was added (279 mg, 1% solution, pyridine/water 98:2 v/v). The mixture was stirred at room temperature for an additional 15 min and the excess iodine destroyed with aqueous $NaHSO_4$ (5% solution). The mixture was evaporated to dryness and the residue taken up in water (10 ml). After addition of concentrated ammonia (10 ml) the mixture set at room temperature for 1 h. The product was purified by DEAE Sephadex A25 (Pharmacia) chromatography (0-1 M triethylammonium bicarbonate buffer). To remove excess buffer salt, the product was repeatedly concentrated from methanol to yield 364 mg (62%) tetrakis (triethylammonium)-2'-amino,2'-deoxyuridine 5'-triphosphate. $^1$H NMR (300 MHz, methanol-$d_4$) δ 7.21 (d, 1H, $H_{6'}$ $J_{H6,H5}$ = 8.1 Hz), 5.33 (d, 1H, H1', $J_{H1',H2'}$ = 7.9 Hz), 5.05 (d, 1H, $H_5$, $J_{H6,H5}$ = 8.1 Hz), 3.82 (dd, $H_3$, , $J_{H3',H2'}$ = 5.3 Hz, $J_{H3',H4'}$ = 1.6 Hz, 3.49 (m, 1H, HA), 3.42 (m, 1H, $H_{4'}$), 3.38 (m, 1H, $H_{5',B}$), 3.08 (dd, 1H, $H_2$, $J_{H1',H2'}$ = 7.9 Hz, $J_{H2',H3'}$ = 5.3 Hz), 2.52 (s, 1H, OH), 2.38 (q, 24H, $-CH_2$), 0.52 (t, 36H, $-CH_3$). $^{31}$P NMR (121.5 MHz, methanol-$d_4$, $H_3PO_4$ as external standard) δ -8.24 (d, $P_\alpha$, $J_{p\gamma p\beta}$ = 21 Hz), -11.27 (d, $P_\alpha$, $J_{P\alpha,P\beta}$ = 21 Hz), -22.43 (t, $P_\beta$, J = 21Hz).

5'Iodo,2'-amino,2'-deoxyuridine 5'triphosphate. To a solution of tetrakis(triethylammonium)-2'-amino,2'-deoxyuridine 5'-triphosphate (35.2 mg, 0.02 mmoles, 0.02 M) in sodium acetate (0.1 M, 2 ml, pH 6.0) was added a solution of mercuric acetate (63.74 mg in 0.1 M sodium acetate, 0.01 M). The mixture was heated at 50 °C for 3 h. After cooling to room temperature, a solution of iodine in ethanol (25.38 mg $I_2$ in 80 μl ethanol) was added. The mixture was stirred at room temperature in the dark for 1 h. The product was purified by DEAE Sephadex-A25 (Pharmacia) chromatography (0-1 M triethyl ammonium bicarbonate buffer). To remove excess buffer salt the product was repeatedly concentrated from methanol to yield 6 mg (30%) tetrakis(triethylammonium)-5-iodo,2'-amino,2'-deoxyuridine 5'-triphosphate. UV: λmax 273 nm.

EXAMPLE 6. IN VIVO STABILITY OF 2'-$NH_2$ MODIFIED RNA LIGANDS OF THROMBIN.

A study was conducted to evaluate the plasma kinetics of a thrombin ligand (38-mer) (PK-5) modified with 2'-$NH_2$ groups on the pyrimidines.

Plasma kinetic curves were obtained for five rats. Each of five male Sprague-Dawley rats was injected intravenously with 1 μCi of $^{32}$P labelled oligonucleotide. Blood samples were collected at 15 and 30 minutes and at 1, 2, 4, 7, and 24 hours into tubes containing EDTA. Plasma was separated from the cells and phenol precipitated. 10 μl samples of the plasma were quantitated for radioactivity by scintillation counting. Figure 11 shows the plasma kinetic curves obtained for each experimental animal.

The shape of the plasma kinetic curve was similar to that observed with a 2'-$OCH_3$ stabilized oligonucleotide of similar length which distributes rapidly into total body water and remains stable in vivo for at least 6 hours. A distribution phase half-life of 4 minutes was reported for an unstabilized, phosphodiester 20-mer oligodeoxynucleotide administered intravenously to rabbits (Goodchild et *al.* (1991) Antisense Res. and Dev. 1:153) . No published in vivo data were found for plasma half-life of oligoribonucleotides. However, in vitro data have shown them to be even less stable than oligodeoxynucleotides in the presence of serum nucleases (Crooke (1992) Annu. Rev. Pharmacol. Toxicol. 32:329).

EXAMPLE 7. INTRODUCTION OF FUNCTIONAL GROUPS.

5-AA-UTP and 5-AA-dUTP contain a primary amine functionality which is useful since a wide variety of other functional groups can be readily introduced by the carbodiimide/N-hydroxysuccinimide coupling, as shown by Muhlegger et al. (1989) Nucleosides & Nucleotides 8:1161 (Figure 5). Global physico-chemical properties of the modified oligonucleotides such as hydrophobicity or the overall charge can be modified with the addition of appropriate "side chains".

Additionally, molecules that are known to bind at well-defined sites on given target molecules may be used in this context to anchor the binding of nucleic acid ligands to specific regions on the target molecule. Such moieties themselves need only modest affinities for their target molecules since the oligonucleotide framework would be evolved to provide additional binding energy. An example of this strategy is given in Figure 5.

EXAMPLE 8. SYNTHESIS OF PEPTIDE CONJUGATED UTP.

Reagents. 5-(3-aminoallyl)-uridine 5'-triphosphate, sodium salt, was obtained from Sigma Chem. Co. (St. Louis, MO). Gly-Arg-Gly-Asp-Thr-Pro (SEQ ID NO:40) was obtained from GIBCO-BRL (Gaithersburg, MD). 1-ethyl-3-(dimethylaminopropyl) carbodiimide hydrochloride (EDC) was obtained from Pierce Chemical Co. (Rockford, IL). Other chemicals were of highest quality available from Aldrich Chemical Company (Milwaukee, WI).

Succinylation of AA-UTP. The peptide Gly-Arg-Gly-Asp-Thr-Pro (SEQ ID NO:40) was coupled to UTP via a two-step reaction through the N-terminal α-amino group of the peptide. This α-amino group is the only reactive amine in the peptide and is not required for integrin binding. Condensation with peptide carboxyl groups could function to block the aspartic acid residue, thus preventing integrin binding.

5-(3-aminoallyl)UTP (5 mg) was dissolved in 250 μl of 0.2 M sodium borate, pH 8.5. The solution was mixed with 750 μl succinic anhydride (97 mg/1.14 ml DMF) and reacted for 2 h at 4°C. An equal volume of 0.1 M triethylammonium bicarbonate, pH 7.6, was added, filtered and purified on Mono Q 5x5 anion exchange FPLC (Pharmacia). The sample was applied in 0.1 M triethylammonium bicarbonate, and eluted with a linear gradient to 1.0 M triethylammonium bicarbonate. The column was eluted at 1.0 ml/min over 20 min. The major peak was pooled, lyophilized, and taken up in dry DMF for the next reaction. The sample was analyzed by TLC by spotting on PEI-F chromatography plates (J.T. Baker) and developing with 0.2 M sodium phosphate, pH 3.5.

Chromatographic isolation of succinyl-UTP. The succinylation reaction mixture was diluted 1:1 with 0.1 M triethylammonium-bicarbonate, pH 7.6 (Buffer A), filtered and applied to a 0.5 cm x 5 cm Mono Q anion exchange column which was equilibrated with the same buffer. The column was eluted with a gradient to 1.0 M triethylammonium-bicarbonate (Buffer B) at a flow rate of 0.5 ml/ml (1 min/fraction) (Figure 13). Succinyl-UTP was easily separated by ion exchange from the starting material. This separation is achieved because succinyl-UTP elutes from Mono Q similar to UTP, whereas AA-UTP partially neutralizes the 5'-triphosphate and elutes earlier in the salt gradient. Succinyl-UTP eluted late in the gradient from the Mono Q column (92% of Buffer B), well separated from reactants and from the AA-UTP (which eluted at approximated 68% of Buffer B). The majority of UV adsorbing material eluted in the major peak, although several other peaks were present. Succinyl-UTP pooled from fractions 43-49 was lyophilized to remove the volatile buffer in preparation for the next step. The yield of this step was 80-95%. The dried sample was dissolved in dry DMF and its UV spectrum determined. This peak had the expected maximum at 290 nm.

Coupling of GRGDTP peptide to 5'(3-succinyl-aminoallyl) uridine 5'-triphosphate. The RGD peptide was condensed with succinyl-UTP using the water soluble carbodiimide. Succinyl-UTP in 58 μl of DMF was cooled to 4°C. 117 μl of N-hydroxy-succinimide (24 mg/1.4 ml DMF) was added and the mixture was reacted for 30 min at 4°C. 175 μl of EDC (0.1 M, pH 4.8) was added and the mixture reacted for 2 h at 4°C. The activated nucleotide was added to 14 mg of lyophilized GRGDTP and the mixture reacted for 2 h at 4°C. The reaction mixture was diluted 1:1 with 0.1 M triethylammonium bicarbonate, purified on a Mono Q column as above, and analyzed by TLC and Fast Atom Bombardment (FAB) Mass Spectrometry (University of Colorado Structural Division).

Chromatographic isolation of RGD-UTP. RGD-UTP was purified by ion exchange chromatography. The peptide coupling reaction mixture was diluted with Buffer A, filtered and purified on the Mono Q column described above. RGD-UTP eluted from the Mono Q column similar to AA-UTP, but ahead of succinyl-UTP, suggesting the arginine side chain also interacts with the phosphate charge. The major peak eluted at approximately 70% of Buffer B, ahead of the peak of residual succinyl-UTP (which eluted at approximately 90% of Buffer B) (Figure 14). Only trace amounts of other peaks were detected, except for the EDC which eluted earlier. Fractions 35-38 were pooled and lyophilized. The sample was dissolved in dry DMF for thin layer chromatography (TLC) and FAB mass spectroscopy. The sample had a maximum adsorption at 290 nm.

Thin layer chromatography of AA-UTP, succinyl-UTP. and RGD-UTP. As shown in Figure 15, RGD-UTP is cleanly separated from both AA-UTP and succinyl-UTP. AA-UTP, succinyl-UTP, and RGD-UTP were spotted onto cellulose PEI-F TLC plates (J. T. Baker, Inc.) and developed with 0.75 M $NaH_2PO_4$, pH 3.5. AA-UTP migrated near the solvent front, succinyl-UTP migrated with an $R_f$ of 0.42, and RGD-UTP remained at the origin. This TLC system is an ideal method for monitoring product formation.

Mass spectrometry analysis. RGD-UTP analyzed by FAB mass spectroscopy yielded a mass of 1223, remarkably close to the calculated formula weight (FW) of 1222. The precision of the mass spectrometry result shows that RGD was coupled in the predicted manner, and that no side reactions occurred introducing alternative or additional derivatives. Further, there were no smaller peaks present which would indicate degradation or dephosphorylation of the triphosphate.

EXAMPLE 9. BINDING OF RGD-RNA TO qpIIbIIIa.

Molecular biology techniques. Agarose electrophoresis, T7 RNA transcription, and AMV reverse transcription were performed generally as described in the SELEX Patent Applications. FPLC purification of RNA oligonucleotides was achieved using a 5x5 Mono Q anion exchange column. The RNA transcription reaction mixture was phenol:chloroform extracted, ethanol precipitated and washed, and dissolved in 50 mM sodium phosphate, 0.2 M NaCl, 6 M urea, 50% formamide (Fisher, Ultrapore, low-UV absorbance), pH 6.0. Following sample application, the column was developed with a linear gradient to 1 M NaCl. The RNA peak was precipitated by addition of 2:1 v/v ethanol, the pellet was washed with 80% ethanol and dried in a speed-vac, then dissolved in SELEX binding buffer (150 mM NaCl, 10 nM HEPES, 2 mM $CaCl_2$, 1 mM $MgCl_2$, 0.1% Tween, pH 7.4). RNA and RNA-gpIIbIIIa (Enzyme Research) complexes were prepared by incubation at 37°C for 10 min, and separated by size exclusion chromatography on a 16 x 100 Superdex 200 column (Pharmacia) which was equilibrated and run in the binding buffer. Concentration and removal of detergent from the samples collected from the Superdex column was achieved by adsorption onto a Resource Q column (Pharmacia) and elution with 1 M NaCl.

RGD-UTP transcription and purification. RGD-UTP was used in a T7 RNA polymerase reaction by direct substitution for normal UTP, and gave yields that were at least 50% of those obtainable with UTP. The concentration of RGD-UTP and other NTPs was 1 mM; the DNA template contained 5' and 3' fixed regions flanking a 30 base pair random sequence. The RNA transcript was purified by adsorption onto an anion exchange column (Mono Q)in a phosphate buffer containing 6 M urea and 50% formamide, and eluted with a gradient to 1 M NaCl (Figure 16). RGD-RNA eluted as one major peak from the column at a position in the gradient slightly ahead of the elution position of an equivalent RNA transcript made with normal UTP, consistent with the altered charge and perhaps altered conformation of the derivatized RNA. The amount of truncated RNA material was only slightly increased compared to normal RNA transcription.

Binding of RGD-30n7 RNA to gpIIbIIIa. Size-based partitioning, used to separate RGD-RNA bound to gpIIbIIIa integrin from unbound RGD-RNA, is a method for identifying high affinity RNA ligands to gpIIbIIIa (Kd < $10^{-8}$). RGD-RNA was incubated with gpIIbIIIa (2 mg/ml) in 0.15 M NaCl, 2 mM $CaCl_2$, 1 mM $mgCl_2$, 20 mM HEPES, 0.1% Tween 20, pH 7.4. The results showed that RGD-RNA incubated with gpIIbIIIa yielded a higher molecular weight RNA peak than obtained for samples not incubated with gpIIbIIIa. Normal RNA made with UTP and partitioned in the same manner did not have the high molecular weight peak, indicating that binding to gpIIbIIIa of RNA not coupled to the RGD peptide was weaker than that of the blended molecule. The estimated molecular mass is consistent with the addition of the RGD peptide at each uridine position (Figure 17). Perhaps more conclusively, chromatographically purified RGD-RNA had an absorbance shoulder, compared to normal RNA, at 310 nm resulting from incorporation of AA-UTP. A small peak of early eluting material was detected in the column void volume which was not present in samples of RGD-RNA chromatographed without protein. The high molecular weight RGD-RNA-gpIIbIIIa peak was concentrated and detergent removed by adsorption and elution from an anion exchange column. The RNA was reversed transcribed and PCR amplified. The DNA obtained yielded a single band of expected size on agarose electrophoresis, indicating that RGD-RNA is reverse transcribed with acceptable efficiency even with the low amounts of RNA extracted during SELEX partitioning.

EXAMPLE 10. SUBSTRATE-COUPLED SELEX-DERIVED BLENDED LIGANDS AS ELASTASE INHIBITORS.

To demonstrate that a high affinity, high specificity nucleic acid ligand coupled to an inhibitor peptide is a viable method of producing a high affinity inhibitor to elastase, a ssDNA ligand to elastase was coupled to the inhibitory substrate peptide chloromethyl ketone, such that the chloromethyl functionality is inactivated by creating a non-hydrolyzable linkage. ssDNA ligand 17 (DNA-17), having the sequence TAGCGAT ACTGCGTGGGTTGGGGCGGGTAGGGCCAGCAGTCTCGTGCGGTACTTGAGCA (SEQ ID NO:42) has a Kd for elastase of 15 nM. Because it was not known which end of DNA-17 is in close proximity to the active site of elastase, blended nucleic acid molecules were prepared in which the substrate peptide was attached to both the 3' and 5' ends of the DNA.

Peptide conjugation. The chemistry of the attachment is shown in Figure 18. An oligonucleotide with four 18-atom ethylene glycol moieties (synthesized by using spacer phosphoramidite, Clonetech) and a thiol group at the 3' end was synthesized by automated DNA synthesis, deprotected by standard methods, and gel purified. Immediately after the deprotection of the 3'-SH group, the oligonucleotide was passed through a Sephadex-G50 spin column equilibrated in 0.5 M triethylammonium acetate buffer (pH 7.5) to remove excess DTT, and then mixed with N-methoxysuccinyl Ala-Ala-Pro-Val-chloromethyl (SEQ ID NO:41) ketone (25 mg/200 ul of DMF). The mixture was incubated at 37°C overnight. 20 μl of 1 M DTT was added to inactivate the unreacted chloromethyl ketone inhibitor. The peptide conjugated DNA was finally purified from the unconjugated peptide either by three successive Sephadex-G50 spin columns or by reverse phase HPLC.

Elastase inhibition assay. The assay for elastase inhibition is based on the use of a chromogenic tetrapeptide substrate. The assay was conducted in a buffer containing 10 nM elastase, 0.5 mM N-methoxysuccinyl-Ala-Ala-Pro-Val-p-nitroanilide (SEQ ID NO:43), 150 mM NaCl, 26 mM KCl, 2 mM $MgCl_2$, 0.02% HSA, 0.05% DMSO, 0.01% Triton X-100, and 100 mM Tris-HCl, at 25°C. The assay measured the generation of elastase-induced release of p-nitroanilide as a function of time by spectroscopy (OD 405 nm). The rate of p-nitroanilide generation in the absence of inhibitor was used as the control. Positive control was established in the presence of the irreversible inhibitor N-methoxysuccinyl-Ala-Ala-Pro-Val-chloromethyl (SEQ ID NO:41) ketone.

The presence of trace amounts of unreacted N-methoxysuccinyl Ala-Ala-Pro-Val-chloromethyl (SEQ ID NO: 41) ketone in the final blended nucleic acid ligand preparation will inhibit the enzyme. However, this possibility will be eliminated by inactivating the inhibitor with DTT. Figure 19 shows the inactivation of N-methoxysuccinyl Ala-Ala-Pro-Val-chloromethyl (SEQ ID NO:41) ketone by DTT. Incubation of 1.95 M N-methoxysuccinyl Ala-Ala-Pro-Val-chloromethyl (SEQ ID NO:41) ketone with 50 mM DTT destroyed the inhibition. The partial inhibition seen with the inactivated inhibitor may be due to competitive inhibition due to the high peptide concentration.

Figure 20 shows the inhibition of the blended nucleic acid ligand in the presence of 20 mM DTT. The inhibition of elastase by the 5' end blended nucleic acid ligand and the 3' end blended nucleic acid ligand is shown in Figure 21. A Lineweaver-Burk plot of elastase inhibition by N-methoxysuccinyl Ala-Ala-Pro-Val (SEQ ID NO:44) or the blended nucleic acid ligand is shown in Figure 22. The resulting Km of 0.16 mM for the substrate is in good agreement with published values. The Ki of the blended nucleic acid ligands was 30 nM versus 920 uM for the inhibitor peptide alone, or a 30,000 fold improvement.

The inhibition of two other human serine proteases--urokinase and thrombin-- by the 3' end blended nucleic acid DNA-17 was also examined, and shown in Figure 23. At 70 nm concentration the 3' end blended nucleic acid has greater than 50% inhibition of elastase, whereas even at 650 nm concentration neither urokinase nor thrombin were inhibited to any detectable extent. These results demonstrate the specificity of the blended nucleic acid ligand for elastase.

EXAMPLE 11. SPLINT BLENDED SELEX.

The splint blended SELEX process was performed by preparing a standard SELEX candidate mixture and a single compound containing a valyl phosphonate attached to a nucleic acid sequence that hybridizes to a portion of the fixed region of the candidate mixture of nucleic acid sequences as shown in Figure 24. Figure 24 also shows the chemical reaction that occurs between the valyl phosphonate and human neutrophil elastase (HNE).

The valyl phosphonate was activated via an NHS ester. This compound was coupled to the 5' hexyl amine linker of a 19-mer DNA oligo complementary to the 5'-fixed region of 40N7.1. A candidate mixture composed of pyrimidine $2'NH_2$-substituted RNA was hybridized to the splint, and reacted with HNE at subsaturating protein concentrations. Covalent complexes were enriched by diluting the reaction 100-fold, then filtering through nitrocellulose.

After five rounds of selection for species that reacted with the HNE, the reactivity of the pool was assayed by gel electrophoresis (5% polyacrylamide/TBE pH 8 or MAE pH 6/0.25% SDS). The results are shown in Figure 25. The analysis of reaction rates indicates biphasic kinetics, with $k_1$obs 0.1 $min^{-1}$, and $k_2$obs 0.01 $min^{-1}$. The reaction plateaus at 30%, presumably because the valyl phosphonate is a racemic mixture and the theoretical limit is 50%. A $k_1$obs of 0.1 $min^{-1}$ at a protein concentration of 50 nM indicates a second order rate constant $\geq 2 \times 10^6$ $M^{-1}min^{-1}$. The reported rate constant for the valyl phosphonate alone is $10^3$-$10^4$ $M^{-1}min^{-1}$ (Oleksyszyn et at. (1989) Biophys. Biochem. Res. Comm. 161: 143-149). Thus, the splint blended nucleic acid mixture increases the reaction rate of the species by at least $10^3$ fold. The reaction of the selected pool is also highly specific. No reaction of the pool with thrombin, another serine protease, is detectable. The second-order rate constant for the thrombin reaction is estimated to be < 200 $M^{-1}min^{-1}$, $10^4$ fold lower than the reaction rate with elastase.

EXAMPLE 12. MATERIALS AND METHODS - NUCLEIC ACID LIGANDS TO VEGF.

Materials. Recombinant human VEGF (165 amino acid form; MW 46,000) was a generous gift from Dr. Napoleone Ferrara (Genentech). All other reagents and chemicals were of the highest purity available and were purchased from commercial sources.

SELEX. The essential features of the SELEX protocol are described in Example 1. To screen for RNA ligands with an affinity for VEGF, an initial pool of random RNAs were generated (each containing a 30 nucleotide random region flanked by fixed sequences for transcription and PCR amplification) by *in vitro* transcription of approximately 200 picomoles ($10^{14}$ molecules) of the double stranded DNA template using T7 RNA polymerase. The DNA templates and the corresponding PCR primers were prepared chemically using established solid phase oligonucleotide synthesis protocols. The random region was generated by utilizing an equimolar mixture of the four unmodified nucleotides during oligonucleotide synthesis. The two constant regions were designed to contain PCR primer annealing sites, primer annealing site for cDNA synthesis, T7 RNA polymerase promoter region and restriction enzyme sites that allow cloning into vectors (Figure 26) (SEQ ID NOS:45-47). Transcription mixtures consisting of 100-300 nM template, 5 units/μl T7 RNA polymerase, 40 mM Tris-Cl buffer (pH 8.0) containing 12 mM $MgCl_2$, 5 mM DTT, 1 mM spermidine, 0.002% Triton X-100, 4% PEG were incubated at 37˚C for 2-3 hours. These conditions typically resulted in transcriptional amplification of 10 to 100-fold. Selections for high affinity RNA ligands were done by incubating VEGF with RNA for 10-20 minutes at 37˚C in 50 ml of phosphate buffered saline (PBS = 10.1 mM $Na_2HPO_4$, 1.8 mM $KH_2PO_4$, 137 mM NaCl, 2.7 mM KCl, pH 7.4) and then separating the protein-RNA complexes from the unbound species by nitrocellulose filter partitioning (Tuerk, C. and Gold, L. (1990) Science 249, 505-510). The selected RNA (which typically amounted to 5-10% of the total input RNA) was then extracted from the filters and reverse transcribed into cDNA by avian myeloblastoma virus reverse transcriptase (AMV RT). Reverse transcriptions were done at 48˚C (60 min) in 50 mM Tris buffer (pH 8.3), 60 mM NaCl, 6 mM $Mg(OAc)_2$, 10 mM DTT and 1 unit/μl AMV RT. Amplification of the cDNA by PCR under standard conditions yielded a sufficient amount of double-stranded DNA for the next round of in vitro transcription.

Nitrocellulose Filter Binding Assays. Nitrocellulose filters (0.2 μm pore size, Schleicher and Schuell, Keene, NH) were secured on a filter manifold and washed with 4-10 ml of buffer. Following incubations of $^{32}P$ labeled RNA with serial dilutions of the protein for 10 min at 37˚C in buffer (PBS) containing 0.01% human serum albumin (HSA), the solutions were applied to the filters under gentle vacuum in 45 ml aliquots and washed with 5 ml of PBS. The filters were than dried under an infrared lamp and counted in a scintillation counter.

Equilibrium Dissociation Constants. In the simplest case, equilibrium binding of RNA (R) to VEGF (P) can be described by eq. 1,

$$R.P \overset{Kd}{\rightleftharpoons} R + P \qquad (1)$$

where Kd =([R][P]/[R.P]) is the equilibrium dissociation constant. Using the mass-balance equations, the fraction of bound RNA at equilibrium (q) can be expressed in terms of measurable quantities (eq. 2),

$$q=(f/2Rt)\{Pt+Rt+Kd-[(Pt+Rt+Kd)^2-4PtRt]^{½}\} \qquad (2)$$

where Pt and Rt are total protein and total RNA concentrations and f reflects the efficiency of retention of the protein-RNA complexes on nitrocellulose filters. The average value of f for VEGF in our assays was 0.7.

Most RNA ligands exhibited biphasic binding to VEGF. For those ligands, binding of RNA to VEGF is described by a model where total RNA is assumed to be partitioned between two non-interconverting components (R1 and R2) that bind to VEGF with different affinities (eqs 3 and 4).

$$R1.P \overset{Kd1}{\rightleftharpoons} R1 + P \qquad (3)$$

$$R2.P \overset{Kd2}{\rightleftharpoons} R2 + P \qquad (4)$$

In this case, the fraction of total bound RNA (q) is given by eq. 5.

$$q=(f/2Rt)\{2Pt+Rt+Kd1+Kd2-[(Pt+\chi 1Rt+Kd1)^2-4Pt\chi 1Rt]^{½}-[(Pt+\chi 2Rt+Kd2)^2-4Pt\chi 2Rt]^{½}\} \qquad (5)$$

where $\chi^1$ and $\chi^2$(=1-c1) are the mole fractions of R1 and R2 and Kd1 and Kd2 are the corresponding dissociation constants.

Internally-labeled RNA ligands used for binding studies were prepared by in vitro transcription using T7 RNA polymerase (Milligan et *al.* (1987) Nucl. Acids Res. 15:8783) and were purified on denaturing polyacrylamide gels to ensure size homogeneity. All RNA ligands were diluted to about 1 nM in PBS, denatured at 90˚C for 2 minutes, and then cooled on ice prior to incubation with the protein. This denaturation/renaturation cycle performed at high dilution is necessary to ensure that the RNA is essentially free from dimers and other higher order aggregates. Concentrations of the stock solutions of VEGF, from which other dilutions were made, were determined from the absorbance reading at 280 nm using the calculated value for $\in_{280}$ of 46,600 $M^{-1}cm^{-1}$ for the VEGF dimer (Gill et al. (1989) Anal. Biochem. 182: 319). Data sets that define the binding curves were fit to either eq. 2 or eq. 5 by the non-linear least squares method using the software package Kaleidagraph (Synergy Software, Reading, PA).

Information Boundary Determinations. High-affinity VEGF ligands were radiolabeled at the 5'-end with $\gamma$- $^{32}$P-ATP (New England Biolabs, Beverly, MA) and T4 polynucleotide kinase (New England Biolabs, Beverly, MA) for the 3'-boundary determinations, or at the 3'-end with $\alpha$-$^{32}$PCp and T4 RNA ligase (New England Biolabs) for the 5'-boundary determination. Radiolabeled RNA ligands were subjected to partial alkaline hydrolysis and then selectively bound in solution to VEGF at 5, 0.5, or 0.125 nM before being passed through nitrocellulose filters. Retained oligonucleotides were resolved on 8% denaturing polyacrylamide gels. In each experiment, the smallest radiolabeled oligonucleotide bound by VEGF at the lowest protein concentration defines the information boundary. Partial digests of the 5'- or the 3'-labelled RNA ligands with RNAse $T_1$ (Boehringer Mannheim Biochemicals, Indianapolis, IN) were used to mark the positions of labeled oligonucleotides ending with a guanosine.

Cloning and Sequencing. Individual members of the enriched pool were cloned into pUC18 vector and sequenced as described (Schneider, D. et al. (1992) J. Mol. Biol. 228:862-869).

Receptor Binding. VEGF was radioiodinated by the lodegen method (Jakeman et *al.* (1992) J. Clin. Invest. 89: 244) to a specific activity of 2.4 x $10^4$ cpm/ng. Human umbilical vein endothelial cells (HUVECs) were plated in 24-well plates at a density of 1-2 x $10^5$ cells/well and grown to confluence in EGM (Clonetics, San Diego, CA) media (24-48 hrs). At confluence, the cells were washed 3 times with PBS and incubated for 2 hrs at 4°C in $\alpha$-MEM serum-free media containing $^{125}$I-labeled VEGF with or without unlabeled competitor (VEGF, EGF, or RNA). For experiments done with RNA, 0.2 units of placental RNase inhibitor (Promega, Madison, WI) were included in the media. It was determined that the RNA ligands were not degraded during the course of the experiment. At the end of the 2 hr incubation period, the supernatant was removed and the wells washed 2 times with PBS. HUVECs were then lysed with 1% triton X-100/1 M NaOH and the amount of cell-associated $^{125}$I-VEGF determined by gamma counting.

EXAMPLE 13. RNA LIGANDS TO VEGF.

Approximately $10^{14}$ RNA molecules randomized at thirty contiguous positions were used in the initial selection targeting VEGF. Random RNA bound to VEGF with an affinity of approximately 0.2 uM. After 13 rounds of SELEX, the observed improvement in affinity of the evolved RNA pool was about two orders of magnitude (data not shown). 64 isolates were cloned and sequenced from this evolved pool, and 37 unique sequences found (sequences differing at only one or two positions were not considered unique). 34 of the 37 unique sequences could be classified into six families based on sequence similarity in the evolved region (Figure 27). Three unique clones, 4 (GGGAUGUUUGGCUAUCUCGGAUAGUGCCCC)(SEQ ID NO:83), 16 (GCUUAAUACGACUCACUNUAGGGAGCUCAG) (SEQ ID NO: 84) and 18 (UUGAGUGAUGUGCUUGACGUAUCGCUGCAC)(SEQ ID NO:85) had a more limited sequence similarity with members of the six families.

Consensus Structures. In addition to allowing determination of consensus primary structures, groups of similar sequences consisting of members that share a defined functional property often contain useful clues for secondary structure prediction (James et al. (1988) Meth. Enzymol. 180:227). The underlying assumption is that ligands with similar primary structures are capable of adopting similar secondary structures in which the conserved residues are organized in unique, well-defined motifs. In this context, ligands which have strong, unambiguous secondary structures can provide good structural leads for other sequences within a similar set where consensus folding may be less obvious. Conserved elements of secondary structure, such as base-pairing, may also be detected through covariation analysis of aligned sequence sets (James et al. (1988) supra; Gutell et al. (1992) Nucl. Acids Res. 20:5785). The predicted consensus secondary structures for the six sequence families are shown in Figure 28 (SEQ ID NOS:86-91).

The most highly conserved residues in the family 1 sequence set (A17, G19 and the CAUC sequence at positions 23-26) can be accommodated in the 9-10 nucleotide loop (SEQ ID NO:86). Base-pairing covariation between positions 16 and 27 (G-C occurs with a frequency of 8 out of 11 times (8/11) and C-G with a frequency of 3/11), positions 15 and 28 (U-G, 7/11; G-C, 3/11; U-A, 1/11) and positions 14 and 29 (G-C, 5/11; U-A, 2/11, and C-G, 1/11) supports the predicted secondary structure. It is worth noting that many ligands in this family have stable extended stems that contain up to 15 base pairs.

In the family 2 sequence set, the strongly conserved UGCCG and UUGAUG(G/U)G sequences (positions 8-12 and 26-33) are circularly permutated. In the consensus secondary structures, these nucleotides are found in an identical arrangement within or adjacent to the asymmetrical internal loop (Figure 28) (SEQ ID NO:87). This result suggests that the nucleotides outside of the consensus motif shown in Figure 28 are unimportant for binding. Base-pairing covariation is noted between positions 5 and 36 (C-G, 2/7; G-C, 2/7; U-A, 1/7; G-U, 1/7), 6 and 35 (A-U, 4/7; C-G, 1/7; G-C, 1/7), 7 and 34 (A-U, 4/7; G-C, 1/7), 11 and 28 (C-G, 6/7; G-C, 1/7), 12 and 27 (G-U, 6/7; C-G, 1/7), 13 and 26 (A-U, 5/7; G-C, 1/7; G-U, 1/7), 14 and 25 (G-C, 4/7; C-G, 2/7) and 15 and 24 (C-G, 4/7; G-C, 2/7).

Family 3 and family 4 sequence sets are characterized by highly conserved contiguous stretches of 21 (GGGAACCUGCGU(C/U)UCGGCACC (SEQ ID NO:92), positions 11-31) and 15 (GGUUGAGUCUGUCCC (SEQ ID NO:93), positions 15-29) arranged in bulged hairpin motifs (Figure 28) (SEQ ID NOS:88-89). Base-pairing covariation is detected in family 3. between positions 8 and 33 (A-U, 2/4; G-C, 2/4), 9 and 32 (A-U, 2/4; U-A, 1/4; G-C, 1/4), and 10 and 31 (A-U, 1/4; G-C, 3/4) and in family 4 between positions 13 and 31 (A-U, 4/7; C-G, 2/7; U-A, 1/7) and 14 and 30 (C-G, 3/7; U-A, 3/7; A-U, 1/7).

Family 5 consensus secondary structure is an asymmetrical internal loop where the conserved UAGUUGG (positions 9-15) and CCG (positions 29-31) sequences are interrupted by less conserved sequences (Figure 28) (SEQ ID NO:90). Modest base-pairing covariation is found between positions 8 and 32 (A-U, 2/4; U-G, 1/4), 16 and 26 (G-C, 2/4; A-U, 1/4), 17 and 25 (A-U, 2/4; G-C, 1/4) and 18 and 24 (C-G, 2/4; G-C, 1/4).

Family 6 has only two sequences and therefore the concept of consensus sequence or consensus structure is less meaningful. Nevertheless, the two sequences are very similar (90% identity) and can be folded into a common motif (Figure 28) (SEQ ID NO:91). Base-pairing covariation is found between positions 1 and 32 (A-U, 1/2; G-U, 1/2), 2 and 31 (C-G, 1/2; G-C, 1/2), 14 and 20 (U-A, 1/2; G-C, 1/2) and 15 and 19 (A-U, 1/2; G-U, 1/2).

Affinities. The affinity of all unique sequence clones for VEGF was screened by determining the amount of RNA bound to VEGF at two protein concentrations (1 and 10 nM). Binding of the best ligands from each of the six sequence families was then analyzed over a range of protein concentrations (Figure 29). Dissociation constants were calculated by fitting the data points to either eq. 2 (monophasic binding) or eq. 5 (biphasic binding) and their values are shown in Table 1.

**Information Boundaries.** In order to determine the minimal sequence information necessary for high-affinity binding to VEGF, deletion analyses were performed with representative members from each of the six families. These experiments were done by radiolabeling RNA ligands at either the 3' end or the 5' end (for the 3' or the 5' boundary determinations, respectively) followed by limited alkaline hydrolysis, partitioning of the free and the bound RNA by nitrocellulose filtration and resolving the hydrolytic fragments that retained high affinity for VEGF on denaturing polyacrylamide gels (Tuerk et *al*. (1990) J. Mol. Biol. 213:749). The combined information from the 3' and the 5' boundary experiments outlines the shortest sequence segment that has high affinity for the protein (Figure 30) (SEQ ID NO:94). It is important to realize that these experiments define boundaries sequentially at the unlabeled ends of ligands in the context of full-length labeled ends. Since the full-length ends may provide additional contacts with the protein or participate in competing secondary structures, ligands truncated at both ends may have lower or higher affinities for the protein than their full-length parent. The following truncated ligands were prepared by in *vitro* transcription from synthetic DNA templates: 100t (Family 1) GGCCGGUAG-UCGCAUGGCCCAUCGCGCCCGG (SEQ ID NO:95), 44t (Family 2) GGaaGCUUGAUGGGUGACACACGUCAUGCCGAGCu (SEQ ID NO:96), 12t (Family 3) GGAAGGGAACCUGCGUCUCGGCACCuucg (SEQ ID NO:97), 40t (Family 4) GGUCAACGGUUGAGUCUGUCCCGuucgac (SEQ ID NO:98), 84t (Family 5) GgcucaaAGUUGGAGGCCUGUCCU-CGCCGUAGAGC (SEQ ID NO:99) and 126t (Family 6) GGaACGGUUCUGUGUGUGGACUAGCCGCGGCCGuu (SEQ ID NO:100) (letter t designates truncated sequences; underlined guanines are not present in the original sequences and were added to increase the transcriptional efficiency (Milligan et *al.* (1990) supra); lowercase letters indicate nucleotides from the constant sequence region). Binding curves for these truncated ligands and their dissociation constants are shown alongside their parent ligands in Figure 29 and Table 1. The dissociation constants of the truncated versus full-length ligands are generally comparable, although ligands 40t (SEQ ID NO:98) and 126t (SEQ ID NO:100) clearly bind to VEGF significantly less well than the corresponding full-length ligands.

Competition experiments revealed that binding of all possible pairwise combinations of truncated ligands representing each of the families is mutually exclusive (100t (SEQ ID NO:95), 44t (SEQ ID NO:96), 12t (SEQ ID NO:97), 40t (SEQ ID NO:98), 84t (SEQ ID NO:99) and 126t (SEQ ID NO:100)). Furthermore, all of these ligands are displaced by low-molecular weight (5,100 Da) heparin (data not shown). Truncated ligands and low-molecular weight heparin were used in these studies in order to maximize the probability of observing non-competing ligand pairs. It appears, therefore, that although there are multiple non-isomorphic solutions to high-affinity binding to VEGF, all examined ligands may bind to the same region of the protein. Proteins in general may have "immunodominant" domains for nucleic acid ligands.

## EXAMPLE 14. SPECIFICITY OF TRUNCATED RNA LIGANDS TO VEGF.

Binding of two truncated high-affinity ligands, 100t (SEQ ID NO:95) and 44t (SEQ ID NO:96), to four other heparin binding proteins (bFGF, PDGF, antithrombin III and thrombin) was tested in order to address the question of specificity. Dissociation constants were determined using the nitrocellulose filter partitioning technique. Results are shown in Table 2. Binding of these ligands to VEGF in a buffer containing 10 mM dithiothreitol is at least 1000-fold weaker.

Receptor Binding. Unlabeled VEGF but not EGF was shown to inhibit binding of $^{125}$I-VEGF to HUVECs in a concentration-dependent manner (data not shown), confirming that $^{125}$I-VEGF binds to specific sites on HUVECs. As previous studies have reported (Myoken et al. (1991) Proc. Natl. Acad. Sci. USA 88:5819), two classes of receptors on HUVECs were observed to bind VEGF with dissociation constants of ~ 5 x $10^{-11}$ M (7,000 receptors/cell) and ~ 5 x $10^{-10}$ M (20,000 receptors/cell) (Figure 31).

A group of truncated RNA ligands representing each of the sequence families (100t (SEQ ID NO:95), family 1; 44t (SEQ ID NO:96), family 2; 12t (SEQ ID NO:97), family 3; 40t (SEQ ID NO:98), family 4; 84t (SEQ ID NO:99), family 5; and 126t (SEQ ID NO:100), family 6), as well as random RNA were tested for their ability to inhibit binding of VEGF to its cell-surface receptors. All high-affinity ligands, but not random RNA, inhibited VEGF-VEGF receptor interaction in a concentration-dependent manner with half-inhibition occurring in the 20-40 nM range (Figure 32).

EXAMPLE 15. MODIFIED 2'-$NH_2$ PYRIMIDINE RNA LIGANDS TO VEGF.

In order to generate ligands with improved stability *in* vivo, two SELEX experiments (A and B) targeting VEGF were initiated with separate pools of randomized RNA containing amino ($NH_2$) functionalities at the 2'-position of each pyrimidine. Starting ligand pools for the two experiments contained approximately $10^{14}$ molecules (500 pmols) of modified RNA randomized at 30 (SELEX experiment A) and 50 (SELEX experiment B) contiguous positions. The starting RNAs and the corresponding PCR primers are defined in Figure 33 (SEQ ID NOS:101-106). Sequences corresponding to the evolved regions of modified RNA are shown in Figure 34.

Ligands with similar primary structures were grouped into 5 families and their consensus sequences are shown below each sequence set Figure 34 (SEQ ID NOS:107-190). Groups of sequences with similar primary structure (families) have been aligned in Figure 34 and their consensus sequences are shown below each set. Pairs of similar/related sequences, sequences that could not be included in any of the families ("other sequences") and sequences that correspond to ligands that bind additionally to nitrocellulose filters with high affinity have been shown in separate groups. Letter N in a sequence indicates an ambiguous position on a sequencing gel. Italicized letter N in a consensus sequence indicates a position that is not conserved (i.e., any nucleotide may be found at that position). Dissociation constants for Random RNA A (30N8), Random RNA B (50N7) and a set of modified 2'-amino pyrimidine high-affinity RNA ligands for VEGF are shown in Table 3.

EXAMPLE 16. MATERIAL AND METHODS - NUCLEIC ACID LIGANDS TO ELASTASE.

Materials. Human neutrophil elastase was purchased from Athens Research and Technology, Athens, Georgia. All oligodeoxynucleotides were synthesized at Operon Technologies by standard chemistry employing cyanoethyl phosphoramidites.

2'-$NH_2$ RNA SELEX. The essential features of the SELEX protocol are described in Example 1. The random nucleotides of the initial candidate mixture were comprised of 2'-$NH_2$ pyrimidine and 2'-OH purine bases. All RNA synthesizing steps by *in vitro* transcription were conducted with 2'-$NH_2$ pyrimidine bases.

Briefly, the first round random pool of RNA was derived from the transcription of a DNA template containing a forty-nucleotide random region flanked by defined regions; the complexity of the pool was in the order of $10^{14}$ molecules. The DNA template and the corresponding PCR primers were synthesized chemically by standard techniques using cyanoethyl phosphoramidites (Operon). The random region was generated by reacting with all four phosphoramidites at optimized concentrations of each to obtain equimolar coupling during oligonucleotide synthesis. Defined nucleotide sequences in the flanking regions of the template served as primer annealing sites for PCR, for reverse transcriptase and as a promoter for T7 RNA polymerase, and restriction enzyme sites that allow cloning into vectors.

The SELEX was done with the primer-template mixture I, shown in Figure 46. 2'-$NH_2$ RNA transcriptions were carried out in 100 $\mu$l transcription reactions containing 2 mM each ATP, GTP, 2'-$NH_2$CTP and 2'-$NH_2$UTP, 40 mM Tris-HCl (pH 8.0), 12 mM $MgCl_2$, 1 mM Spermidine, 5 mM DTT, 0.002% Triton X-100 and 4% polyethylene glycol (w/v) at room temperature for 2 hr. The full length transcripts were gel purified, resuspended in binding buffer (150 mM NaCl, 100 mM Tris-HCl (pH 7.0), 2 mM $MgCl_2$ and 6 mM KCl), heated to 70°C for 3 min., chilled on ice, and incubated with HNE at 37°C for 10 min.

The RNA pool was incubated with elastase (1.7-0.003 $\mu$M) in binding buffer containing 150 mM NaCl, 100 mM Tris-HCl (pH 7.0), 2 mM $MgCl_2$, and 6 mM KCl for 10 minutes at 37°C. The RNA-protein mixture was passed through a nitrocellulose filter (0.45 $\mu$, Millipore) and the RNA from RNA-protein complexes retained on the filter was eluted (Tuerk & Gold (1990) Science 249:505) and recovered. The recovered RNA was reverse transcribed into cDNA by AMV reverse transcriptase at 48°C. The resulting cDNA was amplified by PCR and the amplified DNA used as the template for T7 RNA polymerase to obtain a pool of RNA for the subsequent round of SELEX. To avoid the selection and amplification of undesired nitrocellulose binding RNA molecules, the newly transcribed RNA pool was passed through several layers of nitrocellulose filters (negative selection) before the next round of selection with HNE. The procedure was reiterated until the enriched pool of RNA showed significantly improved affinity to elastase over the initial random pool.

Once significant binding of the 2'-$NH_2$ RNA pool was achieved, PCR-amplified DNA was digested with HindIII and BamHI, and cloned into pUC18 previously digested with the same enzymes by standard techniques (Tuerk & Gold (1990) Science 249:505). Recombinant plasmids were isolated and sequenced.

Single-Stranded DNA SELEX. SELEX was conducted with a candidate library of single-stranded DNA (ssDNA) molecules. The SELEX experiment was carried out by using the primer template-mixture II, shown in Figure 46. Briefly, after nitrocellulose filter partitioning, filter bound ssDNA was recovered by incubating the filter in a mixture of neutralized phenol and 7 M urea at 37°C followed by extraction of the aqueous phase. Recovered ssDNA was precipitated with ethanol, and an aliquot used as a template in a PCR reaction to identify the optimum number of cycles needed to produce double-stranded DNA (dsDNA) molecules of the correct size without creating high molecular weight DNAs. The optimum number of cycles was used for a bulk PCR reaction (800 ul) carried out with the remaining recovered ssDNA as the template and a biotinylated primer (BIO-3G2) and a non-biotinylated primer (5G2) . The PCR products are gel purified to remove unincorporated biotinylated primer. The non-biotinylated strand of the double-stranded DNA was isolated from the complementary strand by incubation of the PCR product with 200 ul of streptavidin beads (Pierce) followed by alkali denaturation (0.15 N NaOH, 30 min at room temperature). The recovered single-stranded DNA was neutralized, precipitated with ethanol, and used for the next round of SELEX. The recovery of ssDNA by streptavidin capture was about 30-40%.

The use of biotinylated primer 3G2 and the non-biotinylated primer 5G2 in PCR yields the bottom DNA strand after streptavidin capture. Therefore, to obtain a fairly large sequence space for the first round of SELEX, 10 nm of HPLC-purified synthetic bottom strand was used. For each SELEX round, a small fraction of ssDNA was end-labelled, gel purified, and used for a mini-binding curve with three different elastase concentrations. These results were then used to obtain the protein concentration at which 5-10% of the DNA remains on the filter above the background level used for the subsequent round.

Except for the first round, selected ssDNA was passed through a layer of 4-5 nitrocellulose filters after every SELEX round to remove those DNA molecules that bound to the filter, not elastase (filter binders). However, even after this prefiltration treatment, the percentage of filter binders increased from 0.2% to as high as 12-15%. To avoid the high background problem, the filters were washed with 0.5 M urea solution immediately after the filtration of the protein-DNA mixture. The urea treatment brought background binding levels to 0.1-0.2%. Therefore, during each SELEX round as well as during determination of binding curves, the filters were washed with 10 ml of 0.5 M urea and 10 ml of binding buffer.

Several polyanions including tRNA have been shown to bind and inhibit elastase (Lestienne and Bieth (1983) Biochime 65:49-52). In order to increase the stringency of selection, tRNA at a concentration 8-10 times higher than that of ssDNA was included in the binding reaction as a competitor after the 9th SELEX round. The apparent Kd of random ssDNA was determined to be approximately 0.25 uM, whereas the Kd of ssDNA after 14 SELEX rounds was 22 nM.

Elastase Inhibition Assay. The assay for elastase inhibition is based on the use of a chromogenic tetrapeptide substrate. The assay was conducted in a solution containing 10 nM elastase, 0.5 mM N-methoxysuccinyl-Ala-Ala-Pro-Val-p-nitroanilide, 150 mM NaCl, 26 mM KCl, 2 mM $MgCl_2$, 0.02% HSA, 0.05% DMSO, 0.01% Triton X-100, and 100 mM Tris-HCl, at 25°C. The assay measured by spectroscopy (OD 405 nm) the generation of elastase-induced release of p-nitroanilide as a function of time. The rate of p-nitroanilide generation in the absence of inhibitor was used as the negative control; the positive control contained the irreversible inhibitor N-methoxysuccinyl-Ala-Ala-Pro-Val-chloromethyl ketone.

OD values were plotted against time, and the slope used to determine the rate of enzyme activity.

Cell detachment assay. An assay was-developed in which elastase-induced endothelial cell detachment is measured in cultured endothelial cells. Endothelial cells were grown under standard culture conditions in 24-well plates to obtain a confluent monolayer. The cells were washed with solution A (PBS, 20 mM HEPES, 10 mM glucose) prior to addition of elastase with and without the test ligand. After incubation at 37°C for 45 minutes, the supernatant was removed and counted for detached cells.

## EXAMPLE 17. 2'-$NH_2$ RNA SELEX.

SELEX was conducted as described above for 17 rounds. The resulting affinity of the enriched pool to elastase was significantly improved (dissociation constant (Kd) of about 70 nM as compared to the unselected random pool having a Kd of 0.8 μM). At this point, PCR-amplified DNA was cloned and sixty individual clones were sequenced (Table 4, SEQ ID NOS:191-215). Ligands were grouped into classes on the basis of primary sequence homology. Kds of representative ligands belonging to different classes are shown in Table 5.

Specificity of ligands. As shown in Table 6, 2'-$NH_2$ ligands to elastase did not exhibit high affinity binding to other proteins such as thrombin and basic fibroblast growth factor (bFGF). The specificity of the ligands to human elastase was tested by comparing representative ligand 14 (SEQ ID NO:200) binding to human and porcine elastase. The homology between these proteins is about 40% overall, but the sequences are essentially identical in the catalytic region. As shown in Figure 35, ligand 14 (SEQ ID NO:200) did not bind porcine elastase at a detectable level at any concentrations.

EXAMPLE 18. IDENTIFICATION OF INHIBITORY LIGANDS TO ELASTASE.

Using the elastase inhibition assay described in Example 16, several ligands with inhibitory activity toward elastase were identified. Molecules belonging to class I and II were found to inhibit elastase. Clone 9 (SEQ ID NO:202) of class III and clone 19 (SEQ ID NO:205) of class IV also exhibited inhibitory activity. Figure 36 shows the elastase inhibitory activity of representative ligand 34 (SEQ ID NO:197). The elastase inhibitory activity of all RNA ligands tested was dependent on RNA concentration and had a maximum inhibition of elastase of approximately 30%.

Since ligands from different classes exhibited similar inhibition of elastase, competition experiments were conducted with ligands 24 and 30 (SEQ ID NOS:198 & 191)(class I and II) and ligands 56 and 19 (SEQ ID NOS:195 & 205)(class II and IV), to determine if these ligands competed with each other. The results (Figure 37A and 37B) indicate that these ligands appear to compete with each other for the same binding site of the elastase protein or for spatially close binding sites.

Experiments were conducted to determine whether ligands bound the active site or the substrate binding site of elastase. The binding of ligand 34 (SEQ ID NO:197) to elastase irreversibly complexed with N-methoxysuccinyl-Ala-Ala-Pro-Val-chloromethyl ketone was studied. The chloromethyl ketone portion of the inhibitor irreversibly binds at the active site of elastase, with the tetrapeptide portion occupying the substrate binding pocket (Navia et *al.* (1989) Proc. Natl. Acad. Sci. USA 86:7). The results, shown in Figure 38, show that ligand 34 (SEQ ID NO:197) bound to complexed elastase to the same extent as to the native enzyme. These results support the conclusion that the ligands do not bind at the active site or the substrate binding pocket of elastase. Ligand-mediated elastase inhibition may come from either ligand binding near the active site or to a remote site of the enzyme surface, inducing an allosteric change in the enzyme which impairs catalytic activity to some extent.

A natural inhibitor for elastase, $\alpha$-1-antitrypsin ($\alpha$-1PI), forms a tight irreversible enzyme-inhibitor complex with elastase. The binding of ligand 34 (SEQ ID NO:197) to $\alpha$-1PI-complexed elastase was examined. The results show (Figure 39) that ligand 34 did not bind the complexed enzyme. One possible explanation for the lack of binding is that the ligand binds to a site on the face of elastase that interacts with $\alpha$-1PI. Alternatively, $\alpha$-1PI binding may induce a conformation change in elastase that abolishes ligand binding.

The effect of ligand binding on $\alpha$-1PI-induced inhibition was examined. Representative ligand 19 (SEQ ID NO: 205) was found to act additively with $\alpha$-1PI inhibition, enhancing the inhibitory activity of the natural inhibitor (Figure 40).

A cell detachment assay was conducted with inhibitory ligands 24, 30, and 19 (SEQ ID NOS:198, 191 & 205). As shown in Figure 41, all of the ligands substantially inhibited elastase activity.

EXAMPLE 19. TRUNCATED ELASTASE LIGANDS.

With the use of PCR primers specific for the 3' sequence of the 2'-$NH_2$ modified RNA ligand, the 3'-fixed regions for four different ligands (class I ligand 12 (SEQ ID NO:191), class II ligand 14 (SEQ ID NO:200), class III ligand 9 (SEQ ID NO:202) and class IV ligand 19 (SEQ ID NO:205)) were eliminated. The results (Table 7) indicate that the 3'-fixed region is not necessary for high affinity binding for ligands 12 (class I) and 14 (class II). Both 3'-truncated ligands (64 nucleotides) had Kds similar to those of the full length ligands (87 nucleotides). However, the removal of the 3'-fixed region was deleterious for ligands 19 and 9. The 3'-fixed end, therefore, appears to be critical for enzyme inhibition by these ligands.

EXAMPLE 20. EFFECT OF MONOVALENT CATIONS ON SECONDARY STRUCTURE FORMATION.

The three-dimensional molecular structure(s) assumed by the 2'-$NH_2$ modified elastase ligands of the present invention in the binding buffer is not known with certainty. However, the following evidence suggests these ligands form G-quartet structures (Figure 42). It has been proposed that 2'-$NH_2$-substituted RNA has an unusually low melting temperature in helix-coil transition compared to that of 2'-OH RNA analogs. If so, this would argue against the formation of stem-loop structures in class I and II ligands. It is possible that SELEX with 2'-$NH_2$ ligands is inherently resistant to the production of RNA ligands with helical structures. This prediction remains to be tested.

The binding buffer employed with 2'-$NH_2$ SELEX contained 6 mM KCl, a monovalent ion known to stabilize the G-quartet structure. The absence of KCl in the binding buffer affects the binding affinity of three clones (21, 24 and 34) (SEQ ID NOS:195, 198 & 197). As shown in Figure 43, there was a significant change in Kd values in the presence and absence of KCl. For example, clone 21 (SEQ ID NO:195) had a Kd for elastase of 39 nM, in the presence of KCl, which dropped to 192 nM in the absence of KCl. This suggests that KCl has an important role in ligand binding, perhaps by favoring the formation of secondary structures with high binding affinity to elastase.

Ligand binding to elastase was then investigated with ligands 30 (SEQ ID N0:191)(class I), 34 (SEQ ID N0: 197)(class II), and 9 (SEQ ID NO:202)(class III) in a binding buffer (150 mM XCl, 100 mM Tris-HCL (pH 7.0), 2 mM $mgcl_2$) where X is Li, K, or Tris (Table 8). The purpose of this experiment was to compare affinity in the presence of K (which favors formation of the G-quartet structure), Li (which does not favor formation of the G-quartet structure), or Tris (which does not affect formation of the G-quartet structure). The affinities of ligands belonging to classes I and II were higher in 150 mM than in 6 mM KC1 buffer. This suggests that the fraction of high affinity secondary structures can be increased by increasing KCl concentration. However, ligand 9 (SEQ ID NO:202) (class III) had lower elastase affinity in the presence of high KCl, suggesting that the secondary structure of ligand 9 may be different from that assumed by class I and II ligands. The lowest affinity of class I and II ligands to elastase was seen in the presence of Li, the monovalent cation that does not favor formation of G-quartet structures.

In the presence of Tris buffer, all three ligands exhibited a moderately high affinity for elastase (however, lower than that of class I and II ligands in KCl buffer). A possible contamination of the Tris with $K^+$ may be responsible, or alternatively, $Mg^{++}$ ions may be involved in establishment of secondary structures in the absence of a high concentration of a monovalent ion. Some evidence for the stabilization of RNA G-quartet structures by $Mg^{++}$ has been published (Sundquist and Heapy (1993) Proc. Natl. Acad. Sci. USA 90:3393).

EXAMPLE 21. EFFECT OF pH ON 2'-$NH_2$ RNA AND NON-MODIFIED SINGLE-STRANDED DNA LIGAND BINDING TO ELASTASE.

The pKa of 2'-$NH_2$-NTPs (nucleotide triphosphates) is approximately 6.5, not distant from the physiological pH range of 7.35-7.45. Therefore, at a physiological pH, a given RNA molecule containing 2'-$NH_2$-modified NTPs should have a distribution of protonated and unprotonated 2'-$NH_2$ groups. Consequently, it is likely that a small change in pH can affect the degree of protonation of a ligand, which may affect its binding to a target molecule.

The binding to elastase of representative 2'-$NH_2$ RNA ligand 14 and non-modified single-stranded DNA ligand 17 was examined over a pH range of 5.0-8.0. The results are shown in Figure 44. The binding of the 2'-$NH_2$ RNA ligand was profoundly affected by pH. For example, the Kd increased more than 10 fold from pH 6.5 to pH 6.25. The optimum binding for the 2'-$NH_2$ ligand is seen at pH 7.0, the pH at which SELEX was conducted. The ssDNA ligand bound better at pH 5 and the Kd increased approximately 3 fold over two pH units.

EXAMPLE 22. NUCLEIC ACID MIMETICS

The connecting loop nucleotides of the G-quartet structure was replaced with a synthetic linker tether group (Figure 45). DNA-17(TAGCGATACTGCGTGGGTTGGGGCGGGT AGGGCCAGCAGTCTCGTGCGGTACTTGAGCA) (SEQ ID NO:52) was synthesized with two loop nucleotides replaced by a 18-carbon ethylene glycol linker (Spacer phosphoramidite; Clonetech) to obtain a mimetic sequence, 17-LQT(TAGCGAT ACTGCGTGGGLGGGLGGGLGGGCCAGCAGTCTCGTGCGGTACTTGAGCA) (SEQ ID NO:53). L stands for the ethylene glycol linker. The length of the internucleotide phosphate is about a 3-carbon distance. Thus, the 18-carbon linker was expected to have enough flexibility to loop back into a G-quartet. 17-LQT was found not to bind elastase with significant affinity, however.

The absence of high affinity binding could be due to lack of loop nucleotides that are crucial for protein recognition or due to the synthetic tether restricting the oligonucleotide to fold into a tetraplex.

EXAMPLE 23. METHODS AND MATERIALS -NUCLEIC ACID LIGANDS TO THEOPHYLLINE AND CAFFEINE.

Materials. 1-carboxypropyl theophylline was provided by Abbott Laboratories, Abbott Park, IL.

SELEX. The essential features of the SELEX protocol are described in Example 1. Briefly, DNA templates for in vitro transcription (that contain a region of forty random positions flanked by constant sequence regions) and the corresponding PCR primers were synthesized chemically (Operon). The random region was generated by utilizing an equimolar mixture of the four nucleotides during oligonucleotide synthesis. The two constant regions were designed to contain PCR primer annealing sites, a primer annealing site for cDNA synthesis, T7 RNA polymerase promoter region, and restriction enzyme sites that allow cloning into vectors. Theophylline SELEX. experiments utilized 100 mM HEPES buffer (100 mM HEPES, pH 7.3, 0.5 M NaCl and 5 mM $MgCl_2$).

Cloning and Sequencing. Individual members of the enriched pools were cloned into pUC18 vector and sequenced as described (Schneider et *al*. (1992) J. Mol. Biol. Proc. Natl. Acad. Sci. USA 89:6992; Tuerk & Gold (1990) supra).

Equilibrium filtration analysis. A rapid procedure was developed to assess theophylline binding by oligonucleotides, called "equilibrium filtration". These assays were performed by the addition of [$^{14}$C]-theophylline and RNA at the indicated concentrations (see Figure 51) in a 150 µl reaction mixture containing 100 mM HEPES (pH 7.3), 5 mM $MgCl_2$, and 50 mM NaCl. Each binding mixture was incubated 5 min at 25 °C. The mixture was then placed on a Microcon 10 (Amicon) filtration device and centrifuged 4 min at 14,000 x g. Under these conditions, the RNA is retained by the filter while theophylline passes freely through the filter. A 25 µl sample was removed from each side of the filter and the radioactivity determined by scintillation counting. Each reaction was performed in triplicate. Other experiments established that this procedure provided results similar to that obtained by equilibrium dialysis. Equilibrium dialysis analysis of binding was carried out under identical reaction conditions to those described above. Each binding mixture was placed in a Spectra dialyzer (Spectrum) for 2 hr at 25 °C. Radioactivity retained inside the chamber was determined. Data were fit by least squares to a quadratic binding equation assuming a 1:1 stoichiometry (Gill et *al*. (1991) J. Mol. Biol. 220:307) (Figure 51).

Selection for RNA ligands to Theophylline. To screen for RNA molecules with affinity for theophylline, a pool of $10^{14}$ random RNAs were generated, each containing a 40 nucleotide random region flanked by fixed sequences for transcription and PCR amplification. These RNAs were generated by transcription of double-stranded DNA by T7 RNA polymerase as described above, and were radiolabelled with $[\alpha]^{32}$P-ATP. The pool of labelled RNA was added to an EAH Sepharose column to which 1-carboxypropyl theophylline (1-cp theophylline) was covalently bound via an amide linkage using the coupling reagents 1-ethyl-3(3-dimethylaminopropyl carbodimide (EDC) and 60 N-hydroxysuccinimide (NHS). In each SELEX selection round, about 0.5 nmoles of RNA was added to columns containing about 50 nmoles of theophylline. The addition of 0.5 M NaCl was found to be required to reduce non-specific RNA binding to the column. After allowing 10 min for interaction, the column was washed with loading buffer to remove non-specifically bound RNA. Bound RNA was partitioned by eluting with 0.1 M theophylline. The eluted RNA was precipitated, reverse transcribed with AMV reverse transcriptase, and the resulting DNA amplified by PCR as described above. This set of procedures constituted one SELEX selection round.

Theophylline SELEX TR8. The general progress of the experiment over eight selection rounds was monitored by determining the percent of the labelled RNA input to each selection round that was specifically eluted from the theophylline column. About 0.05% of the random RNA in the first selection round was eluted from the column. After eight rounds of selection, approximately 62% of the input RNA was eluted by theophylline, representing an affinity enrichment of about 1200-fold, relative to the starting population. This SELEX experiment was designated TR8.

## EXAMPLE 24. COUNTER-SELEX SELECTION FOR THEOPHYLLINE LIGANDS: TCT8.

A variation on the initial SELEX partition protocol was carried out after the fifth SELEX selection round. A different elution protocol was used to increase the stringency of the SELEX process. Rather than eluting with theophylline directly after washing, bound RNAs were first subjected to challenge with 0.1 M caffeine after the fifth SELEX selection round. Remaining RNAs were eluted with 0.1 M theophylline.

In the first counter-SELEX selection round, 99.7% of the theophylline-bound RNA was eluted with caffeine. The remaining 0.3% was eluted with theophylline and amplified. In the second counter-SELEX selection round, 70% of the bound RNAs were eluted with caffeine, the remaining 30% of bound RNAs were eluted with theophylline and amplified. A third and final counter-SELEX selection round generated a pool of RNAs termed "TCT8". Approximately 80% of the TCT8 RNAs bound directly to theophylline columns, representing an affinity enrichment of approximately 4000-fold relative to that of the starting population. Of the bound RNAs, 54% were resistant to caffeine challenge and were eluted subsequently by theophylline.

## EXAMPLE 25. SELEX SELECTION FOR RNA LIGANDS TO CAFFEINE.

RNA ligands with high affinity to caffeine were identified in a similar fashion to the experiments described in Example 23. Eight selection rounds of SELEX were performed using Sepharose-bound caffeine as the molecular target. Bound RNAs were eluted with excess free caffeine. Enrichment of the populations of RNAs for those with affinity for caffeine proceeded similarly to the theophylline SELEX experiment. In the first selection round, about 0.01% of the random RNA was eluted with caffeine. After eight selection rounds, 52.5% of the input RNA was eluted with caffeine. Twenty-one bacterial clones were generated from this population, designated CR8 and sequenced as described in Example 23. CR8 sequences are shown in Figure 54.

An important characteristic of the CR8 sequence family is that it lacks any apparent similarity to the 15 nucleotide conserved motifs of the theophylline RNA binding family (data not shown). Thus, surprisingly, RNAs selected for high affinity to caffeine are distinct from those selected to bind to theophylline. The binding properties of the CR8 RNA pool with $^{14}$C-caffeine were investigated by equilibrium dialysis. The CR8 RNA pool bound caffeine with a Kd of 15 $\mu$M, an affinity not dissimilar from that of the theophylline binding RNA population for theophylline after eight selection rounds.

## EXAMPLE 26. BINDING CHARACTERISTICS OF RNA LIGANDS FOR THEOPHYLLINE.

The binding properties of specific RNA species derived from the theophylline SELEX experiment were investigated by equilibrium filtration analysis with $^{14}$C-theophylline and varying concentrations of a representative SELEX ligand, TCT8-4 (SEQ ID NO:249). The results of these experiments indicated that the Kd of the TCT8-4 RNA-theophylline interaction was 0.58 $\mu$M (Figure 51). This affinity is similar to that observed for some monoclonal antibodies raised against theophylline (Poncelet et *al.* (1990) J. Immunoassay 11:77).

Binding by Truncated RNA ligand to Theophylline (mTCT8-4 (SEQ ID NO:286)). The hypothesis that the determinants in the TCT8-4 ligand responsible for high affinity binding to theophylline resided in the 15 nucleotide conserved domains was tested by determining the binding characteristics of a truncated RNA containing these domains and limited flanking residues. The mini-TCT8-4 RNA construct (mTCT8-4 (SEQ ID NO:286)) (Figure 52) is a 39 nucleotide T7 RNA polymerase transcript produced from a DNA template designed from the sequence of TCT8-4 RNA. The sequences of TCT8-4 present in mTCT8-4 are shown in Figure 49. Essentially, mTCT8-4 contains motifs 1 and 2, and the 6 bp vstem1 and 4 bp vstem2 regions of TCT8-4. It lacks the 24 nucleotide 3' fixed region of the TCT8-4 RNA. mTCT8-4 RNA was produced by T7 RNA polymerase in vitro, gel purified, and tested for its ability to bind to $^{14}C$-theophylline by equilibrium filtration analysis. A Kd of 0.11 μM was determined for mTCT8-4 binding to theophylline (Figure 51). This affinity is roughly seven-fold greater than that of TCT8-4, indicating that the presence of the flanking fixed sequence regions in TCT8-4 reduces affinity of the oligonucleotide, perhaps because of its influence on the conformation of the conserved sequence elements comprising the theophylline binding site. It is concluded that the structural determinants required for theophylline binding are entirely confined to the conserved regions of the oligonucleotide, whose specific three-dimensional orientation is likely dictated by non-conserved flanking stem and stem-loop domains.

EXAMPLE 27. COMPETITION BINDING STUDIES WITH XANTHINE DERIVATIVES.

An attractive feature of a small molecule-RNA interaction is the opportunity to readily investigate quantitatively the relative contribution of various constituents of the small molecule to the overall binding. Competition binding experiments were conducted with a variety of xanthine derivatives and related compounds. In these experiments, the effect of competitor added at various concentrations on the $^{14}C$-theophylline-TCT8-4 RNA interaction was measured. Of particular interest were the roles of $CH_3$ residues at the 1, 3, and 7 positions; these were assessed both individually and in combination. In addition, 1,3-dimethyluric acid probes the 8-position, as it contains a keto oxygen there relative to theophylline, and hypoxanthine, lacking the 2-position keto oxygen of xanthine, permit assessment of this constituent.

Equilibrium filtration assays were performed by the addition of various concentrations of the potential competitor to 1 μM [$^{14}C$]-theophylline and 3.3 μM TCT8-4 RNA (SEQ ID NO:249) in a 150 μl reaction mixture containing 100 mM HEPES buffer. Each reaction was performed as described in Example 23. Competition data were fit using a standard competition equation and fitting procedure (Gill et *al.* (1991) J. Mol. Biol. 220:307) assuming a 1:1 stoichiometry of competitor to RNA. A Kd = 0.45 μM for theophylline to RNA was used. From such data a competitor dissociation constant (Kc) was obtained. Under the condition where theophylline and the specific competitor bind to the same site, Kc = Kd. These data are summarized in Table 10. Competition binding data are shown in Figure 53.

Several features of the RNA binding surface are revealed by these studies. First, these results emphasize the major role played by the 7-position of xanthine derivative in recognition by the SELEX RNA. 7-methylxanthine competes at least 1500-fold less efficiently than xanthine, which itself is about 23-fold less effective than theophylline. The relatively good competition by xanthine indicates that the unadorned planar ring system is tightly accommodated by the RNA binding surface. As expected from the behavior of 7-methylxanthine, theobromine (3,7-dimethylxanthine) is also recognized poorly by TCT8-4 RNA. The 3-position provides positive binding energy as evidenced by the approximately 5-fold better competition of 3-methylxanthine relative to xanthine. In contrast, TCT8-4 RNA is relatively blind to the 1-position methyl, relative to xanthine. Hypoxanthine is bound about 5-fold more poorly by the RNA relative to xanthine, indicating that the 2-position keto oxygen is also important in the recognition. Finally, 1,3-dimethyl uric acid is very poorly recognized compared to theophylline, indicating that the binding site is highly sensitive to the 8-position.

Competition Binding Studies with Caffeine.

Competition binding studies showed that caffeine is an extremely poor competitor of theophylline for RNA binding. The competition data are consistent with a Kd for caffeine of about 6.5 mM. Thus, TCT8-4 (SEQ ID NO:249) can discriminate between theophylline and caffeine at least 9000-fold, corresponding to a ΔG of about 5.6 kcal/mol. Relative to the truncated version of TCT8-4, mTCT8-4 (SEQ ID NO:286), the discrimination is approximately 60,000-fold. From these experiments, it is apparent that TCT8-4 RNA contains a binding surface or pocket that closely inspects the 7- and 8-positions of these xanthine derivatives, and discriminates between theophylline and caffeine largely on the basis of its inability to accommodate the 7-position methyl group of caffeine.

EXAMPLE 28. MOLECULAR MODELING OF HIGH AFFINITY LIGAND FOR THEOPHYLLINE.

The conserved sequences present in each of the class I and II molecules may be arranged in space in a way that ultimately rationalizes their high discrimination in recognizing theophylline. As an initial step, molecular modeling, energy minimization and molecular dynamics of the conserved region and flanking sequences were used to model a small oligonucleotide composed of the conserved sequences and limited flanking regions. This model incorporates A-form helical parameters derived from tRNA crystal data to model those portions predicted to be helical. Bulged nucleotides are believed to introduce local kinking into the helical axis (Riordan et *al*. (1992) J. Mol. Biol. 226:305). The three base pyrimidine bulge was modeled as stacked within the flanking helical regions based on similar tracts that have been analyzed in the HIV TAR RNA by NMR analysis (Puglisi et al. (1992) Science 257:76). In addition, preliminary nuclease mapping experiments indicate that the two C residues in this bulge are not accessible to nuclease CL3 (data not shown). The three base symmetric bulge was modeled using a central UA Watson-Crick base pair, flanked by two non-Watson-Crick CA base pairs, AC and AG. It is noteworthy that the single position of variability in the conserved sequences would allow CA or AA pairs, which are thought to be isosteric from artificial phylogeny experiments exploring RNA recognition of the HIV Rev Response Element by the Rev protein (Bartel et *al.* (1991) Cell 67:529; Ellington (1993) Current Biol. 3: 375). Remarkably, in this model all of the conserved sequences play a coherent structural role in the formation of the major groove of the A-form helix that comprises the majority of the oligonucleotide. The asymmetric CCU bulge, and the non-Watson-Crick base pairs have the effect of slightly opening the major groove compared to that in fully duplex RNA. Such deformations of the major groove by helical perturbations provided by non-Watson-Crick pairs are a common theme of protein-RNA interactions (Bartel et *al*. (1991) *supra;* Ellington (1993) *supra).* While it is clear that theophylline can be physically accommodated in the major groove, the model lacks sufficient detail to rationalize the discrimination against caffeine binding (Figure 53).

TABLE 1. DISSOCIATION CONSTANTS FOR A REPRESENTATIVE SET OF FULL-LENGTH AND TRUNCATED HIGH-AFFINITY RNA LIGANDS FOR VEGF.[A]

| LIGAND[b] | Kd1 (nM)[c] | $\chi^{1\delta}$ | Kd2 (nM)[e] | SEQ ID NOS. |
|---|---|---|---|---|
| 100 | 0.20 ± 0.02 | 0.82 ± 0.02 | 42 ± 30 | 55 |
| 100t | 0.42 ± 0.04 | 0.76 ± 0.03 | 182 ± 94 | 95 |
| 44 | 1.7 ± 0.5 | 0.70 ± 0.11 | 38 ± 32 | 64 |
| 44t | 0.48 ± 0.04 | 0.73 ± 0.01 | 82 ± 23 | 96 |
| 12 | 0.48 ± 0.07 | 0.56 ± 0.03 | 21 ± 5 | 66 |
| 12t | 1.1 ± 0.2 | 0.78 ± 0.04 | 180 ± 160 | 97 |
| 40 | 0.19 ± 0.09 | 0.19 ± 0.04 | 10 ± 1 | 72 |
| 40t[f] | 20 ± 1 | - | - | 98 |
| 84 | 0.82 ± 0.2 | 0.45 ± 0.06 | 21 ± 5 | 80 |
| 84t | 1.8 ± 0.4 | 0.53 ± 0.07 | 31 ± 10 | 99 |
| 126 | 0.14 ± 0.04 | 0.40 ± 0.04 | 11 ± 3 | 82 |
| 126t | 1.4 ± 0.2 | 0.54 ± 0.03 | 181 ± 57 | 100 |

[a]Binding experiments were done as described in Example 13 and errors are given as standard deviations. [b]Full-length and truncated ligands are listed in pairs and represent sequence families 1-6, in order. [c]Dissociation constant of the higher-affinity binding component as defined in eq. 5. [d]mole fraction of the high-affinity binding component as defined in eq. 5.
[e]Dissociation constant of the lower-affinity binding component as defined in eq. 5. fDissociation constant for ligand 40t was determined by fitting the data points to eq. 2.

TABLE 2. Binding of 100t and 44t Truncates

| Target Molecule | 100t (Kd) (SEQ ID NO:95) | 44t (Kd) (SEQ ID NO:96) |
|---|---|---|
| bFGF | 1 uM | 0.6 uM |
| PDGF | 0.6 uM | 0.6 uM |
| antithrombin III | 3 uM | 12 uM |
| thrombin | > 10 uM | > 10 uM |
| plasminogen activator inhibitor I | > 10 uM | > 10 uM |

TABLE 3.

| Ligand | Kd1, nM | $\chi$ [1] | Kd2, nM | SEQ ID NOS. |
|---|---|---|---|---|
| Rndm RNA A | 83 $\pm$ 21 | - | - | |
| Rndm RNA B | 240 $\pm$ 140 | - | - | |
| 14A | 0.70 $\pm$ 0.16 | 0.42 $\pm$ 0.05 | $\approx 10^2$ | 120 |
| 23A | 2.8 $\pm$ 0.3 | - | - | 122 |
| 24A | 0.71 $\pm$ 0.14 | 0.79 $\pm$ 0.5 | $\approx 10^2$ | 123 |
| 41A | 0.86 $\pm$ 0.19 | 0.68 $\pm$ 0.11 | $\approx 10^2$ | 137 |
| 17B | 0.028 $\pm$ 0.008 | 0.62 $\pm$ 0.05 | $\approx 10^2$ | 109 |
| 26B | 0.37 $\pm$ 0.10 | 0.74 $\pm$ 0.15 | $\approx 10^2$ | 126 |
| 30B | 0.034 $\pm$ 0.009 | 0.77 $\pm$ 0.06 | $10^1$-$10^2$ | 112 |
| 32B | 0.050 $\pm$ 0.023 | 0.50 $\pm$ 0.06 | 15 $\pm$ 9 | 148 |
| 34B | 0.068 $\pm$ 0.016 | 0.82 $\pm$ 0.06 | $10^1$-$10^2$ | 114 |
| 14B | 0.14 $\pm$ 0.06 | 0.54 $\pm$ 0.09 | 9 $\pm$ 6 | 139 |

TABLE 4. 2'-$NH_2$ RNA LIGANDS TO ELASTASE.

| CLASS I | | SEQ ID NO: |
|---|---|---|
| Clone 12, 13, 17, 20, 20, 29, 30, 31, 33, 43, 55, 58 | GAGUAGGCUUGACGCCUGGGGGGGUAUGGCUUCGACUGCG | 191 |
| Clone 42 | --------------------------------A------ | 192 |
| Clone 44 | -------------- -------------------U---- | 193 |
| Clone 16 | A---G-------------------------C-------- | 194 |
| **CLASS II** | | |
| Clone 21, 56 | GGUAGACAUACGCCUUGGGGGGGUGUCAGGGUAUAUGGUA | 195 |
| Clone 57 | --C------------------------------------- | 196 |
| Clone 34 | ------------------------C--------------- | 197 |
| Clone 24 | ----------------------A----------------- | 198 |
| Clone 2 | --C------------------------------------C- | 199 |
| Clone 14 | --C-------------------U--------------C- | 200 |
| **CLASS III** | | |
| Clone 45, 10 | AGAAUGAAUGUGAUGAACAGGUGUCGGGGUGGAUGGGUGG | 201 |
| Clone 9 | G--------------------------------------- | 202 |
| Clone 6 | -AA------------------------------------- | 203 |
| Clone 26 | C-CC-U-G-CGCCAU--UGC-------------A----- | 204 |
| **CLASS IV** | | |
| Clone 8, 19, 41 | UGCCCGGCAGUACUGCACGGCCUCGGGGGGGACCAGGGAG | 205 |
| Clone 28 | AU-GGAUAGCGC-GCG-U---G--U--------------- | 206 |
| **CLASS V** | | |
| Clone 60 | GUACUACUGCAAGCCCGUGUGGCGCGGUCAGUGGGUGGC | 207 |
| Clone 35 | ---------------------------------------C | 208 |
| Clone 4 | CGGAUCGGGCGGUCGU-UA-C--GAU--CGU-C------A | 209 |
| Clone 50 | UGAC-GGG-CG-CAUG-C--A--U-U--C----------- | 210 |
| Clone 22 | GGCC--CUG-A--GUCUG----U---U----------●●● | 211 |
| Clone 25 | UGACCGGG-C-AC-UG-UUGAG-UGUG-CC---------- | 212 |
| **CLASS VI** | | |
| Clone 11 | GACGCGUGCUGGCCUCGACCGUGUGGGUGCGGAUGGGUGG | 213 |
| Clone 3 | UGAG--UGA-UAG---UGGUAA---CCA---C----G--GUCGG | 214 |
| Clone 46 | UG-AUA-CGCUG-GU-----C-CU--G-G--U---CA--G | 215 |

TABLE 5. Kds OF REPRESENTATIVE LIGANDS

| CLASS | CLONE NUMBER | SEQ ID NO: | Kd (ηM) |
|---|---|---|---|
| I | 30 | 191 | 10 |
| | 44 | 193 | 28 |
| II | 21 | 195 | 39 |
| | 14 | 200 | 17 |
| | 24 | 198 | 11 |
| | 34 | 197 | 8 |
| III | 9 | 202 | 10 |
| | 26 | 204 | 38 |
| IV | 19 | 205 | 8 |
| | 28 | 206 | 57 |
| V | 4 | 209 | 172 |
| | 22 | 211 | 24 |
| VI | 3 | 214 | 28 |
| | 46 | 215 | 13 |

TABLE 6. SPECIFICITY OF 2'-$NH_2$ LIGANDS TO ELASTASE

| LIGAND | SEQ ID NO: | ELASTASE | Kd for THROMBIN | Kd for bFGF |
|---|---|---|---|---|
| Class II clone 24 | 198 | 11 ηM | 0.6 μM | > 1 μM |
| Class III, clone 9 | 202 | 9.7 ηM | -- | 0.5 μM |

TABLE 7. BINDING OF TRUNCATED 2'-$NH_2$ LIGANDS TO ELASTASE

| CLASS | LIGAND | SEQ ID NO: | Kd (ηM) | |
|---|---|---|---|---|
| | | | Full Length | 3'-Truncated |
| I | 12 | 191 | 10 | 10 |
| II | 14 | 200 | 17 | 14.5 |
| III | 9 | 202 | 10 | 989 |
| IV | 19 | 205 | 8 | 648 |

TABLE 8. THE EFFECT OF $K^+$, $Li^+$, AND TRIS ON BINDING OF 2'-$NH_2$ LIGANDS TO ELASTASE.

| CLASS | LIGAND | SEQ ID NO: | Kd (ηM) | | |
|---|---|---|---|---|---|
| | | | KC1 | LiCl | Tris -HCl |
| I | 30 | 191 | 13.8 | 581 | 38 |
| II | 34 | 197 | 5.5 | 669 | 59 |
| III | 9 | 202 | 378 | 231 | 34 |

TABLE 9. SINGLE-STRANDED DNA LIGANDS TO ELASTASE.

| CLONE | SEQUENCE | SEQ ID NO: |
|---|---|---|
| 1 | ACCTTTTCCTCTCAGTCTTCTTATCTCGCCTATTATTATT | 216 |
| 2 | GCGTGGGTTGGGGCCGGGAGGGCCAGCAGTCTCGTGCGTC | 217 |
| 3 | CATTCATCTTCTCATTCTCGCCTAACTGTACACATCTTT | 218 |
| 4 | GCGTGGGTTGGGGCCGGGAGGGCCAGCAGTCTCGTGCGT | 219 |
| 5 | GCGTGGGTTGGGGCCGGGAGGGCCAACAGTCTCGTGCGTC | 220 |
| 6 | CTACCCTTTCTTGACCACCGCCTCGTTTCATCCACCTTAC | 221 |
| 7 | TTCTTTCTATACCCATATTACCCTTCTTCACACTCGTATC | 222 |
| 14 | CTTTATCCTTTCTCTTTCCTTGCACTCTAACATCCTACTC | 223 |
| 15 | GCGTGGGTTGGGGCCGGTAGGGCCAGCAGTCTCGTTGCGT | 224 |
| 16 | CCTTCTTGTTATATTGGTCGTTTTCTTCTTTTACTTTCTT | 225 |
| 17 | TCTTCATCATTTCACTTCATTCTGTCGGGCTATCTTCGGT | 226 |
| 18 | TTCCACGTCTCCTCAGCCCGGGAGGCCACCTTTTTATCTG | 227 |
| 19 | GAAGGCTTAACCTAATTTTCCACCTTTCATCCACTTTTCC | 228 |
| 20 | TCACCTCCCATTTATATTTTCCCTTAATTTCTTCTTCTTA | 229 |
| 23 | CTTACTATGCATCTTACTTATTATTTTTTTTTACTTTCTA | 230 |
| 24 | TACTTCTTTTACATCATTCCTCGATTTATTCATTCTCCAC | 231 |
| 25 | TTCACCCGTGTCATATCATATTTCCCGGTCCTTCCTTTCCC | 232 |
| 28 | CAATTCAAACCTTTTCTACAATTTTCATCTTACATTCTTC | 233 |
| 44 | TCACTTGATCCTTCTTTACTTTTTTTCTCGTCTAATTATA | 234 |
| 45 | GCGTGGGTTGGGGCGGGATGGGCCAGCAGTCTCGTGCGGT | 235 |
| 46 | CTTTTTATTCCAACCCCCATTCTTACTTACAATATCTTGA | 236 |
| 47 | TATCCTTCTCCTTAACTCCTACTTCTATCTATAAAATTAT | 237 |
| 11 | GCGTGGGTAGGGGCCGGGAGGGCCAGCAGTCACGTGCGTA | 238 |
| 50 | GGGTGGGTTGGGGCCGGGAGGGCTAGCAGTCTCGTGCGTT | 239 |
| 51 | GCGTGGGATGGGGCCGGGAGGGCCAGCAGTCTCGTGCGTT | 240 |
| 58 | GCGTGGGTTGGGGCCGGGAGGGCCAGCAGTCTCGTGCGT | 241 |

TABLE 10. COMPETITION BINDING ANALYSIS WITH TCT8-4 RNA

| COMPETITOR | Kc (μM) | Kc(c)/Kc(t) |
|---|---|---|
| Theophylline | 0.32 ± 0.13 | 1 |
| CP-Theophylline | 0.93 ± 0.20 | 2.9 |
| Xanthine | 8.5 ± 0.40 | 27 |
| 1-methylxanthine | 9.0 ± 0.30 | 28 |
| 3-methylxanthine | 2.0 ± 0.7 | 6.3 |
| 7-methylxanthine | > 500 | > 1500 |
| 3,7-dimethylxanthine | > 500 | > 1500 |
| 1,3-dimethylxanthine | > 1000 | > 3100 |
| Hypoxanthine | 49 ± 10 | 153 |

(continued)

| COMPETITOR | Kc (μM) | Kc(c)/Kc(t) |
|---|---|---|
| Caffeine | 3500 ± 1500 | 10900 |

## Claims

1. A nucleic acid ligand which binds specifically to vascular endothelial growth factor (VEGF) and is an inhibitor of VEGF.

2. The nucleic acid ligand of claim 1 wherein said ligand is single-stranded.

3. The nucleic acid ligand of claim 2 wherein said ligand is an RNA ligand.

4. The nucleic acid ligand of claim 3 wherein said ligand is selected from the group consisting of SEQ ID NOs:48-82 and 94-100.

5. The nucleic acid ligand of claim 3 wherein said ligand is comprised of 2' $NH_2$ (2'-amino) modified nucleotides.

6. The nucleic acid ligand of claim 5 wherein said ligand is selected from the group consisting of SEQ ID NOs:107-154.

7. A pharmaceutical composition comprising the nucleic acid ligand of claim 1, wherein said ligand contains one or more modified bases.

8. The pharmaceutical composition of claim 7 wherein said ligand is an RNA ligand.

9. The pharmaceutical composition of claim 8 wherein said RNA ligand is comprised of 2' $NH_2$ (2'-amino) modified nucleotides.

10. A nucleic acid ligand which binds specifically to vascular endothelial growth factor (VEGF) and is an inhibitor of VEGF identified according to the method comprising:

a) preparing a candidate mixture of nucleic acids;
b) contacting the candidate mixture of nucleic acids with VEGF, wherein nucleic acids having an increased affinity to VEGF relative to the candidate mixture may be partitioned from the remainder of the candidate mixture;
c) partitioning the increased affinity nucleic acids from the remainder of the candidate mixture;
d) amplifying the increased affinity nucleic acids to yield a mixture of nucleic acids enriched for nucleic acids with relatively higher affinity and specificity for binding to VEGF.

11. The nucleic acid ligand of claim 10 wherein said candidate mixture of nucleic acids is comprised of single stranded nucleic acids.

12. The nucleic acid ligand of claim 11 wherein said single stranded nucleic acids are ribonucleic acids.

13. The nucleic acid ligand of claim 11 wherein said candidate mixture of nucleic acids comprises 2'-$NH_2$ (2'-amino)

modified ribonucleic acids.

General Formula of Nucleoside Analogs

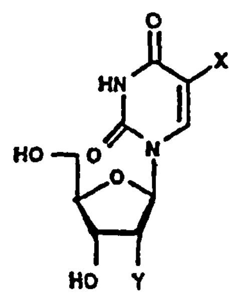

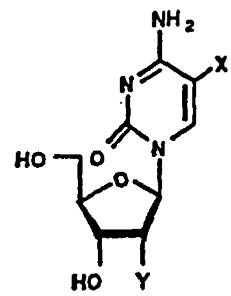

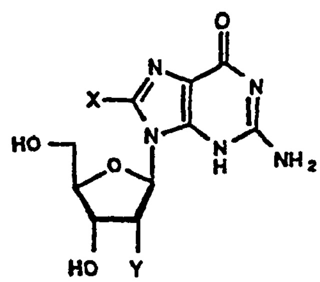

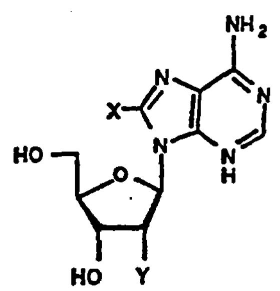

X= I, Br, $Cl_3NNH_2$

Y= $NH_2$, F, $OCH_3$

FIG. 1

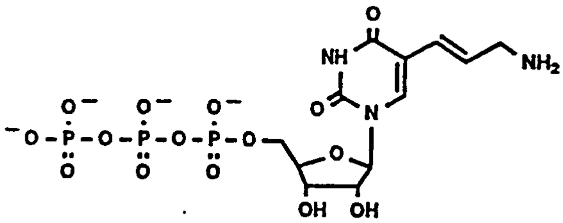

5-allylamino-UTP

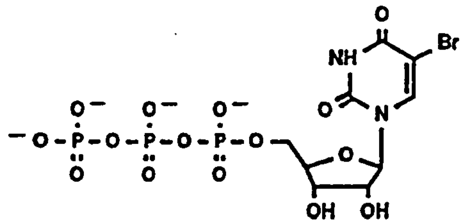

5-Bromo-UTP

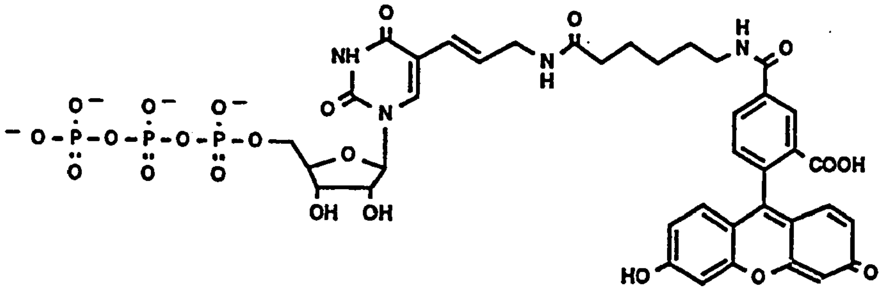

5-Fluorescein-12-UTP

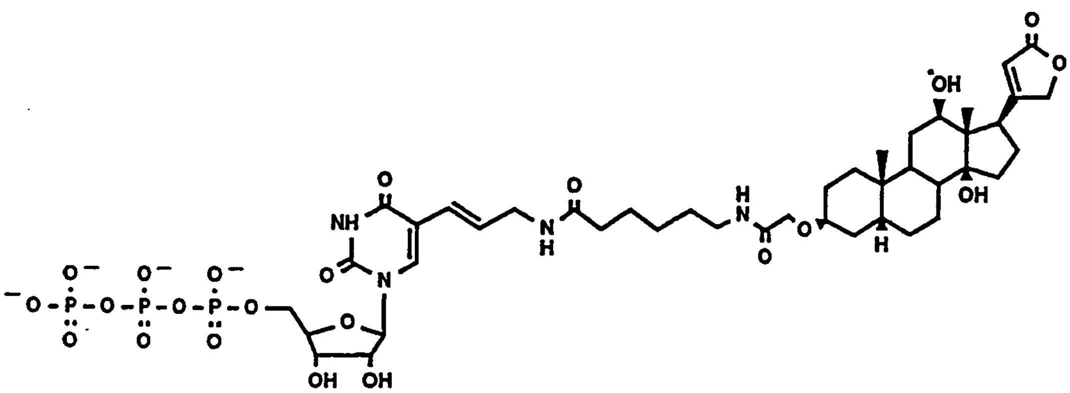

5-Digoxygenin-11-UTP

FIG. 2

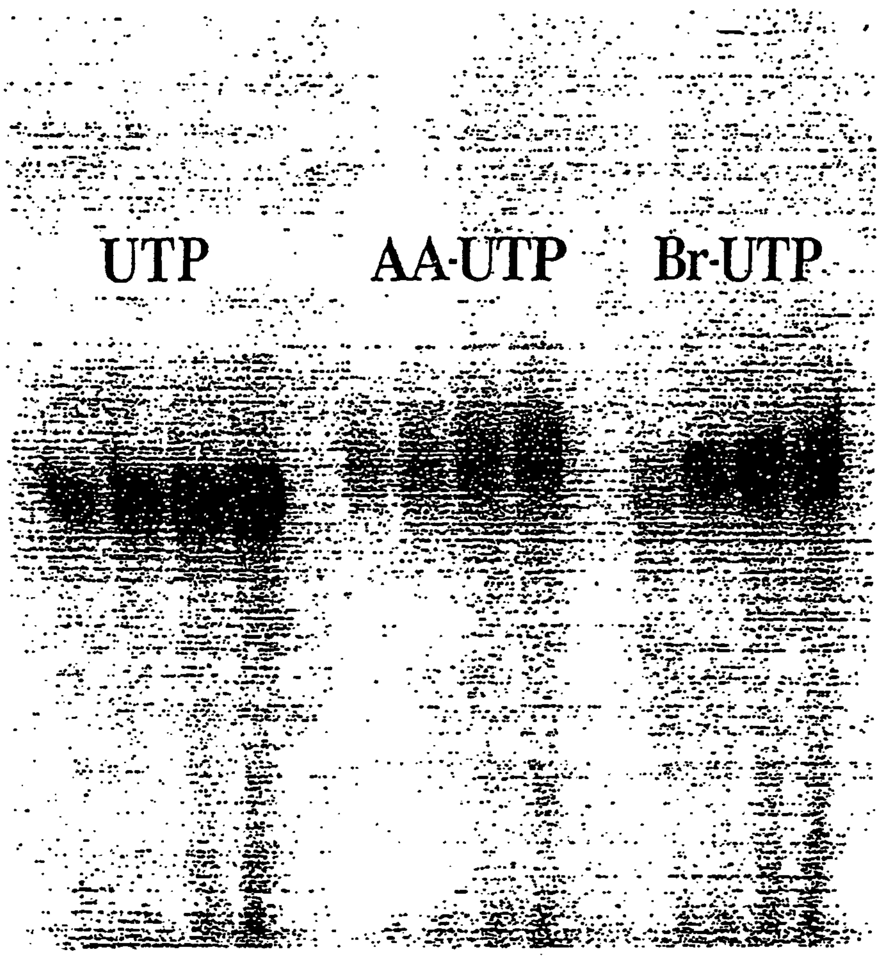


FIG. 3

| Side Chain | Structure | Target | Reference |
|---|---|---|---|
| MQPA | 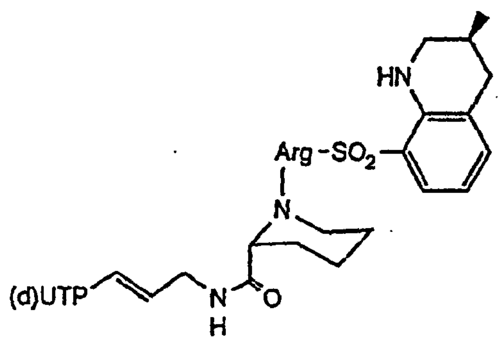 | Thrombin | Kikumoto et al. (1984) *Biochemistry* 23, 85. |
| RGD | 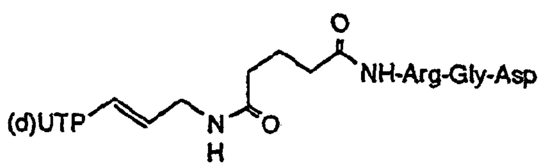 | Integrins (GP IIb/IIIa) | Rouslahti et al. (1986) *Cell* 44, 517. |
| Y | 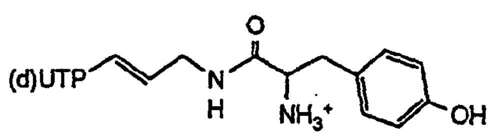 | Tyrosine Kinases | Bolen (1993) *Oncogene* 8, 2025. |

FIG. 4

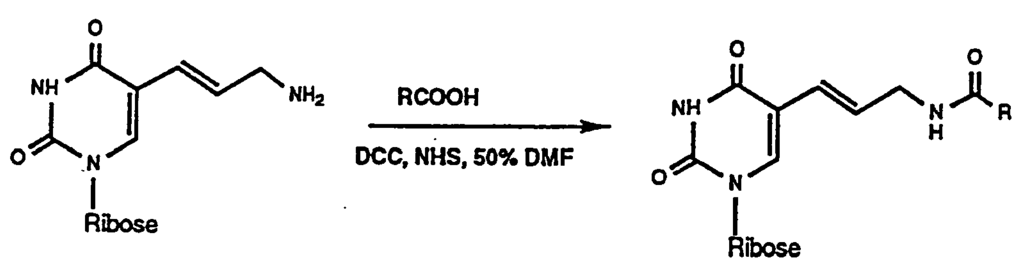


FIG. 5

**THROMBIN 2'OH RNA BINDING SEQUENCES--ROUND 12**

**Class I (22 clones)**

| | | SEQ ID NO |
|---|---|---|
| #1 | GGGAGAUGCCUGUCGAGCAUGCUG A**GGAUCGAAG**UU**AGUAGGC**UUUGUGUGCUC GUAGCUAAACAGCUUUGUCGACUCU | 1 |
| #6 | GGGAGAUGCCUGUCGAGCAUGCUG A**GGAUCGAAG**UU**AGUAGGC**UUUGUGUGCUC GUAGCUAAACAGCUUUGUCGACUCU | 1 |
| #13 | GGGAGAUGCCUGUCGAGCAUGCUG A**GGAUCGAAG**UU**AGUAGGC**UUUGUGUGCUC GUAGCUAAACAGCUUUGUCGACUCU | 1 |
| #19 | GGGAGAUGCCUGUCGAGCAUGCUG A**GGAUCGAAG**UU**AGUAGGC**UUUGUGUGCUC GUAGCUAAACAGCUUUGUCGACUCU | 1 |
| #23 | GGGAGAUGCCUGUCGAGCAUGCUG A**GGAUCGAAG**UU**AGUAGGC**UUUGUGUGCUC GUAGCUAAACAGCUUUGUCGACUCU | 1 |
| #24 | GGGAGAUGCCUGUCGAGCAUGCUG A**GGAUCGAAG**UU**AGUAGGC**UUUGUGUGCUC GUAGCUAAACAGCUUUGUCGACUCU | 1 |
| #25 | GGGAGAUGCCUGUCGAGCAUGCUG A**GGAUCGAAG**UU**AGUAGGC**UUUGUGUGCUC GUAGCUAAACAGCUUUGUCGACUCU | 1 |

FIG. 6A

| | | SEQ ID NO |
|---|---|---|
| #30 | GGGAGAUGCCUGUCGAGCAUGCUG A**GGAUCGAAG**UU**AGUAGGC**UUUGUGUGCUC GUAGCUAAACAGCUUUGUCGACUCU | 1 |
| #2 | GGGAGAUGCCUGUCGAGCAUGCUG UACU**GGAUCGAAG**GU**AGUAGGC**AGUCAC GUAGCUAAACAGCUUUGUCGACUCU | 2 |
| #5 | GGGAGAUGCCUGUCGAGCAUGCUG AUAUCAC**GGAUCGAAG**GA**AGUAGGC**GUG GUAGCUAAACAGCUUUGUCGACUCU | 3 |
| #9 | GGGAGAUGCCUGUCGAGCAUGCUG CCUUUCCCG**GG**U**UCGAAG**UC**AGUAGGC**CGG GUAGCUAAACAGCUUUGUCGACUCU | 4 |
| #10 | GGGAGAUGCCUGUCGAGCAUGCUG CACCC**GGAUCGAAG**UU**AGUAGGC**GUGAGU GUAGCUAAACAGCUUUGUCGACUCU | 5 |
| #15 | GGGAGAUGCCUGUCGAGCAUGCUG UGUAC**GGAUCGAAG**GU**AGUAGGC**AGGUUAC GUAGCUAAACAGCUUUGUCGACUCU | 6 |
| #16 | GGGAGAUGCCUGUCGAGCAUGCUG CAUCC**GGAUCGAAG**UU**AGUAGGC**GGAGUG GUAGCUAAACAGCUUUGUCGACUCU | 7 |
| #18 | GGGAGAUGCCUGUCGAGCAUGCUG AUUGUUGC**GGAUCGAAG**UGG**AGUAGGC**GCA GUAGCUAAACAGCUUUGUCGACUCU | 8 |

FIG. 6A (CONT'D)

| | Sequence | SEQ ID NO |
|---|---|---|
| #21 | GGGAGAUGCCUGUCGAGCAUGCUG UGUACU**GGAUCGAAG**GU**AGUAGGC**AGUCAC GUAGCUAAACAGCUUUGUCGACUCU | 9 |
| #22 | GGGAGAUGCCUGUCGAGCAUGCU**G** **AUCGAAG**UU**AGUAGG**AGCGUGUG GUAGCUAAACAGCUUUGUCGACUCU | 10 |
| #26 | GGGAGAUGCCUGUCGAGCAUGCUG ACGCUAGAGUC**GGAUCGAA**AGGUA**AGUAGGC**GACU GUAGCUAAACAGCUUUGUCGACUCU | 11 |
| #31 | GGGAGAUGCCUGUCGAGCAUGCUG GGGUC**GGAUCGAA**AGGUA**AGUAGGC**GACU GUAGCUAAACAGCUUUGUCGACUCU | 12 |
| #33 | GGGAGAUGCCUGUCGAGCAUGCUG AUAUCAC**GGAUCGAA**AGAG**AGUAGGC**GU GUAGCUAAACAGCUUUGUCGACUCU | 13 |
| #34 | GGGAGAUGCCUGUCGAGCAUGCUG UGUACU**GGAUCGAAG**GU**AGUAGGC**AGGCAC GUAGCUAAACAGCUUUGUCGACUCU | 14 |
| #37 | GGGAGAUGCCUGUCGAGCAUGCUG AUAUCAC**GGAUCGAAG**GAA**AGUAGGC**GUG GUAGCUAAACAGCUUUGUCGACUCU | 15 |

FIG. 6A (CONT'D)

**Class II (6 clones)**

| | | | SEQ ID NO |
|---|---|---|---|
| #3 | GGGAGAUGCCUGUCGAGCAUGCUG GUAGCUAAACAGCUUUGUCGACUCU | GU**GCGGCUUUGGGCGCCGUGCUU**GGC | 16 |
| #20 | GGGAGAUGCCUGUCGAGCAUGCUG GUAGCUAAACAGCUUUGUCGACUCU | GU**GCGGCUUUGGGCGCCGUGCUU**AC | 17 |
| #27 | GGGAGAUGCCUGUCGAGCAUGCUG GUAGCUAAACAGCUUUGUCGACUCU | GU**GCGGCUUUGGGCGCCGUGCUU**GAC | 18 |
| #35 | GGGAGAUGCCUGUCGAGCAUGCUG GUAGCUAAACAGCUUUGUCGACUC | GG**GCGGCUUUGGGCGCCGUGCUU**GAC | 19 |
| #38 | GGGAGAUGCCUGUCGAGCAUGCUG GUAGCUAAACAGCUUUGUCGACUCU | GU**GCGGCUUUGGGCGCCGUGCUU**GAC | 18 |
| #39 | GGGAGAUGCCUGUCGAGCAUGCUG GUAGCUAAACAGCUUUGUCGACUCU | GU**GCGGCUUUGGGCGCCGUGCUU**GAC | 18 |

FIG. 6A (CONT'D)

**THROMBIN 2'$NH_2$ RNA SEQUENCES--ROUND 15**

**Group I (15 clones)**

| Clone | Sequence | SEQ ID NO |
|---|---|---|
| #32 | GGGAGAUGCCUGUCGAGCAUGCUG______UGAGCCUGCCAGUGUGUAUGUGGAAACAAG GUAGCUAAACAGCUUUGUCGAC UCU | 20 |
| #2 | GGGAGAUGCCUGUCGAGCAUGCUG______UGAGCCUGCCAGUGUGCAUGUGGAAACAAG GUAGCUAAACAGCUUUGUCGAC UCU | 21 |
| #12,27,30,31,35,39,48 | GGGAGAUGCCUGUCGAGCAUGCUG______UGAGCCAGCCAGUGUGCAUGUGGAAACAAG GUAGCUAAACAGCUUUGUCGAC UCU | 22 |
| 13,20,28,42 | GGGAGAUGCCUGUCGAGCAUGCUG______UGAGCCAGCCAGUGUGUAUGUGGAAACAAG GUAGCUAAACAGCUUUGUCGAC UCU | 23 |
| #23 | GGGAGAUGCCUGUCGAGCAUGCUG______UGAGCCGGCCAGUGUGCAUGUGGAAACAAG GUAGCUAAACAGCUUUGUCGAC UCU | 24 |
| #46 | GGGAGAUGCCUGUCGAGCAUGCUG______UGAGCCAGCCAGUGCGUAUGUGGAAACAAG GUAGCUAAACAGCUUUGUCGAC UCU | 25 |

FIG. 6B

**THROMBIN 2'NH2 RNA SEQUENCES--ROUND 15**

**Group IIA (25 clones)**

SEQ ID NO

33,43 GGGAGAUGCCUGUCGAGCAUGCUG UUACGGGGAGGUGUUAGGGAGUGUACCC
GUAGCUAAACAGCUUUGUCGACUCU 26

#3,14,8,15,34,38,40,51 (A)
GGGAGAUGCCUGUCGAGCAUGCUG UUGCGGGGAGGUGUUAGGGAGUGUACCC
GUAGCUAAACAGCUUUGUCGACUCU 27

#6 GGGAGAUGCCUGUCGAGCAUGCUG UUGCGGGGAGGUGUUAGXXAGUGUACCC
GUAGCUAAACAGCUUUGUCGACUCU 28

(A)
#24 GGGAGAUGCCUGUCGAGCAUGCUG UUGCGGGGAGGUGUUAGGGAGUUCACCC
GUAGCUAAACAGCUUUGUCGACUCU 29

#7,9,26,36,37,41,52,54
GGGAGAUGCCUGUCGAGCAUGCUG CUGCGGGGAGGUGUUAGGGAGUGUACCC
GUAGCUAAACAGCUUUGUCGACUCU 30

FIG. 6B (CONT'D)

**THROMBIN 2'$NH_2$ RNA SEQUENCES--ROUND 15**

**Group II (30 clones)**

**Group IIA (25 clones)**

| | | SEQ ID NO |
|---|---|---|
| #10,18 | GGGAGAUGCCUGUCGAGCAUGCUG CUGCGGGAGGUGUUAGAGAGUGUACCC GUAGCUAAACAGCUUUGUCGACUCU | 31 |
| #11 | GGGAGAUGCCUGUCGAGCAUGCUG CUGCGGGAGGUGUCAGAGAGUGUACCC GUAGCUAAACAGCUUUGUCGACUCU | 32 |
| #22 | GGGAGAUGCCUGUCGAGCAUGCUG CUACGGGAGGUGUUAGAGAGUGUACCU GUAGCUAAACAGCUUUGUCGACUCU | 33 |
| #53 | GGGAGAUGCCUGUCGAGCAUGCUG CUACGGGAGGUGUCGGAGAGUGUACCU GUAGCUAAACAGCUUUGUCGACUCU | 34 |

FIG. 6B (CONT'D)

**THROMBIN 2'$NH_2$ RNA SEQUENCES--ROUND 15**

**Group IIB (5 clones)**

SEQ ID NO

#4 GGGAGAUGCCUGUCGAGCAUGCUG CACGAGGUGUCAGAGAGUGUAG(G)UUCAGC
GUAGCUAAACAGCUUUGUCGACUCU 35

17,50 GGGAGAUGCCUGUCGAGCAUGCUG CACGAGGUGUCAGAGAGUGUAGUGCAGC
GUAGCUAAACAGCUUUGUCGACUCU 36

#1 GGGAGAUGCCUGUCGAGCAUGCUG CACGAGGUGUAGAGGGUGUAGUGCAGC
AGUACGUAAACAGCUUUGUCGACUCU 37

#5 GGGAGAUGCCUGUCGAGCAUGCUG CACGAGGCGUCAGAGAGUGUA(U)GUGCUGC
GUACGUAAACAGCUUUGUCGACUCU 38

**Group III (2 clones)**

#29 GGGAGAUGCCUGUCGAGCAUGCUG AGGAUCGAAGUUAGUAGGCUUUGUGUACUC
GUAGCUAAACAGCUUUGUCGAC UCU 39

FIG. 6B (CONT'D)

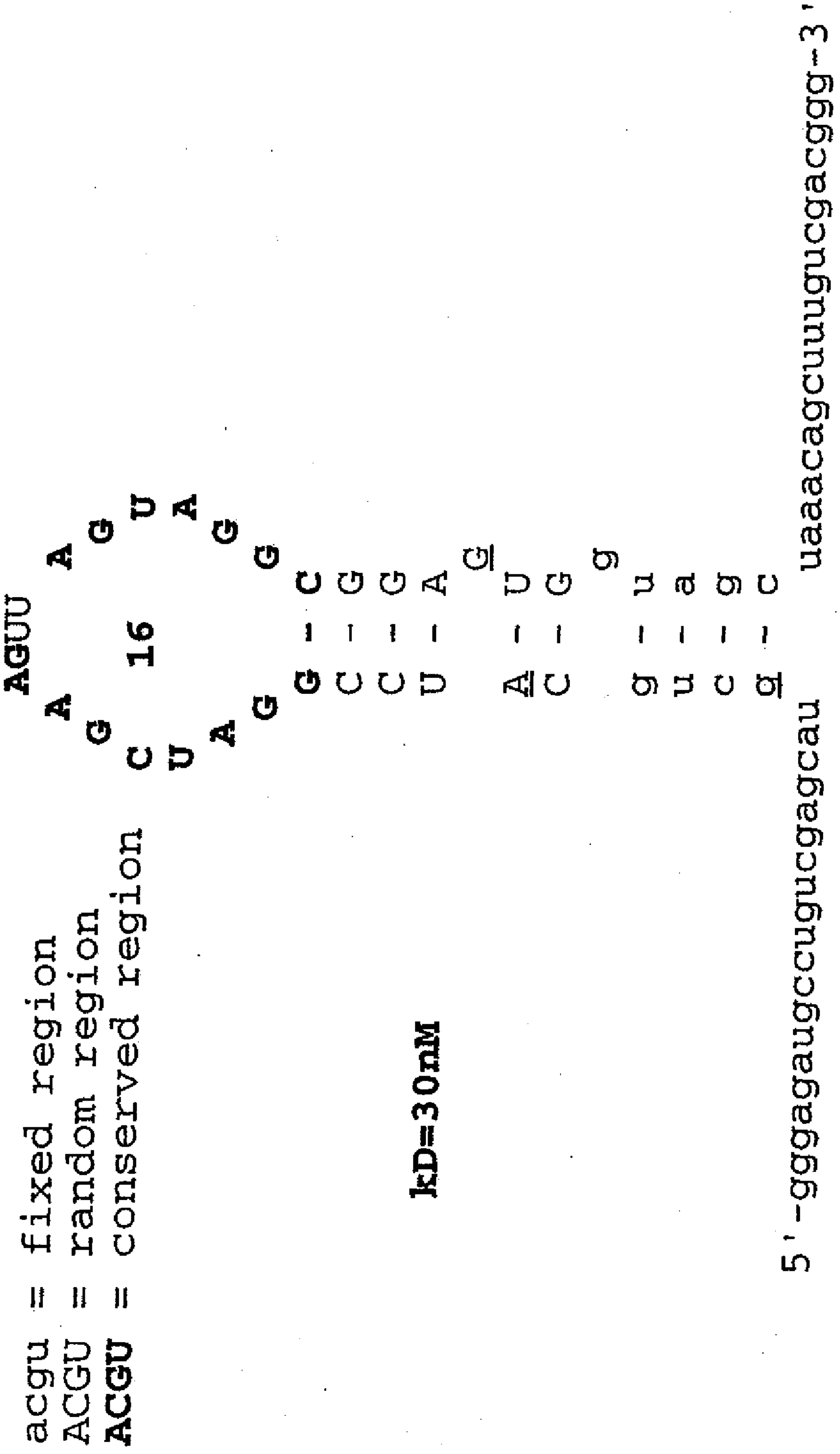


**CLASS I**
**Clone 16**
**SEQ ID NO: 7**
**FIG. 7A**

acgu = fixed region
ACGU = random region
**ACGU** = conserved region

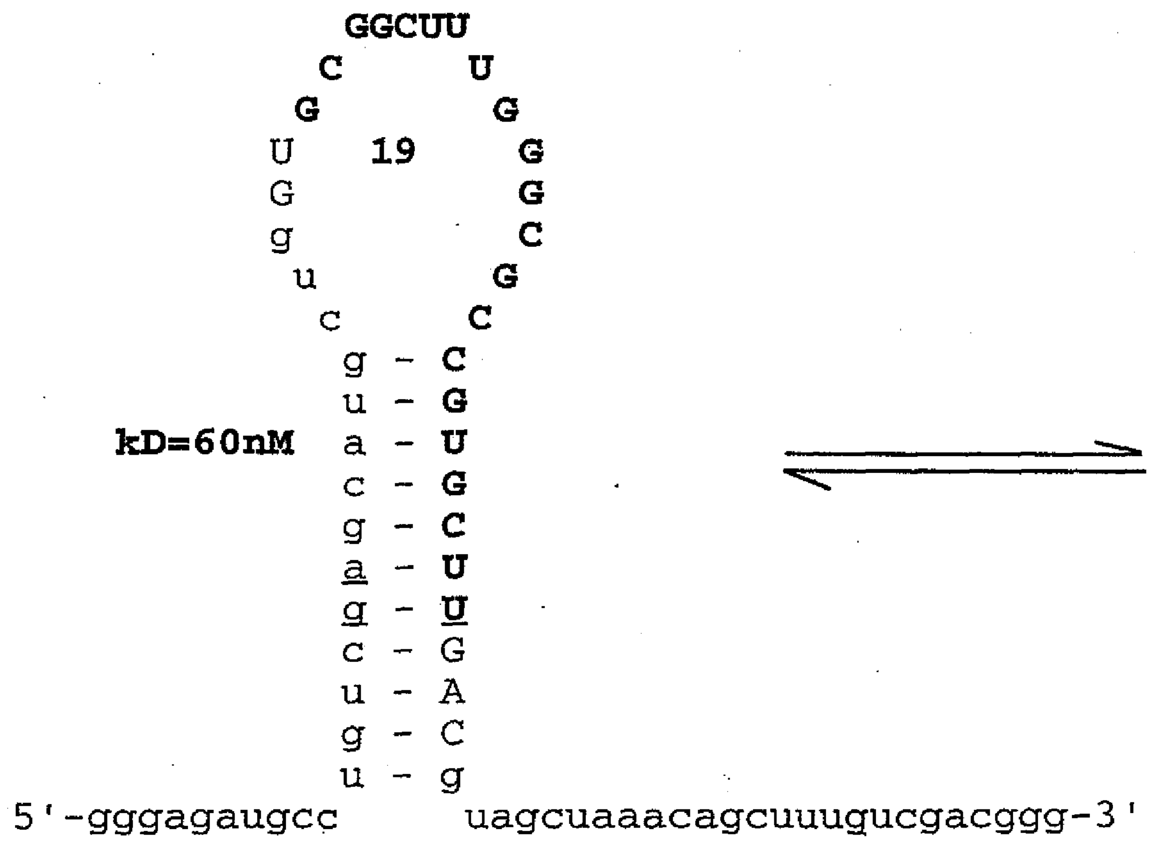


**CLASS II**
**Clone 27**
**SEQ ID NO:18**
**FIG. 7A (CONT'D)**

acgu = fixed region
ACGU = random region
**ACGU** = conserved region

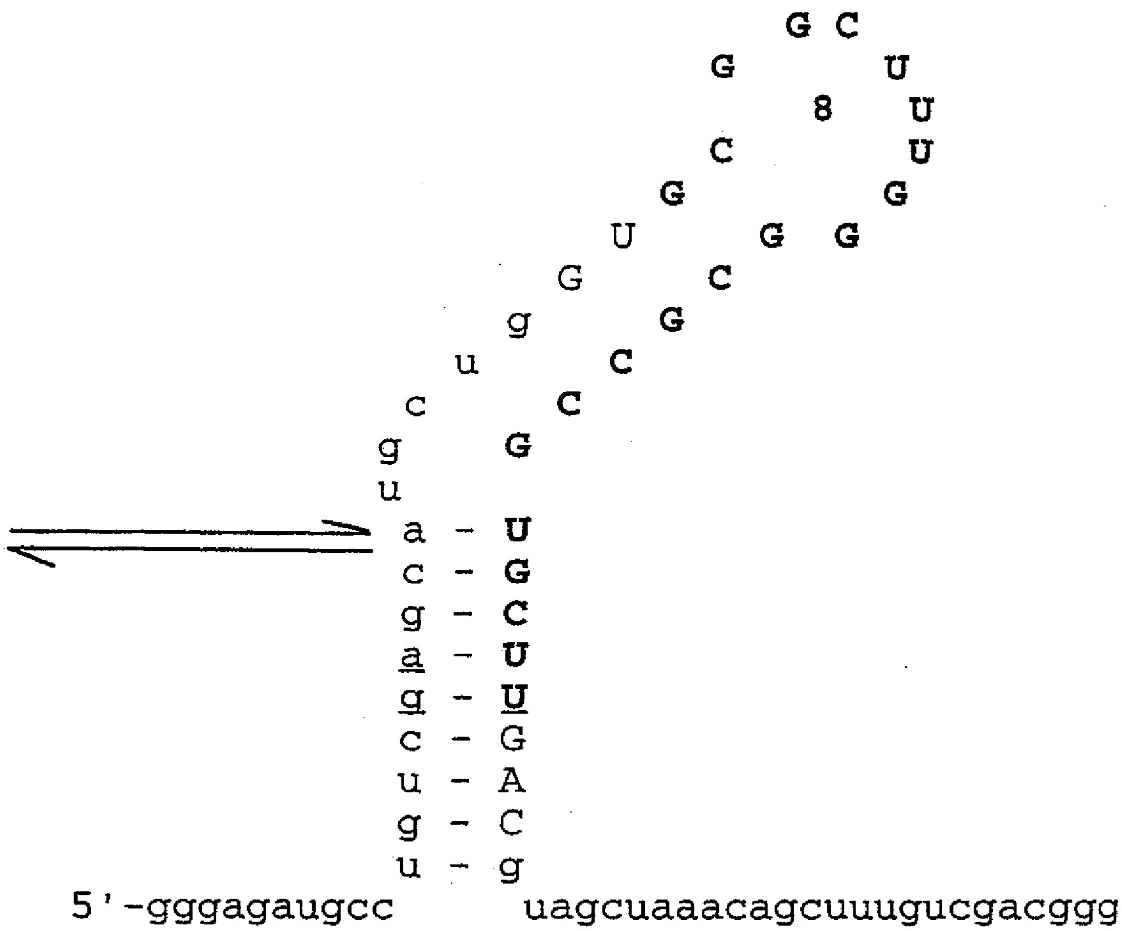


**CLASS II**
**Clone 27**
**SEQ ID NO:18**
**FIG. 7A (CONT'D)**

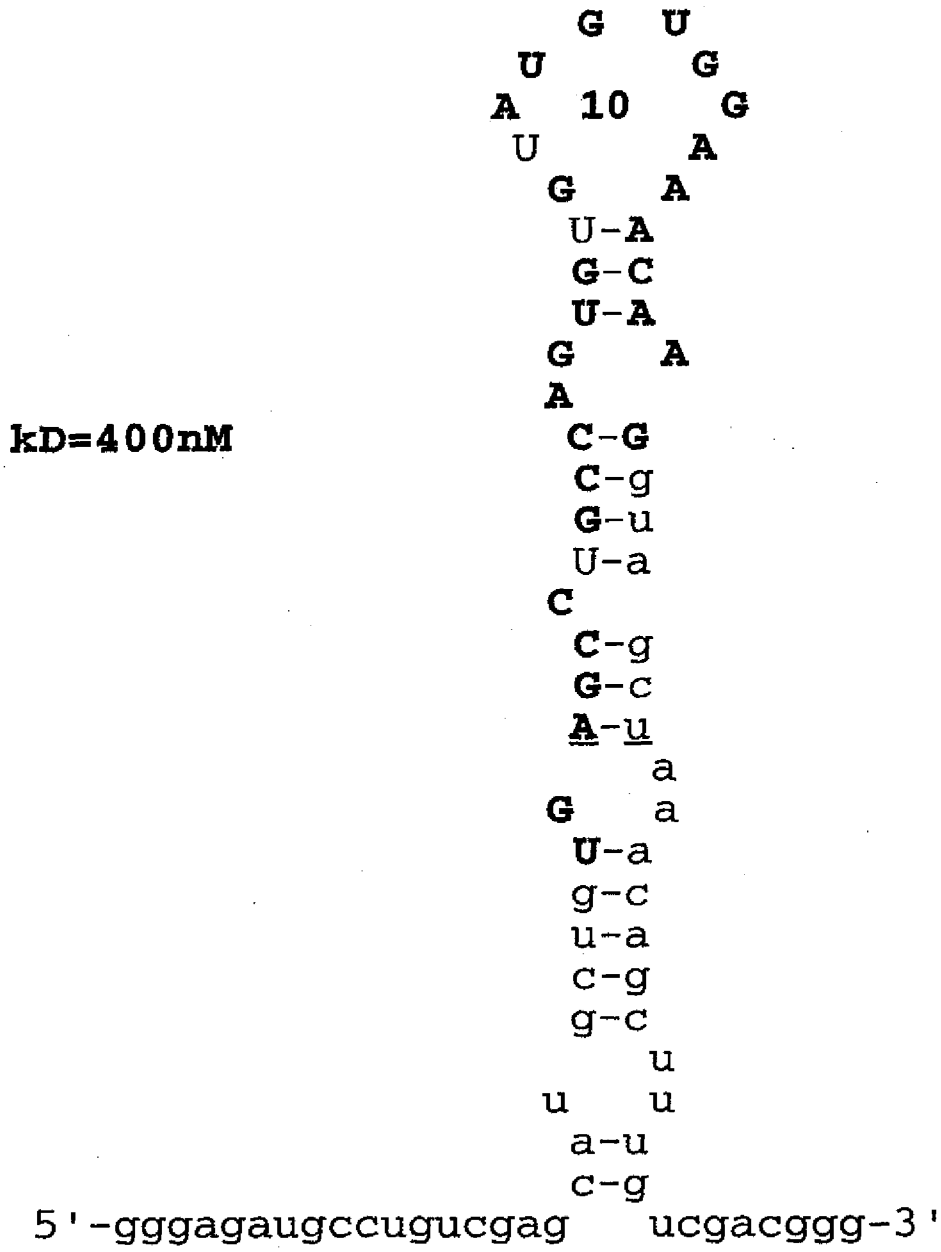


GROUP I
Clone 32
SEQ ID NO: 20
FIG. 7B

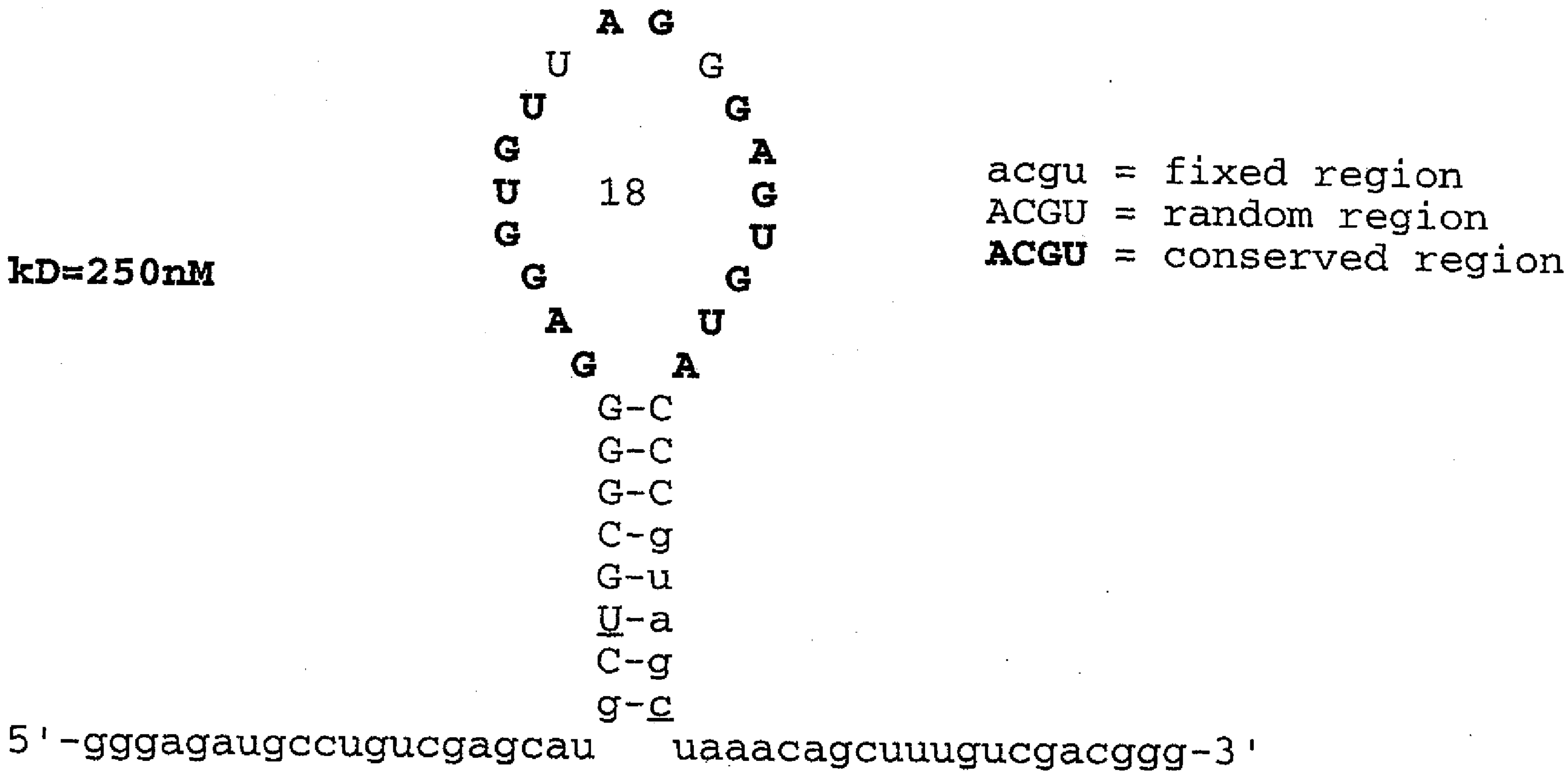


GROUP IIA
CLONE 37
SEQ ID NO:30
FIG. 7B (CONT'D)

kD=200nM

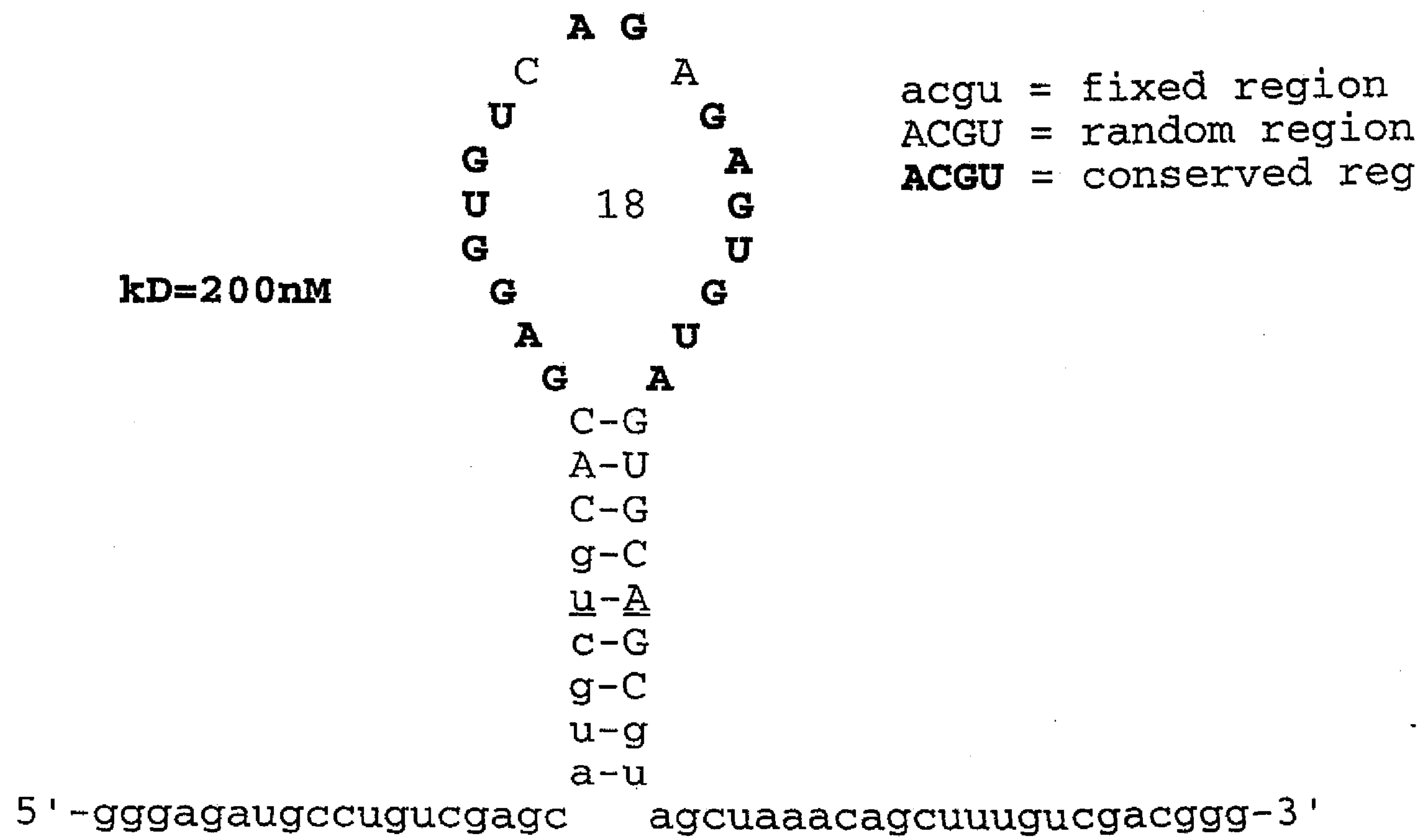


acgu = fixed region
ACGU = random region
**ACGU** = conserved region

GROUP IIB
CLONE 17
SEQ ID NO:36
FIG. 7B (CONT'D)

kDOH=100nM

```
  G U
 A 4 A
  U-G
  U-G
  G-C
  A-U
  A-U
  G-U
  C-G
     U
  U-G
  A-U
 G   G
  G-C
  A-U
  g-C
  u-g
5'-gggagaugccugucgagcaugc   uagcuaaacagcuuugucgacggg-3'
```

acgu = fixed region
ACGU = random region
**ACGU** = conserved region

GROUP III
CLONE 29
SEQ ID NO:39
FIG. 7B (CONT'D)

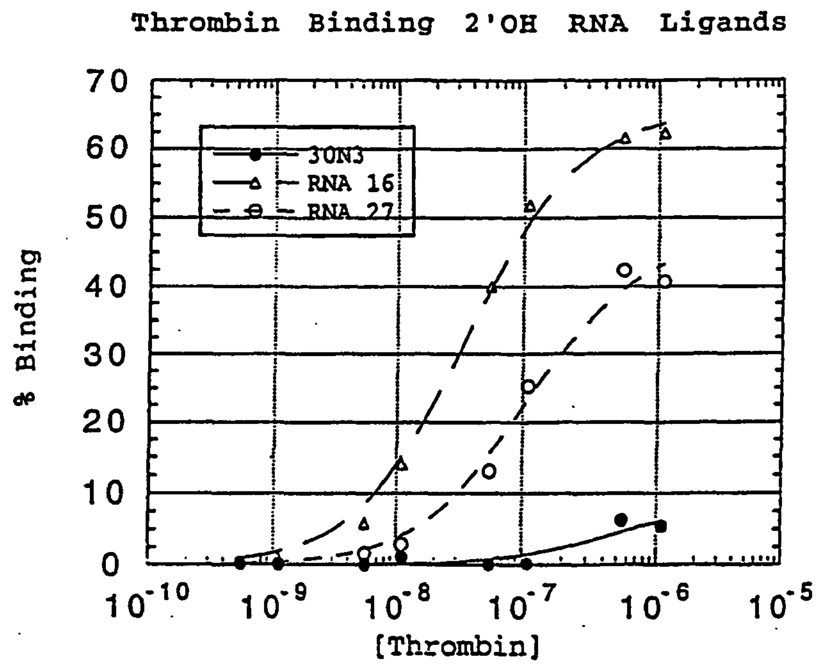
Thrombin Binding 2'OH RNA Ligands
30N3
RNA 16
RNA 27
% Binding
[Thrombin]

FIG. 8A

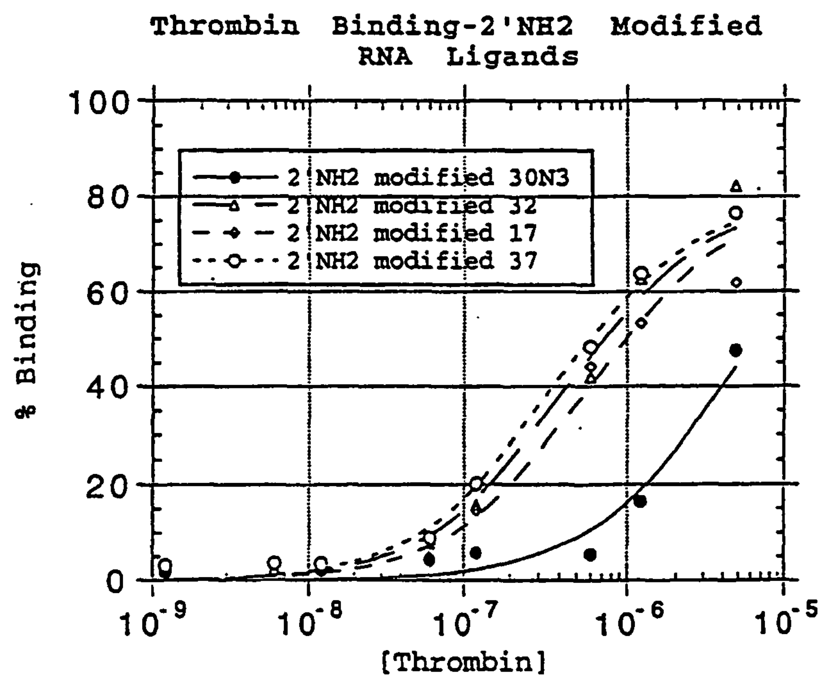
Thrombin Binding-2'NH2 Modified RNA Ligands
2'NH2 modified 30N3
2'NH2 modified 32
2'NH2 modified 17
2'NH2 modified 37
% Binding
[Thrombin]

FIG. 8B

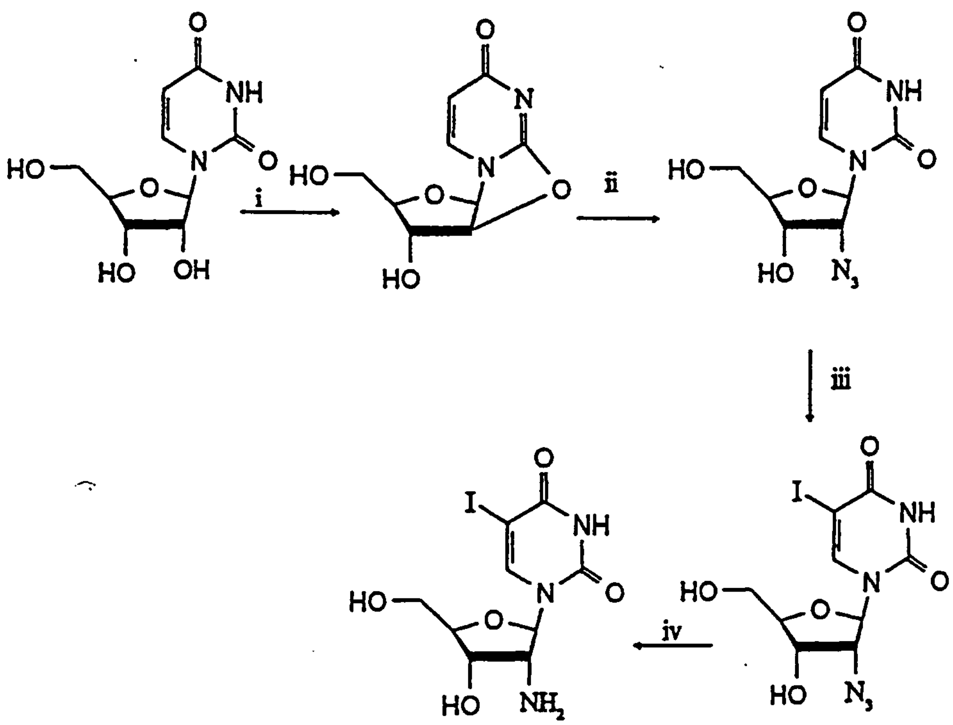


i: Diphenylcarbonate, sodium bicarbonate, DMF; ii: TMS-azide, LiF, 1:1 TMEDA/DMF; iii: ICl, methanol 50 °C, then methanolic ammonia ; iv: $Ph_3P$, dioxane r.t.

FIG. 9

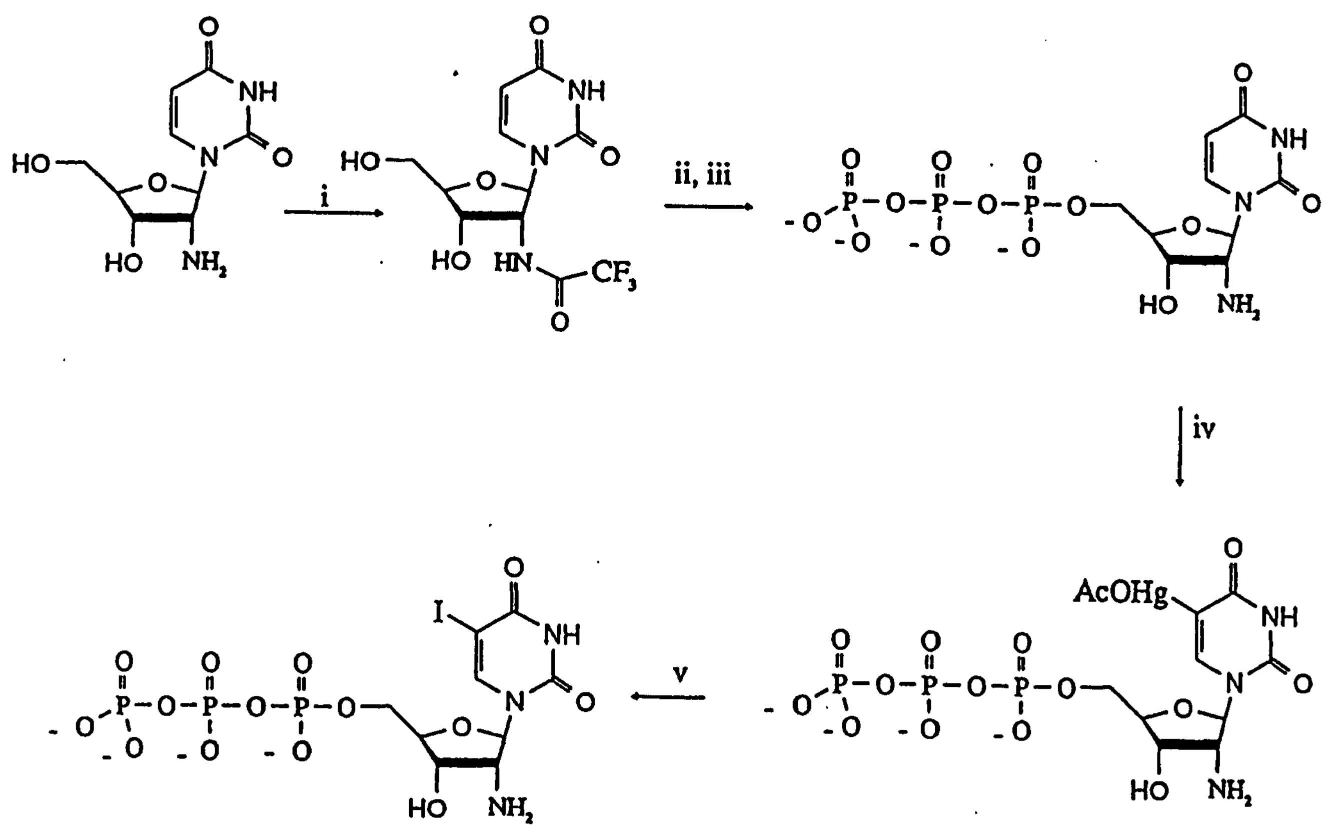


i: S-ethyl-trifluoroacetate, methanol; ii: 2-chloro-4H-1,2,3-benzodioxaphsophorin-4one, dioxane, triethylammonium pyrophosphat, then iodine, water, pyridine; iii: ammonium hydroxide; iv: mercuric acetat; v: iodine, water.

FIG. 10

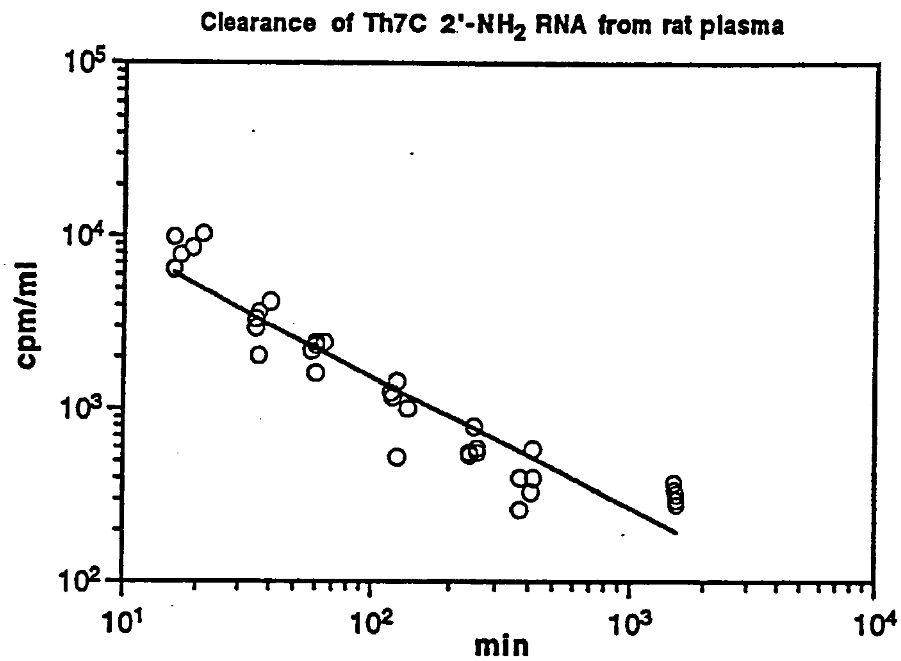

Clearance of Th7C 2'-NH2 RNA from rat plasma
cpm/ml
10^5
10^4
10^3
10^2
10^1
10^2
10^3
10^4
min


FIG. 11

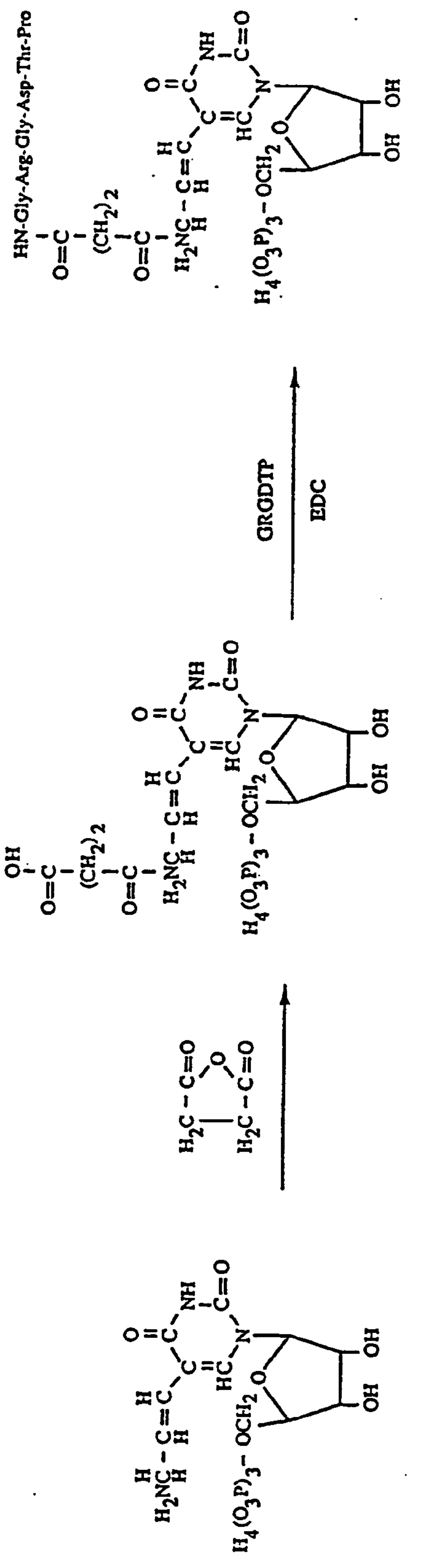


5-(3-Aminoallyl)-Uridine Triphosphate

F.W. 539.2

Succinyl-(Aminoallyl)-UTP

F.W. 639.2

RGD-UTP

F.W. 1222.6

FIG. 12

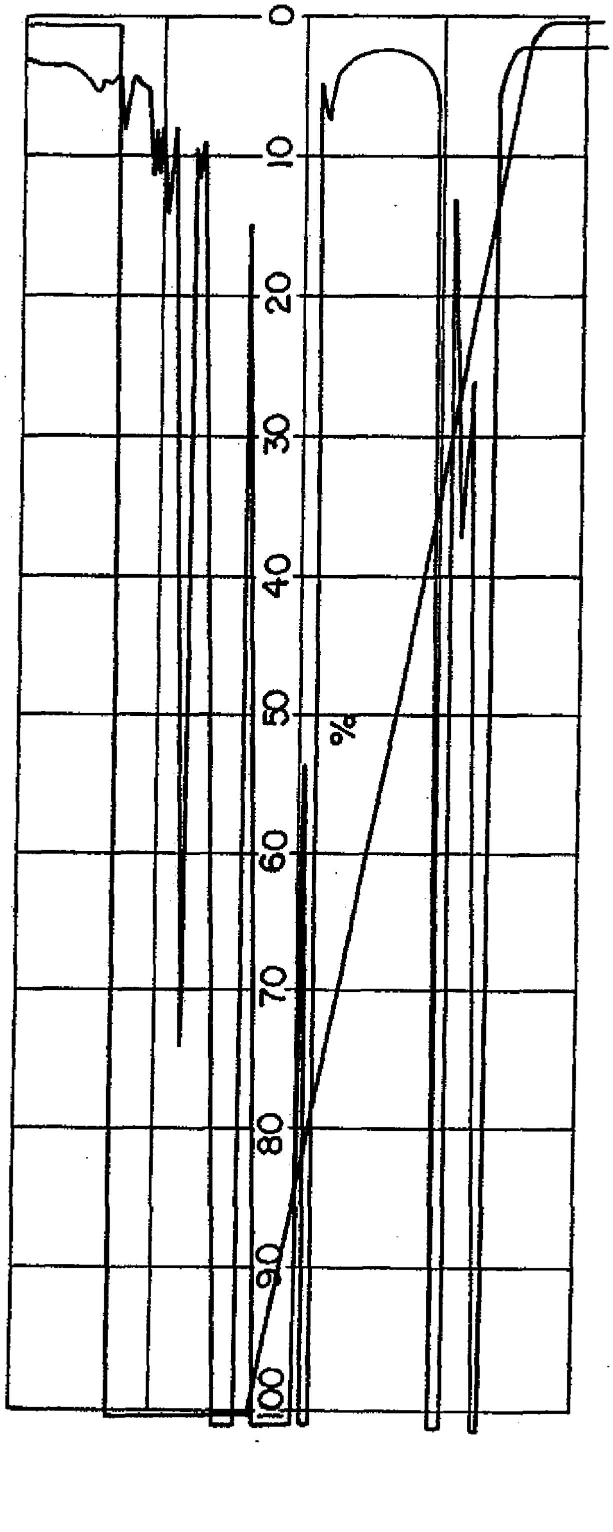


START → OD 280=2 SUCCINYLATION

FIG.13A

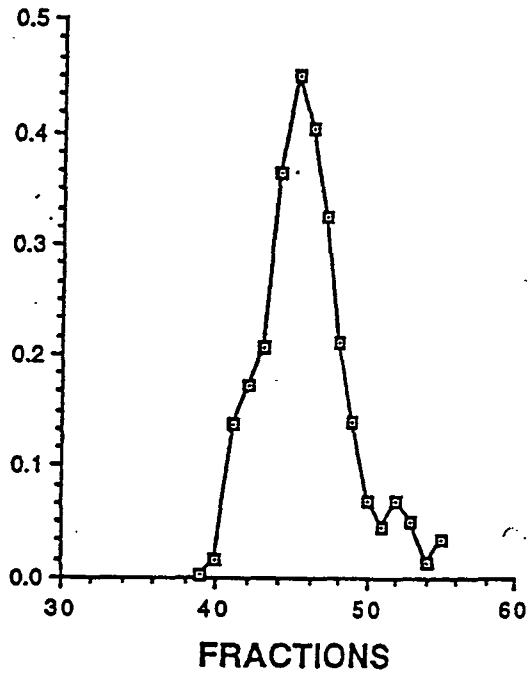


FIG. 13B

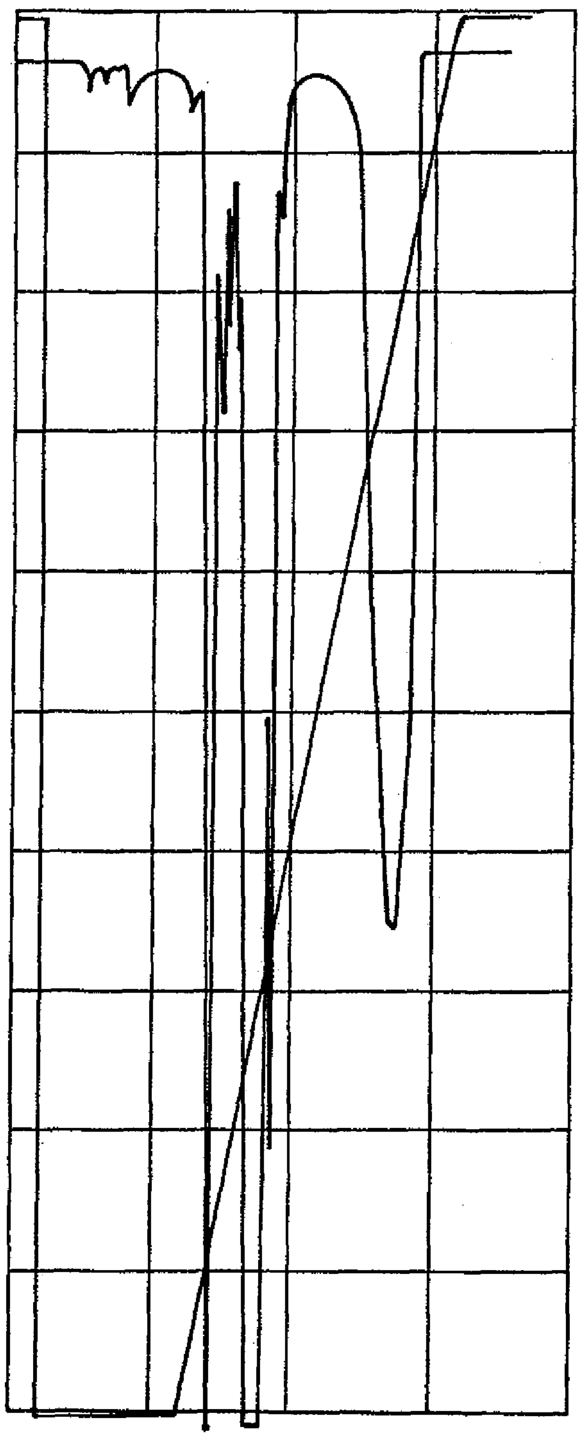

START → OD 280= 2 RGD-UTP (EDC) #2

FIG.14 A

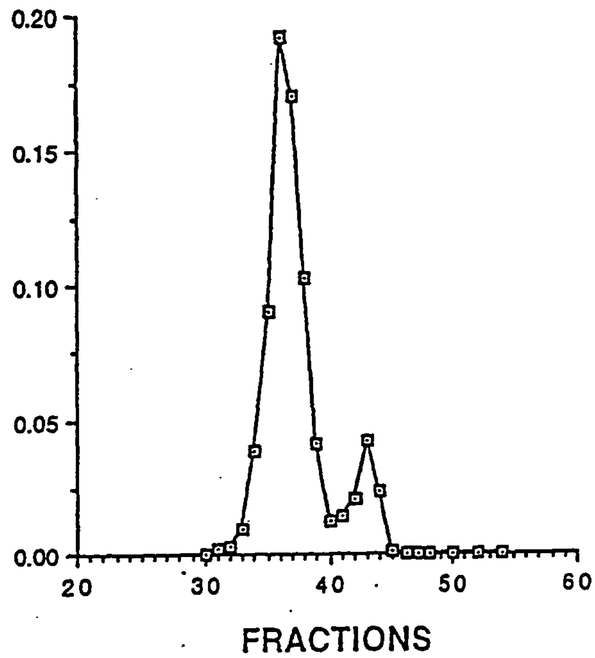


FIG. 14B

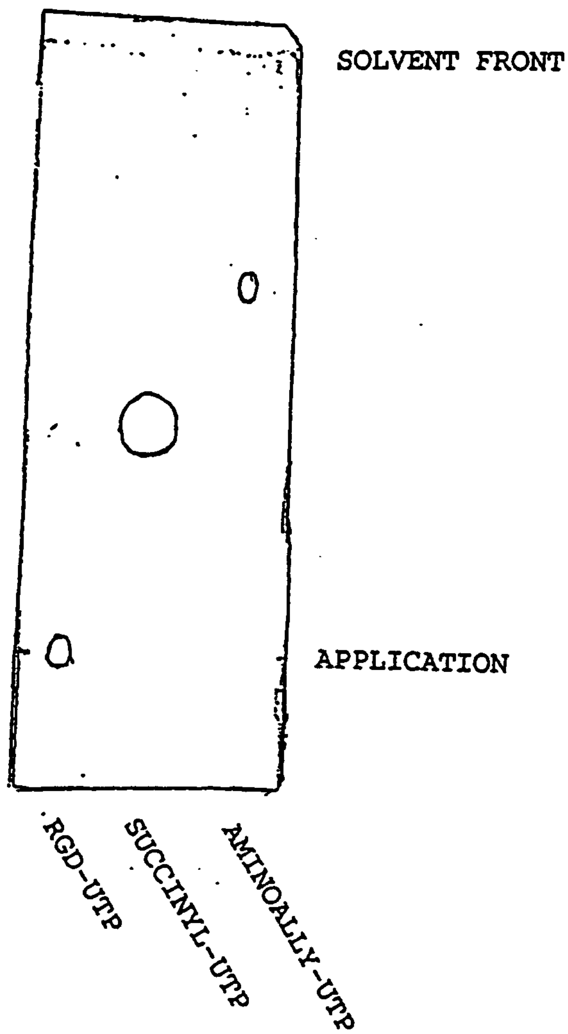
SOLVENT FRONT
APPLICATION
RGD-UTP
SUCCINYL-UTP
AMINOALLY-UTP

FIG. 15

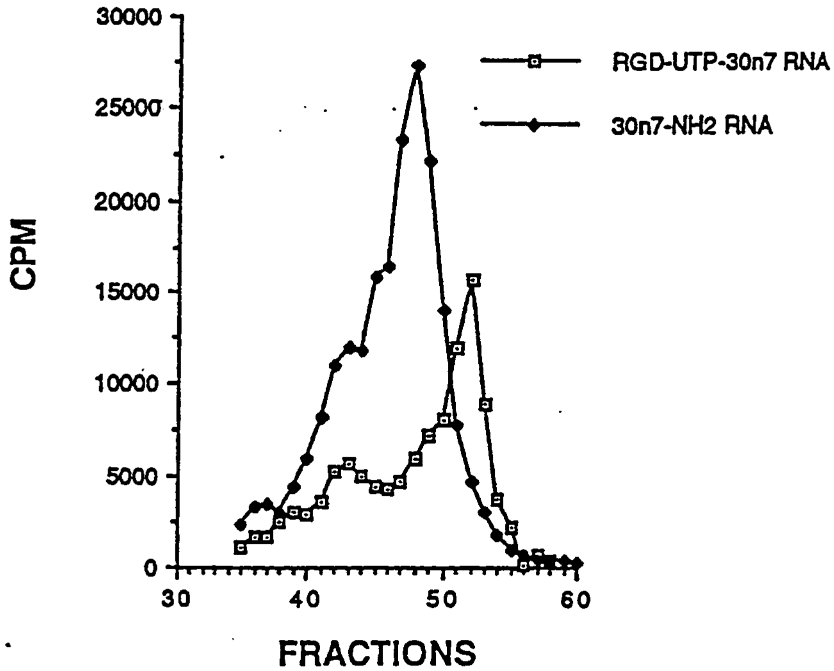

30000
25000
20000
15000
10000
5000
0
CPM
30
40
50
60
FRACTIONS
RGD-UTP-30n7 RNA
30n7-NH2 RNA


FIG. 16

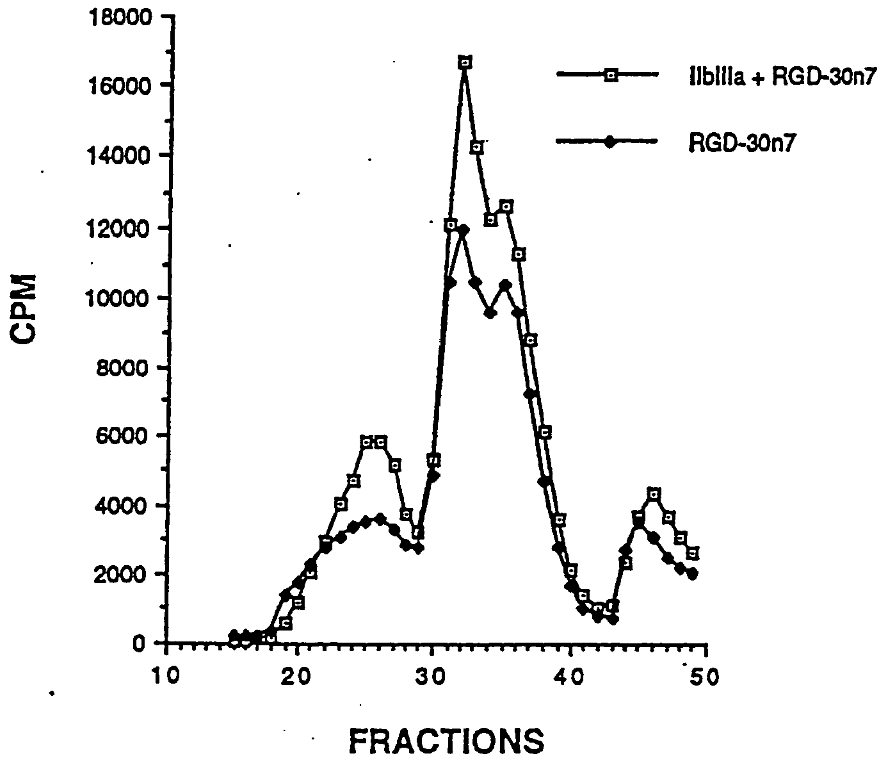
18000
16000
14000
12000
10000
8000
6000
4000
2000
0
CPM
10
20
30
40
50
FRACTIONS
IIbIIIa + RGD-30n7
RGD-30n7

FIG. 17

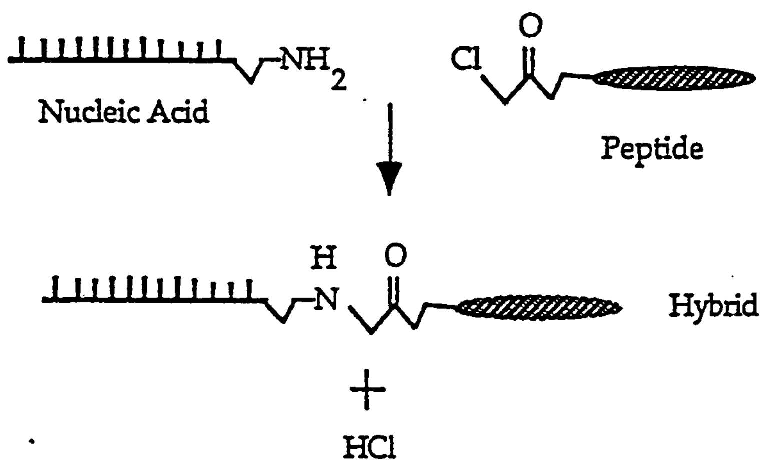


FIG. 18

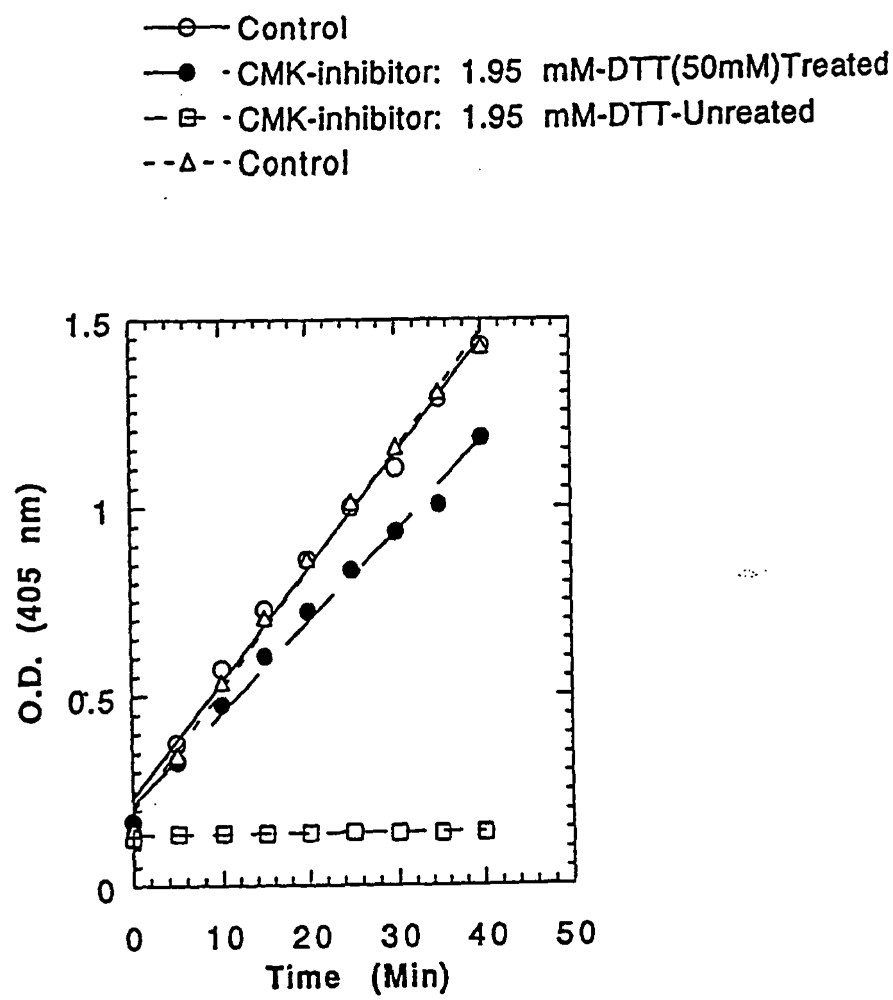
Control
CMK-inhibitor: 1.95 mM-DTT(50mM)Treated
CMK-inhibitor: 1.95 mM-DTT-Unreated
Control
1.5
1
0.5
0
0
10
20
30
40
50
O.D. (405 nm)
Time (Min)

FIG. 19

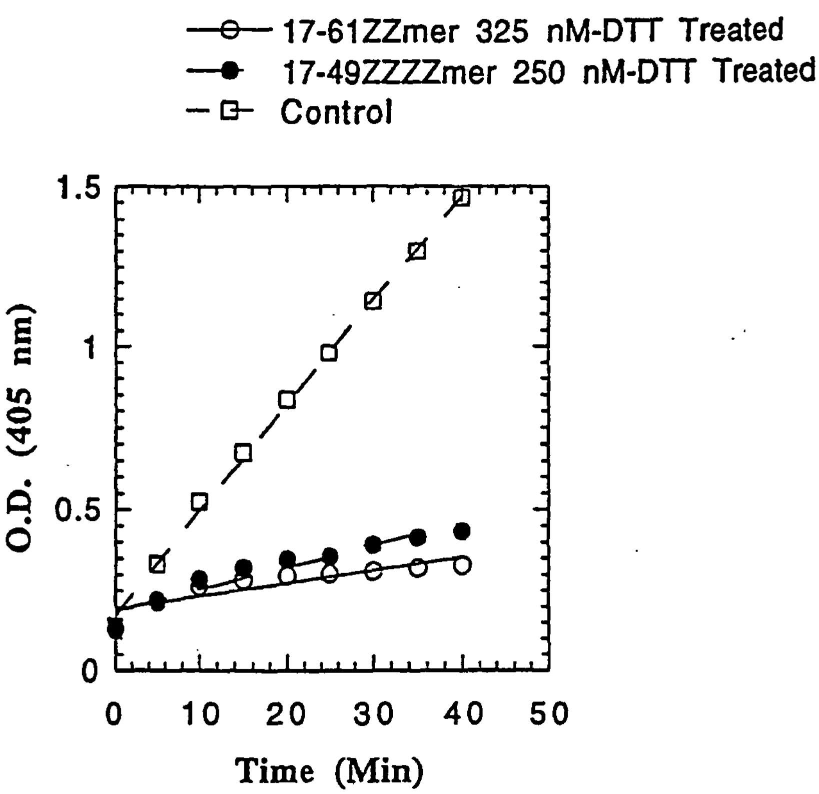

17-61ZZmer 325 nM-DTT Treated
17-49ZZZZmer 250 nM-DTT Treated
Control
O.D. (405 nm)
0
0.5
1
1.5
0
10
20
30
40
50
Time (Min)


FIG. 20

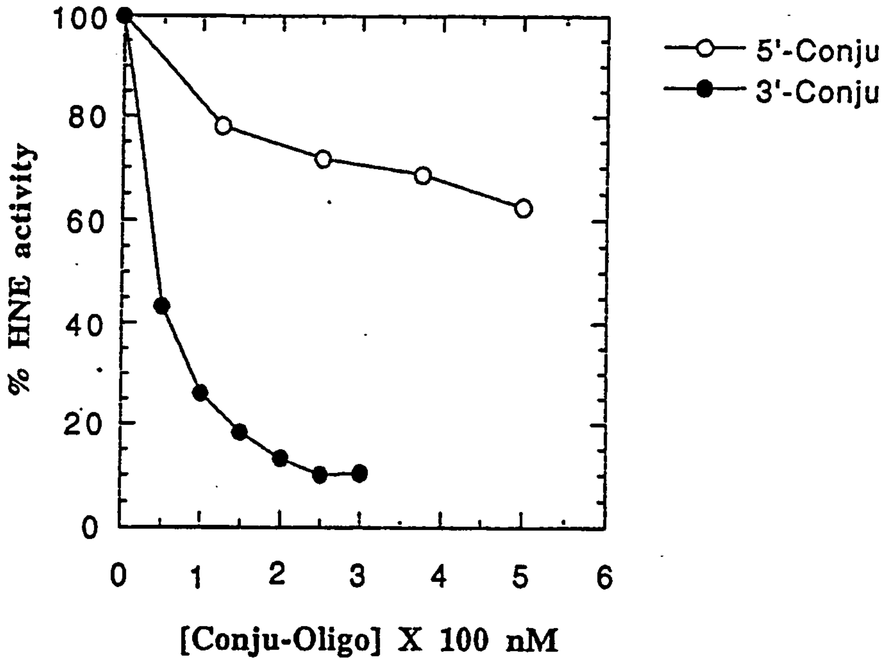
100
80
60
40
20
0
% HNE activity
0
1
2
3
4
5
6
[Conju-Oligo] X 100 nM
5'-Conju
3'-Conju

FIG. 21

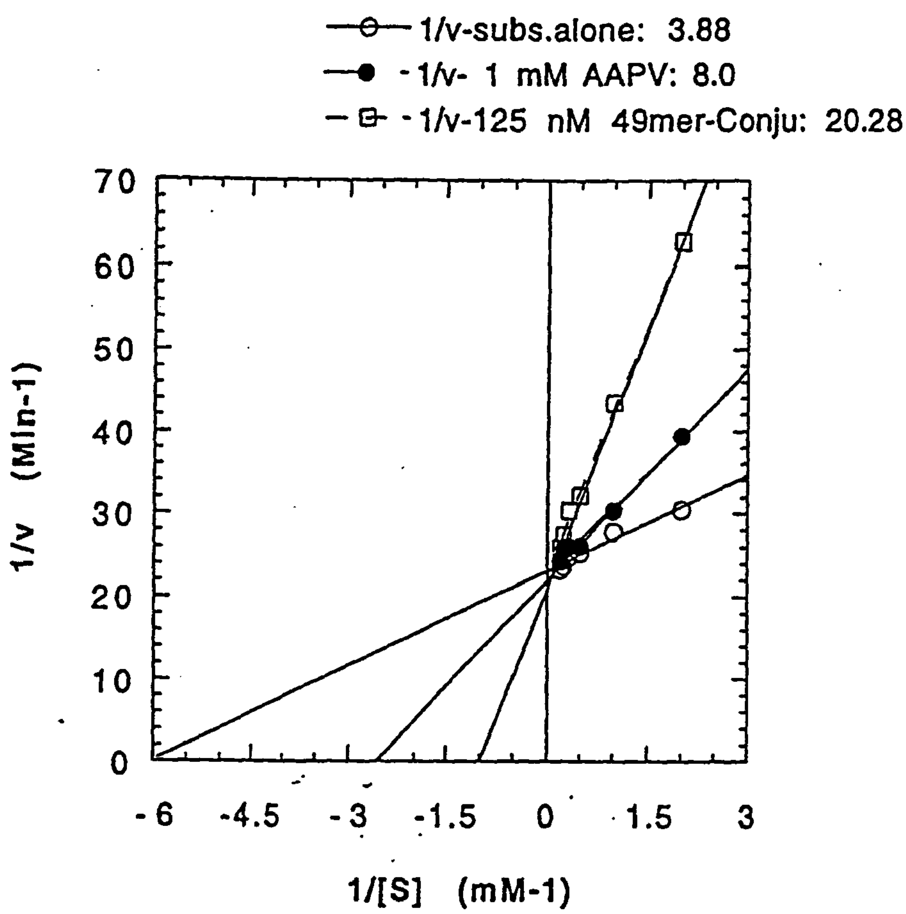
1/v-subs.alone: 3.88
1/v- 1 mM AAPV: 8.0
1/v-125 nM 49mer-Conju: 20.28
70
60
50
40
30
20
10
0
1/v (MIn-1)
-6
-4.5
-3
-1.5
0
1.5
3
1/[S] (mM-1)

FIG. 22

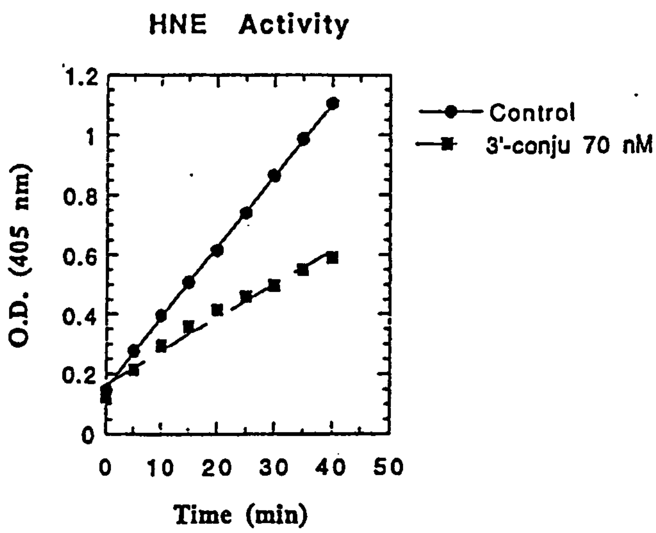


FIG. 23b

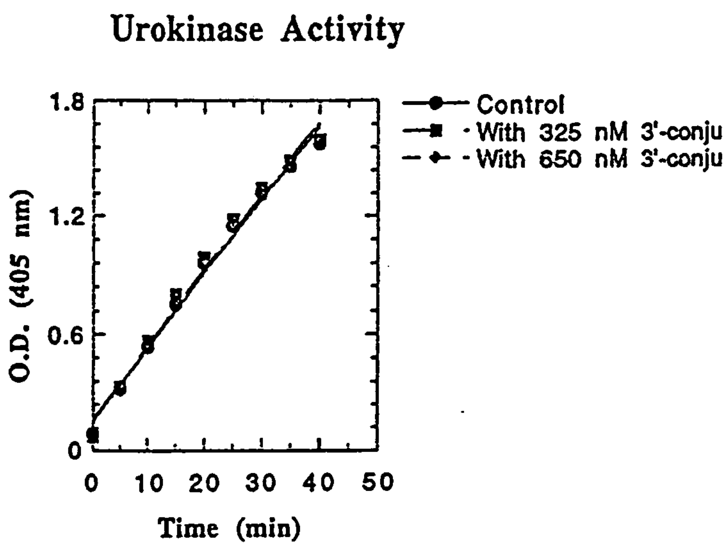


FIG. 23c

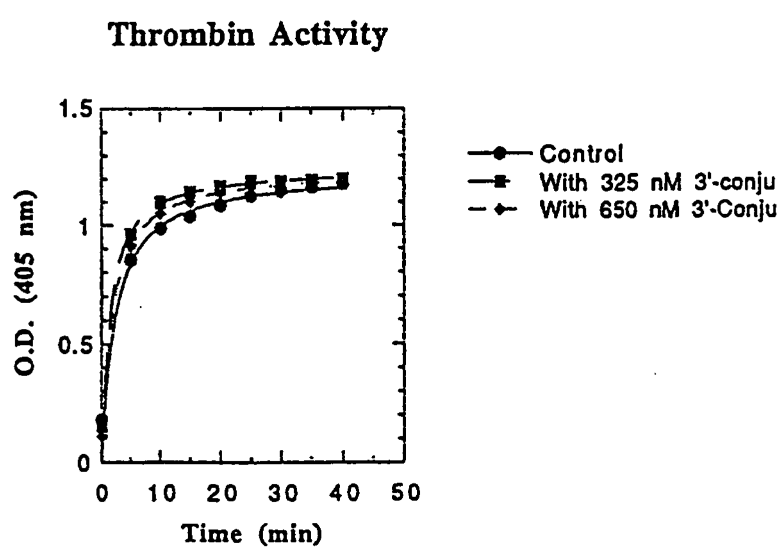

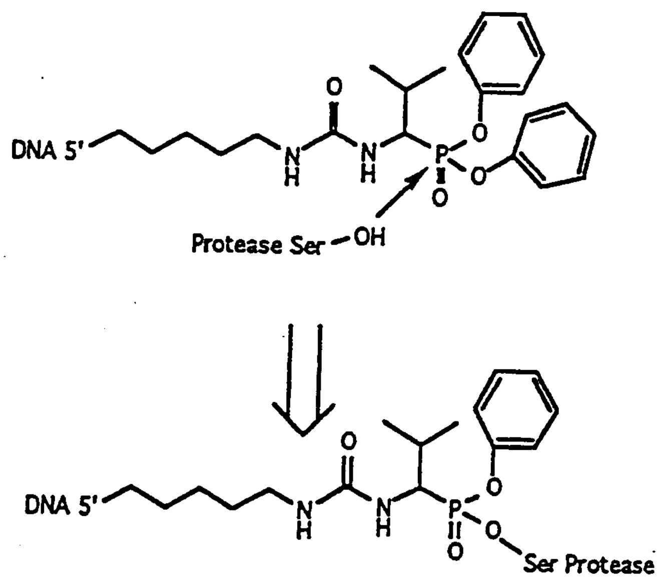


FIG. 24

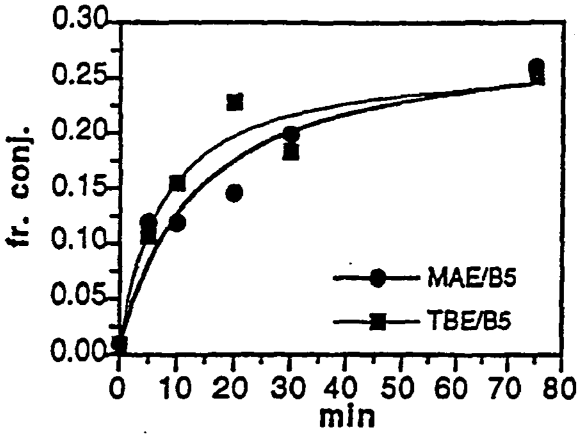
0.30
0.25
0.20
0.15
0.10
0.05
0.00
fr. conj.
0
10
20
30
40
50
60
70
80
min
MAE/B5
TBE/B5

FIG. 25

Starting RNA:

5'-GGGAGCUCAGAAUAAACGCUCAA[-30N-]UUCGACAUGAGGCCCGGAUCCGGC-3'
(SEQ ID NO:45)

PCR Primer 1:

*Hind III*
------
5'-CCGAAGCTTAATACGACTCACTATAGGGAGCTCAGAATAAACGCTCAA-3'
T7 Promoter
(SEQ ID NO:46)

PCR Primer 2:

*Bam H1*
------
5'-GCCGGATCCGGGCCTCATGTCGAA-3'
(SEQ ID NO:47)

FIG. 26

FAMILY 1

| | | SEQ ID NOS. |
|---|---|---|
| 1 | ucaaGAGUGAUGCU-CAUCCGCACUUGGUGACGUU | 48 |
| 3 (9) | caaUACCGGCAUGCAUGUC-CAUCGCUAGCGGUAuucg | 49 |
| 5 | aaUGCGUGUUGUGACGCA-CAUCCGCACGCGCAuu | 50 |
| 7 (4) | ucaaGGAGUGAUGCCCUAUCCGCACCUUGGCCCA | 51 |
| 9 | ucaaGCUUGACNGCCCAUCCGAGCUUGAUCACGC | 52 |
| 46 | aaacgcucaaUCCUUGAUGCG-GAUCCGAGGAUGGGACGUUu | 53 |
| 50 | ACACCGUCGACCUAUGAUGCG-CAUCCGCACuucgac | 54 |
| 100 | aaCCGGUAGUCGCAUGGCCCAUCGCGCCCGGuucgac | 55 |
| 107 | acgcucaaGUCAGCAUGGCCCACCGCGCUUGACGUCUG | 56 |
| 112 | CACGGUUCGAUCUGUGACGUU-CAUCCGCACuucga | 57 |
| 119 | aacgcucaaGGAGCAGUGACGCA-CAUCCACACUCCAGCGuu | 58 |

1 5 10 15 20 25 30 35 40

FAMILY 2

| | | |
|---|---|---|
| 24 (3) | UUCGAAUGCCGAGGCUC--GUGCCUUGACGGGuuc | 59 |
| 34 | UCGCGAAUGCCGACCACU---CAGGUUGAUGGGuucg | 60 |
| 102 | ucaaUGCCGGCCUGA---UCGGCUGAUGGGUUGACCG | 61 |
| 128 | GAAUGCCGAGCCCUAAGAGGCUUGAUGUGGuu | 62 |
| 27 | 5'-aaCCUUNAUGUGGCNCGAAC-<br>UGCGUGCCGAGGuu-3' | 63 |
| 44 | 5'-aaGCUUGAUGGGUGACACAC-<br>GUCAUGCCGAGCuu-3' | 64 |
| 55 | 5'-GUCGUCCUGCAUGGGCCGUAU-<br>CGGUGCGCG-3' | 65 |

1 5 10 15 20 25 30 35 40

FAMILY 3

| | | |
|---|---|---|
| 12 (7) | GCAGACGAAGGG-AACCUGCGUCUCGGCACCuucg | 66 |
| 28 | AAGGAGG-ANCCUGCGUCUCGGCACUCCGCA | 67 |
| 75 (1) | ucaaGGG-AACCUGCGUUUCGGCACCUUGUUCCGU | 68 |
| 137 | aaAUGUGGGUUACCUGCGUUUCGGCACCACGUuu | 69 |

1 5 10 15 20 25 30 35 40

FIG. 27

FAMILY 4

```
                                                              SEQ ID
                                                              NOS.
6               CGACGGUAGAGUCUGUCCCGUCAUCCCCCA                 70
35          AAAGACCCCUGGUUGAGUCUGUCCCAGCCGuu                   71
40       GACCCAUCGUCAACGGUUGAGUCUGUCCCGuucgacaugagg            72
56                gcucaaGGUUGAGUCUGUCCCUUCGAGUAUCUGAUC         73
90          UCGGACAGUUGGUUGAGUCUGUCCCAACUUuu                   74
106       GACCAUGUGACUGGUUGAGCCUGUCCCAGuu                      75
138               AACGGUUGAGUCUGUCCCGUAAGAGAGCGC               76

         |...|....|....|....|....|....|....|....|
         1   5   10   15   20   25   30   35   40
```

FAMILY 5

```
15       UCGGAAUGUAGUUGACGUAUCCUUGU--CCGAuucgacau              77
20         aGGGUGUAGUUGGGACCUA--GUCCGCCGUACCuu                 78
21          GGCAUAGUUGGGACCUC--GUCCGCCGUGCCC                   79
84         gcucaaUAGUUGGAGGCCUGUCCUCGCCGUAGAGCG                80

         |...|....|....|....|....|....|....|....|
         1   5   10   15   20   25   30   35   40
```

FAMILY 6

```
25       aGGGGUUCUA-GUGGAGACUCUGCCGCGGCCCuu                    81
126 (2) aACGGUUCUGUGUGUGGACUA-GCCGCGGCCGuu                    82

         |...|....|....|....|....|....|...
         1   5   10   15   20   25   30
```

FIG. 27 (CONT'D)

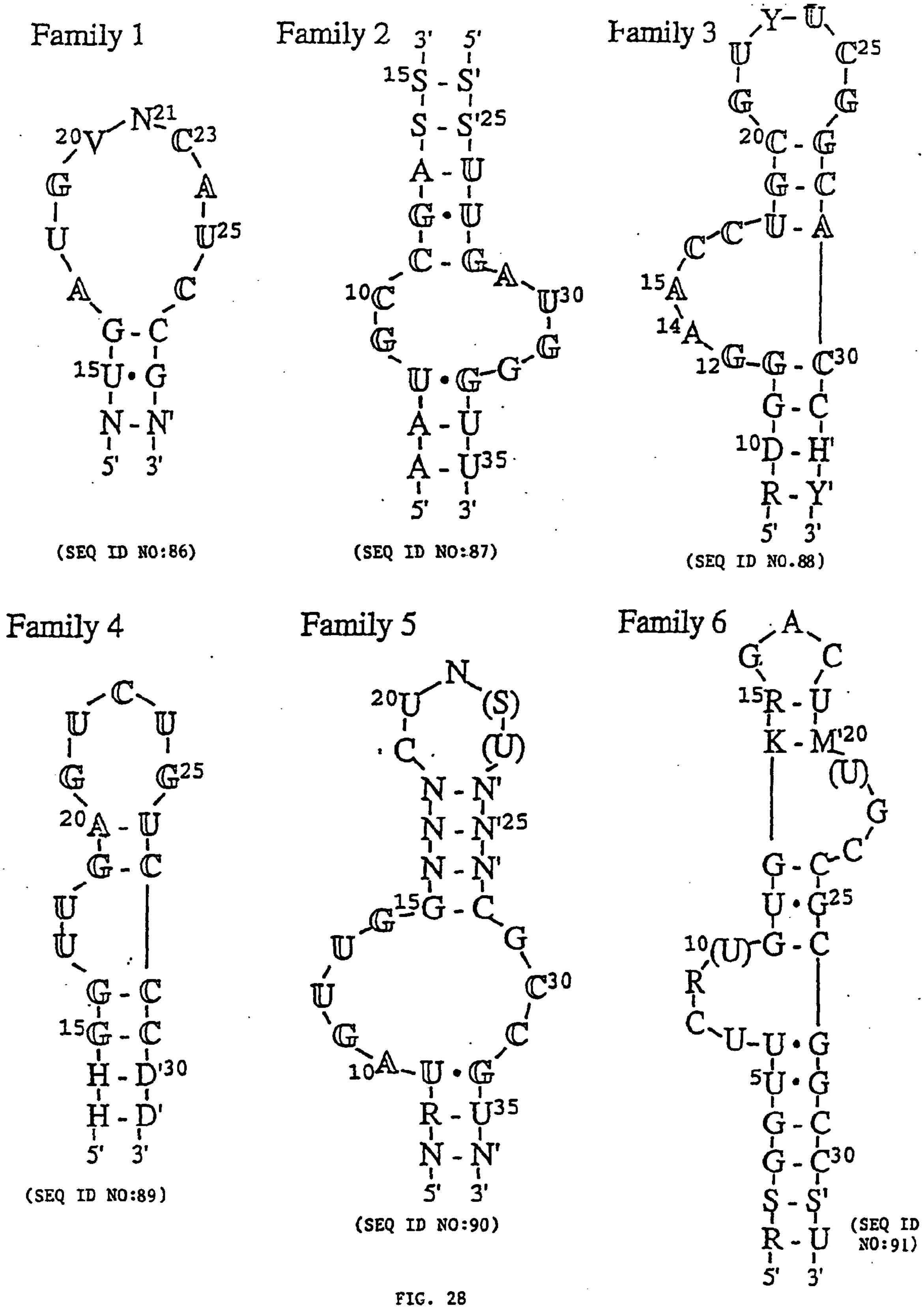
Family 1
(SEQ ID NO:86)
Family 2
(SEQ ID NO:87)
Family 3
(SEQ ID NO.88)
Family 4
(SEQ ID NO:89)
Family 5
(SEQ ID NO:90)
Family 6
(SEQ ID NO:91)

FIG. 28

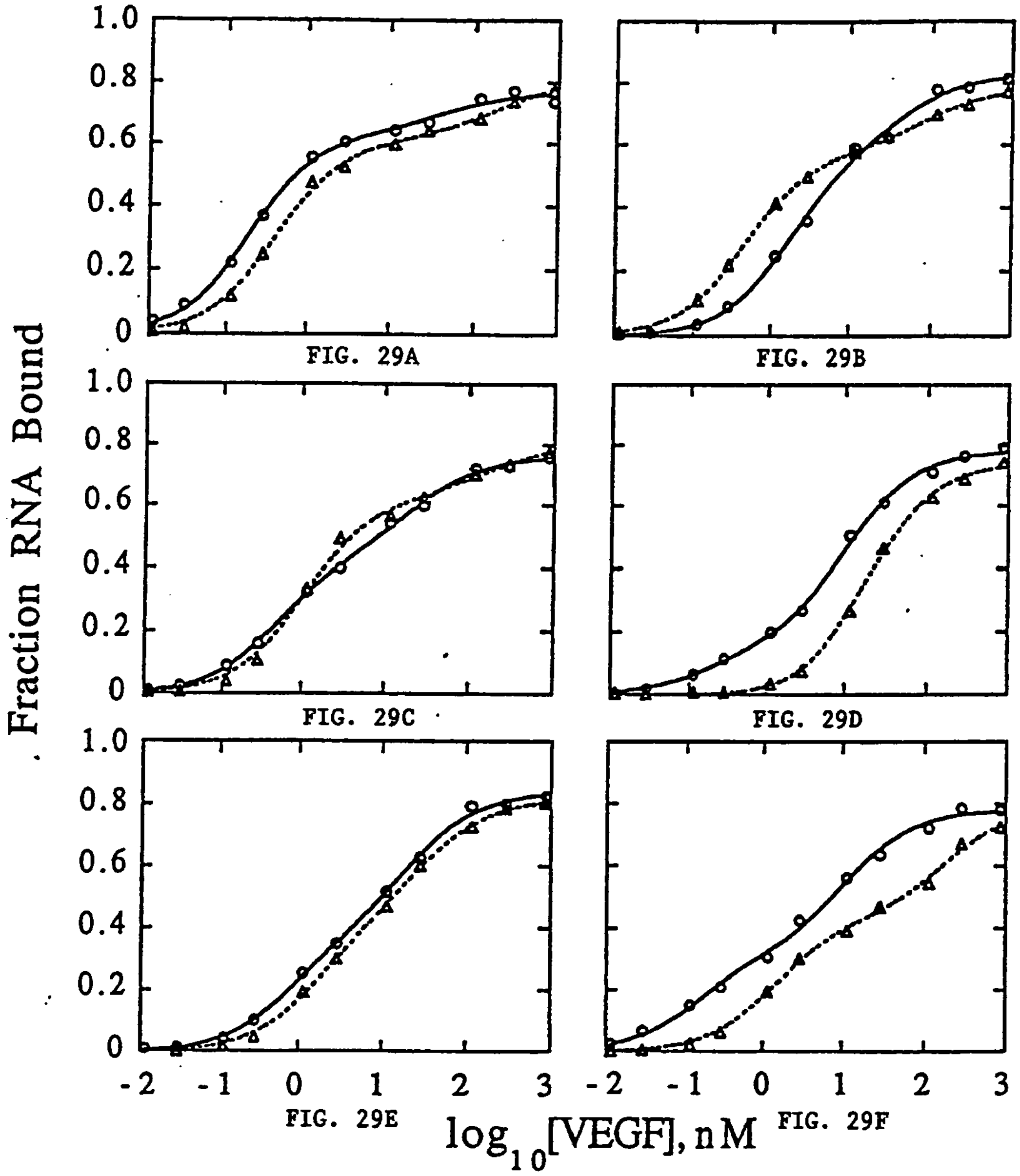


FIG. 29A FIG. 29B FIG. 29C FIG. 29D FIG. 29E FIG. 29F

FIG. 30A

FIG. 30B

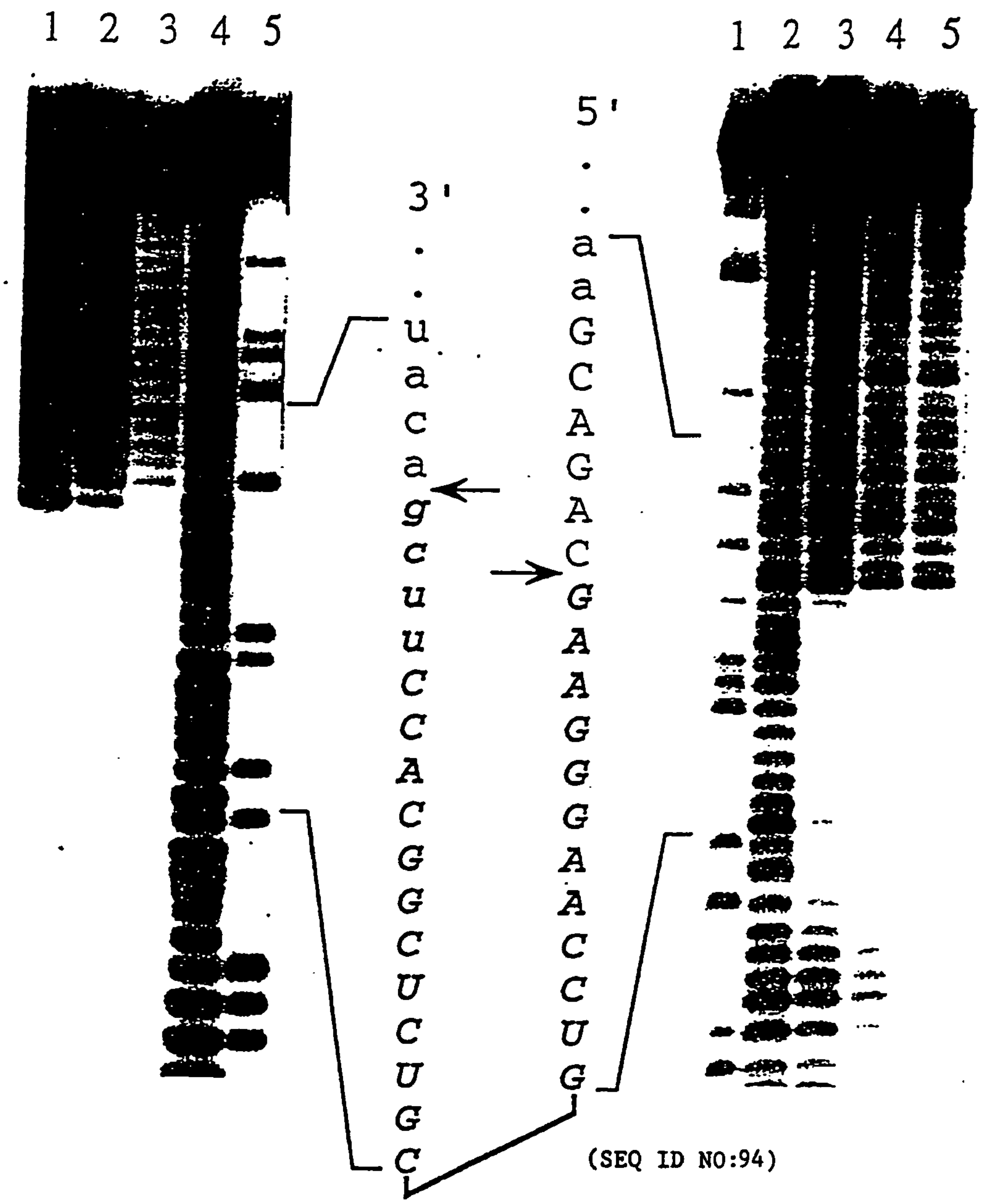

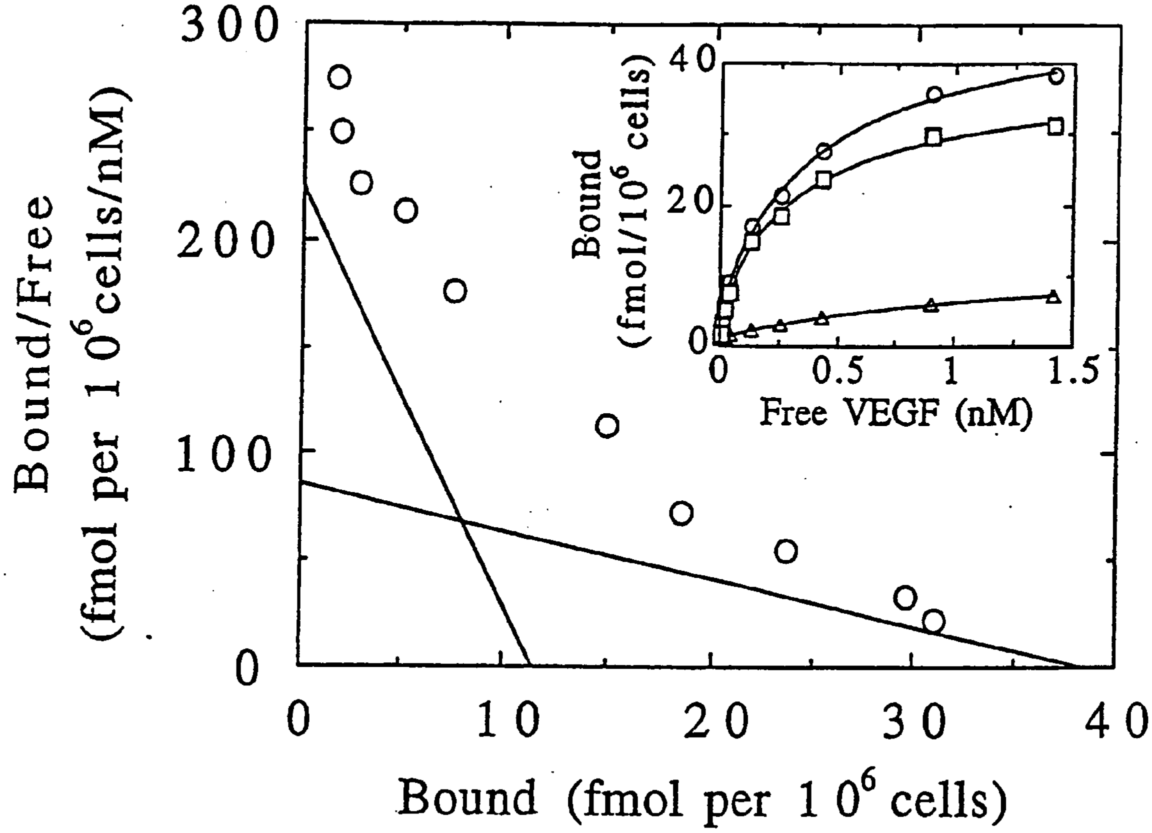
300
200
100
0
Bound/Free
(fmol per 1 0$^6$cells/nM)
0
10
20
30
40
Bound (fmol per 1 0$^6$ cells)
40
20
0
Bound
(fmol/10$^6$ cells)
0
0.5
1
1.5
Free VEGF (nM)

FIG. 31

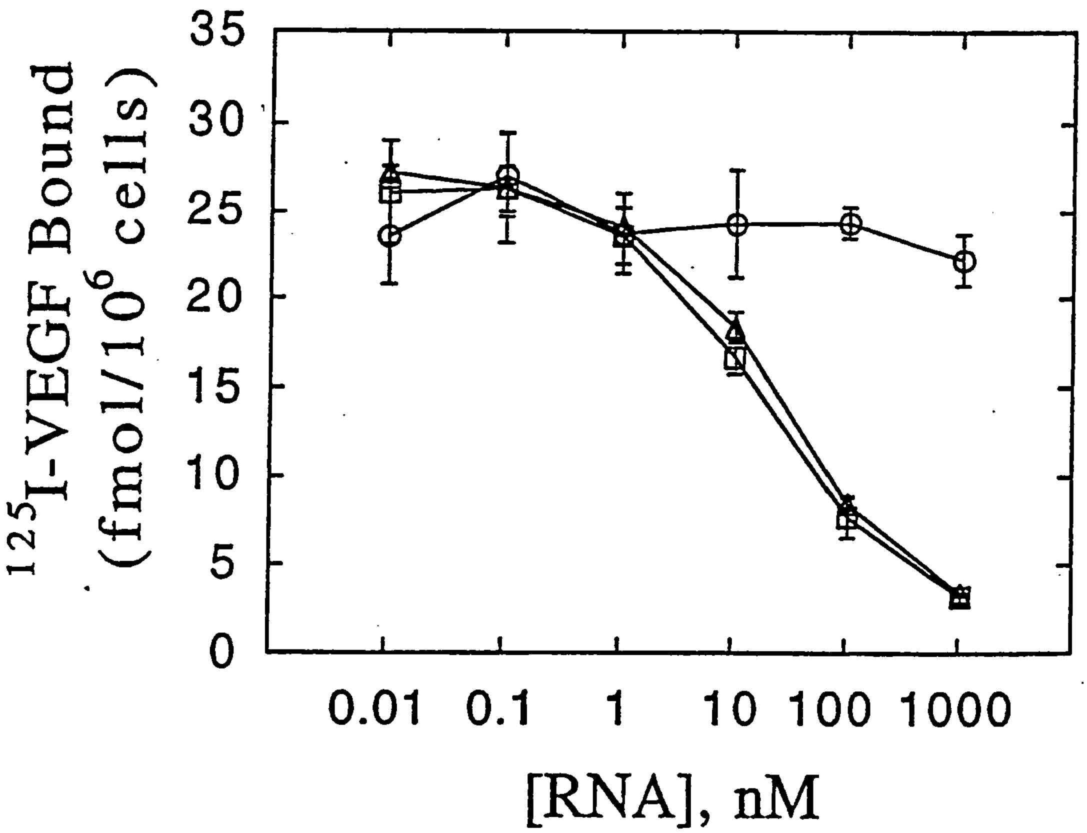

35
30
25
20
15
10
5
0
$^{125}$I-VEGF Bound
(fmol/$10^6$ cells)
0.01
0.1
1
10
100
1000
[RNA], nM


FIG. 32

SELEX Experiment A

Starting RNA:

5'-GGGAGACAAGAAUAACGCUCAA[-30N-
]UUCGACAGGAGGCUCACAACAGGC-3' (SEQ ID NO:101)

PCR Primer 1:

5'-TAATACGACTCACTATAGGGAGACAAGAAUAACGCUCAA-3'
T7 Promoter (SEQ ID NO:102)

PCR Primer 2:

5'-GCCTGTTGTGAGCCTCCTGTCGAA-3' (SEQ ID NO:103)

SELEX Experiment B:

Starting RNA:

5'-GGGAGGACGAUGCGG[-50N-]CAGACGACTCGCCCGA-3'
(SEQ ID NO:104)

PCR Primer 1:

5'-TAATACGACTCACTATAGGGAGGACGAUGCGG-3'
T7 Promoter (SEQ ID NO:105)

PCR Primer 2:

5'-TCGGGCGAGTCGTCTG-3'
(SEQ ID NO:106)

FIG. 33

| FAMILY 1 | | SEQ ID NOS. |
|---|---|---|
| 14B | UGGCUGUGAUCAAUGCGGGGAGGUGAGGAAGGCCUUACAAAUCCUUCGG | 107 |
| 16B | UGUGAUCAAUGCGGUGGCGGGGUAUGGAUGGGAGUCUGGAAUGCUGCGCU | 108 |
| 17B | CGCUGUGUUCAAUGCGGGGAUCGGGCCGGAGGAUGAACAAAUGGCGGGU | 109 |
| 25B | UGUUGAGCAAGCACUCAUGUGGUCAAUGUGGAGUGGGAGCUGGUGGGGU | 110 |
| 28B | CAAGGGAGCGUUAGAGCCAUGUGGUCAAUGAGGGGUGGGAUUGGUUGGGU | 111 |
| 30B | CAUGGUUGUGAACUGUUGUGAUCAAUGCGGGGAGGGUAAUGGUGGGUGGU | 112 |
| 33B | AUGAGUGACACAUGUGCUCAAUGCGGGGUGGGUUGGUAGGGGUAGCACGG | 113 |
| 34B | UGUGGUCAAUGUGGGGUAGGGCUGGUAGGGCAUUCCGUACUGGUGUGGU | 114 |
| 47B | CCGAGUUGUGCUCAAUGUGGGGUCUGGGUACGGACGGGAACAGAUCUGG | 115 |
| 46B | GUGCUCAGCAUUGUGUGCUCAAUGCGGGGGAGUUUGGGUUGGCGACGG | 116 |
| CONSENSUS: | UGUGNUCAAUGNGGGG | 117 |

| FAMILY 2 | | |
|---|---|---|
| 23B | CAUAGGCUUACAACAGAGCGGGGGUUCUGAUGGUAGACGCCGGGACGCCC | 118 |
| 51B | UAUGAUGGUAGACGCCGUACCGCAUCAGGCCAAGUCGUCACAGAUCGUG | 119 |
| 14A | GCAACAGAGGCUGAUGGUAGACGCCGGCC-A | 120 |
| 17A | AGAGUCGCUGAUGGUAGACGCCGGCG-GAUC | 121 |
| 23A | GAGGCUGAUGGCAGACGC-GGCC-GAAGACA | 122 |
| 24A | CCCUGAUGGUAGACGCCGGGGUGCCGGAA | 123 |
| CONSENSUS: | CUGAUGGUAGACGCCGG | 124 |

| FAMILY 3 | | |
|---|---|---|
| 7B | CAGUGCUGAACUAAUCGAACGGGGUCAAGGAGGGUCGAACGAGAUCUGCCG | 125 |
| 26B | CACCUUCGUGGGGUCAAGGAGGGUCGCGAGGCCGCAGGAUCAACCGUGUG | 126 |
| 54B | GGUCAAGUUGGGUCGAGGAAGCGCUCCCGAGUAUCGUAGUGUGCGACUGC | 127 |
| 8A | GAACUUGAUCGGGGUCAAGGCGGGACGAA | 128 |
| 15A | UGGCGGGACCAAGGAGGGACGUGUAGGAAA | 129 |
| 16A | AAAAUGCACAAAUCGGGGUCAAGGAGGGACGA | 130 |
| 45B | AUGGGUUCGUGUGGUGAAUGGAGGAGGUGGGCUCGCAUGCUACUGUG | 131 |
| CONSENSUS: | GGUCAAGGNGGG | 132 |

FIG. 34

| FAMILY 4 | | SEQ ID NOS. |
|---|---|---|
| 43B | UGCACUAAGUCCGGGUAGUGGGAGUGGUUGGGCCUGGAGUGCGC | 133 |
| 2A | AUCAAAGGGUAGAGGGUGGGCUGUGGCAAG | 134 |
| 9A | AAUCGAGGGUAGCGGGCGCGGCUUGGCCAA | 135 |
| 60A | GCCUCGGAUCGGGCAGCGGGUGGGAUGGCAA | 136 |
| 41A | AACGGAGUGGUAGGCGUUGGGUGCCAGGAA | 137 |
| CONSENSUS: | GGUAGNGGGNG | 138 |
| **FAMILY 5** | | |
| 44B | AAC---CG-AGUCGUGUGGGUUGGGGCUCCAGUACAUCCCCGGUCUGGGUGU | 139 |
| 50B | UAACAUACGCAGUCGUGUGGGUAGGGGAU-CACAAACUGCG----UAUCGUGU | 140 |
| CONSENSUS: | AGUCGUGUGGGU-GGGG-U--CA | 141 |
| **OTHER SEQUENCES** | | |
| 12A | AGUGUAGGAUAGGGGAUGGGAGGUCCGGGA | 142 |
| 20A | ACUGUGGGCUCUAGGGCAGUGGGAGUGGAG | 143 |
| 48A | AGUGGGACAGGGAUUGCGGAGGGUGGAAGG | 144 |
| 11A | GUCAGGAGGACUGGAAGGUGGGACUGGUGA | 145 |
| 54A | GCAGGAGAGAGGGUGUUGGGUGCGGAUACA | 146 |
| 8B | AGGGUAGGAGGCUAAGCAUAGUUCAGAGGAGGUGGCGCGUGCCCCCGUG | 147 |
| 32B | CAACAUUGGCACCAAUGCUCUGUGUUAAUGUGGGGUGGGAACGGCGCCG | 148 |
| 22B | ACCAAUGAUUGCAAUGAGGGCAGUGGGGGGAAUUGGGCUCGUGUGGU | 149 |
| 12B | GCAGUGGGUGAGGUCCGGGCACGAUUGAGUUUGAACGGUUCUGGCUUGGU | 150 |
| 53B | GUGGUAGGUGUAGAGUGGAUGGCGGAGGUCCUAGUAGUUCUGUGCCUGGU | 151 |
| 13B | CGCGGGAGAGGGUAGUGGGUGUGGUGCUUGGACAAGCAGCG | 152 |
| 1B | ACCCGCAUACGGACCGCGGAGGGGGAAAUCUAGCCUCAGGGGUGGCGGGC | 153 |
| 5B | UGAAGAAGCGGGGACUGCACGACGGGAUGGAGGGACACGACUGCGGGGU | 154 |

FIG. 34 CONT'D

**NITROCELLULOSE FILTER BINDERS:**

| | | SEQ ID NOS. |
|---|---|---|
| 22A | ACACCAGGAGAGUGGGUUCGGGUGAGGACG | 155 |
| 33A | GUGGCUGAUGGCAGACGCCGGCUGCUGACG | 156 |
| 34A | UCGUGCCAGGACAUGGUGGCUCAUGGGUAA | 157 |
| 30A | AGGUACGGGGGAGGGAAGGAUAUAACGCGA | 158 |
| 32A | UGGAAAGGUGUGGAAAGAGGCAUCGGGGGG | 159 |
| 38A | UCAAUGGGCAGGAAGAGGGAAGGGAUGUGA | 160 |
| 45A | CAUGGGUAAGGGAGUGGGAGUGGUGAAUAG | 161 |
| 46A | GGAACGAGUAGGGCAGUGGGUGGUGAUGGC | 162 |
| 49A | UAGGGCAGAGGGAGUGGUUAGGGCUGUGAU | 163 |
| 55A | GGGUAGUGGGAAGGGUAAGGGCCGAGGUGG | 164 |
| 19A | AAUACACACCGCGGGAAGGGAGGGUGGAAA | 165 |
| 59A | AGACUACAGCGCGGGUUAGGGUUGAGGGAA | 166 |
| 61A | UACGAGCAAGCGGGCGAAGGGUUGAGGGAA | 167 |
| 40A | CAAGGUGGUGGAGGAGGAUACGAUCUGCAG | 168 |
| 18A | GGAGGGAAGGAGGGCAGGUGAUGGGUCAG | 169 |
| 42A | UGAUGGCGGUAGUGGAGGUAAUGAGCGUGA | 170 |
| 1A | GCAACUGGGGGCGGGUGGUGUGAGGA | 171 |
| 4A | GGAGGGGCCUAUAGGGGUGGUGGUGUACGA | 172 |
| 36A | UAUAGGGUAGUGGGUGUAGGUAGGGCGACA | 173 |
| 21A | GAGGGUUGGAGGGCAUGGGGCAGGAACCGG | 174 |
| 44A | CGUAGAACUGGCGGGCAGUGGGGGGGAUGC | 175 |
| 13A | UGAGGGGACGAGGGAUGUGGGGAGCGGGAC | 176 |
| 25A | CGAGGGAUGGGAGGCGUGUGGAAGAUGCAA | 177 |
| 29A | GCAUCCGGGGACAAGAUGGGUCGGUAAGGU | 178 |
| 47A | GUGUGCGGGGUCAAGACGGGUGGCGUGCG | 179 |
| 51A | UCAAACCAUGGGGCGGGUGGUACGAGGAAC | 180 |
| 58A | CGAGUCCGAGGGAUGGGUGGUGUGCGGCAA | 181 |
| 10A | CAGUGUCGGAGAGGAGGAUGGAGGUAUGAA | 182 |
| 2B | CACCACUACGCGGGAAGGGUAGGGUGGAUUACAAGGUGUGACCGCUCCGU | 183 |
| 21B | UACGGUUAACGGGGGUGGUGUGGGAGGACACAAAGCGCGUACCUGCCCC | 184 |
| 52B | AGGUCCUCGAGGGUCUGGGUGUGGGAGUGGCAUGGACCAAUACCGCGUG | 185 |
| 27B | AAACCCAUCCUGCGCGGGAUGGGAGGGUGGAAACACUAGAGCUUCGCCUG | 186 |
| 35B | AACUGGUGGUCACGCGUUGAGGUGGUGGAGGUGGAUUCAACGGUCGAGGG | 187 |
| 38B | CAUGAAAGUAGGGUUAUGAAGGCGGUAGAUGGAGGAGGUUGGGUUGCCGC | 188 |
| 10B | GUCUAUUGGGUAGGUGUUUGCAAGAAUUCCGCACGAUAGGUAAAACAGUG | 189 |
| 19B | UGUAGGGGAAGUACGAGAGUGGGAGCGGCCGUAUAGGUGGGAGUGUGCU | 190 |

FIG. 34 CONT'D

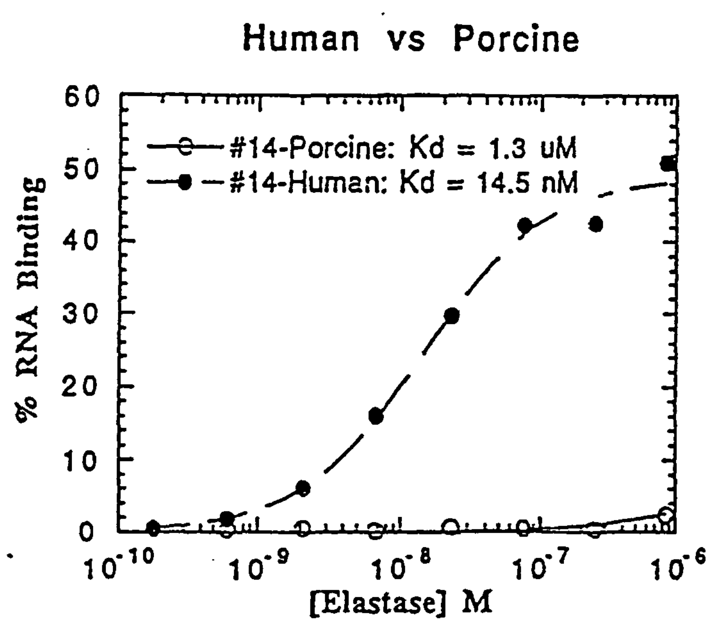
Human vs Porcine
#14-Porcine: Kd = 1.3 uM
#14-Human: Kd = 14.5 nM
% RNA Binding
0
10
20
30
40
50
60
$10^{-10}$
$10^{-9}$
$10^{-8}$
$10^{-7}$
$10^{-6}$
[Elastase] M

FIG. 35

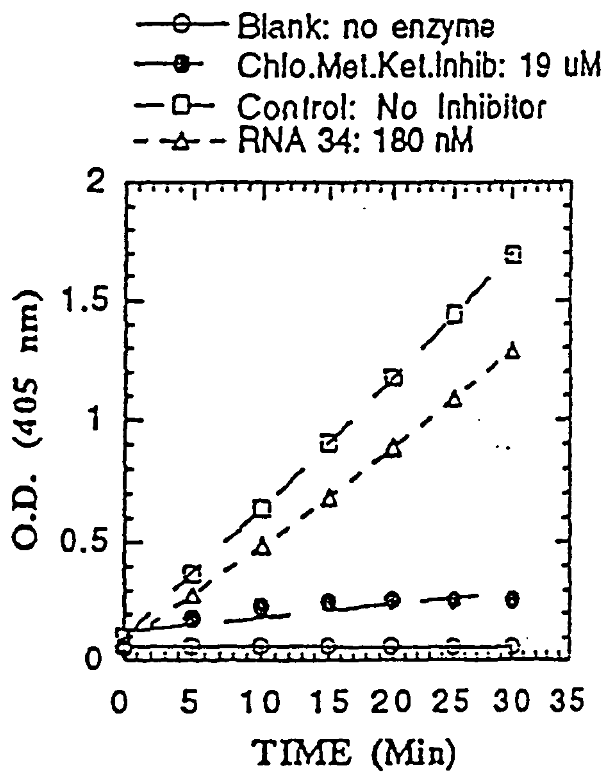
Blank: no enzyme
Chlo.Met.Ket.Inhib: 19 uM
Control: No Inhibitor
RNA 34: 180 nM
O.D. (405 nm)
0
0.5
1
1.5
2
0
5
10
15
20
25
30
35
TIME (Min)

FIG. 36

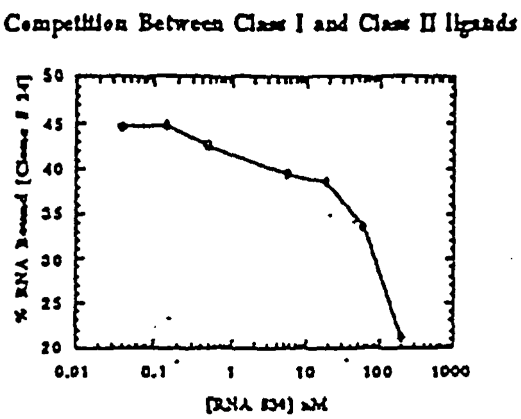


FIG. 37A

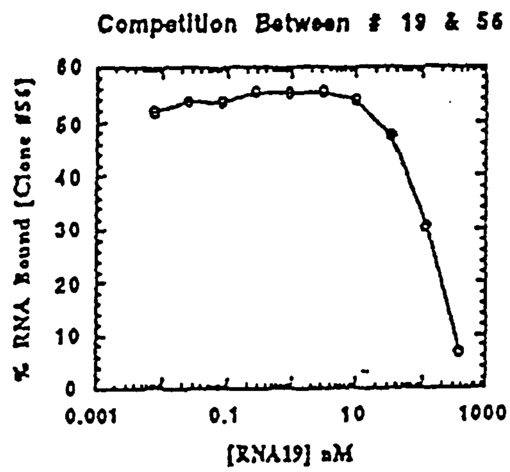


FIG. 37B

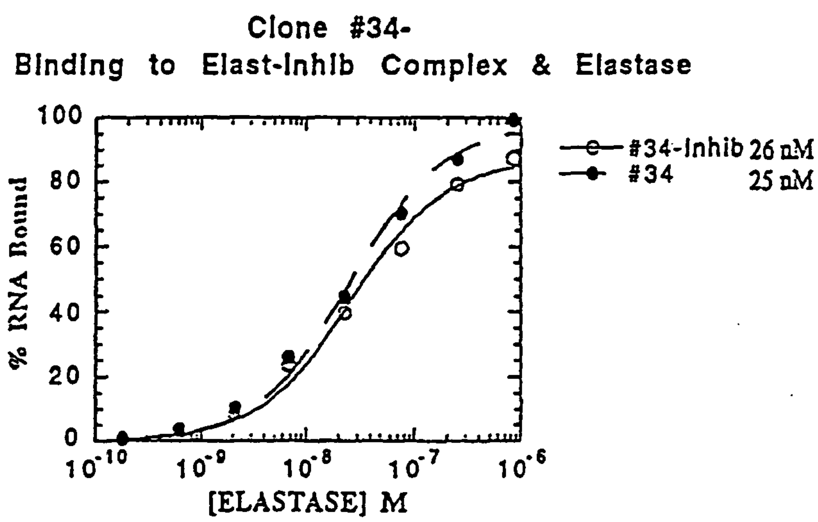
Clone #34-
Binding to Elast-Inhib Complex & Elastase
100
80
60
40
20
0
% RNA Bound
10^-10
10^-9
10^-8
10^-7
10^-6
[ELASTASE] M
#34-Inhib 26 nM
#34 25 nM

FIG. 38

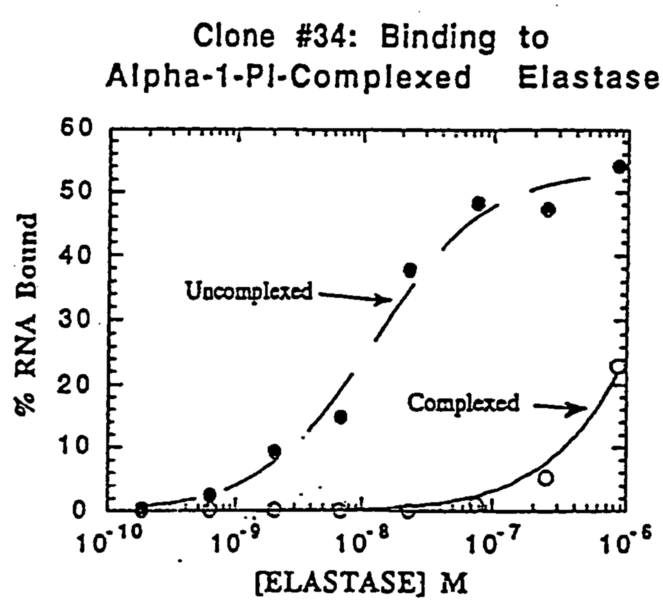
Clone #34: Binding to
Alpha-1-PI-Complexed Elastase
60
50
40
30
20
10
0
% RNA Bound
Uncomplexed
Complexed
10-10
10-9
10-8
10-7
10-6
[ELASTASE] M

FIG. 39

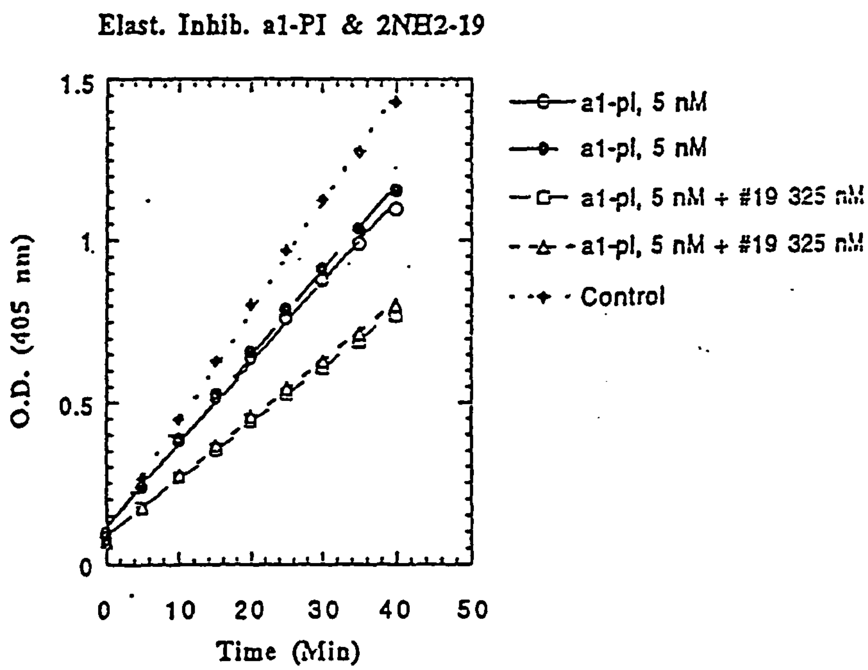
Elast. Inhib. a1-PI & 2NH2-19
O.D. (405 nm)
1.5
1.
0.5
0
0
10
20
30
40
50
Time (Min)
a1-pl, 5 nM
a1-pl, 5 nM
a1-pl, 5 nM + #19 325 nM
a1-pl, 5 nM + #19 325 nM
Control

FIG. 40

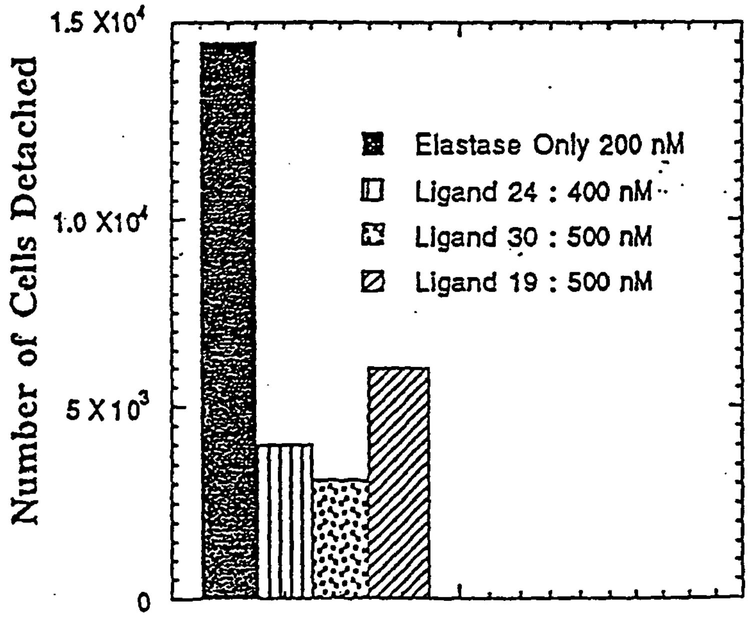
1.5 X10⁴
1.0 X10⁴
5 X10³
0
Number of Cells Detached
Elastase Only 200 nM
Ligand 24 : 400 nM
Ligand 30 : 500 nM
Ligand 19 : 500 nM

FIG. 41

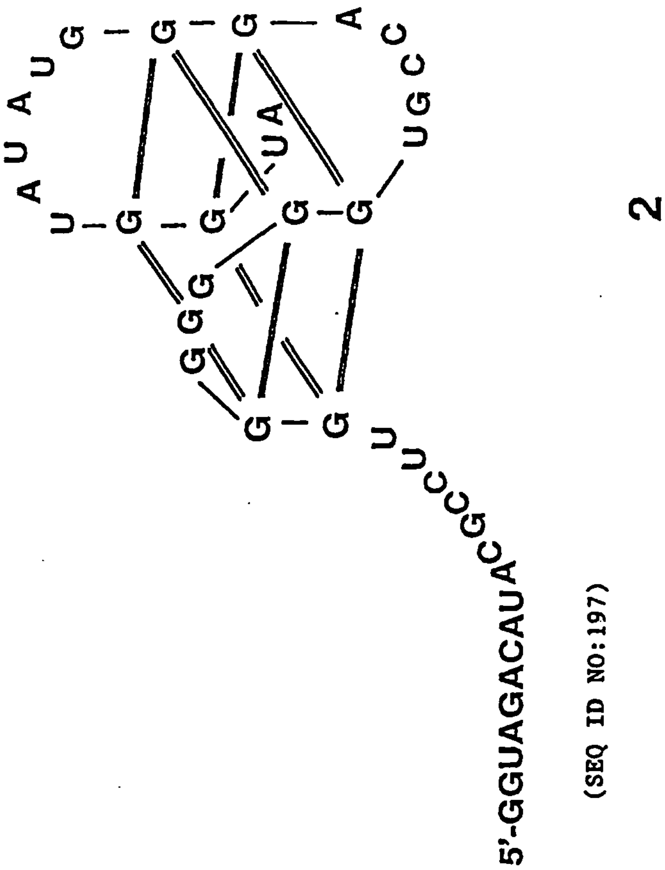


(SEQ ID NO:197)

FIG. 42B

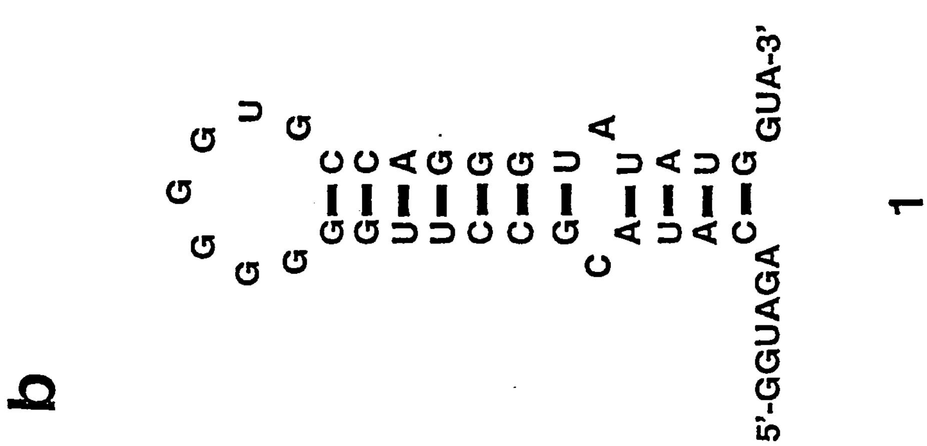


FIG. 42A

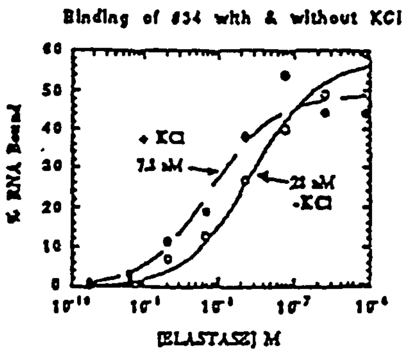


FIG. 43A

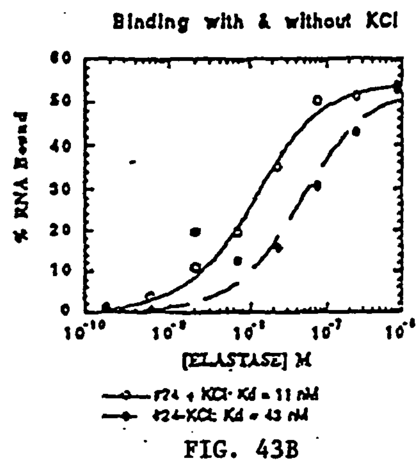


FIG. 43B

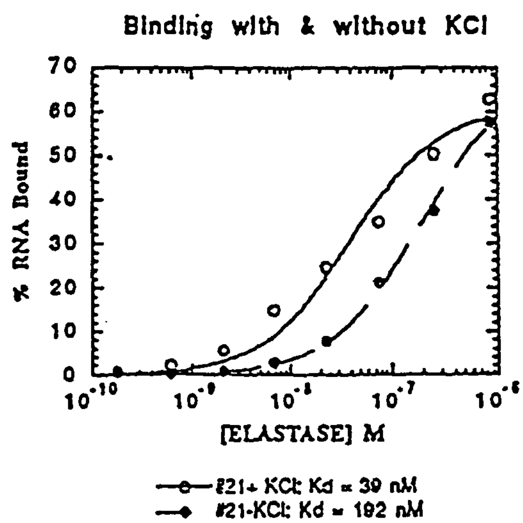


FIG. 43C

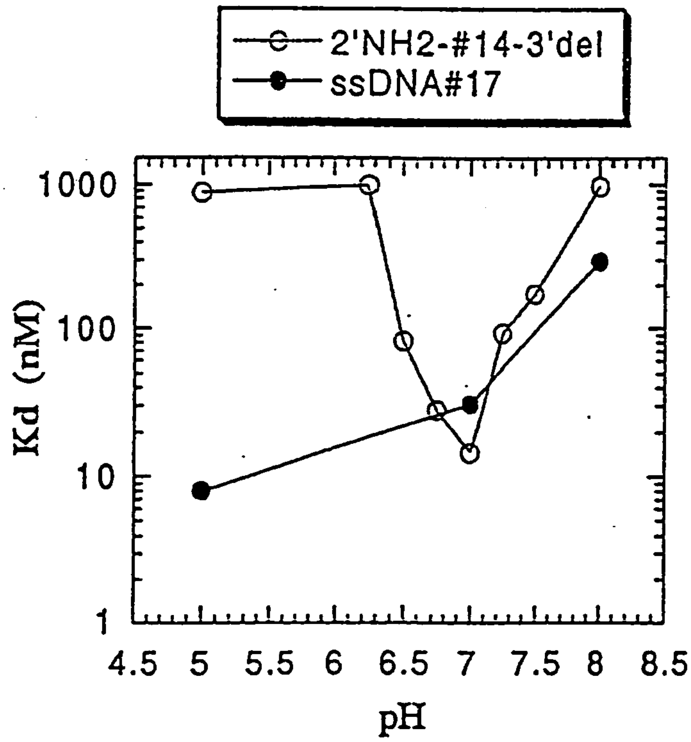


FIG. 44

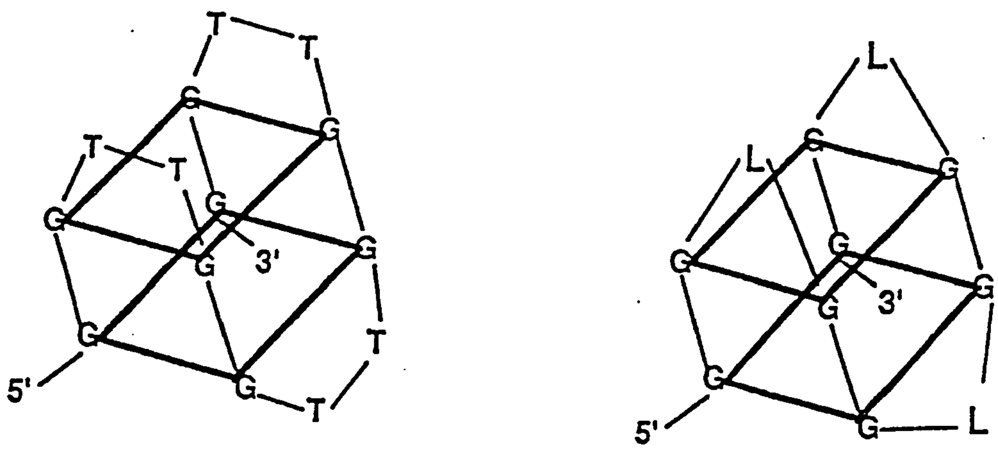


L =

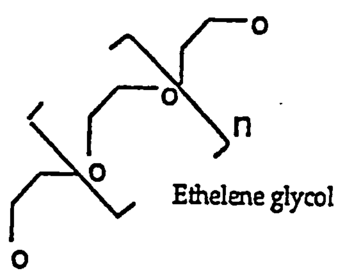

Ethelene glycol linker

FIG. 45

Primer-Template Mixture I
Template

5'-GCCGGATCCGGGCCTCATGTCGAA-[40]n-TTGAGCGTTTATTCTGAGCTCCC
(SEQ ID NO:271)

Primers

5'-CCGAAGCTTAATACGACTCACTATAGGGAGCTCAGAATAAACGCTCAA 5G1
(SEQ ID NO:272)

5'-GCCGGATCCGGGCCTCATGTCGAA 3G1
(SEQ ID NO:273)

For the ssDNA SELEX the following oligomers were used.

Primer-Template Mixture II
Template:

5'-CCCCTGCAGGTGATTTTGCTCAAGT-(40)n-AGTATCGCTAATCAGGCGGATC
(SEQ ID NO:274)

Primers

5'-CCGAAGCTTAATACGACTCACTATAGGGATCCGCCTGATTAGCGATACT 5G2
(SEQ ID NO:275)

5'-CCCCTGCAGGTGATTTTGCTCAAGT 3G2
(SEQ ID NO:276)

FIG. 46

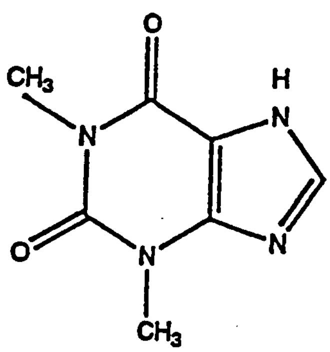

THEOPHYLLINE.

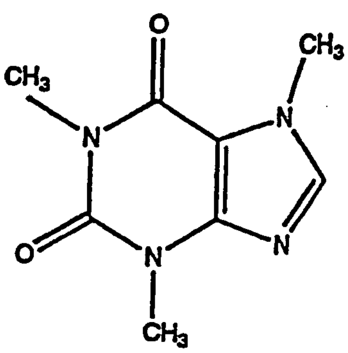

CAFFEINE

FIG. 47

CLASS I

| | | |
|---|---|---|
| TCT8-6,9 | 5'GAGAAAUACCAGUGACAACUCUCGAGAUCACCCUUGGAAG 3' | SEQ ID NO: 246 |
| TCT8-5 | AUACCAUCGUGUAAGCAAGAGCACGACCUUGGCAGUGUGUG | SEQ ID NO: 247 |
| TCT8-1,10 | GAUACCAACAGCAUAU----UUGCUGUCCUUGGAAGCAACGAGA | SEQ ID NO:248 |
| TCT8-4,8 | GUGAUACCAGCAUCGUC-----UUGAUGCCCUUGGCAGCACUUCA | SEQ ID NO: 249 |
| TCT8-7 | UUGUCGAAUCGGAUACCAGCAAU----------GCAGCCCUUGGAAGCAG | SEQ ID NO:250 |
| TR8-14 | GAUACCAACGGCAUAU----UUGCUGUCCUUGGAAGCAACUAUA | SEQ ID NO:251 |
| TR8-8 | CUCUCGAAAUACCAACUACUCUCACA---AUAGUCCUUGGAAG | SEQ ID NO:252 |
| TR8-5 | UUCAUGUCGCUUGAUACCAUCAACA----------AUGACCUUGGAAGCA | SEQ ID NO: 253 |

FIG. 48A

CLASS II

| | | |
|---|---|---|
| TCT8-3 | 5'UGACUCGAACCCUUGGAAGACCUGAGU-----ACAGGUAUACCAG 3' | SEQ ID NO: 254 |
| TCT8-11 | UCCUUGGAAGCCG-----------UACGGAUACCAAUUGAGUGGCCAUAUG | SEQ ID NO: 255 |
| TR8-28 | UAUCGAGUGGCCUUGGCAGACCAGGC--------CCGGUAUACCACCA | SEQ ID NO: 256 |
| TR8-29 | CGAGAUUCAACCUUGGAAGUCAAU----------CGUGAAUACCAUUGUU | SEQ ID NO: 257 |
| TR8-9 | UCAGAACCUUGGAAGCACUGAAUAAGAUCAGUUGAUACCA | SEQ ID NO: 258 |

FIG. 48 B

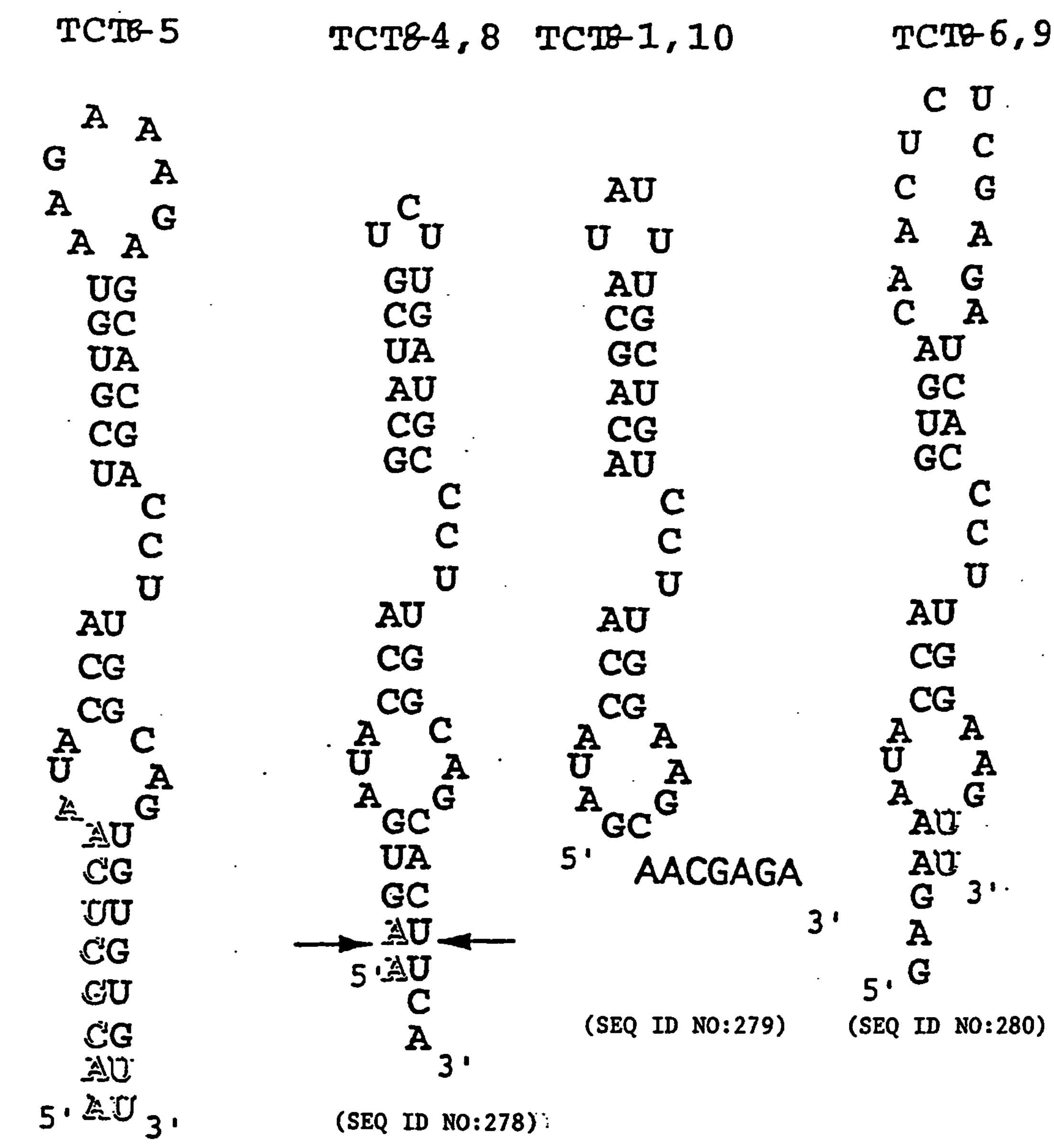
TCT8-5
TCT8-4,8
TCT8-1,10
TCT8-6,9
5' AACGAGA 3'
(SEQ ID NO:277)
(SEQ ID NO:278)
(SEQ ID NO:279)
(SEQ ID NO:280)

FIG. 49

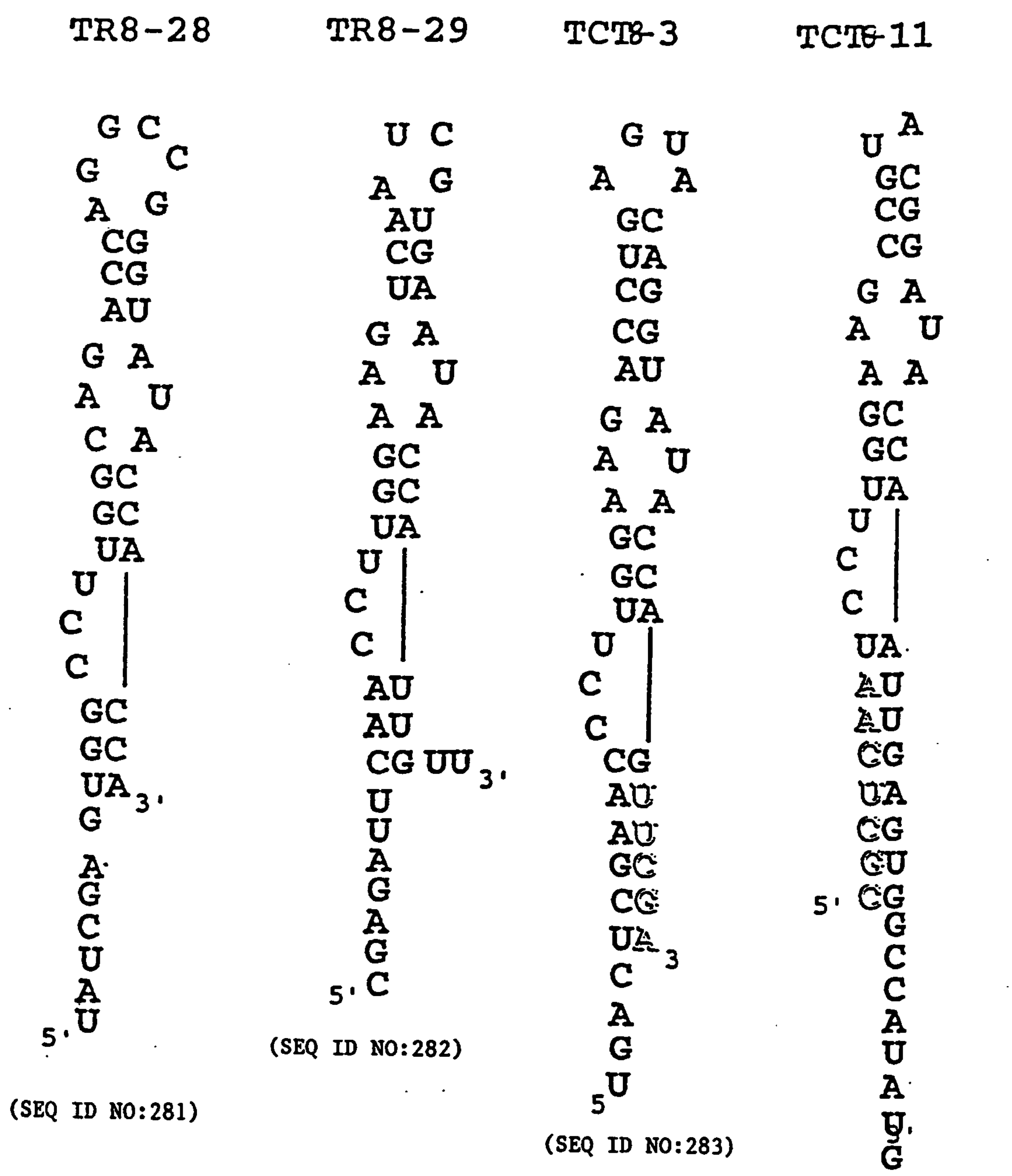


(SEQ ID NO:281)

(SEQ ID NO:282)

(SEQ ID NO:283)

(SEQ ID NO:284)

FIG. 49 CONT'D

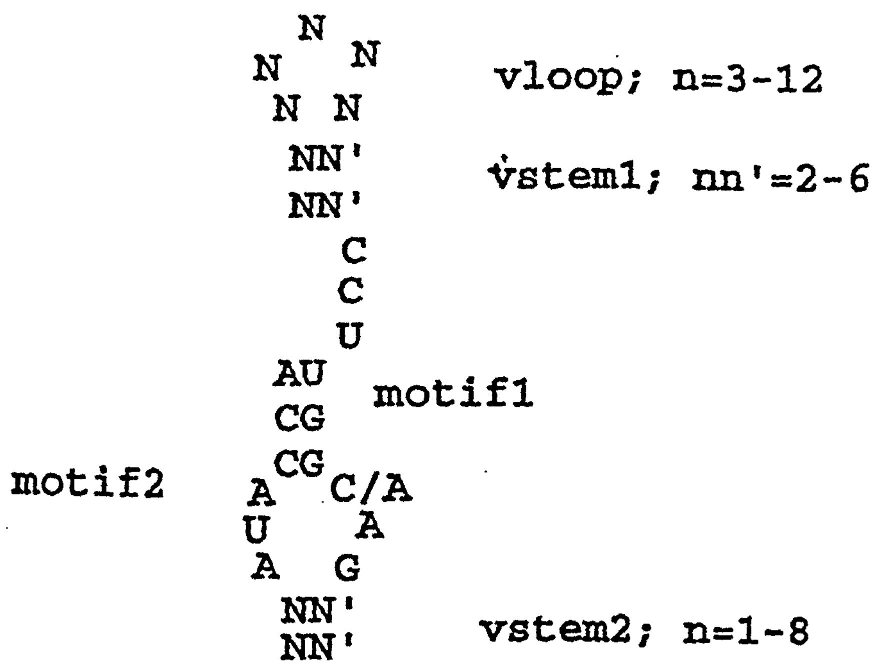


(SEQ ID NO:285)

FIG. 50

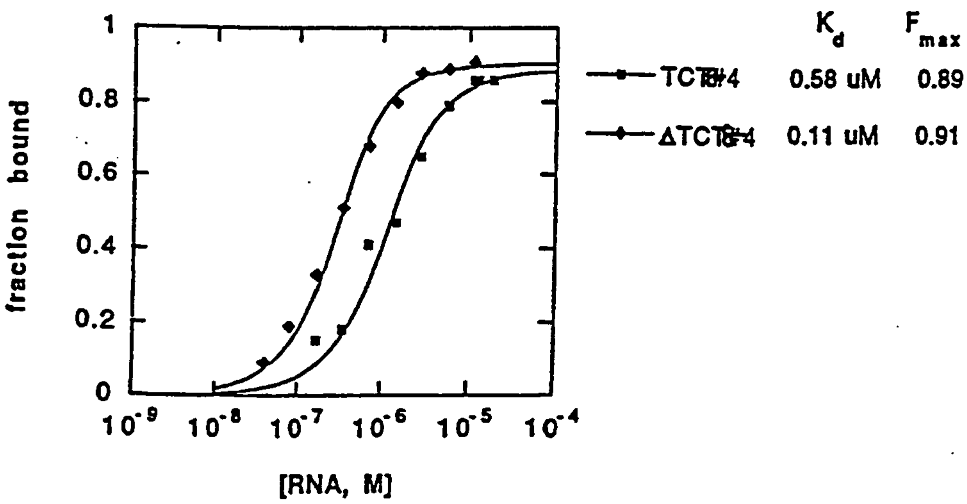
fraction bound
1
0.8
0.6
0.4
0.2
0
$10^{-9}$ $10^{-8}$ $10^{-7}$ $10^{-6}$ $10^{-5}$ $10^{-4}$
[RNA, M]
$K_d$ $F_{max}$
0.58 uM 0.89
0.11 uM 0.91

FIG. 51

mTCT8 - 4

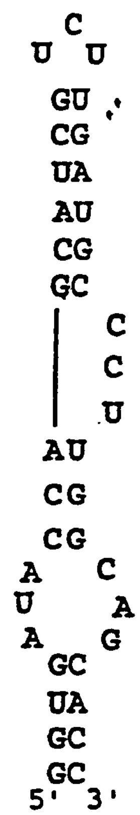


(SEQ ID NO:286)

FIG. 52

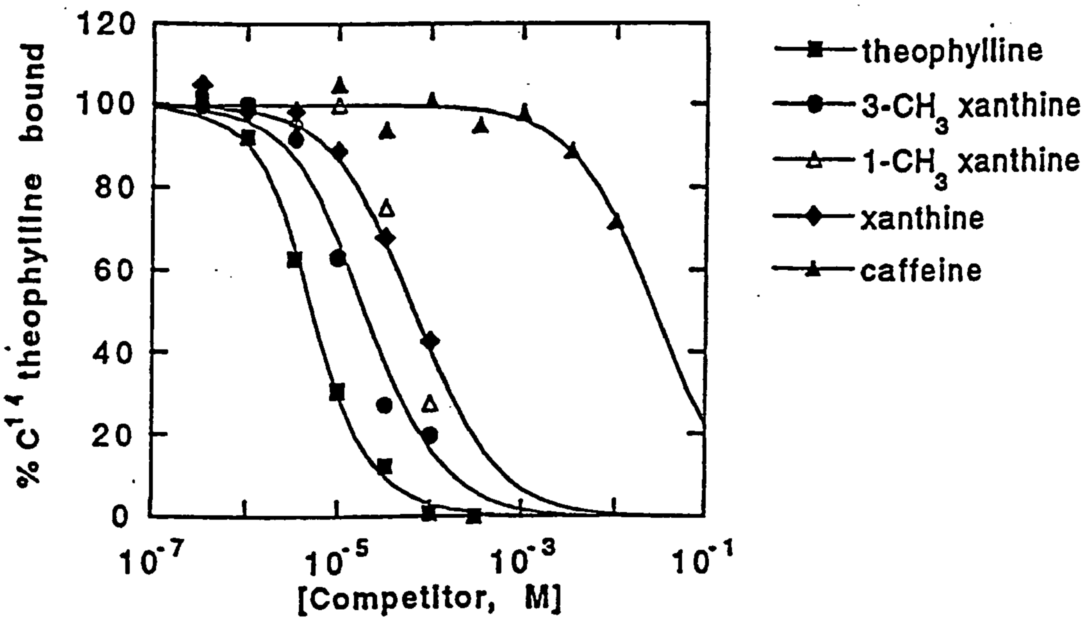
120
100
80
60
40
20
0
% C14 theophylline bound
10-7
10-5
10-3
10-1
[Competitor, M]
theophylline
3-CH3 xanthine
1-CH3 xanthine
xanthine
caffeine

FIG. 53

Group 1

| | | | | | |
|---|---|---|---|---|---|
| CR8-16 | GGACGAGACT | CATCGTAACC | TAGATGGTTG | CCAGCATTTA | SEQ ID NO:259 |
| CR8-18 | GGATGCTTAC | AGCATAATCG | GAATTGATTG | CCAGCGGAAA | SEQ ID NO:260 |
| CR8-19 | GGGGCAATAG | AAGCCAACGC | ACAGTCGTTG | CCAGTGTTCG | SEQ ID NO: 261 |
| CR8-21 | GGGATGGATC | TTCGGATACG | TCAACCAAAG | GTTGCCAGCG | SEQ ID NO: 262 |

Group 2

| | | | | | |
|---|---|---|---|---|---|
| CR8-6,9 | GCGCATCGTA | AAAAGGACAA | ACGTCGTCGT | GACCCCGATA | SEQ ID NO: 263 |
| CR8-12 | GTGCATCGTA | AAAAGGACAA | ACGTCGTCGT | GACCCCGATA | SEQ ID NO: 264 |

Group 3

| | | | | | |
|---|---|---|---|---|---|
| CR8-29 | AGACGGTGAA | ACTGAAATCT | AATCCGTCTG | AACCCTGGAT | SEQ ID NO:265 |
| CR8-40 | AGACGGTGAA | GCTGAAATCT | AATCCGTCTG | AACCCTGGAC | SEQ ID NO:266 |

Others

| | | | | | |
|---|---|---|---|---|---|
| CR8-34 | GGGAGGTCAA | GGACCTCACA | CTTTTGTGTG | CCAGCGCTAT | SEQ ID NO: 267 |
| CR8-37 | GAAAGCGTTG | TGGCGTGCCA | TCGCCCGCAG | GCGAATAACA | SEQ ID NO: 268 |
| CR8-39 | ACGATGGGTT | GTTATAGTGG | AAACGGTAAG | TTCGAGTCTG | SEQ ID NO: 269 |
| CR8-41 | ACGGTGATCC | TCTAATCCGT | CGACAGAATC | GATGTCAATC | SEQ ID NO: 270 |

FIG. 54

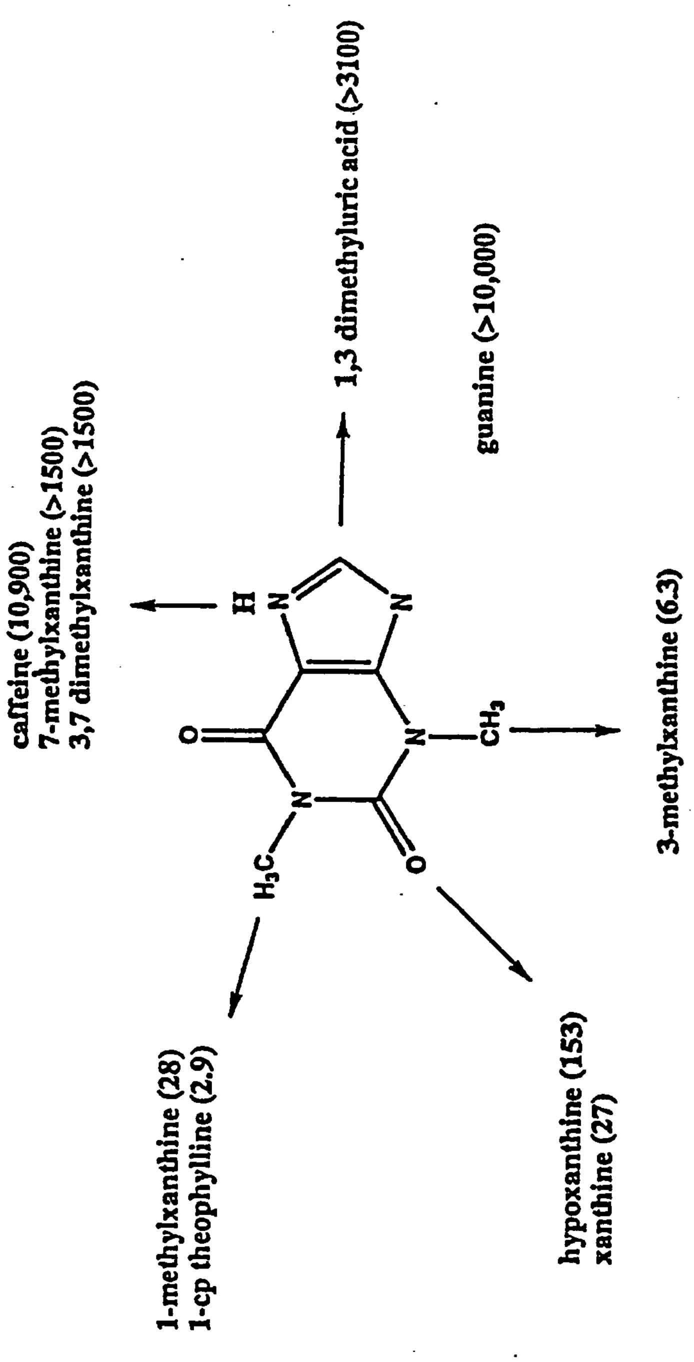
caffeine (10,900)
7-methylxanthine (>1500)
3,7 dimethylxanthine (>1500)
1,3 dimethyluric acid (>3100)
guanine (>10,000)
3-methylxanthine (6.3)
hypoxanthine (153)
xanthine (27)
1-methylxanthine (28)
1-cp theophylline (2.9)

FIG. 55